# EUROPEAN PATENT APPLICATION

(11) **EP 2 416 270 A2**
(43) Date of publication of application: **08.02.2012**
(21) Application number: 11187488.9
(22) Date of filing: 09.12.2006
(51) Int. Cl.: G06F 19/20, C12Q 1/68

(54) **Module-level analysis of peripheral blood leukocyte transcriptional profiles**

(30) Priority: 09.12.2005 US 748884 P; 05.06.2006 US 446825
(62) Divisional of application: 06848531.7
(71) Applicant: Baylor Research Institute, Dallas, TX 75204 (US)
(72) Inventor: Chaussabel, Damien, Richardson TX 75082 (US); Banchereau, Jacques, Dallas TX 75230 (US)
(74) Representative: Sonn & Partner Patentanwälte

(57) **Abstract**

The present invention includes an apparatus, system and method for the development and use of transcriptional modules by obtaining individual gene expression levels from cells obtained from one or more patients with a disease or condition; recording the expression value for each gene in a table that is divided into clusters; iteratively selecting gene expression values for one or more transcriptional modules by: selecting for the module the genes from each cluster that match in every disease or condition; removing the selected genes from the analysis; and repeating the process of gene expression value selection for genes that cluster in a sub-fraction of the diseases or conditions; and iteratively repeating the generation of modules.

## Description

### TECHNICAL FIELD OF THE INVENTION

The present invention relates in general to the transcriptional profiling of cells, and more particularly, to the diagnosis and prognosis of disease from the transcriptional expression profiles of leukocytes.

### BACKGROUND OF THE INVENTION

The widespread utilization of gene expression microarrays holds great promise for biomedical research. This technology has led to the establishment of prognostic signatures in cancer patients¹⁻⁴ and the identification of genes or pathways involved in pathogenesis (for instance, the discovery of the role of interleukin-1 (IL-1) in the pathogenesis of systemic onset juvenile idiopathic arthritis)⁵. However, despite these significant advances, gene expression microarray technology has not lived up to the excitement surrounding its inception, and results derived from the use of microarray platforms have recently been the object of sharp criticisms⁶. Among the chief concerns is the fact that microarray data are particularly prone to noise and could, when over-interpreted, lead to the generation of spurious results⁷. Skepticism also stems from notoriously poor reproducibility of microarray data obtained by different laboratories and across platforms⁸⁻¹². Finally, the limited ability to interpret experimental results in a genome-wide context constitutes another bottleneck in microarray research¹³.

### SUMMARY OF THE INVENTION

Genomic research is facing significant challenges with the analysis of transcriptional data that are notoriously noisy, difficult to interpret and do not compare well across laboratories and platforms. The present inventors have developed an analytical strategy emphasizing the selection of biologically relevant genes at an early stage of the analysis, which are consolidated into analytical modules that overcome the inconsistencies among microarray platforms. The transcriptional modules developed may be used for the analysis of large gene expression datasets. The results derived from this analysis are easily interpretable and particularly robust, as demonstrated by the high degree of reproducibility observed across commercial microarray platforms.

Applications for this analytical process are illustrated through the mining of a large set of PBMC transcriptional profiles. Twenty-eight transcriptional modules regrouping 4742 genes were identified. Using the present invention is it possible to demonstrate that diseases are uniquely characterized by combinations of transcriptional changes in, e.g., blood leukocytes, measured at the modular level. Indeed, module-level changes in blood leukocytes transcriptional levels constitute the molecular fingerprint of a disease or sample.

This invention has a broad range of applications. It can be used to characterize modular transcriptional components of any biological system (e.g., peripheral blood mononuclear cells (PBMCs), blood cells, fecal cells, peritoneal cells, solid organ biopsies, resected tumors, primary cells, cells lines, cell clones, etc.). Modular PBMC transcriptional data generated through this approach can be used for molecular diagnostic, prognostic, assessment of disease severity, response to drug treatment, drug toxicity, etc. Other data processed using this approach can be employed for instance in mechanistic studies, or screening of drug compounds. In fact, the data analysis strategy and mining algorithm can be implemented in generic gene expression data analysis software and may even be used to discover, develop and test new, disease- or condition-specific modules. The present invention may also be used in conjunction with pharmacogenomics, molecular diagnostic, bioinformatics and the like, wherein in-depth expression data may be used to improve the results (e.g., by improving or sub-selecting from within the sample population) that mat be obtained during clinical trails.

More particularly, the present invention includes arrays, apparatuses, systems and method for diagnosing a disease or condition by obtaining the transcriptome of a patient; analyzing the transcriptome based on one or more transcriptional modules that are indicative of a disease or condition; and determining the patient's disease or condition based on the presence, absence or level of expression of genes within the transcriptome in the one or more transcriptional modules. The transcriptional modules may be obtained by: iteratively selecting gene expression values for one or more transcriptional modules by: selecting for the module the genes from each cluster that match in every disease or condition; removing the selected genes from the analysis; and repeating the process of gene expression value selection for genes that cluster in a sub-fraction of the diseases or conditions; and iteratively repeating the generation of modules for each clusters until all gene clusters are exhausted.

Examples of clusters selected for use with the present invention include, but are not limited to, expression value clusters, keyword clusters, metabolic clusters, disease clusters, infection clusters, transplantation clusters, signaling clusters, transcriptional clusters, replication clusters, cell-cycle clusters, siRNA clusters, miRNA clusters, mitochondrial clusters, T cell clusters, B cell clusters, cytokine clusters, lymphokine clusters, heat shock clusters and combinations thereof. Examples of diseases or conditions for analysis using the present invention include, e.g., autoimmune disease, a viral infection a bacterial infection, cancer and transplant rejection. More particularly, diseases for analysis may be selected from one or more of the following conditions: systemic juvenile idiopathic arthritis, systemic lupus erythematosus, type I diabetes, liver transplant recipients, melanoma patients, and patients bacterial infections such as Escherichia coli, Staphylococcus aureus, viral infections such as influenza A, and combinations thereof. Specific array may even be made that detect specific diseases or conditions associated with a bioterror agent.

Cells that may be analyzed using the present invention, include, e.g., peripheral blood mononuclear cells (PBMCs), blood cells, fetal cells, peritoneal cells, solid organ biopsies, resected tumors, primary cells, cells lines, cell clones and combinations thereof. The cells may be single cells, a collection of cells, tissue, cell culture, cells in bodily fluid, e.g., blood. Cells may be obtained from a tissue biopsy, one or more sorted cell populations, cell culture, cell clones, transformed cells, biopies or a single cell. The types of cells may be, e.g., brain, liver, heart, kidney, lung, spleen, retina, bone, neural, lymph node, endocrine gland, reproductive organ, blood, nerve, vascular tissue, and olfactory epithelium cells. After cells are isolated, these mRNA from these cells is obtained and individual gene expression level analysis is performed using, e.g., a probe array, PCR, quantitative PCR, bead-based assays and combinations thereof. The individual gene expression level analysis may even be performed using hybridization of nucleic acids on a solid support using cDNA made from mRNA collected from the cells as a template for reverse transcriptase.

In another embodiment, the present invention includes a method for identifying transcriptional modules by obtaining individual gene expression levels from cells obtained from one or more patients with a disease or condition; recording the expression value for each gene in a table that is divided into clusters; iteratively selecting gene expression values for one or more transcriptional modules by: selecting for the module the genes from each cluster that match in every disease or condition; removing the selected genes from the analysis; and repeating the process of gene expression value selection for genes that cluster in a sub-fraction of the diseases or conditions; and iteratively repeating the generation of modules for each clusters until all gene clusters are exhausted. Examples of transcriptional modules for use with the present invention may be selected from:

| Transcriptional modules |
|---|
| **Plasma cells.** Includes genes encoding for Immunoglobulin chains (*e.g.* IGHM, IGJ, IGLL1, IGKC, IGHD) and the plasma cell marker CD38.; |
| **Platelets.** Includes genes encoding for platelet glycoproteins (ITGA2B, ITGB3, GP6, GP1A/B), and platelet-derived immune mediators such as PPPB (pro-platelet basic protein) and PF4 (platelet factor 4); |
| **B-cells.** Includes genes encoding for B-cell surface markers (CD72, CD79A/B, CD19, CD22) and other B-cell associated molecules: Early B-cell factor (EBF), B-cell linker (BLNK) and B lymphoid tyrosine kinase (BLK); |
| **Undetermined.** This set includes genes encoding regulators and targets of cAMP signaling pathway (JUND, ATF4, CREM, PDE4, NR4A2, VIL2), as well as repressors of TNF-alpha mediated NF-KB activation (CYLD, ASK, TNFAIP3); |
| **Myeloid lineage.** Includes genes encoding molecules expressed by cells of the myeloid lineage (CD86, CD163, FCGR2A), some of which being involved in pathogen recognition (CD14, TLR2, MYD88). This set also includes TNF family members (TNFR2, BAFF); |
| **Undetermined.** This set includes genes encoding for signaling molecules, *e.g.* the zinc finger containing inhibitor of activated STAT (PIAS 1 and PIAS2), or the nuclear factor of activated T-cells NFATC3; |
| **MHC/Ribosomal proteins.** Almost exclusively formed by genes encoding MHC class I molecules (HLA-A,B,C,G,E)+ Beta 2-microglobulin (B2M) or Ribosomal proteins (RPLs, RPSs); |
| **Undetermined.** Includes genes encoding metabolic enzymes (GLS, NSF1, NAT1) and factors involved in DNA replication (PURA, TERF2, EIF2S1); |
| **Cytotoxic cells.** Includes genes encoding cytotoxic T-cells amd NK-cells surface markers (CD8A, CD2, CD 160, NKG7, KLRs), cytolytic molecules (granzyme, perforin, granulysin), chemokines (CCL5, XCL1) and CTL/NK-cell associated molecules (CTSW); |
| **Neutrophils.** This set includes genes encoding innate molecules that are found in neutrophil granules (Lactotransferrin: LTF, defensin: DEAF1, Bacterial Permeability Increasing protein: BPI, Cathelicidin antimicrobial protein: CAMP); |
| **Erythrocytes.** Includes genes encoding hemoglobin genes (HGBs) and other erythrocyte-associated genes (erythrocytic alkirin:ANK1, Glycophorin C: GYPC, hydroxymethylbilane synthase: HMBS, erythroid associated factor: ERAF); |
| **Ribosomal proteins.** Including genes encoding ribosomal proteins (RPLs, RPSs), Eukaryotic Translation Elongation factor family members (EEFs) and Nucleolar proteins (NPM1, NOAL2, NAP1L1); |
| **Undetermined.** This module includes genes encoding immune-related (CD40, CD80, CXCL12, IFNA5, IL4R) as well as cytoskeleton-related molecules (Myosin, Dedicator of Cytokenesis, Syndecan 2, Plexin C1, Distrobrevin); |
| **Myeloid lineage.** Related to M 1.5. Includes genes encoding genes expressed in myeloid lineage cells (IGTB2/CD18, Lymphotoxin beta receptor, Myeloid related proteins 8/14 Formyl peptide receptor 1), such as Monocytes and Neutrophils; |
| **Undetermined.** This module is largely composed of transcripts with no known function. Only 20 genes associated with literature, including a member of the chemokine-like factor superfamily (CKLFSF8); |
| **T-cells.** Includes genes encoding T-cell surface markers (CD5, CD6, CD7, CD26, CD28, CD96) and molecules expressed by lymphoid lineage cells (lymphotoxin beta, IL2-inducible T-cell kinase, TCF7, T-cell differentiation protein mal, GATA3, STAT5B); |
| **Undetermined.** Includes genes encoding molecules that associate to the cytoskeleton (Actin related protein 2/3, MAPK1, MAP3K1, RAB5A). Also present are T-cell expressed genes (FAS, ITGA4/CD49D, ZNF1A1); |
| **Undetermined.** Includes genes encoding for Immune-related cell surface molecules (CD36, CD86, LILRB), cytokines (IL15) and molecules involved in signaling pathways (FYB, TICAM2-Toll-like receptor pathway); |
| **Undetermined.** Includes genes encoding kinases (UHMK1, CSNK1G1, CDK6, WNK1, TAOK1, CALM2, PRKCI, ITPKB, SRPK2, STK17B, DYRK2, PIK3R1, STK4, CLK4, PKN2) and RAS family members (G3BP, RAB14, RASA2, RAP2A, KRAS); |
| **Interferon-inducible.** This set includes genes encoding interferon-inducible genes: antiviral molecules (OAS1/2/3/L, GBP1, G1P2, EIF2AK2/PKR, MX1, PML), chemokines (CXCL10/IP-10), signaling molecules (STAT1, STAt2, IRF7, ISGF3G); |
| **Inflammation I.** Includes genes encoding molecules involved in inflammatory processes (e.g. IL8, ICAM1, C5R1, CD44, PLAUR, IL1A, CXCL16), and regulators of apoptosis (MCL1, FOXO3A, RARA, BCL3/6/2A1, GADD45B); |
| **Inflammation II.** Includes genes encoding molecules inducing or inducible by Granulocyte-Macrophage CSF (SPI1, IL18, ALOX5, ANPEP), as well as lysosomal enzymes (PPT1, CTSB/S, CES1, NEU1, ASAH1, LAMP2, CAST); |
| **Undetermined.** Includes genes encoding protein phosphates (PPP1R12A, PTPRC, PPP1CB, PPM1B) and phosphoinositide 3-kinase (PI3K) family members (PIK3CA, PIK32A, PIP5K3); |
| **Undetermined.** Composed of only a small number of transcripts. Includes genes encoding hemoglobin genes (HBA1, HBA2, HBB); |
| **Undetermined.** This very large set includes genes encoding T-cell surface markers (CD101, CD102, CD103) as well as molecules ubiquitously expressed among blood leukocytes (CXRCR1: fraktalkine receptor, CD47, P-selectin ligand); |
| **Undetermined.** Includes genes encoding proteasome subunits (PSMA2/5, PSMB5/8); ubiquitin protein ligases HIP2, STUB1, as well as components of ubiqutin ligase complexes (SUGT1); |
| **Undetermined.** Includes genes encoding for several enzymes: aminomethyltransferase, arginyltransferase, asparagines synthetase, diacylglycerol kinase, inositol phosphatases, methyltransferases, helicases; and |
| **Undetermined.** Includes genes encoding for protein kinases (PRKPIR, PRKDC, PRKCI) and phosphatases (*e.g.* PTPLB, PPP1R8/2CB). Also includes RAS oncogene family members and the NK cell receptor 2B4 (CD244); |

and combinations thereof, wherein the level of expression of genes in a sample is charted to the modules to determine a disease or condition.

The present invention also includes a disease analysis tool that includes one or more gene modules selected from the group consisting of, for example,

| Transcriptional modules |
|---|
| **Plasma cells.** Includes genes encoding for Immunoglobulin chains (*e.g.* IGHM, IGJ, IGLL1, IGKC, IGHD) and the plasma cell marker CD38.; |
| **Platelets.** Includes genes encoding for platelet glycoproteins (ITGA2B, ITGB3, GP6, GP1A/B), and platelet-derived immune mediators such as PPPB (pro-platelet basic protein) and PF4 (platelet factor 4); |
| **B-cells.** Includes genes encoding for B-cell surface markers (CD72, CD79A/B, CD19, CD22) and other B-cell associated molecules: Early B-cell factor (EBF), B-cell linker (BLNK) and B lymphoid tyrosine kinase (BLK); |
| **Undetermined.** This set includes regulators and targets of cAMP signaling pathway (JUND, ATF4, CREM, PDE4, NR4A2, VIL2), as well as repressors of TNF-alpha mediated NF-KB activation (CYLD, ASK, TNFAIP3); |
| **Myeloid lineage.** Includes molecules expressed by cells of the myeloid lineage (CD86, CD163, FCGR2A), some of which being involved in pathogen recognition (CD14, TLR2, MYD88). This set also includes TNF family members (TNFR2, BAFF); |
| **Undetermined.** This set includes genes encoding for signaling molecules, *e.g.* the zinc finger containing inhibitor of activated STAT (PIAS 1 and PIAS2), or the nuclear factor of activated T-cells NFATC3; |
| **MHC/Ribosomal proteins.** Almost exclusively formed by genes encoding MHC class I molecules (HLA-A,B,C,G,E)+ Beta 2-microglobulin (B2M) or Ribosomal proteins (RPLs, RPSs); |
| **Undetermined.** Includes genes encoding metabolic enzymes (GLS, NSF1, NAT1) and factors involved in DNA replication (PURA, TERF2, EIF2S1); |
| **Cytotoxic cells.** Includes Cytotoxic T-cells amd NK-cells surface markers (CD8A, CD2, CD160, NKG7, KLRs), cytolytic molecules (granzyme, perforin, granulysin), chemokines (CCL5, XCL1) and CTL/NK-cell associated molecules (CTSW); |
| **Neutrophils.** This set includes innate molecules that are found in neutrophil granules (Lactotransferrin: LTF, defensin: DEAF1, Bacterial Permeability Increasing protein: BPI, Cathelicidin antimicrobial protein: CAMP...); |
| **Erythrocytes.** Includes hemoglobin genes (HGBs) and other erythrocyte-associated genes (erythrocytic alkirin:ANK1, Glycophorin C: GYPC, hydroxymethylbilane synthase: HMBS, erythroid associated factor: ERAF); |
| **Ribosomal proteins.** Including genes encoding ribosomal proteins (RPLs, RPSs), Eukaryotic Translation Elongation factor family members (EEFs) and Nucleolar proteins (NPM1, NOAL2, NAP1L1); |
| **Undetermined.** This module includes genes encoding immune-related (CD40, CD80, CXCL12, IFNA5, IL4R) as well as cytoskeleton-related molecules (Myosin, Dedicator of Cytokenesis, Syndecan 2, Plexin C1, Distrobrevin); |
| **Myeloid lineage.** Related to M 1.5. Includes genes expressed in myeloid lineage cells (IGTB2/CD18, Lymphotoxin beta receptor, Myeloid related proteins 8/14 Formyl peptide receptor 1), such as Monocytes and Neutrophils; |
| **Undetermined.** This module is largely composed of transcripts with no known function. Only 20 genes associated with literature, including a member of the chemokine-like factor superfamily (CKLFSF8); |
| **T-cells.** Includes T-cell surface markers (CD5, CD6, CD7, CD26, CD28, CD96) and molecules expressed by lymphoid lineage cells (lymphotoxin beta, IL2-inducible T-cell kinase, TCF7, T-cell differentiation protein mal, GATA3, STAT5B); |
| **Undetermined.** Includes genes encoding molecules that associate to the cytoskeleton (Actin related protein 2/3, MAPK1, MAP3K1, RAB5A). Also present are T-cell expressed genes (FAS, ITGA4/CD49D, ZNF1A1); |
| **Undetermined.** Includes genes encoding for Immune-related cell surface molecules (CD36, CD86, LILRB), cytokines (IL15) and molecules involved in signaling pathways (FYB, TICAM2-Toll-like receptor pathway); |
| **Undetermined.** Includes kinases (UHMK1, CSNK1G1, CDK6, WNK1, TAOK1, CALM2, PRKCI, ITPKB, SRPK2, STK17B, DYRK2, PIK3R1, STK4, CLK4, PKN2) and RAS family members (G3BP, RAB14, RASA2, RAP2A, KRAS); |
| **Interferon-inducible.** This set includes interferon-inducible genes: antiviral molecules (OAS1/2/3/L, GBP1, G1P2, EIF2AK2/PKR, MX1, PML), chemokines (CXCL10/IP-10), signaling molecules (STAT1, STAt2, IRF7, ISGF3G); |
| **Inflammation I.** Includes genes encoding molecules involved in inflammatory processes (e.g. IL8, ICAM1, C5R1, CD44, PLAUR, IL1A, CXCL16), and regulators of apoptosis (MCL1, FOXO3A, RARA, BCL3/6/2A1, GADD45B); |
| **Inflammation II.** Includes molecules inducing or inducible by Granulocyte-Macrophage CSF (SPI1, IL18, ALOX5, ANPEP), as well as lysosomal enzymes (PPT1, CTSB/S, CES1, NEU1, ASAH1, LAMP2, CAST); |
| **Undetermined.** Includes protein phosphates (PPP1R12A, PTPRC, PPP1CB, PPM1B) and phosphoinositide 3-kinase (PI3K) family members (PIK3CA, PIK32A, PIP5K3); |
| **Undetermined.** Composed of only a small number of transcripts. Includes hemoglobin genes (HBA1, HBA2, HBB); |
| **Undetermined.** This very large set includes T-cell surface markers (CD101, CD102, CD103) as well as molecules ubiquitously expressed among blood leukocytes (CXRCR1: fraktalkine receptor, CD47, P-selectin ligand); |
| **Undetermined.** Includes genes encoding proteasome subunits (PSMA2/5, PSMB5/8); ubiquitin protein ligases HIP2, STUB1, as well as components of ubiqutin ligase complexes (SUGT1); |
| **Undetermined.** Includes genes encoding for several enzymes: aminomethyltransferase, arginyltransferase, asparagines synthetase, diacylglycerol kinase, inositol phosphatases, methyltransferases, helicases; and |
| **Undetermined.** Includes genes encoding for protein kinases (PRKPIR, PRKDC, PRKCI) and phosphatases (*e.g.* PTPLB, PPP1R8/2CB). Also includes RAS oncogene family members and the NK cell receptor 2B4 (CD244); |

sufficient to distinguish between an autoimmune disease, a viral infection a bacterial infection, cancer and transplant rejection. The modules are used to distinguish between Systemic Lupus erythematosus, Influenza infection, melanoma and transplant rejection.

In one embodiment, the modules selected may be selected from:
**Plasma cells.** Includes genes encoding for Immunoglobulin chains (*e.g.* IGHM, IGJ, IGLL1, IGKC, IGHD) and the plasma cell marker CD38; and
**Platelets.** Includes genes encoding for platelet glycoproteins (ITGA2B, ITGB3, GP6, GP1A/B), and platelet-derived immune mediators such as PPPB (pro-platelet basic protein) and PF4 (platelet factor 4);
and the modules are used to identify Systemic Lupus erythematosus by having a positive vector at these two modules.

In another embodiment, the modules selected may be selected from:
**Plasma cells.** Includes genes encoding for Immunoglobulin chains (*e.g.* IGHM, IGJ, IGLL1, IGKC, IGHD) and the plasma cell marker CD38; and
**Platelets.** Includes genes encoding for platelet glycoproteins (ITGA2B, ITGB3, GP6, GP1A/B), and platelet-derived immune mediators such as PPPB (pro-platelet basic protein) and PF4 (platelet factor 4);
and the modules are used to identify Influenza infection by having neither a positive nor a negative vector at these two modules.

In another embodiment, the modules selected may be selected from:
**Plasma cells.** Includes genes encoding for Immunoglobulin chains (*e.g.* IGHM, IGJ, IGLL1, IGKC, IGHD) and the plasma cell marker CD38; and
**Platelets.** Includes genes encoding for platelet glycoproteins (ITGA2B, ITGB3, GP6, GP1A/B), and platelet-derived immune mediators such as PPPB (pro-platelet basic protein) and PF4 (platelet factor 4);
and the modules are used to identify melanoma by having a negative vector for the plasma cell markers and a positive vector for the platelet markers.

In another embodiment, the modules selected may be selected from:
**Plasma cells.** Includes genes encoding for Immunoglobulin chains (*e.g.* IGHM, IGJ, IGLL1, IGKC, IGHD) and the plasma cell marker CD38; and
**Platelets.** Includes genes encoding for platelet glycoproteins (ITGA2B, ITGB3, GP6, GP1A/B), and platelet-derived immune mediators such as PPPB (pro-platelet basic protein) and PF4 (platelet factor 4);
and the modules are used to identify transplant rejection by having a negative vectors at these two modules.

In another embodiment, the modules selected may be selected from:
**Plasma cells.** Includes genes encoding for Immunoglobulin chains (*e.g.* IGHM, IGJ, IGLL1, IGKC, IGHD) and the plasma cell marker CD38; and
**Platelets.** Includes genes encoding for platelet glycoproteins (ITGA2B, ITGB3, GP6, GP1A/B), and platelet-derived immune mediators such as PPPB (pro-platelet basic protein) and PF4 (platelet factor 4);
and the modules are used to identify Influenza infection by having a negative vector at these two modules.

Yet another embodiment of the present invention is a prognostic gene array that includes a customized gene array that has a combination of genes that are representative of one or more transcriptional modules, wherein the transcriptome of a patient that is contacted with the customized gene array is prognostic of one or more disease or conditions that match the transcriptional modules. In one example, the patient's immune response to the disease or condition is determined based on the presence, absence or level of expression of genes of the transcriptome based on a correlation of the transcriptional modules with a specific disease or condition. The array can distinguish between an autoimmune disease, a viral infection a bacterial infection, cancer and transplant rejection. The array may even be organized into two or more transcriptional modules. For example, the array may be organized into three transcriptional modules that include one or more submodules selected from:

| Submodule | Number of probe sets | Keyword selection | Assessment |
|---|---|---|---|
| M 1.1 | 69 | Ig, Immunoglobulin, Bone, Marrow, PreB, IgM,Mu. | Plasma cells. Includes genes encoding for Immunoglobulin chains (e.g. IGHM, IGJ, IGLL1, IGKC, IGHD) and the plasma cell marker CD38; |
| M 1.2 | 96 | Platelet, Adhesion, Aggregation, Endothelial, Vascular | Platelets. Includes genes encoding for platelet glycoproteins (ITGA2B, ITGB3, GP6, GP1A/B), and platelet-derived immune mediators such as PPPB (pro-platelet basic protein) and PF4 (platelet factor 4); |
| M 1.3 | 47 | Immunoreceptor, BCR, B-cell, IgG | B-cells. Includes genes encoding for B-cell surface markers (CD72, CD79A/B, CD19, CD22) and other B-cell associated molecules: Early B-cell factor (EBF), B-cell linker (BLNK) and B lymphoid tyrosine kinase (BLK); |
| M 1.4 | 87 | Replication, Repression, Repair, CREB, Lymphoid, TNF-alpha | Undetermined. This set includes regulators and targets of cAMP signaling pathway (JUND, ATF4, CREM, PDE4, NR4A2, VIL2), as well as repressors of TNF-alpha mediated NF-KB activation (CYLD, ASK, TNFAIP3); |
| M 1.5 | 130 | Monocytes, Dendritic, MHC, Costimulatory, TLR4, MYD88 | Myeloid lineage. Includes molecules expressed by cells of the myeloid lineage (CD86, CD163, FCGR2A), some of which being involved in pathogen recognition (CD14, TLR2, MYD88). This set also includes TNF family members (TNFR2, BAFF); |
| M 1.6 | 28 | Zinc, Finger, P53, RAS | Undetermined. This set includes genes encoding for signaling molecules, e.g. the zinc finger containing inhibitor of activated STAT (PIAS1 and PIAS2), or the nuclear factor of activated T-cells NFATC3; |
| M 1.7 | 127 | Ribosome, Translational, 40S, 60S, HLA | MHC/Ribosomal proteins. Almost exclusively formed by genes encoding MHC class I molecules (HLA-A,B,C,G,E)+ Beta 2-microglobulin (B2M) or Ribosomal proteins (RPLs, RPSs); |
| M 1.8 | 86 | Metabolism, Biosynthesis, Replication, Helicase | Undetermined. Includes genes encoding metabolic enzymes (GLS, NSF1, NAT1) and factors involved in DNA replication (PURA, TERF2, EIF2S 1); |
| M 2.1 | 72 | NK, Killer, Cytolytic, CD8, Cell-mediated, T-cell, CTL, IFN-g | Cytotoxic cells. Includes cytotoxic T-cells and NK-cells surface markers (CD8A, CD2, CD160, NKG7, KLRs), cytolytic molecules (granzyme, perforin, granulysin), chemokines (CCL5, XCL1) and CTL/NK-cell associated molecules (CTSW); |
| M 2.2 | 44 | Granulocytes, Neutrophils, Defense, Myeloid, Marrow | Neutrophils. This set includes innate molecules that are found in neutrophil granules (Lactotransferrin: LTF, defensin: DEAF1, Bacterial Permeability Increasing protein: BPI, Cathelicidin antimicrobial protein: CAMP); |
| M 2.3 | 94 | Erythrocytes, Red, Anemia, Globin, Hemoglobin | Erythrocytes. Includes hemoglobin genes (HGBs) and other erythrocyte-associated genes (erythrocytic alkirin:ANK1, Glycophorin C: GYPC, hydroxymethylbilane synthase: HMBS, erythroid associated factor: ERAF); |
| M 2.4 | 118 | Ribonucleoprotein, 60S, nucleolus, Assembly, Elongation | Ribosomal proteins. Including genes encoding ribosomal proteins (RPLs, RPSs), Eukaryotic Translation Elongation factor family members (EEFs) and Nucleolar proteins (NPM1, NOAL2, NAP1L1); |
| M 2.5 | 242 | Adenoma, Interstitial, Mesenchyme, Dendrite, Motor | Undetermined. This module includes genes encoding immune-related (CD40, CD80, CXCL12, IFNA5, IL4R) as well as cytoskeleton-related molecules (Myosin, Dedicator of Cytokenesis, Syndecan 2, Plexin C1, Distrobrevin); |
| M 2.6 | 110 | Granulocytes, Monocytes, Myeloid, ERK, Necrosis | Myeloid lineage. Related to M 1.5. Includes genes expressed in myeloid lineage cells (IGTB2/CD 18, Lymphotoxin beta receptor, Myeloid related proteins 8/14 Formyl peptide receptor 1), such as Monocytes and Neutrophils; |
| M 2.7 | 43 | No keywords extracted. | Undetermined. This module is largely composed of transcripts with no known function. Only 20 genes associated with literature, including a member of the chemokine-like factor superfamily (CKLFSF8); |
| M 2.8 | 104 | Lymphoma, T-cell, CD4, CD8, TCR, Thymus, Lymphoid, IL2 | T-cells. Includes T-cell surface markers (CD5, CD6, CD7, CD26, CD28, CD96) and molecules expressed by lymphoid lineage cells (lymphotoxin beta, IL2-inducible T-cell kinase, TCF7, T-cell differentiation protein mal, GATA3, STAT5B) ; |
| M 2.9 | 122 | ERK, Transactivation, Cytoskeletal, MAPK, JNK | Undetermined. Includes genes encoding molecules that associate to the cytoskeleton (Actin related protein 2/3, MAPK1, MAP3K1, RAB5A). Also present are T-cell expressed genes (FAS, ITGA4/CD49D, ZNF1A1); |
| M2.10 | 44 | Myeloid, Macrophage, Dendritic, Inflammatory, Interleukin | Undetermined. Includes genes encoding for Immune-related cell surface molecules (CD36, CD86, LILRB), cytokines (IL15) and molecules involved in signaling pathways (FYB, TICAM2-Toll-like receptor pathway); |
| M 2.11 | 77 | Replication, Repress, RAS, Autophosphorylati on, Oncogenic | Undetermined. Includes kinases (UHMK1, CSNK1G1, CDK6, WNK1, TAOK1, CALM2, PRKCI, ITPKB, SRPK2, STK17B, DYRK2, PIK3R1, STK4, CLK4, PKN2) and RAS family members (G3BP, RAB14, RASA2, RAP2A, KRAS); |
| M 3.1 | 80 | ISRE, Influenza, Antiviral, IFN-gamma, IFN-alpha, Interferon | Interferon-inducible. This set includes interferon-inducible genes: antiviral molecules (OAS1/2/3/L, GBP1, G1P2, EIF2AK2/PKR, MX1, PML), chemokines (CXCL10/IP-10), signaling molecules (STAT1, STAt2, IRF7, ISGF3G); |
| M 3.2 | 230 | TGF-beta, TNF, Inflammatory, Apoptotic, Lipopolysaccharide | Inflammation I. Includes genes encoding molecules involved in inflammatory processes (e.g. IL8, ICAM1, C5R1, CD44, PLAUR, IL1A, CXCL16), and regulators of apoptosis (MCL1, FOXO3A, RARA, BCL3/6/2A1, GADD45B); |
| M 3.3 | 230 | Granulocyte, Inflammatory, Defense, Oxidize, Lysosomal | Inflammation II. Includes molecules inducing or inducible by Granulocyte-Macrophage CSF (SPI1, IL18, ALOX5, ANPEP), as well as lysosomal enzymes (PPT1, CTSB/S, CES1, NEU1, ASAH1, LAMP2, CAST); |
| M 3.4 | 323 | No keyword extracted | Undetermined. Includes protein phosphates (PPP1R12A, PTPRC, PPP1CB, PPM1B) and phosphoinositide 3-kinase (PI3K) family members (PIK3CA, PIK32A, PIP5K3); |
| M 3.5 | 19 | No keyword extracted | Undetermined. Composed of only a small number oftranscripts. Includes hemoglobin genes (HBA1, HBA2, HBB); |
| M 3.6 | 233 | Complement, Host, Oxidative, Cytoskeletal, T-cell | Undetermined. This very large set includes T-cell surface markers (CD 101, CD 102, CD 103) as well as molecules ubiquitously expressed among blood leukocytes (CXRCR1: fraktalkine receptor, CD47, P-selectin ligand); |
| M 3.7 | 80 | Spliceosome, Methylation, Ubiquitin, Beta-catenin | Undetermined. Includes genes encoding proteasome subunits (PSMA2/5, PSMB5/8); ubiquitin protein ligases HIP2, STUB1, as well as components ofubiqutin ligase complexes (SUGT1); |
| M 3.8 | 182 | CDC, TCR, CREB, Glycosylase | Undetermined. Includes genes encoding for several enzymes: aminomethyltransferase, arginyltransferase, asparagines synthetase, diacylglycerol kinase, inositol phosphatases, methyltransferases, helicases; and |
| M 3.9 | 261 | Chromatin, Checkpoint, Replication, Transactivation | Undetermined. Includes genes encoding for protein kinases (PRKPIR, PRKDC, PRKCI) and phosphatases (e.g. PTPLB, PPP1R8/2CB). Also includes RAS oncogene family members and the NK cell receptor 2B4 (CD244); |

wherein one or more probes from each that bind specifically one or more of the genes in the module.

Yet another invention includes a gene analysis tool that includes one or more gene modules selected from a combination of one group selected from the left column and one group selected from the right column including:

| Keyword selection | Transcriptional modules |
|---|---|
| Ig, Immunoglobulin, Bone, Marrow, PreB, IgM,Mu. | Plasma cells. Includes genes encoding for Immunoglobulin chains (e.g. IGHM, IGJ, IGLL1, IGKC, IGHD) and the plasma cell marker CD38.; |
| Platelet, Adhesion, Aggregation, Endothelial, Vascular | Platelets. Includes genes encoding for platelet glycoproteins (ITGA2B, ITGB3, GP6, GP1A/B), and platelet-derived immune mediators such as PPPB (pro-platelet basic protein) and PF4 (platelet factor 4); |
| Immunoreceptor, BCR, B-cell, IgG | B-cells. Includes genes encoding for B-cell surface markers (CD72, CD79A/B, CD19, CD22) and other B-cell associated molecules: Early B-cell factor (EBF), B-cell linker (BLNK) and B lymphoid tyrosine kinase (BLK); |
| Replication, Repression, Repair, CREB, Lymphoid, TNF-alpha | Undetermined. This set includes regulators and targets of cAMP signaling pathway (JUND, ATF4, CREM, PDE4, NR4A2, VIL2), as well as repressors of TNF-alpha mediated NF-KB activation (CYLD, ASK, TNFAIP3); |
| Monocytes, Dendritic, MHC, Costimulatory, TLR4, MYD88 | Myeloid lineage. Includes molecules expressed by cells of the myeloid lineage (CD86, CD163, FCGR2A), some of which being involved in pathogen recognition (CD14, TLR2, MYD88). This set also includes TNF family members (TNFR2, BAFF); |
| Zinc, Finger, P53, RAS | Undetermined. This set includes genes encoding for signaling molecules, e.g. the zinc finger containing inhibitor of activated STAT (PIAS1 and PIAS2), or the nuclear factor of activated T-cells NFATC3; |
| Ribosome, Translational, 40S, 60S, HLA | MHC/Ribosomal proteins. Almost exclusively formed by genes encoding MHC class I molecules (HLA-A,B,C,G,E)+ Beta 2-microglobulin (B2M) or Ribosomal proteins (RPLs, RPSs); |
| Metabolism, Biosynthesis, Replication, Helicase | Undetermined. Includes genes encoding metabolic enzymes (GLS, NSF1, NAT1) and factors involved in DNA replication (PURA, TERF2, EIF2S1); |
| NK, Killer, Cytolytic, CD8, Cell-mediated, T-cell, CTL, IFN-g | Cytotoxic cells. Includes Cytotoxic T-cells amd NK-cells surface markers (CD8A, CD2, CD160, NKG7, KLRs), cytolytic molecules (granzyme, perforin, granulysin), chemokines (CCL5, XCL1) and CTL/NK-cell associated molecules (CTSW); |
| Granulocytes, Neutrophils, Defense, Myeloid, Marrow | Neutrophils. This set includes innate molecules that are found in neutrophil granules (Lactotransferrin: LTF, defensin: DEAF1, Bacterial Permeability Increasing protein: BPI, Cathelicidin antimicrobial protein: CAMP...); |
| Erythrocytes, Red, Anemia, Globin, Hemoglobin | Erythrocytes. Includes hemoglobin genes (HGBs) and other erythrocyte-associated genes (erythrocytic alkirin:ANK1, Glycophorin C: GYPC, hydroxymethylbilane synthase: HMBS, erythroid associated factor: ERAF); |
| Ribonucleoprotein, 60S, nucleolus, Assembly, Elongation | Ribosomal proteins. Including genes encoding ribosomal proteins (RPLs, RPSs), Eukaryotic Translation Elongation factor family members (EEFs) and Nucleolar proteins (NPM1, NOAL2, NAP1L1); |
| Adenoma, Interstitial, Mesenchyme, Dendrite, Motor | Undetermined. This module includes genes encoding immune-related (CD40, CD80, CXCL12, IFNA5, IL4R) as well as cytoskeleton-related molecules (Myosin, Dedicator of Cytokenesis, Syndecan 2, Plexin C1, Distrobrevin); |
| Granulocytes, Monocytes, Myeloid, ERK, Necrosis | Myeloid lineage. Related to M 1.5. Includes genes expressed in myeloid lineage cells (IGTB2/CD 18, Lymphotoxin beta receptor, Myeloid related proteins 8/14 Formyl peptide receptor 1), such as Monocytes and Neutrophils; |
| No keywords extracted. | Undetermined. This module is largely composed of transcripts with no known function. Only 20 genes associated with literature, including a member of the chemokine-like factor superfamily (CKLFSF8); |
| Lymphoma, T-cell, CD4, CD8, TCR, Thymus, Lymphoid, IL2 | T-cells. Includes T-cell surface markers (CD5, CD6, CD7, CD26, CD28, CD96) and molecules expressed by lymphoid lineage cells (lymphotoxin beta, IL2-inducible T-cell kinase, TCF7, T-cell differentiation protein mal, GATA3, STAT5B); |
| ERK, Transactivation, Cytoskeletal, MAPK, JNK | Undetermined. Includes genes encoding molecules that associate to the cytoskeleton (Actin related protein 2/3, MAPK1, MAP3K1, RAB5A). Also present are T-cell expressed genes (FAS, ITGA4/CD49D, ZNF1A1); |
| Myeloid, Macrophage, Dendritic, Inflammatory, Interleukin | Undetermined. Includes genes encoding for Immune-related cell surface molecules (CD36, CD86, LILRB), cytokines (IL15) and molecules involved in signaling pathways (FYB, TICAM2-Toll-like receptor pathway); |
| Replication, Repress, RAS, Autophosphorylation , Oncogenic | Undetermined. Includes kinases (UHMK1, CSNK1G1, CDK6, WNK1, TAOK1, CALM2, PRKCI, ITPKB, SRPK2, STK17B, DYRK2, PIK3R1, STK4, CLK4, PKN2) and RAS family members (G3BP, RAB14, RASA2, RAP2A, KRAS); |
| ISRE, Influenza, Antiviral, IFN-gamma, IFN-alpha, Interferon | Interferon-inducible. This set includes interferon-inducible genes: antiviral molecules (OAS1/2/3/L, GBP1, G1P2, EIF2AK2/PKR, MX1, PML), chemokines (CXCL10/IP-10), signaling molecules (STAT1, STAt2, IRF7, ISGF3G); |
| TGF-beta, TNF, Inflammatory, Apoptotic, Lipopolysaccharide | Inflammation I. Includes genes encoding molecules involved in inflammatory processes (e.g. IL8, ICAM1, C5R1, CD44, PLAUR, IL1A, CXCL16), and regulators of apoptosis (MCL1, FOXO3A, RARA, BCL3/6/2A1, GADD45B); |
| Granulocyte, Inflammatory, Defense, Oxidize, Lysosomal | Inflammation II. Includes molecules inducing or inducible by Granulocyte-Macrophage CSF (SPI1, IL18, ALOX5, ANPEP), as well as lysosomal enzymes (PPT1, CTSB/S, CES1, NEU1, ASAH1, LAMP2, CAST); |
| No keyword extracted | Undetermined. Includes protein phosphates (PPP1R12A, PTPRC, PPP1CB, PPM1B) and phosphoinositide 3-kinase (PI3K) family members (PIK3CA, PIK32A, PIP5K3); |
| No keyword extracted | Undetermined. Composed of only a small number of transcripts. Includes hemoglobin genes (HBA1, HBA2, HBB); |
| Complement, Host, Oxidative, Cytoskeletal, T-cell | Undetermined. This very large set includes T-cell surface markers (CD101, CD102, CD103) as well as molecules ubiquitously expressed among blood leukocytes (CXRCR1: fraktalkine receptor, CD47, P-selectin ligand); |
| Spliceosome, Methylation, Ubiquitin, Beta-catenin | Undetermined. Includes genes encoding proteasome subunits (PSMA2/5, PSMB5/8); ubiquitin protein ligases HIP2, STUB1, as well as components ofubiqutin ligase complexes (SUGT1); |
| CDC, TCR, CREB, Glycosylase | Undetermined. Includes genes encoding for several enzymes: aminomethyltransferase, arginyltransferase, asparagines synthetase, diacylglycerol kinase, inositol phosphatases, methyltransferases, helicases; and |
| Chromatin, Checkpoint, Replication, Transactivation | Undetermined. Includes genes encoding for protein kinases (PRKPIR, PRKDC, PRKCI) and phosphatases (e.g. PTPLB, PPP1R8/2CB). Also includes RAS oncogene family members and the NK cell receptor 2B4 (CD244); |

and combinations thereof, wherein the level of expression of genes in a sample is charted to the modules to determine a disease or condition.

The arrays, methods and systems of the present invetnion may even be used to select patients for a clinical trial by obtaining the transcriptome of a prospective patient; comparing the transcriptome to one or more transcriptional modules that are indicative of a disease or condition that is to be treated in the clinical trial; and determining the likelihood that a patient is a good candidate for the clinical trial based on the presence, absence or level of one or more genes that are expressed in the patient's transcriptome within one or more transcriptional modules that are correlated with success in a clinical trial. Generally, for each module a vector that correlates with a sum of the proportion of transcripts in a sample may be used, e.g., when each module includes a vector and wherein one or more diseases or conditions is associated with the one or more vectors. Therefore, each module may include a vector that correlates to the expression level of one or more genes within each module.

The present invention also includes arrays, e.g., custom microarrays, that include nucleic acid probes immobilized on a solid support that includes sufficient probes from one or more modules to provide a sufficient proportion of differentially expressed genes to distinguish between one or more diseases, the probes being selected from Table 3. For example, an array of nucleic acid probes immobilized on a solid support, in which the array includes at least two sets of probe modules selected from:

| Module I.D. | Transcriptional Modules |
|---|---|
| M 1.1 | Plasma cells. Includes genes encoding for Immunoglobulin chains (e.g. IGHM, IGJ, IGLL1, IGKC, IGHD) and the plasma cell marker CD38. |
| M 1.2 | Platelets. Includes genes encoding for platelet glycoproteins (ITGA2B, ITGB3, GP6, GP1A/B), and platelet-derived immune mediators such as PPPB (pro-platelet basic protein) and PF4 (platelet factor 4). |
| M 1.3 | B-cells. Includes genes encoding for B-cell surface markers (CD72, CD79A/B, CD19, CD22) and other B-cell associated molecules: Early B-cell factor (EBF), B-cell linker (BLNK) and B lymphoid tyrosine kinase (BLK). |
| M 1.4 | Undetermined. This set includes regulators and targets of cAMP signaling pathway (JUND, ATF4, CREM, PDE4, NR4A2, VIL2), as well as repressors of TNF-alpha mediated NF-KB activation (CYLD, ASK, TNFAIP3). |
| M 1.5 | Myeloid lineage. Includes molecules expressed by cells of the myeloid lineage (CD86, CD163, FCGR2A), some of which being involved in pathogen recognition (CD14, TLR2, MYD88). This set also includes TNF family members (TNFR2, BAFF). |
| M 1.6 | Undetermined. This set includes genes encoding for signaling molecules, e.g. the zinc finger containing inhibitor of activated STAT (PIAS 1 and PIAS2), or the nuclear factor of activated T-cells NFATC3. |
| M 1.7 | MHC/Ribosomal proteins. Almost exclusively formed by genes encoding MHC class I molecules (HLA-A,B,C,G,E)+ Beta 2-microglobulin (B2M) or Ribosomal proteins (RPLs, RPSs). |
| M 1.8 | Undetermined. Includes genes encoding metabolic enzymes (GLS, NSF1, NAT1) and factors involved in DNA replication (PURA, TERF2, EIF2S1). |
| M 2.1 | Cytotoxic cells. Includes cytotoxic T-cells amd NK-cells surface markers (CD8A, CD2, CD160, NKG7, KLRs), cytolytic molecules (granzyme, perforin, granulysin), chemokines (CCL5, XCL1) and CTL/NK-cell associated molecules (CTSW). |
| M 2.2 | Neutrophils. This set includes innate molecules that are found in neutrophil granules (Lactotransferrin: LTF, defensin: DEAF1, Bacterial Permeability Increasing protein: BPI, Cathelicidin antimicrobial protein: CAMP...). |
| M 2.3 | Erythrocytes. Includes hemoglobin genes (HGBs) and other erythrocyte-associated genes (erythrocytic alkirin:ANK1, Glycophorin C: GYPC, hydroxymethylbilane synthase: HMBS, erythroid associated factor: ERAF). |
| M 2.4 | Ribosomal proteins. Including genes encoding ribosomal proteins (RPLs, RPSs), Eukaryotic Translation Elongation factor family members (EEFs) and Nucleolar proteins (NPM1, NOAL2, NAP1L1). |
| M 2.5 | Undetermined. This module includes genes encoding immune-related (CD40, CD80, CXCL12, IFNA5, IL4R) as well as cytoskeleton-related molecules (Myosin, Dedicator of Cytokenesis, Syndecan 2, Plexin C1, Distrobrevin). |
| M 2.6 | Myeloid lineage. Related to M 1.5. Includes genes expressed in myeloid lineage cells (IGTB2/CD18, Lymphotoxin beta receptor, Myeloid related proteins 8/14 Formyl peptide receptor 1), such as Monocytes and Neutrophils. |
| M 2.7 | Undetermined. This module is largely composed of transcripts with no known function. Only 20 genes associated with literature, including a member of the chemokine-like factor superfamily (CKLFSF8). |
| M 2.8 | T-cells. Includes T-cell surface markers (CD5, CD6, CD7, CD26, CD28, CD96) and molecules expressed by lymphoid lineage cells (lymphotoxin beta, IL2-inducible T-cell kinase, TCF7, T-cell differentiation protein mal, GATA3, STAT5B). |
| M 2.9 | Undetermined. Includes genes encoding molecules that associate to the cytoskeleton (Actin related protein 2/3, MAPK1, MAP3K1, RAB5A). Also present are T-cell expressed genes (FAS, ITGA4/CD49D, ZNF1A1). |
| M2.10 | Undetermined. Includes genes encoding for Immune-related cell surface molecules (CD36, CD86, LILRB), cytokines (IL15) and molecules involved in signaling pathways (FYB, TICAM2-Toll-like receptor pathway). |
| M 2.11 | Undetermined. Includes kinases (UHMK1, CSNK1G1, CDK6, WNK1, TAOK1, CALM2, PRKCI, ITPKB, SRPK2, STK17B, DYRK2, PIK3R1, STK4, CLK4, PKN2) and RAS family members (G3BP, RAB14, RASA2, RAP2A, KRAS). |
| M 3.1 | Interferon-inducible. This set includes interferon-inducible genes: antiviral molecules (OAS1/2/3/L, GBP1, G1P2, EIF2AK2/PKR, MX1, PML), chemokines (CXCL10/IP-10), signaling molecules (STAT1, STAt2, IRF7, ISGF3G). |
| M 3.2 | Inflammation I. Includes genes encoding molecules involved in inflammatory processes (e.g. IL8, ICAM1, C5R1, CD44, PLAUR, IL1A, CXCL16), and regulators of apoptosis (MCL1, FOXO3A, RARA, BCL3/6/2A1, GADD45B). |
| M 3.3 | Inflammation II. Includes molecules inducing or inducible by Granulocyte-Macrophage CSF (SPI1, IL18, ALOX5, ANPEP), as well as lysosomal enzymes (PPT1, CTSB/S, CES1, NEU1, ASAH1, LAMP2, CAST). |
| M 3.4 | Undetermined. Includes protein phosphates (PPP1R12A, PTPRC, PPP1CB, PPM1B) and phosphoinositide 3-kinase (PI3K) family members (PIK3CA, PIK32A, PIP5K3). |
| M 3.5 | Undetermined. Composed of only a small number of transcripts. Includes hemoglobin genes (HBA1, HBA2, HBB). |
| M 3.6 | Undetermined. This very large set includes T-cell surface markers (CD101, CD102, CD103) as well as molecules ubiquitously expressed among blood leukocytes (CXRCR1: fraktalkine receptor, CD47, P-selectin ligand). |
| M 3.7 | Undetermined. Includes genes encoding proteasome subunits (PSMA2/5, PSMB5/8); ubiquitin protein ligases HIP2, STUB1, as well as components of ubiqutin ligase complexes (SUGT1). |
| M 3.8 | Undetermined. Includes genes encoding for several enzymes: aminomethyltransferase, arginyltransferase, asparagines synthetase, diacylglycerol kinase, inositol phosphatases, methyltransferases, helicases... |
| M 3.9 | Undetermined. Includes genes encoding for protein kinases (PRKPIR, PRKDC, PRKCI) and phosphatases (e.g. PTPLB, PPP1R8/2CB). Also includes RAS oncogene family members and the NK cell receptor 2B4 (CD244). |

wherein the probes in the first probe set have one or more interrogation positions respectively corresponding to one or more diseases. The array may have between 100 and 100,000 probes, and each probe may be, e.g., 9-21 nucleotides long. When separated into organized prose sets, these may be interrogated separately.

The present invention also includes one or more nucleic acid probes immobilized on a solid support to form a module array that includes at least one pair of first and second probe groups, each group having one or more probes as defined by Table 3. The probe groups are selected to provide a composite transcriptional marker vector that is consistent across microarray platforms. In fact, the probe groups may even be used to provide a composite transcriptional marker vector that is consistent across microarray platforms and displayed in a summary for regulatory approval. The skilled artisan will appreciate that using the modules of the present invention it is possible to rapidly develop one or more disease specific arrays that may be used to rapidly diagnose or distinguish between different disease and/or conditions.

### BRIEF DESCRIPTION OF THE DRAWINGS

For a more complete understanding of the features and advantages of the present invention, reference is now made to the detailed description of the invention along with the accompanying figures and in which:

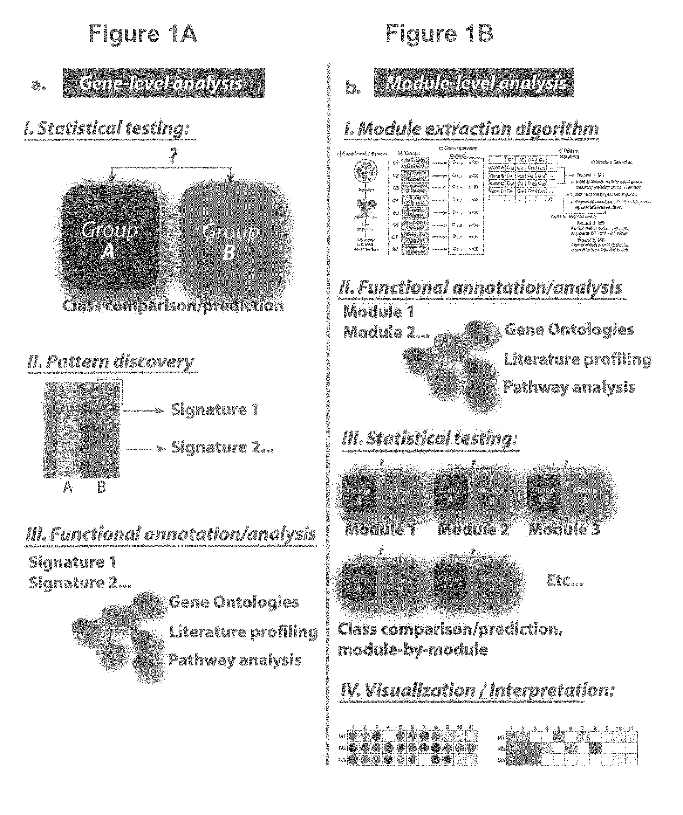

Figures 1A to 1C show the basic microarray data mining strategy steps involved in accepted gene-level microarray data analysis (Figure 1A), the modular mining strategy of the present invention

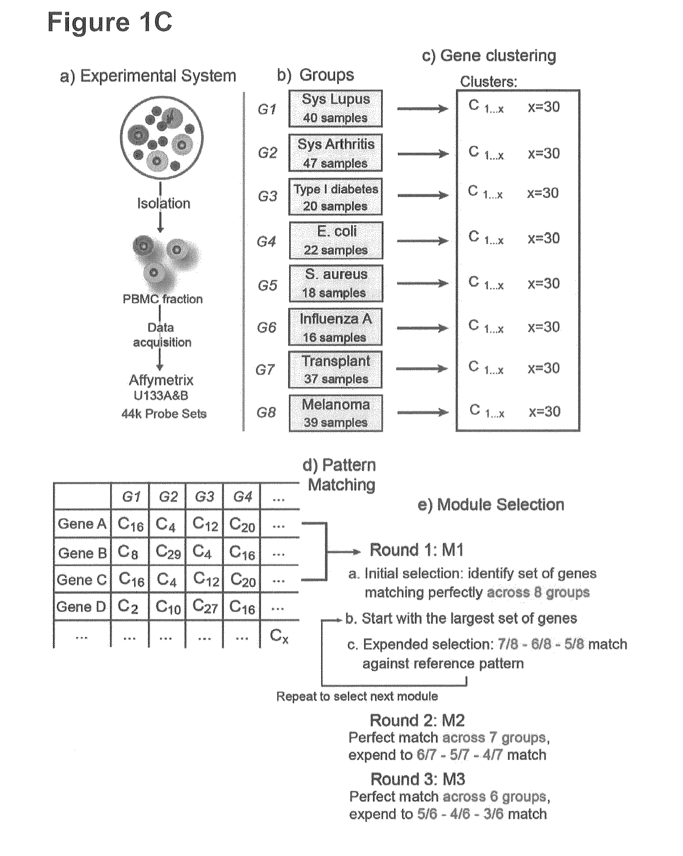

Figure 1b and a full size representation of the module extraction algorithm Figure 1C. Figure 1C provides a more detailed view of the module extraction algorithm in which step (a) shows examples of data are generated in the context of a defined experimental system (*e.g*. ex-vivo PBMCs); step (b) shows that the transcriptional profiles are obtained for several experimental groups (e.g. S1-8); step (c) shows that for each group, genes are distributed among x clusters (*e.g*. x=30) based on similarity of expression profiles (using K-means clustering algorithms); step (d) shows the cluster distribution of each gene across the different experimental groups is recorded into a table and distribution patterns are matched; and step (e) shows that modules are selected through an iterative process, starting with the largest set of genes distributed among the same cluster across all experimental groups (are found in the same cluster for eight out of eight groups). The selection is expanded from this core reference pattern to include genes with 7/8, 6/8 and 5/8 matches. Once a module has been formed, the genes are withdrawn from the selection pool. The process is then repeated, starting with the second largest group of genes, progressively reducing levels of stringency.

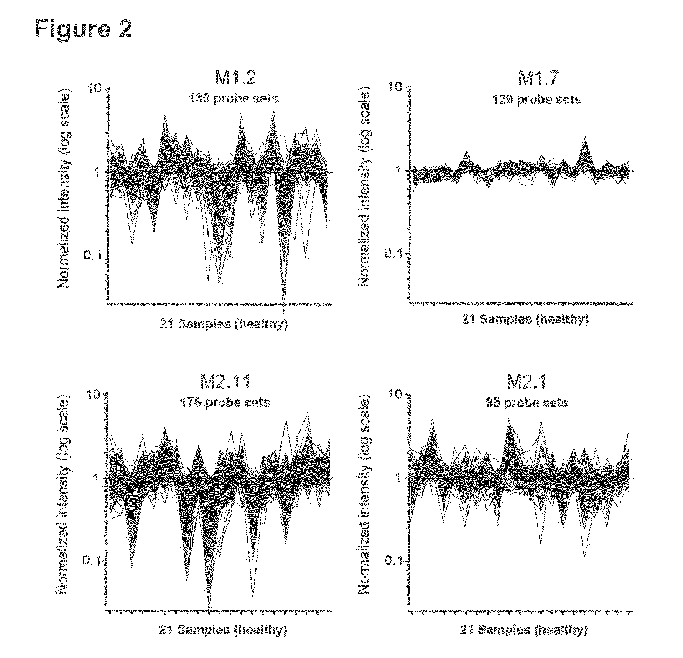

Figure 2: Modular gene expression profiles across an independent group of samples. Differences in transcriptional behavior between modules are illustrated in a set of samples obtained from twenty-one healthy volunteers. The samples were not used in the module selection process. The graphs represent transcriptional profiles, with each line showing levels of expression (y-axis) of a single transcript across multiple conditions (samples, x-axis). Transcriptional profiles of Modules 1.2, 1.7, 2.1 and 2.11 are shown. The expression of each gene is normalized to the median of the measurements obtained across all samples.

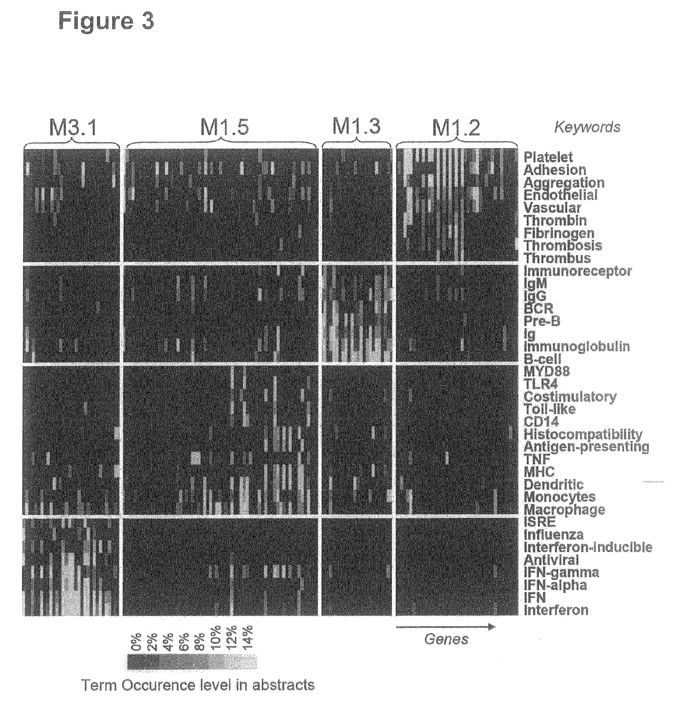

Figure 3: Distribution of keyword occurrence in the literature obtained for four sets of coordinately expressed genes. Term occurrence levels in abstracts were computed for all the genes in M3.1, M1.5, M1.3 and M1.2 associated with at least ten publications (representing more than 26,000 abstracts). Keyword profiles were extracted for each module and a selection was used to generate this figure. Levels of keyword occurrence in abstracts are indicated by color scale, with yellow representing high occurrence. M3.1 is associated to interferon, M1.5 is associated to pathogen recognition molecules /myeloid lineage cells, M1.3 is associated with B-cells and M1.2 is associated with platelets.

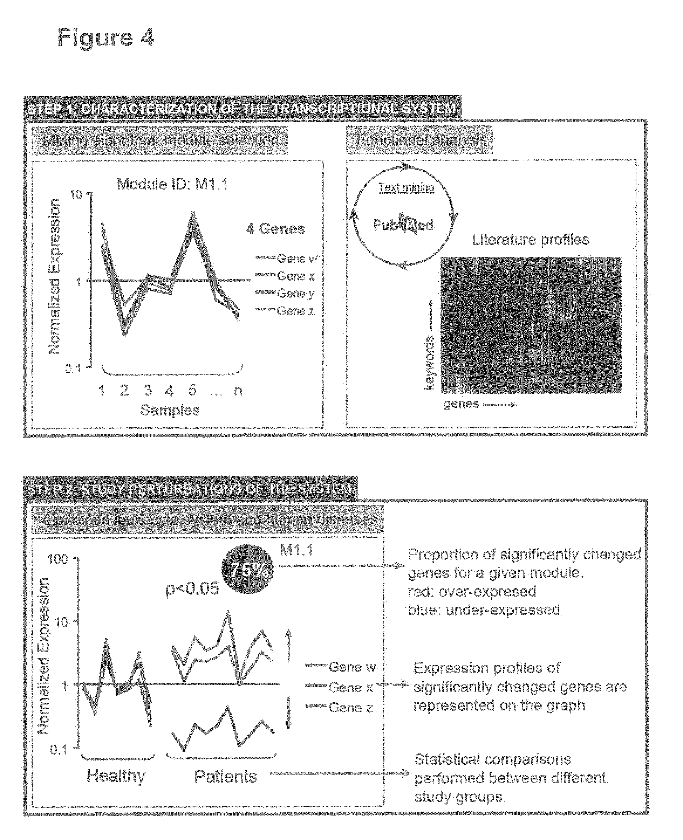

Figure 4: Modular microarray analysis strategy. The proposed microarray data analysis strategy includes two basic steps: 1. Characterization of the transcriptional system: Transcriptional components are extracted through an unsupervised "clustering meta-analysis" (Figure 1). The genes that form each module (designated by a unique ID, *e.g*. M1.1) possess a consistent transcriptional behavior across all conditions for a defined experimental system. Transcriptional modules are identified by a two digit ID (*e.g*. 1.1). A graph represents the expression profile of the genes forming a module across multiple conditions (samples). Each module is in turn functionally characterized (*e.g*. through the analysis of literature profiles). The result is a collection of biologically meaningful transcriptional determinants. 2. Study perturbations of the system: Comparisons between study groups are performed independently for each module. This analysis permitted identification of changes in expression levels for different conditions (*e.g*. comparing samples from patients and healthy controls). The results obtained for each module are represented on a graph. The proportion of genes that meet the significance criteria (class comparison) is indicated in a circle, with red being the proportion of significantly over-expressed genes and blue the proportion of significantly under-expressed genes. In this theoretical example 3/4 genes (75%) with p<0.05 were represented on the graph. Two of these genes are over-expressed (50% - red) and one is under-expressed (25% - blue).

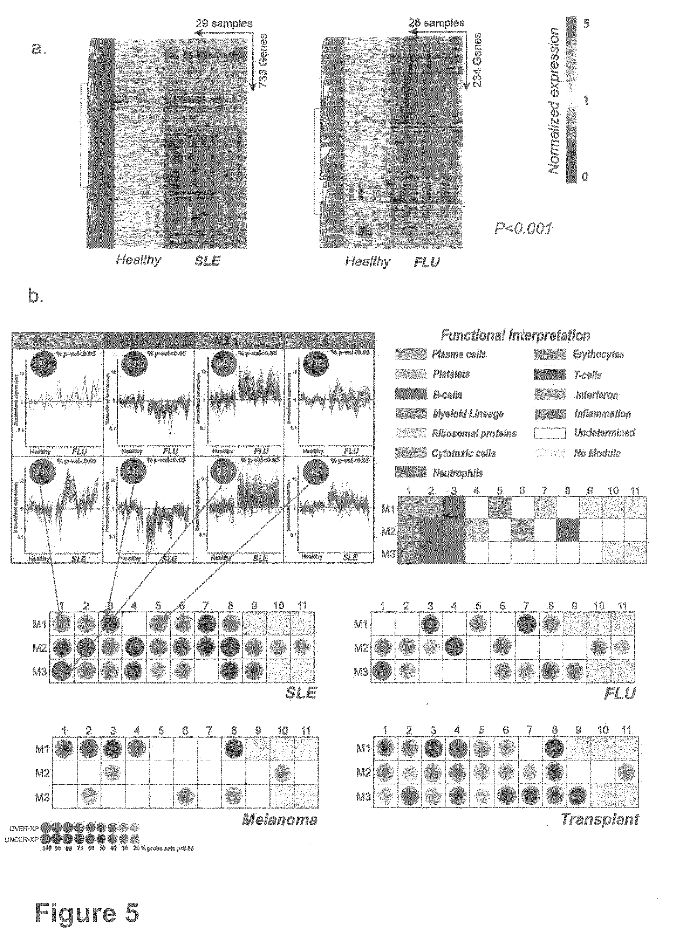

Figure 5 is an analysis of patient blood leukocyte transcriptional profiles. a) Gene level analysis. The upper panel shows a Statistical comparisons identified differentially expressed transcripts between patients with SLE or acute influenza infection and their respective control (p<0.001, Mann Whitney U test, Benjamini and Hochberg False Discovery Rate: SLE = 733 transcripts, FLU=234 transcripts). Clustering analysis grouped genes based on expression patterns and results are represented by a heatmap. The lower panel is a module level analysis. For each module, gene expression levels obtained for patients (SLE or FLU) and respective healthy volunteer PBMCs were compared (p<0.05, Mann-Whitney rank test). Pie charts indicate the proportion of genes that were significantly changed. Graphs represent transcriptional profiles of the genes that were significantly changed, with each line showing levels of expression (y-axis) of a single transcript across multiple conditions (samples, x-axis). The expression of each gene is normalized to the median of the measurements obtained across all samples. Results obtained for the 28 PBMC transcriptional modules are displayed on a grid. The coordinates are used to indicate module IDs (e.g. M2.8 is row M2, column 8). Spots indicate the proportion of genes that were significantly changed for each module. Red spots: proportion of over-expressed genes, Blue spots: proportion of under-expressed genes. Functional interpretation is indicated on a grid by a color code.

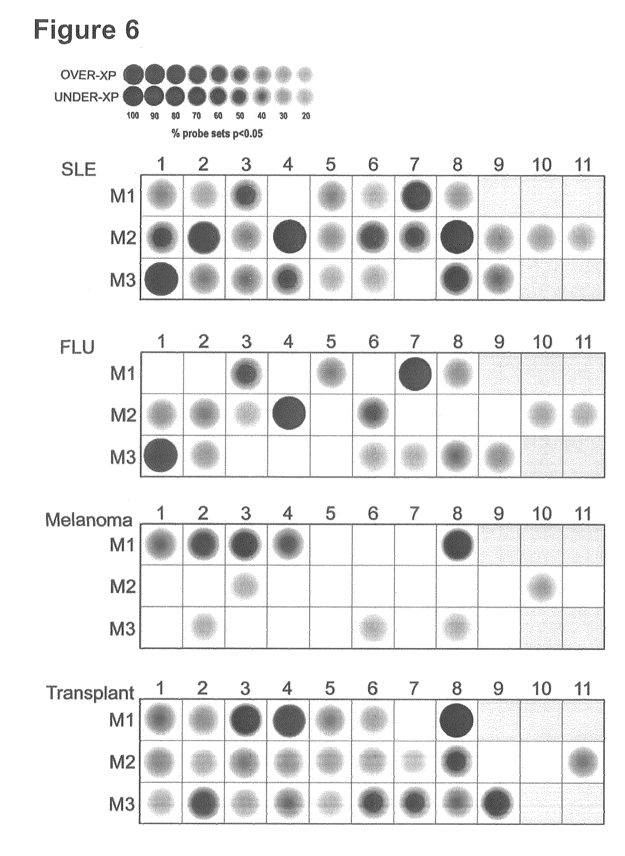

Figure 6: Module maps of transcriptional changes caused by disease. For each module, expression levels measured in PBMCs isolated from patients and their respective healthy control group were compared (Mann Whitney Rank test, p<0.05 between: eighteen patients with SLE and eleven healthy volunteers; sixteen patients with acute influenza infection and ten volunteers; sixteen patients with metastatic melanoma and ten volunteers; and sixteen liver transplant recipients vs. ten volunteers). Spots indicate the proportion of genes that were significantly changed for each module. Red spots: proportion of over-expressed genes, Blue spots: proportion of under-expressed genes. Results obtained for the twenty-eight PBMC transcriptional modules are displayed on a grid. The coordinates are used to indicate module IDs (*e.g*. M2.8 is row M2, column 8).

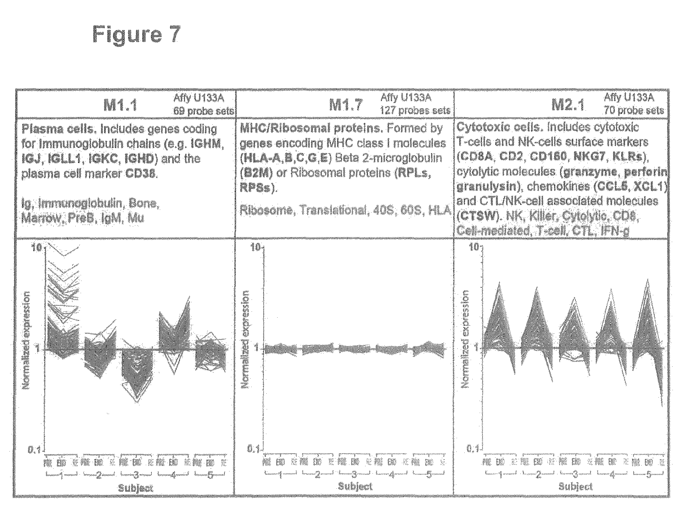

Figure 7: Analysis of a third-party dataset. Modular microarray data analysis was carried out for a published PBMC gene expression dataset. The study investigated the effects of exercise on gene expression. Blood samples were obtained for fifteen subjects, pre-exercise (Pre), end-exercise (End), and 60 min into recovery (Re). Transcriptional profiles were generated for five pools of three subjects each. Expression profiles are shown for three transcriptional modules. The expression of each gene is normalized to the median of the measurements obtained across all samples. Keywords extracted from the literature are indicated in green.

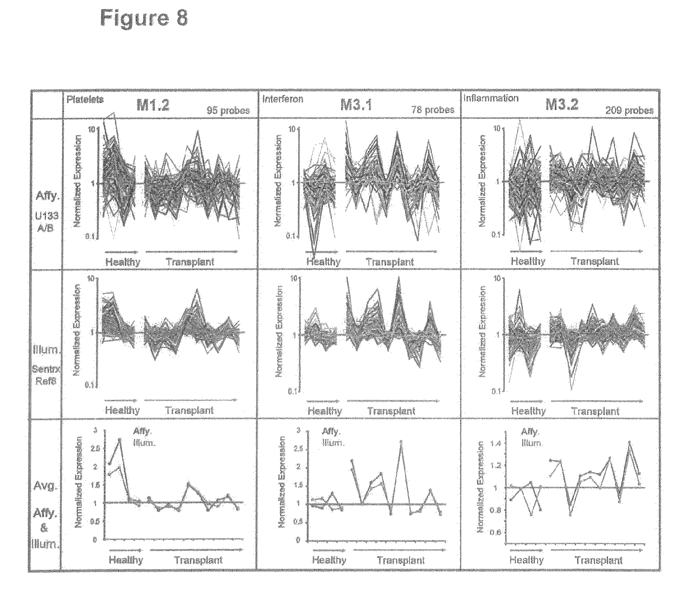

Figure 8: Cross-platform validation. PBMC samples from healthy donors and liver transplant recipient were analyzed on two different microarray platforms: Affymetrix U133A&B GeneChips and Illumina Sentrix Human Ref8 BeadChips. The same pools of total RNA were used to independently prepare biotin-labeled cRNA targets. Results are shown for a set of transcripts shared by the two platforms (Affymetrix: upper panel; Illumina: middle panel). The expression of each gene is normalized to the median of the measurements obtained across all samples. The averaged expression values for all the genes forming each transcriptional module are shown in the bottom panel for both Affymetrix and Illumina platforms.

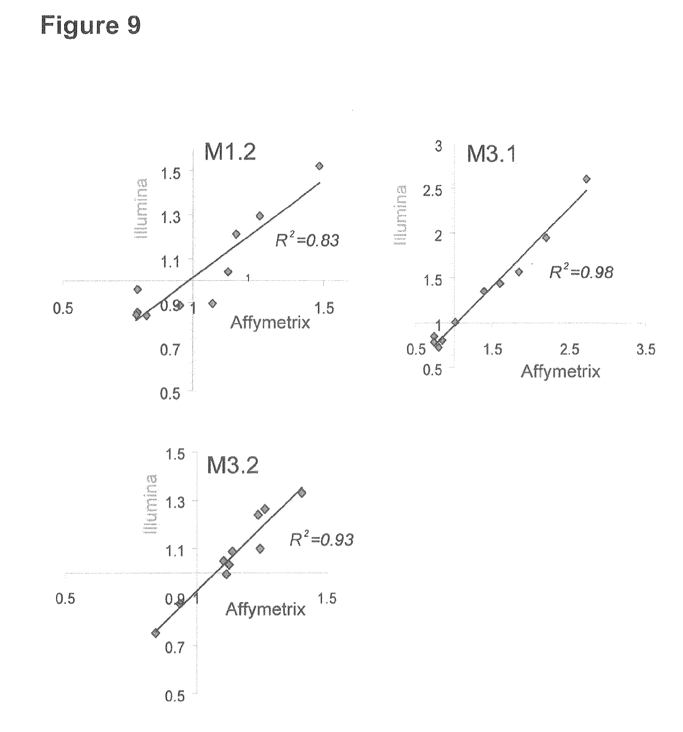

Figure 9 includes three graphs that the reproducibility of module-level expression data across microarray platforms. PBMC samples from healthy donors and liver transplant recipient were analyzed on two different microarray platforms: Affymetrix U133A&B GeneChips and Illumina Sentrix Human Ref8 BeadChips. The same source of total RNA was used to independently prepare biotin-labeled cRNA targets. Normalized "Modular expression levels" were obtained for each sample by averaging expression values of the genes forming each module. The modular expression levels derived from data generated by Affymetrix and Illumina platforms were highly comparable: Pearson correlation coefficient R2 = 0.83, 0.98 and 0.93, for M1.2, M3.1 and M3.2 respectively; p<0.0001).

### DETAILED DESCRIPTION OF THE INVENTION

While the making and using of various embodiments of the present invention are discussed in detail below, it should be appreciated that the present invention provides many applicable inventive concepts that can be embodied in a wide variety of specific contexts. The specific embodiments discussed herein are merely illustrative of specific ways to make and use the invention and do not delimit the scope of the invention.

To facilitate the understanding of this invention, a number of terms are defined below. Terms defined herein have meanings as commonly understood by a person of ordinary skill in the areas relevant to the present invention. Terms such as "a", "an" and "the" are not intended to refer to only a singular entity, but include the general class of which a specific example may be used for illustration. The terminology herein is used to describe specific embodiments of the invention, but their usage does not delimit the invention, except as outlined in the claims. Unless defined otherwise, all technical and scientific terms used herein have the meaning commonly understood by a person skilled in the art to which this invention belongs. The following references provide one of skill with a general definition of many of the terms used in this invention: Singleton et al., DICTIONARY OF MICROBIOLOGY AND MOLECULAR BIOLOGY (2d ed. 1994); THE CAMBRIDGE DICTIONARY OF SCIENCE AND TECHNOLOGY (Walker ed., 1988); THE GLOSSARY OF GENETICS, 5TH ED., R. Rieger et al. (eds.), Springer Verlag (1991); and Hale & Marham, THE HARPER COLLINS DICTIONARY OF BIOLOGY (1991).

Various biochemical and molecular biology methods are well known in the art. For example, methods of isolation and purification of nucleic acids are described in detail in WO 97/10365, WO 97/27317, Chapter 3 of Laboratory Techniques in Biochemistry and Molecular Biology: Hybridization With Nucleic Acid Probes, Part I. Theory and Nucleic Acid Preparation, (P. Tijssen, ed.) Elsevier, N.Y. (1993); Chapter 3 of Laboratory Techniques in Biochemistry and Molecular Biology: Hybridization With Nucleic Acid Probes, Part 1. Theory and Nucleic Acid Preparation, (P. Tijssen, ed.) Elsevier, N.Y. (1993); and Sambrook et al., Molecular Cloning: A Laboratory Manual, Cold Spring Harbor Press, N.Y., (1989); and Current Protocols in Molecular Biology, (Ausubel, F. M. et al., eds.) John Wiley & Sons, Inc., New York (1987-1999), including supplements such as supplement 46 (April 1999).

### BIOINFORMATICS DEFINTIONS

As used herein, an "object" refers to any item or information of interest (generally textual, including noun, verb, adjective, adverb, phrase, sentence, symbol, numeric characters, etc.). Therefore, an object is anything that can form a relationship and anything that can be obtained, identified, and/or searched from a source. "Objects" include, but are not limited to, an entity of interest such as gene, protein, disease, phenotype, mechanism, drug, etc. In some aspects, an object may be data, as further described below.

As used herein, a "relationship" refers to the co-occurrence of objects within the same unit (e.g., a phrase, sentence, two or more lines of text, a paragraph, a section of a webpage, a page, a magazine, paper, book, etc.). It may be text, symbols, numbers and combinations, thereof

As used herein, "meta data content" refers to information as to the organization of text in a data source. Meta data can comprise standard metadata such as Dublin Core metadata or can be collection-specific. Examples of metadata formats include, but are not limited to, Machine Readable Catalog (MARC) records used for library catalogs, Resource Description Format (RDF) and the Extensible Markup Language (XML). Meta objects may be generated manually or through automated information extraction algorithms.

As used herein, an "engine" refers to a program that performs a core or essential function for other programs. For example, an engine may be a central program in an operating system or application program that coordinates the overall operation of other programs. The term "engine" may also refer to a program containing an algorithm that can be changed. For example, a knowledge discovery engine may be designed so that its approach to identifying relationships can be changed to reflect new rules of identifying and ranking relationships.

As used herein, "semantic analysis" refers to the identification of relationships between words that represent similar concepts, e.g., though suffix removal or stemming or by employing a thesaurus. "Statistical analysis" refers to a technique based on counting the number of occurrences of each term (word, word root, word stem, n-gram, phrase, etc.). In collections unrestricted as to subject, the same phrase used in different contexts may represent different concepts. Statistical analysis of phrase co-occurrence can help to resolve word sense ambiguity. "Syntactic analysis" can be used to further decrease ambiguity by part-of-speech analysis. As used herein, one or more of such analyses are referred to more generally as "lexical analysis." "Artificial intelligence (AI)" refers to methods by which a non-human device, such as a computer, performs tasks that humans would deem noteworthy or "intelligent." Examples include identifying pictures, understanding spoken words or written text, and solving problems.

As used herein, the term "database" refers to repositories for raw or compiled data, even if various informational facets can be found within the data fields. A database is typically organized so its contents can be accessed, managed, and updated (e.g., the database is dynamic). The term "database" and "source" are also used interchangeably in the present invention, because primary sources of data and information are databases. However, a "source database" or "source data" refers in general to data, e.g., unstructured text and/or structured data, that are input into the system for identifying objects and determining relationships. A source database may or may not be a relational database. However, a system database usually includes a relational database or some equivalent type of database which stores values relating to relationships between objects.

As used herein, a "system database" and "relational database" are used interchangeably and refer to one or more collections of data organized as a set of tables containing data fitted into redefined categories. For example, a database table may comprise one or more categories defined by columns (e.g. attributes), while rows of the database may contain a unique object for the categories defined by the columns. Thus, an object such as the identity of a gene might have columns for its presence, absence and/or level of expression of the gene. A row of a relational database may also be referred to as a "set" and is generally defined by the values of its columns. A "domain" in the context of a relational database is a range of valid values a field such as a column may include.

As used herein, a "domain of knowledge" refers to an area of study over which the system is operative, for example, all biomedical data. It should be pointed out that there is advantage to combining data from several domains, for example, biomedical data and engineering data, for this diverse data can sometimes link things that cannot be put together for a normal person that is only familiar with one area or research/study (one domain). A "distributed database" refers to a database that may be dispersed or replicated among different points in a network.

Terms such "data" and "information" are often used interchangeably, as are "information" and "knowledge." As used herein, "data" is the most fundamental unit that is an empirical measurement or set of measurements. Data is compiled to contribute to information, but it is fundamentally independent of it. Information, by contrast, is derived from interests, e.g., data (the unit) may be gathered on ethnicity, gender, height, weight and diet for the purpose of finding variables correlated with risk of cardiovascular disease. However, the same data could be used to develop a formula or to create "information" about dietary preferences, i.e., likelihood that certain products in a supermarket have a higher likelihood of selling.

As used herein, "information" refers to a data set that may include numbers, letters, sets of numbers, sets of letters, or conclusions resulting or derived from a set of data. "Data" is then a measurement or statistic and the fundamental unit of information. "Information" may also include other types of data such as words, symbols, text, such as unstructured free text, code, etc. "Knowledge" is loosely defined as a set of information that gives sufficient understanding of a system to model cause and effect. To extend the previous example, information on demographics, gender and prior purchases may be used to develop a regional marketing strategy for food sales while information on nationality could be used by buyers as a guideline for importation of products. It is important to note that there are no strict boundaries between data, information, and knowledge; the three terms are, at times, considered to be equivalent. In general, data comes from examining, information comes from correlating, and knowledge comes from modeling.

As used herein, "a program" or "computer program" refers generally to a syntactic unit that conforms to the rules of a particular programming language and that is composed of declarations and statements or instructions, divisible into, "code segments" needed to solve or execute a certain function, task, or problem. A programming language is generally an artificial language for expressing programs.

As used herein, a "system" or a "computer system" generally refers to one or more computers, peripheral equipment, and software that perform data processing. A "user" or "system operator" in general includes a person, that uses a computer network accessed through a "user device" (e.g., a computer, a wireless device, etc) for the purpose of data processing and information exchange. A "computer" is generally a functional unit that can perform substantial computations, including numerous arithmetic operations and logic operations without human intervention.

As used herein, "application software" or an "application program" refers generally to software or a program that is specific to the solution of an application problem. An "application problem" is generally a problem submitted by an end user and requiring information processing for its solution.

As used herein, a "natural language" refers to a language whose rules are based on current usage without being specifically prescribed, e.g., English, Spanish or Chinese. As used herein, an "artificial language" refers to a language whose rules are explicitly established prior to its use, e.g., computer-programming languages such as C, C++, Java, BASIC, FORTRAN, or COBOL.

As used herein, "statistical relevance" refers to using one or more of the ranking schemes (O/E ratio, strength, etc.), where a relationship is determined to be statistically relevant if it occurs significantly more frequently than would be expected by random chance.

As used herein, the terms "coordinately regulated genes" or "transcriptional modules" are used interchangeably to refer to grouped, gene expression profiles (e.g., signal values associated with a specific gene sequence) of specific genes. Each transcriptional module correlates two key pieces of data, a literature search portion and actual empirical gene expression value data obtained from a gene microarray. The set of genes that is selected into a transcriptional modules is based on the analysis of gene expression data (module extraction algorithm described above). Additional steps are taught by Chaussabel, D. & Sher, A. Mining microarray expression data by literature profiling. Genome Biol 3, RESEARCH0055 (2002), (http://genomebiology.com/2002/3/10/research/0055) relevant portions incorporated herein by reference and expression data obtained from a disease or condition of interest, e.g., Systemic Lupus erythematosus, arthritis, lymphoma, carcinoma, melanoma, acute infection, autoimmune disorders, autoinflammatory disorders, etc.).

The Table below lists examples of keywords that were used to develop the literature search portion or contribution to the transcription modules. The skilled artisan will recognize that other terms may easily be selected for other conditions, e.g., specific cancers, specific infectious disease, transplantation, etc. For example, genes and signals for those genes associated with T cell activation are described hereinbelow as Module ID "M 2.8" in which certain keywords (e.g., Lymphoma, T-cell, CD4, CD8, TCR, Thymus, Lymphoid, IL2) were used to identify key T-cell associated genes, e.g., T-cell surface markers (CD5, CD6, CD7, CD26, CD28, CD96); molecules expressed by lymphoid lineage cells (lymphotoxin beta, IL2-inducible T-cell kinase, TCF7; and T-cell differentiation protein mal, GATA3, STAT5B). Next, the complete module is developed by correlating data from a patient population for these genes (regardless of platform, presence/absence and/or up or downregulation) to generate the transcriptional module. In some cases, the gene profile does not match (at this time) any particular clustering of genes for these disease conditions and data, however, certain physiological pathways (e.g., cAMP signaling, zinc-finger proteins, cell surface markers, etc.) are found within the "Underdetermined" modules. In fact, the gene expression data set may be used to extract genes that have coordinated expression prior to matching to the keyword search, i.e., either data set may be correlated prior to cross-referencing with the second data set.

**Table 1. Examples of Transcriptional Modules**

| **Example Module I.D.** | **Example Keyword selection** | **Gene Profile Assessment** |
|---|---|---|
| M1.1 | Ig, Immunoglobulin, Bone, Marrow, PreB, IgM, Mu. | **Plasma cells.** Includes genes encoding for Immunoglobulin chains (*e.g*. IGHM, IGJ, IGLL1, IGKC, IGHD) and the plasma cell marker CD38. |
| M1.2 | Platelet, Adhesion, Aggregation, Endothelial, Vascular | **Platelets.** Includes genes encoding for platelet glycoproteins (ITGA2B, ITGB3, GP6, GP1A/B), and platelet-derived immune mediators such as PPPB (pro-platelet basic protein) and PF4 (platelet factor 4). |
| M1.3 | Immunoreceptor, BCR, B-cell, IgG | **B-cells.** Includes genes encoding for B-cell surface markers (CD72, CD79AB, CD19, CD22) and other B-cell associated molecules: Early B-cell factor (EBF), B-cell linker (BLNK) and B lymphoid tyrosine kinase (BLK). |
| M 1.4 | Replication, Repression, Repair, CREB, Lymphoid, TNF-alpha | **Undetermined.** This set includes regulators and targets of cAMP signaling pathway (JUND, ATF4, CREM, PDE4, NR4A2, VIL2), as well as repressors of TNF-alpha mediated NF-KB activation (CYLD, ASK, TNFAIP3). |
| M 1.5 | Monocytes, Dendritic, MHC, Costimulatory, TLR4, MYD88 | **Myeloid lineage.** Includes molecules expressed by cells of the myeloid lineage (CD86, CD163, FCGR2A), some of which being involved in pathogen recognition (CD14, TLR2, MYD88). This set also includes TNF family members (TNFR2, BAFF). |
| M 1.6 | Zinc, Finger, P53, RAS | **Undetermined.** This set includes genes encoding for signaling molecules, e.g., the zinc finger containing inhibitor of activated STAT (PIAS1 and PIAS2), or the nuclear factor of activated T-cells NFATC3. |
| M 1.7 | Ribosome, Translational, 40S, 60S, HLA | **MHC/Ribosomal proteins.** Almost exclusively formed by genes encoding MHC class I molecules (HLA-A,B,C,G,E)+ Beta 2-microglobulin (B2M) or Ribosomal proteins (RPLs, RPSs). |
| M 1.8 | Metabolism, Biosynthesis, Replication, Helicase | **Undetermined.** Includes genes encoding metabolic enzymes (GLS, NSF1, NAT1) and factors involved in DNA replication (PURA, TERF2, EIF2S1). |
| M 2.1 | NK, Killer, Cytolytic, CD8, Cell-mediated, T-cell, CTL, IFN-g | **Cytotoxic cells.** Includes cytotoxic T-cells and NK-cells surface markers (CD8A, CD2, CD160, NKG7, KLRs), cytolytic molecules (granzyme, perforin, granulysin), chemokines (CCL5, XCL1) and CTL/NK-cell associated molecules (CTSW). |
| M 2.2 | Granulocytes, Neutrophils, Defense, Myeloid, Marrow | **Neutrophils.** This set includes innate molecules that are found in neutrophil granules (Lactotransferrin: LTF, defensin: DEAF1, Bacterial Permeability Increasing protein: BPI, Cathelicidin antimicrobial protein: CAMP). |
| M 2.3 | Erythrocytes, Red, Anemia, Globin, Hemoglobin | **Erythrocytes.** Includes hemoglobin genes (HGBs) and other erythrocyte-associated genes (erythrocytic alkirin:ANKI, Glycophorin C: GYPC, hydroxymethylbilane synthase: HMBS, erythroid associated factor: ERAF). |
| M 2.4 | Ribonucleoprotein, 60S, nucleolus, Assembly, Elongation | **Ribosomal proteins.** Including genes encoding ribosomal proteins (RPLs, RPSs), Eukaryotic Translation Elongation factor family members (EEFs) and Nucleolar proteins (NPM1, NOAL2, NAP1L1). |
| M 2.5 | Adenoma, Interstitial, Mesenchyme, Dendrite, Motor | **Undetermined.** This module includes genes encoding immune-related (CD40, CD80, CXCL12, IFNA5, IL4R) as well as cytoskeleton-related molecules (Myosin, Dedicator of Cytokenesis, Syndecan 2, Plexin C1, Distrobrevin). |
| M 2.6 | Granulocytes, Monocytes, Myeloid, ERK, Necrosis | **Myeloid lineage.** Related to M 1.5. Includes genes expressed in myeloid lineage cells (IGTB2/CD18, Lymphotoxin beta receptor, Myeloid related proteins 8/14 Formyl peptide receptor 1), such as Monocytes and Neutrophils. |
| M 2.7 | No keywords extracted. | **Undetermined.** This module is largely composed of transcripts with no known function. Only 20 genes associated with literature, including a member of the chemokine-like factor superfamily (CKLFSF8). |
| M 2.8 | Lymphoma, T-cell, CD4, CD8, TCR, Thymus, Lymphoid, IL2 | **T-cells.** Includes T-cell surface markers (CD5, CD6, CD7, CD26, CD28, CD96) and molecules expressed by lymphoid lineage cells (lymphotoxin beta, IL2-inducible T-cell kinase, TCF7, T-cell differentiation protein mal, GATA3, STAT5B). |
| M 2.9 | ERK, Transactivation, Cytoskeletal, MAPK, JNK | **Undetermined.** Includes genes encoding molecules that associate to the cytoskeleton (Actin related protein 2/3, MAPK1, MAP3K1, RAB5A). Also present are T-cell expressed genes (FAS, ITGA4/CD49D, ZNF1A1). |
| M2.10 | Myeloid, Macrophage, Dendritic, Inflammatory, Interleukin | **Undetermined.** Includes genes encoding for Immune-related cell surface molecules (CD36, CD86, LILRB), cytokines (IL15) and molecules involved in signaling pathways (FYB, TICAM2-Toll-like receptor pathway). |
| M 2.11 | Replication, Repress, RAS, Autophosphorylation, Oncogenic | **Undetermined.** Includes kinases (UHMK1, CSNK1G1, CDK6, WNK1, TAOK1, CALM2, PRKCI, ITPKB, SRPK2, STK17B, DYRK2, PIK3R1, STK4, CLK4, PKN2) and RAS family members (G3BP, RAB14, RASA2, RAP2A, KRAS). |
| M 3.1 | ISRE, Influenza, Antiviral, IFN-gamma, IFN-alpha, Interferon | **Interferon-inducible.** This set includes interferon-inducible genes: antiviral molecules (OAS1/2/3/L, GBP1, G1P2, EIF2AK2/PKR, MX1, PML), chemokines (CXCL10/IP-10), signaling molecules (STAT1, STAt2, IRF7, ISGF3G). |
| M 3.2 | TGF-beta, TNF, Inflammatory, Apoptotic, Lipopolysaccharide | **Inflammation I.** Includes genes encoding molecules involved in inflammatory processes (e.g., IL8, ICAM1, C5R1, CD44, PLAUR, IL1A, CXCL16), and regulators of apoptosis (MCL1, FOXO3A, RARA, BCL3/6/2A1, GADD45B). |
| M 3.3 | Granulocyte, Inflammatory, Defense, Oxidize, Lysosomal | **Inflammation II.** Includes molecules inducing or inducible by Granulocyte-Macrophage CSF (SPI1, IL18, ALOX5, ANPEP), as well as lysosomal enzymes (PPT1, CTSB/S, CES1, NEU1, ASAH1, LAMP2, CAST). |
| M 3.4 | No keyword extracted | **Undetermined.** Includes protein phosphates (PPP1R12A, PTPRC, PPP1CB, PPM1B) and phosphoinositide 3-kinase (PI3K) family members (PIK3CA, PIK32A, PIP5K3). |
| M 3.5 | No keyword extracted | **Undetermined.** Composed of only a small number of transcripts. Includes hemoglobin genes (HBA1, HBA2, HBB). |
| M 3.6 | Complement, Host, Oxidative, Cytoskeletal, T-cell | **Undetermined.** Large set that includes T-cell surface markers (CD 101, CD 102, CD 103) as well as molecules ubiquitously expressed among blood leukocytes (CXRCR1: fraktalkine receptor, CD47, P-selectin ligand). |
| M 3.7 | Spliceosome, Methylation, Ubiquitin, Beta-catenin | **Undetermined.** Includes genes encoding proteasome subunits (PSMA2/5, PSMB5/8); ubiquitin protein ligases HIP2, STUB1, as well as components of ubiqutin ligase complexes (SUGT1). |
| M 3.8 | CDC, TCR, CREB, Glycosylase | **Undetermined.** Includes genes encoding for several enzymes: aminomethyltransferase, arginyltransferase, asparagines synthetase, diacylglycerol kinase, inositol phosphatases, methyltransferases, helicases... |
| M 3.9 | Chromatin, Checkpoint, Replication, Transactivation | **Undetermined.** Includes genes encoding for protein kinases (PRKPIR, PRKDC, PRKCI) and phosphatases (e.g., PTPLB, PPP1R8/2CB). Also includes RAS oncogene family members and the NK cell receptor 2B4 (CD244). |

### BIOLOGICAL DEFINTIONS

As used herein, the term "array" refers to a solid support or substrate with one or more peptides or nucleic acid probes attached to the support. Arrays typically have one or more different nucleic acid or peptide probes that are coupled to a surface of a substrate in different, known locations. These arrays, also described as "microarrays" or "gene-chips" that may have 10,000; 20,000, 30,000; or 40,000 different identifiable genes based on the known genome, e.g., the human genome. These pan-arrays are used to detect the entire "transcriptome" or transcriptional pool of genes that are expressed or found in a sample, e.g., nucleic acids that are expressed as RNA, mRNA and the like that may be subjected to RT and/or RT-PCR to made a complementary set of DNA replicons. Arrays may be produced using mechanical synthesis methods, light directed synthesis methods and the like that incorporate a combination of non-lithographic and/or photolithographic methods and solid phase synthesis methods.

Various techniques for the synthesis of these nucleic acid arrays have been described, e.g., fabricated on a surface of virtually any shape or even a multiplicity of surfaces. Arrays may be peptides or nucleic acids on beads, gels, polymeric surfaces, fibers such as fiber optics, glass or any other appropriate substrate. Arrays may be packaged in such a manner as to allow for diagnostics or other manipulation of an all inclusive device, see for example, U.S. Pat. No. 6,955,788, relevant portions incorporated herein by reference.

As used herein, the term "disease" refers to a physiological state of an organism with any abnormal biological state of a cell. Disease includes, but is not limited to, an interruption, cessation or disorder of cells, tissues, body functions, systems or organs that may be inherent, inherited, caused by an infection, caused by abnormal cell function, abnormal cell division and the like. A disease that leads to a "disease state" is generally detrimental to the biological system, that is, the host of the disease. With respect to the present invention, any biological state, such as an infection (e.g., viral, bacterial, fungal, helminthic, etc.), inflammation, autoinflammation, autoimmunity, anaphylaxis, allergies, premalignancy, malignancy, surgical, transplantation, physiological, and the like that is associated with a disease or disorder is considered to be a disease state. A pathological state is generally the equivalent of a disease state.

Disease states may also be categorized into different levels of disease state. As used herein, the level of a disease or disease state is an arbitrary measure reflecting the progression of a disease or disease state as well as the physiological response upon, during and after treatment. Generally, a disease or disease state will progress through levels or stages, wherein the affects of the disease become increasingly severe. The level of a disease state may be impacted by the physiological state of cells in the sample.

As used herein, the terms "therapy" or "therapeutic regimen" refer to those medical steps taken to alleviate or alter a disease state, e.g., a course of treatment intended to reduce or eliminate the affects or symptoms of a disease using pharmacological, surgical, dietary and/or other techniques. A therapeutic regimen may include a prescribed dosage of one or more drugs or surgery. Therapies will most often be beneficial and reduce the disease state but in many instances the effect of a therapy will have non-desirable or side-effects. The effect of therapy will also be impacted by the physiological state of the host, e.g., age, gender, genetics, weight, other disease conditions, etc.

As used herein, the term "pharmacological state" or "pharmacological status" refers to those samples that will be, are and/or were treated with one or more drugs, surgery and the like that may affect the pharmacological state of one or more nucleic acids in a sample, e.g., newly transcribed, stabilized and/or destabilized as a result of the pharmacological intervention. The pharmacological state of a sample relates to changes in the biological status before, during and/or after drug treatment and may serve a diagnostic or prognostic function, as taught herein. Some changes following drug treatment or surgery may be relevant to the disease state and/or may be unrelated side-effects of the therapy. Changes in the pharmacological state are the likely results of the duration of therapy, types and doses of drugs prescribed, degree of compliance with a given course of therapy, and/or un-prescribed drugs ingested.

As used herein, the term "biological state" refers to the state of the transcriptome (that is the entire collection of RNA transcripts) of the cellular sample isolated and purified for the analysis of changes in expression. The biological state reflects the physiological state of the cells in the sample by measuring the abundance and/or activity of cellular constituents, characterizing according to morphological phenotype or a combination of the methods for the detection of transcripts.

As used herein, the term "expression profile" refers to the relative abundance of RNA, DNA or protein abundances or activity levels. The expression profile can be a measurement for example of the transcriptional state or the translational state by any number of methods and using any of a number of gene-chips, gene arrays, beads, multiplex PCR, quantitiative PCR, run-on assays, Northern blot analysis, Western blot analysis, protein expression, fluorescence activated cell sorting (FACS), enzyme linked immunosorbent assays (ELISA), chemiluminescence studies, enzymatic assays, proliferation studies or any other method, apparatus and system for the determination and/or analysis of gene expression that are readily commercially available.

As used herein, the term "transcriptional state" of a sample includes the identities and relative abundances of the RNA species, especially mRNAs present in the sample. The entire transcriptional state of a sample, that is the combination of identity and abundance of RNA, is also referred to herein as the transcriptome. Generally, a substantial fraction of all the relative constituents of the entire set of RNA species in the sample are measured.

As used herein, the term "modular transcriptional vectors" refers to transcriptional expression data that reflects the "proportion of differentially expressed genes." For example, for each module the proportion of transcripts differentially expressed between at least two groups (e.g. healthy subjects vs patients). This vector is derived from the comparison of two groups of samples. The first analytical step is used for the selection of disease-specific sets of transcripts within each module. Next, there is the "expression level." The group comparison for a given disease provides the list of differentially expressed transcripts for each module. It was found that different diseases yield different subsets of modular transcripts. With this expression level it is then possible to calculate vectors for each module(s) for a single sample by averaging expression values of disease-specific subsets of genes identified as being differentially expressed. This approach permits the generation of maps of modular expression vectors for a single sample, e.g., those described in the module maps disclosed herein. These vector module maps represent an averaged expression level for each module (instead of a proportion of differentially expressed genes) that can be derived for each sample.

Using the present invention it is possible to identify and distinguish diseases not only at the module-level, but also at the gene-level; i.e., two diseases can have the same vector (identical proportion of differentially expressed transcripts, identical "polarity"), but the gene composition of the vector can still be disease-specific. Gene-level expression provides the distinct advantage of greatly increasing the resolution of the analysis.

Furthermore, the present invention takes advantage of composite transcriptional markers. As used herein, the term "composite transcriptional markers" refers to the average expression values of multiple genes (subsets of modules) as compared to using individual genes as markers (and the composition of these markers can be disease-specific). The composite transcriptional markers approach is unique because the user can develop multivariate microarray scores to assess disease severity in patients with, e.g., SLE, or to derive expression vectors disclosed herein. Most importantly, it has been found that using the composite modular transcriptional markers of the present invention the results found herein are reproducible across microarray platform, thereby providing greater reliability for regulatory approval.

Gene expression monitoring systems for use with the present invention may include customized gene arrays with a limited and/or basic number of genes that are specific and/or customized for the one or more target diseases. Unlike the general, pan-genome arrays that are in customary use, the present invention provides for not only the use of these general pan-arrays for retrospective gene and genome analysis without the need to use a specific platform, but more importantly, it provides for the development of customized arrays that provide an optimal gene set for analysis without the need for the thousands of other, non-relevant genes. One distinct advantage of the optimized arrays and modules of the present invention over the existing art is a reduction in the financial costs (e.g., cost per assay, materials, equipment, time, personnel, training, etc.), and more importantly, the environmental cost of manufacturing pan-arrays where the vast majority of the data is irrelevant. The modules of the present invention allow for the first time the design of simple, custom arrays that provide optimal data with the least number of probes while maximizing the signal to noise ratio. By eliminating the total number of genes for analysis, it is possible to, e.g., eliminate the need to manufacture thousands of expensive platinum masks for photolithography during the manufacture of pan-genetic chips that provide vast amounts of irrelevant data. Using the present invention it is possible to completely avoid the need for microarrays if the limited probe set(s) of the present invention are used with, e.g., digital optical chemistry arrays, ball bead arrays, beads (e.g., Luminex), multiplex PCR, quantitiative PCR, run-on assays, Northern blot analysis, or even, for protein analysis, e.g., Western blot analysis, 2-D and 3-D gel protein expression, MALDI, MALDI-TOF, fluorescence activated cell sorting (FACS) (cell surface or intracellular), enzyme linked immunosorbent assays (ELISA), chemiluminescence studies, enzymatic assays, proliferation studies or any other method, apparatus and system for the determination and/or analysis of gene expression that are readily commercially available.

The "molecular fingerprinting system" of the present invention may be used to facilitate and conduct a comparative analysis of expression in different cells or tissues, different subpopulations of the same cells or tissues, different physiological states of the same cells or tissue, different developmental stages of the same cells or tissue, or different cell populations of the same tissue against other diseases and/or normal cell controls. In some cases, the normal or wild-type expression data may be from samples analyzed at or about the same time or it may be expression data obtained or culled from existing gene array expression databases, e.g., public databases such as the NCBI Gene Expression Omnibus database.

As used herein, the term "differentially expressed" refers to the measurement of a cellular constituent (e.g., nucleic acid, protein, enzymatic activity and the like) that varies in two or more samples, e.g., between a disease sample and a normal sample. The cellular constituent may be on or off (present or absent), upregulated relative to a reference or downregulated relative to the reference. For use with gene-chips or gene-arrays, differential gene expression of nucleic acids, e.g., mRNA or other RNAs (miRNA, siRNA, hnRNA, rRNA, tRNA, etc.) may be used to distinguish between cell types or nucleic acids. Most commonly, the measurement of the transcriptional state of a cell is accomplished by quantitative reverse transcriptase (RT) and/or quantitative reverse transcriptase-polymerase chain reaction (RT-PCR), genomic expression analysis, post-translational analysis, modifications to genomic DNA, translocations, in situ hybridization and the like.

For some disease states it is possible to identify cellular or morphological differences, especially at early levels of the disease state. The present invention avoids the need to identify those specific mutations or one or more genes by looking at modules of genes of the cells themselves or, more importantly, of the cellular RNA expression of genes from immune effector cells that are acting within their regular physiologic context, that is, during immune activation, immune tolerance or even immune anergy. While a genetic mutation may result in a dramatic change in the expression levels of a group of genes, biological systems often compensate for changes by altering the expression of other genes. As a result of these internal compensation responses, many perturbations may have minimal effects on observable phenotypes of the system but profound effects to the composition of cellular constituents. Likewise, the actual copies of a gene transcript may not increase or decrease, however, the longevity or half-life of the transcript may be affected leading to greatly increases protein production. The present invention eliminates the need of detecting the actual message by, in one embodiment, looking at effector cells (e.g., leukocytes, lymphocytes and/or sub-populations thereof) rather than single messages and/or mutations.

The skilled artisan will appreciate readily that samples may be obtained from a variety of sources including, e.g., single cells, a collection of cells, tissue, cell culture and the like. In certain cases, it may even be possible to isolate sufficient RNA from cells found in, e.g., urine, blood, saliva, tissue or biopsy samples and the like. In certain circumstances, enough cells and/or RNA may be obtained from: mucosal secretion, feces, tears, blood plasma, peritoneal fluid, interstitial fluid, intradural, cerebrospinal fluid, sweat or other bodily fluids. The nucleic acid source, e.g., from tissue or cell sources, may include a tissue biopsy sample, one or more sorted cell populations, cell culture, cell clones, transformed cells, biopies or a single cell. The tissue source may include, e.g., brain, liver, heart, kidney, lung, spleen, retina, bone, neural, lymph node, endocrine gland, reproductive organ, blood, nerve, vascular tissue, and olfactory epithelium.

The present invention includes the following basic components, which may be used alone or in combination, namely, one or more data mining algorithms; one or more module-level analytical processes; the characterization of blood leukocyte transcriptional modules; the use of aggregated modular data in multivariate analyses for the molecular diagnostic/prognostic of human diseases; and/or visualization of module-level data and results. Using the present invention it is also possible to develop and analyze composite transcriptional markers, which may be further aggregated into a single multivariate score.

An explosion in data acquisition rates has spurred the development of mining tools and algorithms for the exploitation of microarray data and biomedical knowledge. Approaches aimed at uncovering the modular organization and function of transcriptional systems constitute promising methods for the identification of robust molecular signatures of disease¹⁴⁻¹⁶, ¹⁷. Indeed, such analyses can transform the perception of large scale transcriptional studies by taking the conceptualization of microarray data past the level of individual genes or lists of genes.

The present inventors have recognized that current microarray-based research is facing significant challenges with the analysis of data that are notoriously "noisy," that is, data that is difficult to interpret and does not compare well across laboratories and platforms. A widely accepted approach for the analysis of microarray data begins with the identification of subsets of genes differentially expressed between study groups. Next, the users try subsequently to "make sense" out of resulting gene lists using pattern discovery algorithms and existing scientific knowledge.

Rather than deal with the great variability across platforms, the present inventors have developed a strategy that emphasized the selection of biologically relevant genes at an early stage of the analysis. Briefly, the method includes the identification of the transcriptional components characterizing a given biological system for which an improved data mining algorithm was developed to analyze and extract groups of coordinately expressed genes, or transcriptional modules, from large collections of data.

In one example, twenty-eight transcriptional modules regrouping 4742 probe sets were obtained from 239 blood leukocyte transcriptional profiles. Functional convergence among genes forming these modules was demonstrated through literature profiling. The second step consisted of studying perturbations of transcriptional systems on a modular basis. To illustrate this concept, leukocyte transcriptional profiles obtained from healthy volunteers and patients were obtained, compared and analyzed. Further validation of this gene fingerprinting strategy was obtained through the analysis of a published microarray dataset. Remarkably, the modular transcriptional apparatus, system and methods of the present invention using pre-existing data showed a high degree of reproducibility across two commercial microarray platforms.

The present invention includes the implementation of a widely applicable, two-step microarray data mining strategy designed for the modular analysis of transcriptional systems. This novel approach was used to characterize transcriptional signatures of blood leukocytes, which constitutes the most accessible source of clinically relevant information.

As demonstrated herein, it is possible to determine, differential and/or distinguish between two disease based on two vectors even if the vector is identical (+/+) for two diseases - e.g. M1.3 = 53% down for both SLE and FLU because the composition of each vector can still be used to differentiate them. For example, even though the proportion and polarity of differentially expressed transcripts is identical between the two diseases for M1.3, the gene composition can still be disease-specific. The combination of gene-level and module-level analysis considerably increases resolution. Furthermore, it is possible to use 2, 3, 4, 5, 10, 15, 20, 25, 28 or more modules to differentiate diseases.

Material and methods. Processing of blood samples. All blood samples were collected in acid citrate dextrose tubes (BD Vacutainer) and immediately delivered at room temperature to the Baylor Institute for Immunology Research, Dallas, TX, for processing. Peripheral blood mononuclear cells (PBMCs) from 3-4 ml of blood were isolated *via* Ficoll gradient and immediately lysed in RLT reagent (Qiagen, Valencia, CA) with beta-mercaptoethanol (BME) and stored at -80°C prior to the RNA extraction step.

Microarray analysis. Total RNA was isolated using the RNeasy kit (Qiagen) according to the manufacturer's instructions and RNA integrity was assessed using an Agilent 2100 Bioanalyzer (Agilent, Palo Alto, CA).

Affymetrix GeneChips: These microarrays include short oligonucleotide probe sets synthesized *in situ* on a quartz wafer. Target labeling was performed according to the manufacturer's standard protocol (Affymetrix Inc., Santa Clara, CA). Biotinylated cRNA targets were purified and subsequently hybridized to Affymetrix HG-U133A and U133B GeneChips (>44,000 probe sets). Arrays were scanned using an Affymetrix confocal laser scanner. Microarray Suite, Version 5.0 (MAS 5.0; Affymetrix) software was used to assess fluorescent hybridization signals, to normalize signals, and to evaluate signal detection calls. Normalization of signal values per chip was achieved using the MAS 5.0 global method of scaling to the target intensity value of 500 per GeneChip. A gene expression analysis software program, GeneSpring, Version 7.1 (Agilent), was used to perform statistical analysis and hierarchical clustering.

Illumina BeadChips: These microarrays include 50mer oligonucleotide probes attached to 3 µm beads, which are lodged into microwells at the surface of a glass slide. Samples were processed and acquired by Illumina Inc. (San Diego, CA) on the basis of a service contract. Targets were prepared using the Illumina RNA amplification kit (Ambion, Austin, TX). cRNA targets were hybridized to Sentrix HumanRef8 BeadChips (>25,000 probes), which were scanned on an Illumina BeadStation 500. Illumina's Beadstudio software was used to assess fluorescent hybridization signals.

Literature profiling. The literature profiling algorithm employed in this study has been previously described in detail¹⁸. This approach links genes sharing similar keywords. It uses hierarchical clustering, a popular unsupervised pattern discovery algorithm, to analyze patterns of term occurrence in literature abstracts. Step 1: A gene:literature index identifying pertinent publications for each gene is created. Step 2: Term occurrence frequencies were computed by a text processor. Step 3: Stringent filter criteria are used to select relevant keywords (i.e., eliminate terms with either high or low frequency across all genes and retain the few discerning terms characterized by a pattern of high occurrence for only a few genes). Step 4: Two-way hierarchical clustering groups genes and relevant keywords based on occurrence patterns, providing a visual representation of functional relationships existing among a group of genes.

Modular data mining algorithm. First, one or more transcriptional components are identified that permit the characterization of biological systems beyond the level of single genes. Sets of coordinately regulated genes, or transcriptional modules, were extracted using a novel mining algorithm, which was applied to a large set of blood leukocyte microarray profiles (Figure 1). Gene expression profiles from a total of 239 peripheral blood mononuclear cells (PBMCs) samples were generated using Affymetrix U133A&B GeneChips (>44,000 probe sets). Transcriptional data were obtained for eight experimental groups (systemic juvenile idiopathic arthritis, systemic lupus erythematosus, type I diabetes, liver transplant recipients, melanoma patients, and patients with acute infections: *Escherichia coli, Staphylococcus aureus* and influenza A). For each group, transcripts with an absent flag call across all conditions were filtered out. The remaining genes were distributed among thirty sets by hierarchical clustering (clusters C1 through C30). The cluster assignment for each gene was recorded in a table and distribution patterns were compared among all the genes. Modules were selected using an iterative process, starting with the largest set of genes that belonged to the same cluster in all study groups (*i.e*. genes that were found in the same cluster in eight of the eight experimental groups). The selection was then expanded from this core reference pattern to include genes with 7/8, 6/8 and 5/8 matches. The resulting set of genes formed a transcriptional module and was withdrawn from the selection pool. The process was then repeated starting with the second largest group of genes, progressively reducing the level of stringency. This analysis led to the identification of 5348 transcripts that were distributed among twenty-eight modules (a complete list is provided as supplementary material). Each module is assigned a unique identifier indicating the round and order of selection (*i.e.* M3.1 was the first module identified in the third round of selection).

Modules display distinct "transcriptional behavior". It is widely assumed that co-expressed genes are functionally linked. This concept of "guilt by association" is particularly compelling in cases where genes follow complex expression patterns across many samples. The present inventors discovered that transcriptional modules form coherent biological units and, therefore, predicted that the co-expression properties identified in our initial dataset would be conserved in an independent set of samples. Data were obtained for PBMCs isolated from the blood of twenty-one healthy volunteers. These samples were not used in the module selection process described above.

Figure 2 shows gene expression profiles of four different modules are shown (Figure 2: M1.2, M1.7, M2.11 and M2.1). In the graphs of Figure 2, each line represents the expression level (y-axis) of a single gene across multiple samples (21 samples on the x-axis). Differences in gene expression in this example represent inter-individual variation between "healthy" individuals. It was found that within each module genes display a coherent "transcriptional behavior". Indeed, the variation in gene expression appeared to be consistent across all the samples (for some samples the expression of all the genes was elevated and formed a peak, while in others levels were low for all the genes which formed a dip). Importantly, inter-individual variations appeared to be module-specific as peaks and dips formed for different samples in M1.2, M2.11 and M2.1. Furthermore, the amplitude of variation was also characteristic of each module, with levels of expression being more variable for M1.2 and M2.11 than M2.1 and especially M1.7. Thus, we find that transcriptional modules constitute independent biological variables.

Functional characterization of transcriptional modules. Next, the modules were characterized at a functional level. A text mining approach was employed to extract keywords from the biomedical literature collected for each gene (described in ¹⁸). The distribution of keywords associated to the four modules that were analyzed is clearly distinct (Figure 3). The following is a list of keywords that may be associated with certain modules.

Keywords highly specific for M1.2 included Platelet, Aggregation or Thrombosis, and were associated with genes such as ITGA2B (Integrin alpha 2b, platelet glycoprotein IIb), PF4 (platelet factor 4), SELP (Selectin P) and GP6 (platelet glycoprotein 6).

Keywords highly specific for M1.3 included B-cell, Immunoglobulin or IgG and were associated with genes such as CD19, CD22, CD72A, BLNK (B cell linker protein), BLK (B lymphoid tyrosine kinase) and PAX5 (paired box gene 5, a B-cell lineage specific activator).

Keywords highly specific for M1.5 included Monocyte, Dendritic, CD14 or Toll-like and were associated with genes such as MYD88 (myeloid differentiation primary response gene 88), CD86, TLR2 (Toll-like receptor 2), LILRB2 (leukocyte immunoglobulin-like receptor B2) and CD163. Keywords highly specific for M3.1 included Interferon, IFN-alpha, Antiviral, or ISRE and were associated with genes such as STAT1 (signal transducer and activator of transcription 1), CXCL10 (CXC chemokine ligand 10, IP-10), OAS2 (oligoadenylate synthetase 2) and MX2 (myxovirus resistance 2).

This contrasted pattern of term occurrence denotes the remarkable functional coherence of each module. Information extracted from the literature for all the modules that have been identified permit a comprehensive functional characterization of the PBMC system at a transcriptional level. A description of functional associations identified for each of the twenty-eight sample PBMC transcriptional modules is provided in Table 2.

**Table 2: Complete Functional assessment of 28 transcriptional modules**

| **Module I.D.** | **Number of probe sets** | **Keyword selection** | **Assessment** |
|---|---|---|---|
| M 1.1 | 69 | Ig, Immunoglobulin, Bone, Marrow, PreB, IgM,Mu. | **Plasma cells.** Includes genes encoding for Immunoglobulin chains (*e.g*. IGHM, IGJ, IGLL1, IGKC, IGHD) and the plasma cell marker CD38. |
| M 1.2 | 96 | Platelet, Adhesion, Aggregation, Endothelial, Vascular | **Platelets.** Includes genes encoding for platelet glycoproteins (ITGA2B, ITGB3, GP6, GP1A/B), and platelet-derived immune mediators such as PPPB (pro-platelet basic protein) and PF4 (platelet factor 4). |
| M 1.3 | 47 | Immunoreceptor, BCR, B-cell, IgG | **B-cells.** Includes genes encoding for B-cell surface markers (CD72, CD79A/B, CD19, CD22) and other B-cell associated molecules: Early B-cell factor (EBF), B-cell linker (BLNK) and B lymphoid tyrosine kinase (BLK). |
| M 1.4 | 87 | Replication, Repression, Repair, CREB, Lymphoid, TNF-alpha | **Undetermined.** This set includes regulators and targets of cAMP signaling pathway (JUND, ATF4, CREM, PDE4, NR4A2, VIL2), as well as repressors of TNF-alpha mediated NF-KB activation (CYLD, ASK, TNFAIP3). |
| M 1.5 | 130 | Monocytes, Dendritic, MHC, Costimulatory, TLR4, MYD88 | **Myeloid lineage.** Includes molecules expressed by cells of the myeloid lineage (CD86, CD163, FCGR2A), some of which being involved in pathogen recognition (CD14, TLR2, MYD88). This set also includes TNF family members (TNFR2, BAFF). |
| M 1.6 | 28 | Zinc, Finger, P53, RAS | **Undetermined.** This set includes genes encoding for signaling molecules, e.g. the zinc finger containing inhibitor of activated STAT (PIAS 1 and PIAS2), or the nuclear factor of activated T-cells NFATC3. |
| M 1.7 | 127 | Ribosome, Translational, 40S, 60S, HLA | **MHC/Ribosomal proteins.** Almost exclusively formed by genes encoding MHC class I molecules (HLA-A,B,C,G,E)+ Beta 2-microglobulin (B2M) or Ribosomal proteins (RPLs, RPSs). |
| M 1.8 | 86 | Metabolism, Biosynthesis, Replication, Helicase | **Undetermined.** Includes genes encoding metabolic enzymes (GLS, NSF1, NAT1) and factors involved in DNA replication (PURA, TERF2, EIF2S1). |
| M 2.1 | 72 | NK, Killer, Cytolytic, CD8, Cell-mediated, T-cell, CTL, IFN-g | **Cytotoxic cells.** Includes cytotoxic T-cells amd NK-cells surface markers (CD8A, CD2, CD160, NKG7, KLRs), cytolytic molecules (granzyme, perforin, granulysin), chemokines (CCL5, XCL1) and CTL/NK-cell associated molecules (CTSW). |
| M 2.2 | 44 | Granulocytes, Neutrophils, Defense, Myeloid, Marrow | **Neutrophils.** This set includes innate molecules that are found in neutrophil granules (Lactotransferrin: LTF, defensin: DEAF1, Bacterial Permeability Increasing protein: BPI, Cathelicidin antimicrobial protein: CAMP...). |
| M 2.3 | 94 | Erythrocytes, Red, Anemia, Globin, Hemoglobin | **Erythrocytes.** Includes hemoglobin genes (HGBs) and other erythrocyte-associated genes (erythrocytic alkirin:ANK1, Glycophorin C: GYPC, hydroxymethylbilane synthase: HMBS, erythroid associated factor: ERAF). |
| M 2.4 | 118 | Ribonucleoprotein, 60S, nucleolus, Assembly, Elongation | **Ribosomal proteins.** Including genes encoding ribosomal proteins (RPLs, RPSs), Eukaryotic Translation Elongation factor family members (EEFs) and Nucleolar proteins (NPM1, NOAL2, NAP1L1). |
| M 2.5 | 242 | Adenoma, Interstitial, Mesenchyme, Dendrite, Motor | **Undetermined.** This module includes genes encoding immune-related (CD40, CD80, CXCL12, IFNA5, IL4R) as well as cytoskeleton-related molecules (Myosin, Dedicator of Cytokenesis, Syndecan 2, Plexin C1, Distrobrevin). |
| M 2.6 | 110 | Granulocytes, Monocytes, Myeloid, ERK, Necrosis | **Myeloid lineage.** Related to M 1.5. Includes genes expressed in myeloid lineage cells (IGTB2/CD 18, Lymphotoxin beta receptor, Myeloid related proteins 8/14 Formyl peptide receptor 1), such as Monocytes and Neutrophils. |
| M 2.7 | 43 | No keywords extracted. | **Undetermined.** This module is largely composed of transcripts with no known function. Only 20 genes associated with literature, including a member of the chemokine-like factor superfamily (CKLFSF8). |
| M 2.8 | 104 | Lymphoma, T-cell, CD4, CD8, TCR, Thymus, Lymphoid, IL2 | **T-cells.** Includes T-cell surface markers (CD5, CD6, CD7, CD26, CD28, CD96) and molecules expressed by lymphoid lineage cells (lymphotoxin beta, IL2-inducible T-cell kinase, TCF7, T-cell differentiation protein mal, GATA3, STAT5B). |
| M 2.9 | 122 | ERK, Transactivation, Cytoskeletal, MAPK, JNK | **Undetermined.** Includes genes encoding molecules that associate to the cytoskeleton (Actin related protein 2/3, MAPK1, MAP3K1, RAB5A). Also present are T-cell expressed genes (FAS, ITGA4/CD49D, ZNF1A1). |
| M2.10 | 44 | Myeloid, Macrophage, Dendritic, Inflammatory, Interleukin | **Undetermined.** Includes genes encoding for Immune-related cell surface molecules (CD36, CD86, LILRB), cytokines (IL15) and molecules involved in signaling pathways (FYB, TICAM2-Toll-like receptor pathway). |
| M 2.11 | 77 | Replication, Repress, RAS, Autophosphorylation , Oncogenic | **Undetermined.** Includes kinases (UHMK1, CSNK1G1, CDK6, WNK1, TAOK1, CALM2, PRKCI, ITPKB, SRPK2, STK17B, DYRK2, PIK3R1, STK4, CLK4, PKN2) and RAS family members (G3BP, RAB14, RASA2, RAP2A, KRAS). |
| M 3.1 | 80 | ISRE, Influenza, Antiviral, IFN-gamma, IFN-alpha, Interferon | **Interferon-inducible.** This set includes interferon-inducible genes: antiviral molecules (OAS1/2/3/L, GBP1, G1P2, EIF2AK2/PKR, MX1, PML), chemokines (CXCL10/IP-10), signaling molecules (STAT1, STAt2, IRF7, ISGF3G). |
| M 3.2 | 230 | TGF-beta, TNF, Inflammatory, Apoptotic, Lipopolysaccharide | **Inflammation I.** Includes genes encoding molecules involved in inflammatory processes (e.g. IL8, ICAM1, C5R1, CD44, PLAUR, IL1A, CXCL16), and regulators of apoptosis (MCL1, FOXO3A, RARA, BCL3/6/2A1, GADD45B). |
| M 3.3 | 230 | Granulocyte, Inflammatory, Defense, Oxidize, Lysosomal | **Inflammation II.** Includes molecules inducing or inducible by Granulocyte-Macrophage CSF (SPI1, IL18, ALOX5, ANPEP), as well as lysosomal enzymes (PPT1, CTSB/S, CES1, NEU1, ASAH1, LAMP2, CAST). |
| M 3.4 | 323 | No keyword extracted | **Undetermined.** Includes protein phosphates (PPP1R12A, PTPRC, PPP1CB, PPM1B) and phosphoinositide 3-kinase (PI3K) family members (PIK3CA, PIK32A, PIP5K3). |
| M 3.5 | 19 | No keyword extracted | **Undetermined.** Composed of only a small number of transcripts. Includes hemoglobin genes (HBA1, HBA2, HBB). |
| M 3.6 | 233 | Complement, Host, Oxidative, Cytoskeletal, T-cell | **Undetermined.** This very large set includes T-cell surface markers (CD101, CD102, CD103) as well as molecules ubiquitously expressed among blood leukocytes (CXRCR1: fraktalkine receptor, CD47, P-selectin ligand). |
| M 3.7 | 80 | Spliceosome, Methylation, Ubiquitin, Beta-catenin | **Undetermined.** Includes genes encoding proteasome subunits (PSMA2/5, PSMB5/8); ubiquitin protein ligases HIP2, STUB1, as well as components of ubiqutin ligase complexes (SUGT1). |
| M 3.8 | 182 | CDC, TCR, CREB, Glycosylase | **Undetermined.** Includes genes encoding for several enzymes: aminomethyltransferase, arginyltransferase, asparagines synthetase, diacylglycerol kinase, inositol phosphatases, methyltransferases, helicases... |
| M 3.9 | 261 | Chromatin, Checkpoint, Replication, Transactivation | **Undetermined.** Includes genes encoding for protein kinases (PRKPIR, PRKDC, PRKCI) and phosphatases (e.g. PTPLB, PPP1R8/2CB). Also includes RAS oncogene family members and the NK cell receptor 2B4 (CD244). |

Module-based microarray data mining strategy. Results from "traditional" microarray analyses are notoriously noisy and difficult to interpret. A widely accepted approach for microarray data analyses includes three basic steps: 1) Use of a statistical test to select genes differentially expressed between study groups; 2) Apply pattern discovery algorithms to identify signatures among the resulting gene lists; and 3) Interpret the data using knowledge derived from the literature or ontology databases.

The present invention uses a novel microarray data mining strategy emphasizing the selection of biologically relevant transcripts at an early stage of the analysis. This first step can be carried out using for instance the modular mining algorithm described above in combination with a functional mining tool used for in-depth characterization of each transcriptional module (Figure 4: top panel, Step 1). The analysis does not take into consideration differences in gene expression levels between groups. Rather, the present invention focuses instead on complex gene expression patterns that arise due to biological variations (e.g. inter-individual variations among a patient population). After defining the transcriptional components associated to a given biological system the second step of the analysis includes the analysis of changes in gene expression through the comparison of different study groups (Figure 4: bottom panel, Step 2). Group comparison analyses are carried out independently for each module. Changes at the module level are expressed as the proportion of genes that meet the significance criteria (represented by a pie chart in Figure 5 or a spot in Figure 6). Notably, carrying out comparisons at the modular level permits to avoid the noise generated when thousands of tests are performed on "random" collections of genes.

Perturbation of modular PBMC transcriptional profiles in human diseases. To illustrate the second step of the microarray data mining strategy described above (Figure 4), gene expression data for PBMC samples obtained from two pediatric patient populations composed of eighteen children with systemic lupus erythematosus (SLE) and sixteen children with acute influenza A infection was obtained, compared and analyzed. Each patient cohort was matched to its respective control group (healthy volunteers: eleven and ten donors were matched to the SLE and influenza groups, respectively). Following the analytical scheme depicted in Figure 4, a statistical group comparisons between patient and healthy groups for each individual module and measured the proportion of genes significantly changed in each module (Figure 5) was performed. The statistical group comparison approach allows the user to focus the analysis on well defined groups of genes that contain minimal amounts of noise and carry identifiable biological meaning. A key to the graphical representation of these results is provided in Figure 4.

The following findings were made: (1) that a large proportion of genes in M3.1 ("interferon-associated") met the significance level in both Flu and SLE groups (84% and 94%, respectively). This observation confirms earlier work with SLE patients ¹⁹ and identifies the presence of an interferon signature in patients with acute influenza infection. (2) Equivalent proportions of genes in M1.3 ("B-cell-associated") were significantly changed in both groups (53%), with over 50% overlap between the two lists. This time, genes were consistently under-expressed in patient compared to healthy groups. (3) Modules were also found that differentiate the two diseases. The proportion of genes significantly changed in Module 1.1 reaches 39% in SLE patients and is only 7% in Flu patients, which at a significance level of 0.05 is very close to the proportion of genes that would be expected to be differentially expressed only by chance. Interestingly, this module is almost exclusively composed of genes encoding immunoglobin chains and has been associated with plasma cells. However, this module is clearly distinct from the B-cell associated module (M1.3), both in terms of gene expression level and pattern (not shown). (4) As illustrated by module M1.5, gene-level analysis of individual modules can be used to further discriminate the two diseases. It is also the case for M1.3, where, despite the absence of differences at the module-level (Figure 4: 53% under-expressed transcripts), differences between Flu and SLE groups could be identified at the gene-level (only 51% of the under-expressed transcripts in M1.3 were common to the two disease groups). These examples illustrate the use of a modular framework to streamline the analysis and interpretation of microarray results.

Mapping changes in gene expression at the modular level. Data visualization is paramount for the interpretation of complex datasets and we sought to provide a comprehensive graphical illustration of changes that occur at the modular level. Changes in gene expression levels caused by different diseases were represented for the twenty-eight PBMC transcriptional modules (Figure 6). Each disease group is compared to its respective control group composed of healthy donors who were matched for age and sex (eighteen patients with SLE, sixteen with acute influenza infection, sixteen with metastatic melanoma and sixteen liver transplant recipients receiving immunosuppressive drug treatment were compared to control groups composed of ten to eleven healthy subjects). Module-level data were represented graphically by spots aligned on a grid, with each position corresponding to a different module (See Table 1 for functional annotations on each of the modules).

The spot intensity indicates the proportion of genes significantly changed for each module. The spot color indicates the polarity of the change (red: proportion of over-expressed genes, blue: proportion of under-expressed genes; modules containing a significant proportion of both over- and under-expressed genes would be purple-though none were observed). This representation permits a rapid assessment of perturbations of the PBMC transcriptional system. Such "module maps" were generated for each disease. When comparing the four maps, we found that diseases were characterized by a unique modular combination. Indeed, results for M1.1 and M1.2 alone sufficed to distinguish all four diseases (M1.1/M1.2: SLE = +/+; FLU=0/0; Melanoma=-/+; transplant=-/-). A number of genes in M3.2 ("inflammation") were over-expressed in all diseases (particularly so in the transplant group), while genes in M3.1 (interferon) were over-expressed in patients with SLE, influenza infection and, to some extent, transplant recipients. "Ribosomal protein" module genes (M1.7 and M2.4) were under-expressed in both SLE and Flu groups. The level of expression of these genes was recently found to be inversely correlated to disease activity in SLE patients (Bennett et al., submitted). M2.8 includes T-cell transcripts which are under-expressed in lymphopenic SLE patients and transplant recipients treated with immunosuppressive drugs targeting T-cells.

Interestingly, differentially expressed genes in each module were predominantly either under-expressed or over-expressed (Figure 5 and Figure 6). Yet, modules were purely selected on the basis of similarities in gene expression profiles, not changes in expression levels between groups. The fact that changes in gene expression appear highly polarized within each module denotes the functional relevance of modular data. Thus, the present invention enables disease fingerprinting by a modular analysis of patient blood leukocyte transcriptional profiles.

Validation of PBMC modules in a published dataset. Next, the validity of the PBMC transcriptional modules described above in a "third-party" dataset was tested. The study from Connolly, *et al.,* who investigated the effects of exercise on gene expression in human PBMCs²⁰ was tested.

Briefly, samples were obtained from fifteen healthy men prior to and immediately after performing thirty minutes of constant work rate cycle ergometry and one hour after the end of the exercise. Transcriptional profiles were generated for five RNA pools of three subjects each, using Affymetrix U133A gene chips. Raw expression data was downloaded from the NCBI Gene Expression Omnibus website ²¹ and analyzed changes in gene expression on a module-by-module basis. Figure 7 shows transcriptional profiles of modules M1.1 ("plasma cells"), M1.7 ("ribosomal proteins") and M2.1 ("cytotoxic cells"). Gene transcriptional behavior for each of these modules was clearly distinct. Interestingly, differences were found between subject pools (M1.1), experimental conditions (M2.1), or no differences (M1.7). These data clearly indicate an increase in expression of cytotoxic cell associated genes (M2.1) immediately after exercise, followed by a decrease to levels comparable to baseline after recuperation. This finding is consistent with the elevation in circulating natural killer cells observed after exercise in sedentary subjects^{22,23}. Some of the genes included in M2.1 were listed by Connolly *et al.* under the category "inflammatory response", but the author did not make the link with a possible change in cellular composition. Very few genes belonging to "inflammatory" modules (M3.2, M3.3) were found to be changed after exercise, despite the fact that levels of expression of the genes composing these modules are increased in a wide range of diseases (Chaussabel *et al.*, submitted). Interestingly, however, immunosuppressive molecules specifically over-expressed in patients with stage IV melanoma and transplant patients (Chaussabel *et al.*, submitted) were found to be transiently increased after exercise (not shown, M1.4; *e.g.* TCF8, CREM, RGS 1, TNFAIP3).

Taken together the results from this analysis demonstrate the validity of the proposed modular mining strategy in the context of data generated by an independent group of investigators. Using the present invention, it was found that modular transcriptional data are reproducible across microarray platforms.

First, modular transcriptional profiles obtained using two commercial microarray platforms were compared. PBMCs were isolated from fourteen samples donated by four healthy volunteers and ten liver transplant recipients. Starting from the same source of total RNA, targets were generated independently and analyzed using Affymetrix U133 GeneChips (at the Baylor Institute for Immunology Research) and Illumina Human Ref8 BeadChips (at the Illumina service core). Fundamental differences exist between the two microarray technologies (see Methods for details). Probe IDs provided by each manufacturer were converted into a unique ID (NCBI Entrez gene ID) that was used for matching gene expression profiles. Data obtained for shared sets of genes are shown in Figure 8 for modules M1.2 ("platelets"), M3.1 ("interferon") and M3.2 ("inflammation"). Profiles derived from data obtained with Illumina beadchips show a very high level of co-expression among genes within each module. This observation is particularly meaningful since the selection of transcriptional modules was exclusively based on gene expression data generated using Affymetrix GeneChips. Furthermore, averaged gene expression values for each module were highly reproducible across microarray platforms (Figure 8).

These results demonstrate the robustness of modular transcriptional signatures and clearly indicate that module-level analysis has the potential to address concerns regarding the reproducibility of microarray data generated at different locations and with different platforms.

Microarray gene expression data produce a comprehensive, but disorganized view of biological systems. Challenges faced by microarray-based research are threefold: (1) Noise, (2) data interpretation and (3) reproducibility. As regards noise, the present invention successfully compared tens of thousands of genes, which the prior art methods invariably produce results that include a large proportion of noise²⁴. As regards data interpretation, the present invention overcomes the problem of information overload. Indeed, interpreting microarray data often requires investigators to examine experimental data in the context of existing biomedical knowledge, on a genome-wide scale¹³. More unsettling is the possibility of generating spurious results through the over-interpretation of noisy data⁷. Finally, as regards reproducibility it is well documented that a key problem with existing technology is the poor reproducibility of microarray results obtained by different laboratories and across platforms has been disconcerting and remains, to this date, a major concern ^{6,7,10-12}.

Mainstream microarray analysis strategies have had limited success in addressing this triad of issues, for several reasons. First of all, because statistical tests are considered as the prerequisite initial step of the analysis. As a consequence, biological considerations come into play only once a list of differentially expressed genes has been generated. Data subsets resulting from the testing of tens of thousands of variables will, however, invariably contain noise and are, therefore, particularly difficult to interpret. The system and method of the present invention takes the cellular and molecular biology of the cells into consideration when determining the features of the modules. In the present invention the first step is to take into account the biology of the system in the very first step of the analysis, thereby selecting sets of functionally-linked genes found to be coordinately expressed across hundreds of samples. Statistical testing is then applied to modular datasets which are considerably enriched in biologically meaningful genes. An additional benefit of this approach is that it transcends gene level analysis by using transcriptional modules as elementary units. Transcriptional modules constitute a framework for the analysis of perturbations that occur in the context of a defined biological system. This modular data format helps streamline the interpretation of microarray studies. It requires, however, the preliminary characterization of each experimental system under a broad range of biological variables, e.g., different experimental conditions, inter-individual variations, and cost or access to biological material can be a limitation.

Interestingly, the data derived from module-level analyses proved to be particularly robust, as indicated by the excellent reproducibility obtained across two commercial microarray platforms. Furthermore, multivariate analysis of PBMC transcriptional modules led to the establishment of a "genomic score," which provided an accurate assessment of disease severity in patients with systemic lupus erythematosus (Bennett, et al., submitted). The identification of reliable blood leukocyte transcriptional markers constitutes an important step towards the application of microarrays in clinical settings.

Working with samples formed by multiple cell types adds a level of complexity to the analysis of microarray gene expression data. Indeed, differences of gene expression levels can be explained not only by changes in transcriptional activity but also changes in cellular composition. Modular signatures obtained analyzing PBMC samples reflect this fact and permit us to distinguish cellular components (including genes associated to platelets - M1.2 -, erythrocytes - M2.3 or T-cells - M2.8) from components related to activation (including genes associated to interferon - M3.1, inflammation M3.2, or signaling - M2.11). This type of consideration is relevant to patient-based research, as the bulk of microarray analyses performed in this context involve multicellular samples.

The modular expression data generated by Affymetrix and Illumina platforms were highly comparable (Figure 9; transplant group Pearson correlation coefficient R² = 0.83, 0.98 and 0.93, for M1.2, M3.1 and M3.2 respectively; p<0.0001). Taken together, these results demonstrate that modular transcriptional data can be reproduced across microarray platforms. This finding is of importance because it indicates that the "modular microarray scores" can be used to assess disease severity in patients derived independently of the microarray platform being used.

The module-level mining strategy described in this work may be used with a broad range of biological systems, and is particularly well suited for the analysis of other clinically relevant samples, such as tumors or solid organ biopsies.

Expression level vectors may be obtained from one or more of the modules and/or one or more of the genes provided in Table 3. Furthermore, depending on the disease expression profile and using the methods of the present invention it is possible to develop and further refine the modules and genes within the modules, as will be apparent to the skilled artisan based on the present invention. For example, depending on the level of specificity required, the number of data set, the number of patients, and the like, one or more new of different module that includes a different proportion of differentially expressed genes within the context of a given disease may be used to develop new modules based on the new data to form and organize arrays based on the new subset of transcripts, which define new vectors that represent an average expression level.

Tables 1, 2 and 3 are LENGTHY TABLES. The patent application contains a lengthy table section. A copy of the table is available in electronic form from the USPTO web site. An electronic copy of the table will also be available from the USPTO upon request and payment of the fee set forth in 37 CFR 1.19(b)(3), which is attached to this EFS filing and Tables 1, 2 and 3.

It will be understood that particular embodiments described herein are shown by way of illustration and not as limitations of the invention. The principal features of this invention can be employed in various embodiments without departing from the scope of the invention. Those skilled in the art will recognize, or be able to ascertain using no more than routine experimentation, numerous equivalents to the specific procedures described herein.

All publications and patent applications mentioned in the specification are indicative of the level of skill of those skilled in the art to which this invention pertains.

In the claims, all transitional phrases such as "comprising," "including, " "carrying," "having," "containing," "involving," and the like are to be understood to be open-ended, i.e., to mean including but not limited to. Only the transitional phrases "consisting of" and "consisting essentially of," respectively, shall be closed or semi-closed transitional phrases.

Preferably the present invention is defined as follows:
1. A method for diagnosing a disease or condition comprising the steps of:
obtaining a transcriptome from a patient;
analyzing the transcriptome based on one or more transcriptional modules that are indicative of a disease or condition; and
determining the patient's disease or condition based on the presence, absence or level of expression of genes within one or more transcriptional modules of the transcriptome.
2. The method of definition 1, wherein the transcriptional modules is obtained by:
iteratively selecting gene expression values for one or more transcriptional modules by:
   selecting for the module the genes from each cluster that match in every disease or condition;
   removing the selected genes from the analysis; and
   repeating the process of gene expression value selection for genes that cluster in a sub-fraction of the diseases or conditions; and
iteratively repeating the generation of modules for each clusters until all gene clusters are exhausted.
3. The method of definition 1, wherein the clusters are selected from expression value clusters, keyword clusters, metabolic clusters, disease clusters, infection clusters, transplantation clusters, signaling clusters, transcriptional clusters, replication clusters, cell-cycle clusters, siRNA clusters, miRNA clusters, mitochondrial clusters, T cell clusters, B cell clusters, cytokine clusters, lymphokine clusters, heat shock clusters and combinations thereof.
4. The method of definition 1, wherein the one or more diseases or conditions are selected from one or more of the following conditions: systemic juvenile idiopathic arthritis, systemic lupus erythematosus, type I diabetes, liver transplant recipients, melanoma patients, and patients bacterial infections such as Escherichia coli, Staphylococcus aureus, viral infections such as influenza A, and combinations thereof.
5. The method of definition 1, wherein the one or more diseases or conditions are selected infections with a bioterror agent.
6. The method of definition 1, wherein the cells comprise peripheral blood mononuclear cells (PBMCs), blood cells, fetal cells, peritoneal cells, solid organ biopsies, resected tumors, primary cells, cells lines, cell clones and combinations thereof.
7. The method of definition 1, wherein the cells comprise single cells, a collection of cells, tissue, cell culture, urine and blood.
8. The method of definition 1, wherein the cells comprise a tissue biopsy, one or more sorted cell populations, cell culture, cell clones, transformed cells, biopies or a single cell.
9. The method of definition 1, wherein the cells comprise brain, liver, heart, kidney, lung, spleen, retina, bone, neural, lymph node, endocrine gland, reproductive organ, blood, nerve, vascular tissue, and olfactory epithelium cells.
10. The method of definition 1, wherein the step of obtaining individual gene expression levels is performed using a probe array, PCR, quantitative PCR, bead-based assays and combinations thereof.
11. The method of definition 1, wherein the step of obtaining individual gene expression levels is performed using hybridization of nucleic acids on a solid support.
12. The method of definition 1, wherein the step of obtaining individual gene expression levels is performed using cDNA from mRNA collected from the cells as a template.
13. The method of definition 1, wherein the modules can distinguish between an autoimmune disease, a viral infection a bacterial infection, cancer and transplant rejection.
14. A method for identifying transcriptional modules comprising the steps of:
obtaining individual gene expression levels from cells obtained from one or more patients with a disease or condition;
recording the expression value for each gene in a table that is divided into clusters;
iteratively selecting gene expression values for one or more transcriptional modules by:
   selecting for the module the genes from each cluster that match in every disease or condition;
   removing the selected genes from the analysis; and
   repeating the process of gene expression value selection for genes that cluster in a sub-fraction of the diseases or conditions; and
iteratively repeating the generation of modules for each clusters until all gene clusters are exhausted.
15. The method of definition 14, wherein the clusters are selected from expression value clusters, keyword clusters, metabolic clusters, disease clusters, infection clusters, transplantation clusters, signaling clusters, transcriptional clusters, replication clusters, cell-cycle clusters, siRNA clusters, miRNA clusters, mitochondrial clusters, T cell clusters, B cell clusters, cytokine clusters, lymphokine clusters, heat shock clusters and combinations thereof.
16. The method of definition 14, wherein the one or more diseases or conditions are selected from one or more of the following conditions: systemic juvenile idiopathic arthritis, systemic lupus erythematosus, type I diabetes, liver transplant recipients, melanoma patients, and patients bacterial infections such as Escherichia coli, Staphylococcus aureus, viral infections such as influenza A, and combinations thereof.
17. The method of definition 14, wherein the one or more diseases or conditions are selected infections with a bioterror agent.
18. The method of definition 14, wherein the cells comprise peripheral blood mononuclear cells (PBMCs), blood cells, fetal cells, peritoneal cells, solid organ biopsies, resected tumors, primary cells, cells lines, cell clones and combinations thereof.
19. The method of definition 14, wherein the cells comprise single cells, a collection of cells, tissue, cell culture, urine and blood.
20. The method of definition 14, wherein the cells comprise a tissue biopsy, one or more sorted cell populations, cell culture, cell clones, transformed cells, biopies or a single cell.
21. The method of definition 14, wherein the cells comprise brain, liver, heart, kidney, lung, spleen, retina, bone, neural, lymph node, endocrine gland, reproductive organ, blood, nerve, vascular tissue, and olfactory epithelium cells.
22. The method of definition 14, wherein the step of obtaining individual gene expression levels is performed using an array of oligonucleotides.
23. The method of definition 14, wherein the step of obtaining individual gene expression levels is performed using hybridization of nucleic acids on a solid support.
24. The method of definition 14, wherein the step of obtaining individual gene expression levels is performed using cDNA from mRNA collected from the cells as a template.
25. The method of definition 14, wherein the one or more transcriptional modules are selected from:

| Transcriptional modules |
|---|
| **Plasma cells:** genes encoding for Immunoglobulin chains (IGHM, IGJ, IGLL1, IGKC, IGHD) and the plasma cell marker CD38.; |
| **Platelets:** genes encoding for platelet glycoproteins (ITGA2B, ITGB3, GP6, GP1A/B), and platelet-derived immune mediators such as PPPB (pro-platelet basic protein) and PF4 (platelet factor 4); |
| **B-cells:** genes encoding for B-cell surface markers (CD72, CD79A/B, CD19, CD22) and other B-cell associated molecules: Early B-cell factor (EBF), B-cell linker (BLNK) and B lymphoid tyrosine kinase (BLK); |
| genes encoding regulators and targets of cAMP signaling pathway (JUND, ATF4, CREM, PDE4, NR4A2, VIL2), as well as repressors of TNF-alpha mediated NF-KB activation (CYLD, ASK, TNFAIP3); |
| **Myeloid lineage:** genes encoding molecules expressed by cells of the myeloid lineage (CD86, CD 163, FCGR2A), some of which being involved in pathogen recognition (CD14, TLR2, MYD88). This set also includes TNF family members (TNFR2, BAFF); |
| genes encoding for signaling molecules, the zinc finger containing inhibitor of activated STAT (PIAS1 and PIAS2), or the nuclear factor of activated T-cells NFATC3; |
| **MHC/Ribosomal proteins:** genes encoding MHC class I molecules (HLA-A,B,C,G,E)+ Beta 2-microglobulin (B2M) or Ribosomal proteins (RPLs, RPSs); |
| genes encoding metabolic enzymes (GLS, NSF1, NAT1) and factors involved in DNA replication (PURA, TERF2, EIF2S1); |
| **Cytotoxic cells:** genes encoding Cytotoxic T-cells amd NK-cells surface markers (CD8A, CD2, CD160, NKG7, KLRs), cytolytic molecules (granzyme, perforin, granulysin), chemokines (CCL5, XCL1) and CTL/NK-cell associated molecules (CTSW); |
| **Neutrophils:** genes encoding innate molecules that are found in neutrophil granules (Lactotransferrin: LTF, defensin: DEAF1, Bacterial Permeability Increasing protein: BPI, Cathelicidin antimicrobial protein: CAMP...); |
| **Erythrocytes:** genes encoding hemoglobin genes (HGBs) and other erythrocyte-associated genes (erythrocytic alkirin:ANK1, Glycophorin C: GYPC, hydroxymethylbilane synthase: HMBS, erythroid associated factor: ERAF); |
| **Ribosomal proteins:** genes encoding ribosomal proteins (RPLs, RPSs), Eukaryotic Translation Elongation factor family members (EEFs) and Nucleolar proteins (NPM1, NOAL2, NAP1L1); |
| genes encoding immune-related (CD40, CD80, CXCL12, IFNA5, IL4R) as well as cytoskeleton-related molecules (Myosin, Dedicator of Cytokenesis, Syndecan 2, Plexin C1, Distrobrevin); |
| **Myeloid lineage:** Related to M 1.5. Includes genes expressed in myeloid lineage cells (IGTB2/CD18, Lymphotoxin beta receptor, Myeloid related proteins 8/14 Formyl peptide receptor 1), such as Monocytes and Neutrophils; |
| genes encoding chemokine-like factor superfamily (CKLFSF8); |
| **T-cells:** genes encoding T-cell surface markers (CD5, CD6, CD7, CD26, CD28, CD96) and molecules expressed by lymphoid lineage cells (lymphotoxin beta, IL2-inducible T-cell kinase, TCF7, T-cell differentiation protein mal, GATA3, STAT5B); |
| genes encoding molecules that associate to the cytoskeleton (Actin related protein 2/3, MAPK1, MAP3K1, RAB5A). Also present are T-cell expressed genes (FAS, ITGA4/CD49D, ZNF1A1); |
| genes encoding for Immune-related cell surface molecules (CD36, CD86, LILRB), cytokines (IL15) and molecules involved in signaling pathways (FYB, TICAM2-Toll-like receptor pathway); |
| genes encoding kinases (UHMK1, CSNK1G1, CDK6, WNK1, TAOK1, CALM2, PRKCI, ITPKB, SRPK2, STK17B, DYRK2, PIK3R1, STK4, CLK4, PKN2) and RAS family members (G3BP, RAB14, RASA2, RAP2A, KRAS); |
| **Interferon-inducible:** genes encoding interferon-inducible genes: antiviral molecules (OAS1/2/3/L, GBP1, G1P2, EIF2AK2/PKR, MX1, PML), chemokines (CXCL10/IP-10), signaling molecules (STAT1, STAt2, IRF7, ISGF3G); |
| **Inflammation I:** genes encoding molecules involved in inflammatory processes (IL8, ICAM1, C5R1, CD44, PLAUR, IL1A, CXCL16), and regulators of apoptosis (MCL1, FOX03A, RARA, BCL3/6/2A1, GADD45B); |
| **Inflammation II:** genes encoding molecules inducing or inducible by Granulocyte-Macrophage CSF (SPI1, IL18, ALOX5, ANPEP), as well as lysosomal enzymes (PPT1, CTSB/S, CES1, NEU1, ASAH1, LAMP2, CAST); |
| genes encoding protein phosphates (PPP1R12A, PTPRC, PPP1CB, PPM1B) and phosphoinositide 3-kinase (PI3K) family members (PIK3CA, PIK32A, PIP5K3); |
| genes encoding hemoglobin genes (HBA1, HBA2, HBB); |
| genes encoding T-cell surface markers (CD101, CD102, CD103) as well as molecules ubiquitously expressed among blood leukocytes (CXRCR1: fraktalkine receptor, CD47, P-selectin ligand); |
| genes encoding proteasome subunits (PSMA2/5, PSMB5/8); ubiquitin protein ligases HIP2, STUB1, as well as components of ubiqutin ligase complexes (SUGT1); |
| genes encoding for several enzymes: aminomethyltransferase, arginyltransferase, asparagines synthetase, diacylglycerol kinase, inositol phosphatases, methyltransferases, helicases; and |
| genes encoding for protein kinases (PRKPIR, PRKDC, PRKCI) and phosphatases (PTPLB, PPP1R8/2CB). Also includes RAS oncogene family members and the NK cell receptor 2B4 (CD244); |

and combinations thereof, wherein the level of expression of genes in a sample is charted to the modules to determine a disease or condition.
26. A disease analysis tool comprising:
one or more gene modules selected from the group consisting of:

| Transcriptional modules |
|---|
| **Plasma cells:** genes encoding for Immunoglobulin chains (IGHM, IGJ, IGLL1, IGKC, IGHD) and the plasma cell marker CD38.; |
| **Platelets:** genes encoding for platelet glycoproteins (ITGA2B, ITGB3, GP6, GP1A/B), and platelet-derived immune mediators such as PPPB (pro-platelet basic protein) and PF4 (platelet factor 4); |
| **B-cells:** genes encoding for B-cell surface markers (CD72, CD79A/B, CD19, CD22) and other B-cell associated molecules: Early B-cell factor (EBF), B-cell linker (BLNK) and B lymphoid tyrosine kinase (BLK); |
| Genes encoding regulators and targets of cAMP signaling pathway (JUND, ATF4, CREM, PDE4, NR4A2, VIL2), repressors of TNF-alpha mediated NF-KB activation (CYLD, ASK, TNFAIP3); |
| **Myeloid lineage:** Genes encoding molecules expressed by cells of the myeloid lineage (CD86, CD 163, FCGR2A), some of which being involved in pathogen recognition (CD14, TLR2, MYD88). This set also includes TNF family members (TNFR2, BAFF); |
| genes encoding for signaling molecules, the zinc finger containing inhibitor of activated STAT (PIAS1 and PIAS2), or the nuclear factor of activated T-cells NFATC3; |
| **MHC/Ribosomal proteins:** genes encoding MHC class I molecules (HLA-A,B,C,G,E)+ Beta 2-microglobulin (B2M) or Ribosomal proteins (RPLs, RPSs); |
| genes encoding metabolic enzymes (GLS, NSF1, NAT1) and factors involved in DNA replication (PURA, TERF2, EIF2S1); |
| **Cytotoxic cells:** Gene encoding for Cytotoxic T-cells and NK-cells surface markers (CD8A, CD2, CD160, NKG7, KLRs), cytolytic molecules (granzyme, perforin, granulysin), chemokines (CCL5, XCL1) and CTL/NK-cell associated molecules (CTSW); |
| **Neutrophils:** Gene encoding innate molecules that are found in neutrophil granules (Lactotransferrin: LTF, defensin: DEAF1, Bacterial Permeability Increasing protein: BPI, Cathelicidin antimicrobial protein: CAMP); |
| **Erythrocytes:** Gene encoding hemoglobin genes (HGBs) and other erythrocyte-associated genes (erythrocytic alkirin:ANK1, Glycophorin C: GYPC, hydroxymethylbilane synthase: HMBS, erythroid associated factor: ERAF); |
| **Ribosomal proteins:** genes encoding ribosomal proteins (RPLs, RPSs), Eukaryotic Translation Elongation factor family members (EEFs) and Nucleolar proteins (NPM1, NOAL2, NAP1L1); |
| genes encoding immune-related (CD40, CD80, CXCL12, IFNA5, IL4R) as well as cytoskeleton-related molecules (Myosin, Dedicator of Cytokenesis, Syndecan 2, Plexin C1, Distrobrevin); |
| **Myeloid lineage:** Related to M 1.5. Includes genes expressed in myeloid lineage cells (IGTB2/CD18, Lymphotoxin beta receptor, Myeloid related proteins 8/14 Formyl peptide receptor 1), such as Monocytes and Neutrophils; |
| genes encoding members of the chemokine-like factor superfamily (CKLFSF8); |
| **T-cells:** genes encoding T-cell surface markers (CD5, CD6, CD7, CD26, CD28, CD96) and molecules expressed by lymphoid lineage cells (lymphotoxin beta, IL2-inducible T-cell kinase, TCF7, T-cell differentiation protein mal, GATA3, STAT5B); |
| genes encoding molecules that associate to the cytoskeleton (Actin related protein 2/3, MAPK1, MAP3K1, RAB5A). Also present are T-cell expressed genes (FAS, ITGA4/CD49D, ZNF1A1); |
| genes encoding for immune-related cell surface molecules (CD36, CD86, LILRB), cytokines (IL15) and molecules involved in signaling pathways (FYB, TICAM2-Toll-like receptor pathway); |
| genes encoding kinases (UHMK1, CSNK1G1, CDK6, WNK1, TAOK1, CALM2, PRKCI, ITPKB, SRPK2, STK17B, DYRK2, PIK3R1, STK4, CLK4, PKN2) and RAS family members (G3BP, RAB14, RASA2, RAP2A, KRAS); |
| **Interferon-inducible:** genes encoding interferon-inducible genes: antiviral molecules (OAS1/2/3/L, GBP1, G1P2, EIF2AK2/PKR, MX1, PML), chemokines (CXCL10/IP-10), signaling molecules (STAT1, STAt2, IRF7, ISGF3G); |
| **Inflammation I:** genes encoding molecules involved in inflammatory processes (IL8, ICAM1, C5R1, CD44, PLAUR, IL1A, CXCL16), and regulators of apoptosis (MCL1, FOX03A, RARA, BCL3/6/2A1, GADD45B); |
| **Inflammation II:** genes encoding molecules inducing or inducible by Granulocyte-Macrophage CSF (SPI1, IL18, ALOX5, ANPEP), as well as lysosomal enzymes (PPT1, CTSB/S, CES1, NEU1, ASAH1, LAMP2, CAST); |
| genes encoding protein phosphates (PPP1R12A, PTPRC, PPP1CB, PPM1B) and phosphoinositide 3-kinase (PI3K) family members (PIK3CA, PIK32A, PIP5K3); |
| genes encoding hemoglobin genes (HBA1, HBA2, HBB); |
| genes encoding T-cell surface markers (CD101, CD102, CD103) as well as molecules ubiquitously expressed among blood leukocytes (CXRCR1, fraktalkine receptor, CD47, P-selectin ligand); |
| genes encoding proteasome subunits (PSMA2/5, PSMB5/8); ubiquitin protein ligases HIP2, STUB1, as well as components of ubiqutin ligase complexes (SUGT1); |
| genes encoding for several enzymes: aminomethyltransferase, arginyltransferase, asparagines synthetase, diacylglycerol kinase, inositol phosphatases, methyltransferases, helicases; and |
| genes encoding for protein kinases (PRKPIR, PRKDC, PRKCI) and phosphatases (PTPLB, PPP1R8/2CB), RAS oncogene family members and the NK cell receptor 2B4 (CD244); |

and are sufficient to distinguish between an autoimmune disease, a viral infection a bacterial infection, cancer and transplant rejection.
27. The method of definition 26, wherein the modules are used to distinguish between Systemic Lupus erythematosus, Influenza infection, melanoma and transplant rejection.
28. The method of definition 26, wherein the modules selected are selected from:
**Plasma cells:** genes encoding for Immunoglobulin chains (IGHM, IGJ, IGLL1, IGKC, IGHD) and the plasma cell marker CD38; and
**Platelets:** genes encoding for platelet glycoproteins (ITGA2B, ITGB3, GP6, GP1A/B), and platelet-derived immune mediators PPPB (pro-platelet basic protein) and PF4 (platelet factor 4);
and the modules are used to identify Systemic Lupus erythematosus by having a positive vector at these two modules.
29. The method of definition 26, wherein the modules selected are selected from:
**Plasma cells:** genes encoding for Immunoglobulin chains (IGHM, IGJ, IGLL1, IGKC, IGHD) and the plasma cell marker CD38; and
**Platelets:** genes encoding for platelet glycoproteins (ITGA2B, ITGB3, GP6, GP1A/B), and platelet-derived immune mediators PPPB (pro-platelet basic protein) and PF4 (platelet factor 4);
and the modules are used to identify Influenza infection by having neither a positive nor a negative vector at these two modules.
30. The method of definition 26, wherein the modules selected are selected from:
**Plasma cells:** genes encoding for Immunoglobulin chains (IGHM, IGJ, IGLL1, IGKC, IGHD) and the plasma cell marker CD38; and
**Platelets:** genes encoding for platelet glycoproteins (ITGA2B, ITGB3, GP6, GP1A/B), and platelet-derived immune mediators such as PPPB (pro-platelet basic protein) and PF4 (platelet factor 4);
and the modules are used to identify melanoma by having a negative vector for the plasma cell markers and a positive vector for the platelet markers.
31. The method of definition 26, wherein the modules selected are selected from:
**Plasma cells:** genes encoding for Immunoglobulin chains (IGHM, IGJ, IGLL1, IGKC, IGHD) and the plasma cell marker CD38; and
**Platelets:** genes encoding for platelet glycoproteins (ITGA2B, ITGB3, GP6, GP1A/B), and platelet-derived immune mediators PPPB (pro-platelet basic protein) and PF4 (platelet factor 4);
and the modules are used to identify transplant rejection by having a negative vectors at these two modules.
32. The method of definition 26, wherein the modules selected are selected from:
**Plasma cells:** genes encoding for Immunoglobulin chains (IGHM, IGJ, IGLL1, IGKC, IGHD) and the plasma cell marker CD38; and
**Platelets:** genes encoding for platelet glycoproteins (ITGA2B, ITGB3, GP6, GP1A/B), and platelet-derived immune mediators PPPB (pro-platelet basic protein) and PF4 (platelet factor 4);
and the modules are used to identify Influenza infection by having a negative vector at these two modules.
33. A prognostic gene array comprising:
a customized gene array that comprises a combination of genes that are representative of one or more transcriptional modules, wherein the transcriptome of a patient that is contacted with the customized gene array is prognostic of one or more disease or conditions that match the transcriptional modules.
34. The array of definition 33, wherein the patient's immune response to the disease or condition is determined based on the presence, absence or level of expression of genes of the transcriptome based on a correlation of the transcriptional modules with a specific disease or condition.
35. The array of definition 33, wherein the array can distinguish between an autoimmune disease, a viral infection a bacterial infection, cancer and transplant rejection.
36. The array of definition 33, wherein the array is organized into two or more transcriptional modules.
37. The array of definition 33, wherein the array is organized into three transcriptional modules comprising one or more submodules selected from:

| **Submodule** | **Number of probe sets** | **Keyword selection** | **Assessment** |
|---|---|---|---|
| M 1.1 | 69 | Ig, Immunoglobulin, Bone, Marrow, PreB, IgM, Mu. | **Plasma cells:** genes encoding for Immunoglobulin chains (IGHM, IGJ, IGLL1, IGKC, IGHD) and the plasma cell marker CD38; |
| M 1.2 | 96 | Platelet, Adhesion, Aggregation, Endothelial, Vascular | **Platelets:** genes encoding for platelet glycoproteins (ITGA2B, ITGB3, GP6, GP1A/B), and platelet-derived immune mediators such as PPPB (pro-platelet basic protein) and PF4 (platelet factor 4); |
| M 1.3 | 47 | Immunoreceptor, BCR, B-cell, IgG | **B-cells:** genes encoding for B-cell surface markers (CD72, CD79A/B, CD19, CD22) and other B-cell associated molecules: Early B-cell factor (EBF), B-cell linker (BLNK) and B lymphoid tyrosine kinase (BLK); |
| M 1.4 | 87 | Replication, Repression, Repair, CREB, Lymphoid, TNF-alpha | genes encoding regulators and targets of cAMP signaling pathway (JUND, ATF4, CREM, PDE4, NR4A2, VIL2), as well as repressors of TNF-alpha mediated NF-KB activation (CYLD, ASK, TNFAIP3); |
| M 1.5 | 130 | Monocytes, Dendritic, MHC, Costimulatory, TLR4, MYD88 | **Myeloid lineage:** Molecules expressed by cells of the myeloid lineage (CD86, CD163, FCGR2A), some of which being involved in pathogen recognition (CD14, TLR2, MYD88). This set also includes TNF family members (TNFR2, BAFF); |
| M 1.6 | 28 | Zinc, Finger, P53, RAS | genes encoding for signaling molecules, the zinc finger containing inhibitor of activated STAT (PIAS 1 and PIAS2), or the nuclear factor of activated T-cells NFATC3; |
| M 1.7 | 127 | Ribosome, Translational, 40S, 60S, HLA | **MHC/Ribosomal proteins:** genes encoding MHC class I molecules (HLA-A,B,C,G,E)+ Beta 2-microglobulin (B2M) or Ribosomal proteins (RPLs, RPSs); |
| M 1.8 | 86 | Metabolism, Biosynthesis, Replication, Helicase | genes encoding metabolic enzymes (GLS, NSF1, NAT1) and factors involved in DNA replication (PURA, TERF2, EIF2S1); |
| M 2.1 | 72 | NK, Killer, Cytolytic, CD8, Cell-mediated, T-cell, CTL, IFN-g | **Cytotoxic cells:** genes encoding cytotoxic T-cell and NK-cell surface markers (CD8A, CD2, CD160, NKG7, KLRs), cytolytic molecules (granzyme, perforin, granulysin), chemokines (CCL5, XCL1) and CTL/NK-cell associated molecules (CTSW); |
| M 2.2 | 44 | Granulocytes, Neutrophils, Defense, Myeloid, Marrow | **Neutrophils:** genes encoding innate molecules that are found in neutrophil granules (Lactotransferrin: LTF, defensin: DEAF1, Bacterial Permeability Increasing protein: BPI, Cathelicidin antimicrobial protein: CAMP); |
| M 2.3 | 94 | Erythrocytes, Red, Anemia, Globin, Hemoglobin | **Erythrocytes:** hemoglobin genes (HGBs) and other erythrocyte-associated genes (erythrocytic alkirin:ANK1, Glycophorin C: GYPC, hydroxymethylbilane synthase: HMBS, erythroid associated factor: ERAF); |
| M 2.4 | 118 | Ribonucleoprotein, 60S, nucleolus, Assembly, Elongation | **Ribosomal proteins:** genes encoding ribosomal proteins (RPLs, RPSs), Eukaryotic Translation Elongation factor family members (EEFs) and Nucleolar proteins (NPM1, NOAL2, NAP1L1); |
| M 2.5 | 242 | Adenoma, Interstitial, Mesenchyme, Dendrite, Motor | genes encoding immune-related (CD40, CD80, CXCL12, IFNA5, IL4R) as well as cytoskeleton-related molecules (Myosin, Dedicator of Cytokenesis, Syndecan 2, Plexin C1, Distrobrevin); |
| M 2.6 | 110 | Granulocytes, Monocytes, Myeloid, ERK, Necrosis | genes encoding molecules expressed in myeloid lineage cells (IGTB2/CD18, Lymphotoxin beta receptor, Myeloid related proteins 8/14 Formyl peptide receptor 1), Monocytes and Neutrophils; |
| M 2.7 | 43 | No keywords extracted. | genes encoding one or more members of the chemokine-like factor superfamily (CKLFSF8); |
| M 2.8 | 104 | Lymphoma, T-cell, CD4, CD8, TCR, Thymus, Lymphoid, IL2 | **T-cells:** genes encoding T-cell surface markers (CD5, CD6, CD7, CD26, CD28, CD96) and molecules expressed by lymphoid lineage cells (lymphotoxin beta, IL2-inducible T-cell kinase, TCF7, T-cell differentiation protein mal, GATA3, STAT5B); |
| M 2.9 | 122 | ERK, Transactivation, Cytoskeletal, MAPK, JNK | genes encoding molecules that associate to the cytoskeleton (Actin related protein 2/3, MAPK1, MAP3K1, RAB5A). Also present are T-cell expressed genes (FAS, ITGA4/CD49D, ZNF1A1); |
| M2.10 | 44 | Myeloid, Macrophage, Dendritic, Inflammatory, Interleukin | genes encoding for Immune-related cell surface molecules (CD36, CD86, LILRB), cytokines (IL15) and molecules involved in signaling pathways (FYB, TICAM2-Toll-like receptor pathway); |
| M 2.11 | 77 | Replication, Repress, RAS, Autophosphorylati on, Oncogenic | genes encoding kinases (UHMK1, CSNK1G1, CDK6, WNK1, TAOK1, CALM2, PRKCI, ITPKB, SRPK2, STK17B, DYRK2, PIK3R1, STK4, CLK4, PKN2) and RAS family members (G3BP, RAB14, RASA2, RAP2A, KRAS); |
| M 3.1 | 80 | ISRE, Influenza, Antiviral, IFN-gamma, IFN-alpha, Interferon | **Interferon-inducible:** genes encoding interferon-inducible genes: antiviral molecules (OAS1/2/3/L, GBP1, G1P2, EIF2AK2/PKR, MX1, PML), chemokines (CXCL10/IP-10), signaling molecules (STAT1, STAt2, IRF7, ISGF3G); |
| M 3.2 | 230 | TGF-beta, TNF, Inflammatory, Apoptotic, Lipopolysaccharide | **Inflammation I:** genes encoding molecules involved in inflammatory processes (IL8, ICAM1, C5R1, CD44, PLAUR, IL1A, CXCL16), and regulators of apoptosis (MCL1, FOXO3A, RARA, BCL3/6/2A1, GADD45B); |
| M 3.3 | 230 | Granulocyte, Inflammatory, Defense, Oxidize, Lysosomal | **Inflammation II:** genes encoding molecules inducing or inducible by Granulocyte-Macrophage CSF (SPI1, IL18, ALOX5, ANPEP), as well as lysosomal enzymes (PPT1, CTSB/S, CES1, NEU1, ASAH1, LAMP2, CAST); |
| M 3.4 | 323 | No keyword extracted | genes encoding protein phosphates (PPP1R12A, PTPRC, PPP1CB, PPM1B) and phosphoinositide 3-kinase (PI3K) family members (PIK3CA, PIK32A, PIP5K3); |
| M 3.5 | 19 | No keyword extracted | genes encoding hemoglobin genes (HBA1, HBA2, HBB); |
| M 3.6 | 233 | Complement, Host, Oxidative, Cytoskeletal, T-cell | genes encoding T-cell surface markers (CD101, CD102, CD103) as well as molecules ubiquitously expressed among blood leukocytes (CXRCR1: fraktalkine receptor, CD47, P-selectin ligand); |
| M 3.7 | 80 | Spliceosome, Methylation, Ubiquitin, Beta-catenin | genes encoding proteasome subunits (PSMA2/5, PSMB5/8); ubiquitin protein ligases HIP2, STUB1, as well as components of ubiqutin ligase complexes (SUGT1); |
| M 3.8 | 182 | CDC, TCR, CREB, Glycosylase | genes encoding for several enzymes: aminomethyltransferase, arginyltransferase, asparagines synthetase, diacylglycerol kinase, inositol phosphatases, methyltransferases, helicases; and |
| M 3.9 | 261 | Chromatin, Checkpoint, Replication, Transactivation | genes encoding for protein kinases (PRKPIR, PRKDC, PRKCI) and phosphatases (PTPLB, PPP1R8/2CB). Also includes RAS oncogene family members and the NK cell receptor 2B4 (CD244); |

and comprising probes that bind specifically one or more of the genes in the module.
38. A gene analysis tool comprising:
one or more gene modules selected from a combination of one group selected from the left column and one group selected from the right column comprising:

| Keyword selection | Transcriptional modules |
|---|---|
| Ig, Immunoglobulin, Bone, Marrow, PreB, IgM,Mu. | Plasma cells: genes encoding for Immunoglobulin chains (IGHM, IGJ, IGLL1, IGKC, IGHD) and the plasma cell marker CD38.; |
| Platelet, Adhesion, Aggregation, Endothelial, Vascular | Platelets: genes encoding for platelet glycoproteins (ITGA2B, ITGB3, GP6, GP1A/B), and platelet-derived immune mediators such as PPPB (pro-platelet basic protein) and PF4 (platelet factor 4); |
| Immunoreceptor, BCR, B-cell, IgG | B-cells: genes encoding for B-cell surface markers (CD72, CD79A/B, CD19, CD22) and other B-cell associated molecules: Early B-cell factor (EBF), B-cell linker (BLNK) and B lymphoid tyrosine kinase (BLK); |
| Replication, Repression, Repair, CREB, Lymphoid, TNF-alpha | genes encoding regulators and targets of cAMP signaling pathway (JUND, ATF4, CREM, PDE4, NR4A2, VIL2), as well as repressors of TNF-alpha mediated NF-KB activation (CYLD, ASK, TNFAIP3); |
| Monocytes, Dendritic, MHC, Costimulatory, TLR4, MYD88 | Myeloid lineage: genes encoding molecules expressed by cells of the myeloid lineage (CD86, CD163, FCGR2A), some of which being involved in pathogen recognition (CD14, TLR2, MYD88) and TNF family members (TNFR2, BAFF); |
| Zinc, Finger, P53, RAS | genes encoding for signaling molecules, the zinc finger containing inhibitor of activated STAT (PIAS1 and PIAS2), or the nuclear factor of activated T-cells NFATC3; |
| Ribosome, Translational, 40S, 60S, HLA | MHC/Ribosomal proteins: genes encoding MHC class I molecules (HLA-A,B,C,G,E)+ Beta 2-microglobulin (B2M) or Ribosomal proteins (RPLs, RPSs); |
| Metabolism, Biosynthesis, Replication, Helicase | genes encoding metabolic enzymes (GLS, NSF1, NAT1) and factors involved in DNA replication (PURA, TERF2, EIF2S 1); |
| NK, Killer, Cytolytic, CD8, Cell-mediated, T-cell, CTL, IFN-g | Cytotoxic cells: Cytotoxic T-cells and NK-cells surface markers (CD8A, CD2, CD160, NKG7, KLRs), cytolytic molecules (granzyme, perforin, granulysin), chemokines (CCL5, XCL1) and CTL/NK-cell associated molecules (CTSW); |
| Granulocytes, Neutrophils, Defense, Myeloid, Marrow | Neutrophils: genes encoding innate molecules that are found in neutrophil granules (Lactotransferrin: LTF, defensin: DEAF1, Bacterial Permeability Increasing protein: BPI, Cathelicidin antimicrobial protein: CAMP...); |
| Erythrocytes, Red, Anemia, Globin, Hemoglobin | Erythrocytes: genes encoding hemoglobin genes (HGBs) and other erythrocyte-associated genes (erythrocytic alkirin:ANK1, Glycophorin C: GYPC, hydroxymethylbilane synthase: HMBS, erythroid associated factor: ERAF); |
| Ribonucleoprotein, 60S, nucleolus, Assembly, Elongation | Ribosomal proteins: genes encoding ribosomal proteins (RPLs, RPSs), Eukaryotic Translation Elongation factor family members (EEFs) and Nucleolar proteins (NPM1, NOAL2, NAP1L1); |
| Adenoma, Interstitial, Mesenchyme, Dendrite, Motor | genes encoding immune-related (CD40, CD80, CXCL12, IFNA5, IL4R) as well as cytoskeleton-related molecules (Myosin, Dedicator of Cytokenesis, Syndecan 2, Plexin C1, Distrobrevin); |
| Granulocytes, Monocytes, Myeloid, ERK, Necrosis | Myeloid lineage: genes expressed in myeloid lineage cells (IGTB2/CD18, Lymphotoxin beta receptor, Myeloid related proteins 8/14 Formyl peptide receptor 1), such as Monocytes and Neutrophils; |
| No keywords extracted. | genes encoding one or more members of the chemokine-like factor superfamily (CKLFSF8); |
| Lymphoma, T-cell, CD4, CD8, TCR, Thymus, Lymphoid, IL2 | T-cells: genes encoding T-cell surface markers (CD5, CD6, CD7, CD26, CD28, CD96) and molecules expressed by lymphoid lineage cells (lymphotoxin beta, IL2-inducible T-cell kinase, TCF7, T-cell differentiation protein mal, GATA3, STAT5B); |
| ERK, Transactivation, Cytoskeletal, MAPK, JNK | genes encoding molecules that associate to the cytoskeleton (Actin related protein 2/3, MAPK1, MAP3K1, RAB5A). Also present are T-cell expressed genes (FAS, ITGA4/CD49D, ZNF1A1); |
| Myeloid, Macrophage, Dendritic, Inflammatory, Interleukin | genes encoding for Immune-related cell surface molecules (CD36, CD86, LILRB), cytokines (IL15) and molecules involved in signaling pathways (FYB, TICAM2-Toll-like receptor pathway); |
| Replication, Repress, RAS, Autophosphorylation , Oncogenic | genes encoding kinases (UHMK1, CSNK1G1, CDK6, WNK1, TAOK1, CALM2, PRKCI, ITPKB, SRPK2, STK17B, DYRK2, PIK3R1, STK4, CLK4, PKN2) and RAS family members (G3BP, RAB14, RASA2, RAP2A, KRAS); |
| ISRE, Influenza, Antiviral, IFN-gamma, IFN-alpha, Interferon | Interferon-inducible: genes encoding interferon-inducible genes: antiviral molecules (OAS1/2/3/L, GBP1, G1P2, EIF2AK2/PKR, MX1, PML), chemokines (CXCL10/IP-10), signaling molecules (STAT1, STAt2, IRF7, ISGF3G); |
| TGF-beta, TNF, Inflammatory, Apoptotic, Lipopolysaccharide | Inflammation I: genes encoding molecules involved in inflammatory processes (IL8, ICAM1, C5R1, CD44, PLAUR, IL1A, CXCL16), and regulators of apoptosis (MCL1, FOXO3A, RARA, BCL3/6/2A1, GADD45B); |
| Granulocyte, Inflammatory, Defense, Oxidize, Lysosomal | Inflammation II: genes encoding molecules inducing or inducible by Granulocyte-Macrophage CSF (SPI1, IL18, ALOX5, ANPEP), as well as lysosomal enzymes (PPT1, CTSB/S, CES1, NEU1, ASAH1, LAMP2, CAST); |
| No keyword extracted | genes encoding protein phosphates (PPP1R12A, PTPRC, PPP1CB, PPM1B) and phosphoinositide 3-kinase (PI3K) family members (PIK3CA, PIK32A, PIP5K3); |
| No keyword extracted | genes encoding hemoglobin genes (HBA1, HBA2, HBB); |
| Complement, Host, Oxidative, Cytoskeletal, T-cell | genes encoding T-cell surface markers (CD101, CD102, CD103) as well as molecules ubiquitously expressed among blood leukocytes (CXRCR1: fraktalkine receptor, CD47, P-selectin ligand); |
| Spliceosome, Methylation, Ubiquitin, Beta-catenin | genes encoding proteasome subunits (PSMA2/5, PSMB5/8); ubiquitin protein ligases HIP2, STUB1, as well as components of ubiqutin ligase complexes (SUGT1); |
| CDC, TCR, CREB, Glycosylase | genes encoding for several enzymes: aminomethyltransferase, arginyltransferase, asparagines synthetase, diacylglycerol kinase, inositol phosphatases, methyltransferases, helicases; and |
| Chromatin, Checkpoint, Replication, Transactivation | genes encoding for protein kinases (PRKPIR, PRKDC, PRKCI) and phosphatases (PTPLB, PPP1R8/2CB). Also includes RAS oncogene family members and the NK cell receptor 2B4 (CD244); |

and combinations thereof, wherein the level of expression of genes in a sample in a module is displayed to diagnose a disease or condition.
39. A method for selecting patients for a clinical trial comprising the steps of:
obtaining the transcriptome of a prospective patient;
comparing the transcriptome to one or more transcriptional modules that are indicative of a disease or condition that is to be treated in the clinical trial; and
determining the likelihood that a patient is a good candidate for the clinical trial based on the presence, absence or level of one or more genes that are expressed in the patient's transcriptome within one or more transcriptional modules that are correlated with success in a clinical trial.
40. The method of definition 39, wherein each module comprises a vector that correlates with a sum of the proportion of transcripts in a sample.
41. The method of definition 39, wherein each module comprises a vector and wherein one or more diseases or conditions is associated with the one or more vectors.
42. The method of definition 39, wherein each module comprises a vector that correlates to the expression level of one or more genes within each module.
43. The method of definition 39, wherein each module comprises a vector and wherein the modules selected are:
**Plasma cells:** genes encoding for Immunoglobulin chains (IGHM, IGJ, IGLL1, IGKC, IGHD) and the plasma cell marker CD38; and
**Platelets:** genes encoding for platelet glycoproteins (ITGA2B, ITGB3, GP6, GP1A/B), and platelet-derived immune mediators such as PPPB (pro-platelet basic protein) and PF4 (platelet factor 4);
and the modules are used to distinguish between Systemic Lupus erythematosus by having a positive vector at these two modules; Influenza infection by having neither a positive nor a negative vector at these two modules; melanoma by having a negative vector for the plasma cell markers and a positive vector for the platelet markers; identify transplant rejection by having a negative vectors at these two modules.
44. An array of nucleic acid probes immobilized on a solid support comprising sufficient probes from one or more modules to provide a sufficient proportion of differentially expressed genes to distinguish between one or more diseases, the probes being selected from Tables 1, 2, 3 or combinations thereof.
45. The array of definition 44, wherein data obtained from a sample contacted with the nucleic acid probes immobilized on the solid support, is sorted by modules selected from:

| Module I.D. | Transcriptional Modules |
|---|---|
| M 1.1 | Plasma cells: genes encoding for Immunoglobulin chains (IGHM, IGJ, IGLL1, IGKC, IGHD) and the plasma cell marker CD38. |
| M 1.2 | Platelets: genes encoding for platelet glycoproteins (ITGA2B, ITGB3, GP6, GP1A/B), and platelet-derived immune mediators such as PPPB (pro-platelet basic protein) and PF4 (platelet factor 4). |
| M 1.3 | B-cells: genes encoding for B-cell surface markers (CD72, CD79A/B, CD19, CD22) and other B-cell associated molecules: Early B-cell factor (EBF), B-cell linker (BLNK) and B lymphoid tyrosine kinase (BLK). |
| M 1.4 | genes encoding regulators and targets of cAMP signaling pathway (JUND, ATF4, CREM, PDE4, NR4A2, VIL2), as well as repressors of TNF-alpha mediated NF-KB activation (CYLD, ASK, TNFAIP3). |
| M 1.5 | Myeloid lineage: genes encoding molecules expressed by cells of the myeloid lineage (CD86, CD163, FCGR2A), some of which being involved in pathogen recognition (CD14, TLR2, MYD88). This set also includes TNF family members (TNFR2, BAFF). |
| M1.6 | genes encoding for signaling molecules, the zinc finger containing inhibitor of activated STAT (PIAS 1 and PIAS2), or the nuclear factor of activated T-cells NFATC3. |
| M1.7 | MHC/Ribosomal proteins: genes encoding MHC class I molecules (HLA-A,B,C,G,E)+ Beta 2-microglobulin (B2M) or Ribosomal proteins (RPLs, RPSs). |
| M 1.8 | Undetermined. genes encoding metabolic enzymes (GLS, NSF1, NAT1) and factors involved in DNA replication (PURA, TERF2, EIF2S1). |
| M 2.1 | Cytotoxic cells: genes encoding cytotoxic T-cells amd NK-cells surface markers (CD8A, CD2, CD160, NKG7, KLRs), cytolytic molecules (granzyme, perforin, granulysin), chemokines (CCL5, XCL1) and CTL/NK-cell associated molecules (CTSW). |
| M 2.2 | Neutrophils: genes encoding innate molecules that are found in neutrophil granules (Lactotransferrin: LTF, defensin: DEAF1, Bacterial Permeability Increasing protein: BPI, Cathelicidin antimicrobial protein: CAMP...). |
| M 2.3 | Erythrocytes: genes encoding hemoglobin genes (HGBs) and other erythrocyte-associated genes (erythrocytic alkirin:ANK1, Glycophorin C: GYPC, hydroxymethylbilane synthase: HMBS, erythroid associated factor: ERAF). |
| M 2.4 | Ribosomal proteins: genes encoding ribosomal proteins (RPLs, RPSs), Eukaryotic Translation Elongation factor family members (EEFs) and Nucleolar proteins (NPM1, NOAL2, NAP1L1). |
| M 2.5 | genes encoding immune-related (CD40, CD80, CXCL12, IFNA5, IL4R) as well as cytoskeleton-related molecules (Myosin, Dedicator of Cytokenesis, Syndecan 2, Plexin C1, Distrobrevin). |
| M 2.6 | Myeloid lineage: genes expressed in myeloid lineage cells (IGTB2/CD18, Lymphotoxin beta receptor, Myeloid related proteins 8/14 Formyl peptide receptor 1), such as Monocytes and Neutrophils: |
| M 2.7 | genes encoding one or more members of the chemokine-like factor superfamily (CKLFSF8). |
| M 2.8 | T-cells: genes encoding T-cell surface markers (CD5, CD6, CD7, CD26, CD28, CD96) and molecules expressed by lymphoid lineage cells (lymphotoxin beta, IL2-inducible T-cell kinase, TCF7, T-cell differentiation protein mal, GATA3, STAT5B). |
| M 2.9 | genes encoding molecules that associate to the cytoskeleton (Actin related protein 2/3, MAPK1, MAP3K1, RAB5A). Also present are T-cell expressed genes (FAS, ITGA4/CD49D, ZNF1A1). |
| M2.10 | genes encoding for Immune-related cell surface molecules (CD36, CD86, LILRB), cytokines (IL15) and molecules involved in signaling pathways (FYB, TICAM2-Toll-like receptor pathway). |
| M 2.11 | genes encoding kinases (UHMK1, CSNK1G1, CDK6, WNK1, TAOK1, CALM2, PRKCI, ITPKB, SRPK2, STK17B, DYRK2, PIK3R1, STK4, CLK4, PKN2) and RAS family members (G3BP, RAB14, RASA2, RAP2A, KRAS). |
| M 3.1 | Interferon-inducible: genes encoding interferon-inducible genes: antiviral molecules (OAS1/2/3/L, GBP1, G1P2, EIF2AK2/PKR, MX1, PML), chemokines (CXCL10/IP-10), signaling molecules (STAT1, STAt2, IRF7, ISGF3G). |
| M 3.2 | Inflammation I: genes encoding molecules involved in inflammatory processes (IL8, ICAM1, C5R1, CD44, PLAUR, IL1A, CXCL16), and regulators ofapoptosis (MCL1, FOXO3A, RARA, BCL3/6/2A1, GADD45B). |
| M 3.3 | Inflammation II: genes encoding molecules inducing or inducible by Granulocyte-Macrophage CSF (SPI1, IL18, ALOX5, ANPEP), as well as lysosomal enzymes (PPT1, CTSB/S, CES1, NEU1, ASAH1, LAMP2, CAST). |
| M 3.4 | genes encoding protein phosphates (PPP1R12A, PTPRC, PPP1CB, PPM1B) and phosphoinositide 3-kinase (PI3K) family members (PIK3CA, PIK32A, PIP5K3). |
| M 3.5 | genes encoding hemoglobin genes (HBA1, HBA2, HBB). |
| M 3.6 | genes encoding T-cell surface markers (CD101, CD102, CD103) as well as molecules ubiquitously expressed among blood leukocytes (CXRCR1: fraktalkine receptor, CD47, P-selectin ligand). |
| M 3.7 | genes encoding proteasome subunits (PSMA2/5, PSMB5/8); ubiquitin protein ligases HIP2, STUB1, and ubiqutin ligase complexes (SUGT1). |
| M 3.8 | genes encoding for several enzymes: aminomethyltransferase, arginyltransferase, asparagines synthetase, diacylglycerol kinase, inositol phosphatases, methyltransferases, helicases |
| M 3.9 | genes encoding for protein kinases (PRKPIR, PRKDC, PRKCI) and phosphatases (PTPLB, PPP1R8/2CB), RAS oncogenes and the NK cell receptor 2B4 (CD244). |

wherein the probes in the first probe set have one or more interrogation positions respectively corresponding to one or more diseases.
46. The array of definition 44, wherein the array has between 100 and 100,000 probes.
47. The array of definition 44, wherein each probe is 9-21 nucleotides.
48. The array of definition 44, wherein the probes in the second, third and fourth probe sets positioned to be interrogated.
49. An array of nucleic acid probes immobilized on a solid support, the array comprising at least one pair of first and second probe groups, each group comprising one or more probes as defined by Tables 1, 2, 3 or combinations thereof.
50. The array of definition 49, wherein the groups provide a composite transcriptional marker vector that is consistent across microarray platforms.
51. The array of definition 49, wherein the groups provide a composite transcriptional marker vector that is consistent across microarray platforms and displayed in a summary for regulatory approval.
TABLES

**Table 1**

| module ID | affy ID | Entrez ID | Gene Symbol | Gene Title |
|---|---|---|---|---|
| 1.1 | 205692_s_at | 952 | CD38 | CD38 antigen (p45) |
| 1.1 | 214677_x_at | 3535 /// 3538 | IGL@ /// IGLC2 | immunoglobulin lambda locus /// immunoglobulin |
| | | | | lambda constant 1 (Mcg marker) /// immunoglobulin |
| | | | | lambda constant 2 (Kern-Oz- marker) /// immunoglobulin lambda variable 2-14 /// immunoglobulin lambda joining 3 |
| 1.1 | 215946_x_at | 3543 /// 91316 | IGLL1 /// LOC91316 | PREDICTED: Homo sapiens similar to omega protein |
| | | | | (LOC91353), mRNA |
| 1.1 | 221253_s_at | 81567 | TXNDC5 | thioredoxin domain containing 5 /// thioredoxin domain |
| | | | | containing 5 |
| 1.1 | 217480_x_at | 339562 | LOC339562 | similar to Ig kappa chain |
| 1.1 | 211430_s_at | 3492 /// 3500 /// 3501 | IGH@ /// IGHG1 /// | immunoglobulin heavy locus /// immunoglobulin heavy |
| | | /// 3507 | IGHG2 /// IGHM | constant gamma 1 (Glm marker) /// immunoglobulin |
| | | | | heavy constant gamma 2 (G2m marker) /// immunoglobulin heavy constant gamma 3 (G3m marker) /// immunoglobulin heavy constant mu |
| 1.1 | 200599_s_at | 7184 | TRA1 | tumor rejection antigen (gp96) 1 |
| 1.1 | 203560_at | 8836 | GGH | gamma-glutamyl hydrolase (conjugase, folylpolygammaglutamyl hydrolase) |
| 1.1 | 221739_at | 56005 | C19orf10 | chromosome 19 open reading frame 10 |
| 1.1 | 202503_s_at | 9768 | KIAA0101 | KIAA0101 |
| 1.1 | 211868_x_at | 3500 | IGHG1 | immunoglobulin heavy constant alpha 1 /// immunoglobulin heavy constant gamma 1 (Glm marker) /// immunoglobulin heavy constant gamma 3 |
| | | | | (G3m marker) |
| 1.1 | 234764_x_at | 3538 | IGLC2 | Ig lambda chain V-region (VL-AIG) /// Immunoglobulin |
| | | | | lambda variable 3-21 |
| 1.1 | 223299-at | 90701 | SEC11L3 | SEC11-like 3 (S. cerevisiae) |
| 1.1 | 214768_x_at | --- | --- | immunoglobulin kappa variable 1-5 |
| 1.1 | 216557_x_at | 3500 | IGHG1 | immunoglobulin heavy constant alpha 1 /// immunoglobulin heavy constant gamma 1 (Glm marker) /// immunoglobulin heavy constant gamma 3 |
| | | | | (G3m marker) |
| 1.1 | 214777_at | --- | --- | Anti-HIV-1 gp120 V3 loop antibody DO142-10 light |
| | | | | chain variable region |
| 1.1 | 224795_x_at | 3514 | IGKC | immunoglobulin kappa constant /// immunoglobulin |
| | | | | kappa variable 1-5 |
| 1.1 | 209374_s_at | 3507 | IGHM | immunoglobulin heavy constant mu |
| 1.1 | 219551_at | 55840 | EAF2 | ELL associated factor 2 |
| 1.1 | 213502_x_at | 91316 | LOC91316 | similar to bK246H3.1 (immunoglobulin lambda-like |
| | | | | polypeptide 1, pre-B-cell specific) |
| 1.1 | 215214_at | 3538 | IGLC2 | Immunoglobulin lambda variable 3-21 |
| 1.1 | 202655_at | 7873 | ARMET | arginine-rich, mutated In early stage tumors |
| 1.1 | 217022_s_at | 283650 /// 3494 | IGHA2 /// MGC27165 | immunoglobulin heavy constant alpha 1 /// immunoglobulin heavy constant alpha 2 (A2m marker) |
| | | | | /// hypothetical protein MGC27165 |
| 1.1 | 216640_s_at | 10130 | TXNDC7 | protein disulfide isomerase-associated 6 |
| 1.1 | 217179_x_at | --- | --- | Ig rearranged lambda-chain gene V-J12/13-region |
| 1.1 | 221004_s_at | 81618 | ITM2C | integral membrane protein 2c /// integral membrane |
| 1.1 | 213399_x_at | 6185 | RPN2 | protein 2C ribophorin II |
| 1.1 | 216491_x_at | 3500 /// 3507 | IGHM /// IGHG1 | immunoglobulin heavy constant mu |
| 1.1 | 214836_x_at | --- | --- | HRV Fab N8-VL |
| 1.1 | 221651_x_at | 3514 | IGKC | immunoglobulin kappa constant /// immunoglobulin |
| | | | | kappa variable 1-5 |
| 1.1 | 214669_x_at | 440871 | LOC440871 | Immunoglobulin kappa constant |
| 1.1 | 211635_x_at | --- | --- | IgM rheumatoid factor RF-TT1, variable heavy chain /// |
| | | | | IgM rheumatoid factor RF-TT1, variable heavy chain |
| 1.1 | 217235_x_at | --- | --- | Immunoglobulin lambda variable 3-21 |
| 1.1 | 211650_x_at | 3500 /// 390714 | IGHG1 /// | IgM VDJ-region /// IgM VDJ-region |
| 1.1 | 202107_s_at | 4171 | MCM2 | MCM2 minichromosome maintenance deficient mitotin (S. cerevisiae) 2, |
| 1.1 | 217236_x_at | 3500 | IGHG1 | Immunoglobulin heavy constant gamma 1 (Glm |
| 1.1 | 217148_x_at | 3538 | IGLC2 | immunoglobulin lambda variable 2-14 |
| 1.1 | 214916_x_at | 283650 /// 3492 /// | IGH@ /// IGHG1 /// | immunoglobulin heavy locus /// immunoglobulin heavy |
| | | 3500 /// 3501 /// 3507 | IGHG2 /// IGHM /// | constant alpha 1 /// immunoglobulin heavy constant |
| | | /// 390714 | MGC27165 /// | alpha 2 (A2m marker) /// immunoglobulin heavy |
| | | | LOC390714 | constant gamma 1 (Glm marker) /// immunoglobulin |
| | | | | heavy constant gamma 2 (G2m marker) /// immunoglobulin he |
| 1.1 | 211_881_x_-at | 28831 | IGLJ3 | immunoglobulin lambda joining 3 |
| 1.1 | 228377_at | 57565 | KLHL14 | kelch-like 14 (Drosophila) |
| 1.1 | 234366_x_at | --- | --- | Immunoglobulin lambda variable 3-21 |
| 1.1 | 211644_x_at | 3514 | IGKC | Anti-rabies virus immunoglobulin rearranged kappa |
| | | | | chain V-region /// Anti-rabies virus immunoglobulin |
| | | | | rearranged kappa chain V-region /// HRV Fab 027-VL |
| | | | | /// HRV Fab 027-VL /// HRV Fab 026-VL /// HRV Fab |
| | | | | 026-VL /// Antibody light chain variable region to H |
| 1.1 | 216576_x_at | --- | --- | HRV Fab 027-VL |
| 1.1 | 212311_at | 23231 | KIAA0746 | KIAA0746 protein |
| 1.1 | 224342_x_at /// Ig | --- | --- | Ig rearranged lambda-chain gene V-J12/13-region |
| | | | | rearranged lambda-chain gene V-J12/13-region |
| 1.1 | 211634_x_at | 3507 | IGHM | immunoglobulin heavy constant mu /// immunoglobulin heavy constant mu |
| 1.1 | 216542_x_at | 283650 | MGC27165 | immunoglobulin heavy constant gamma 1 (Glm marker) /// hypothetical protein MGC27165 |
| 1.1 | 215379_x_at | 3535 /// 3538 | IGL@ /// IGLC2 | immunoglobulin lambda locus /// immunoglobulin |
| | | | | lambda constant 1 (Mcg marker) /// immunoglobulin |
| | | | | lambda constant 2 (Kern-Oz- marker) /// immunoglobulin lambda variable 2-14 /// immunoglobulin lambda joining 3 |
| 1.1 | 211908_x_at | 3500 /// 390714 | IGHG1 /// | IgM VDJ-region /// IgM VDJ-region |
| 1.1 | 200598-s-at | 7184 | TRA1 | tumor rejection antigen (gp96) 1 |
| 1.1 | 216207_x_at | 28902 | IGKV1D-13 | immunoglobulin kappa variable 1D-13 |
| 1.1 | 216510_x_at | 3500 /// 390714 | IGHG1 /// | immunoglobulin heavy constant gamma 1 (Glm marker) /// immunoglobulin heavy constant mu /// |
| | | | | similar to Ig heavy chain V-III region VH26 precursor |
| 1.1 | 217157_x_at | --- | --- | Immunoglobulin light chain |
| 1.1 | 211641_x_at | 3500 | IGHG1 | Immunoglobulin heavy chain VH3 (H11) /// Immunoglobulin heavy chain VH3 (H11) |
| 1.1 | 216401_x_at | --- | --- | --- |
| 1.1 | 215777_at | --- | --- | Ig lambda light chain variable region |
| 1.1 | 211639_x_at | 3507 | IGHM | Ig heavy chain V-region (VH-MAR) /// Ig heavy chain |
| | | | | V-region (VH-MAR) |
| 1.1 | 211637_x_at /// | 388078 | LOC388078 | Immunoglobulin heavy chain V region (Humha448) |
| | | | | Immunoglobulin heavy chain V region (Humha448) |
| 1.1 | 211647_x_at | 3500 | IGHG1 | Immunoglobulin heavy constant gamma 1 (Glm marker) /// Immunoglobulin heavy constant gamma 1 |
| | | | | (Glm marker) |
| 1.1 | 215176_x_at | --- | --- | HRV Fab 027-VL |
| 1.1 | 217227_x_at | 3535 | IGL@ | Hepatitis B surface antigen antibody variable domain |
| 1.1 | 217258_x_at | --- | --- | IgG to Puumala virus G2, light chain variable region |
| 1.1 | 217281_x_at | 283650 /// 3500 /// | IGHG1 /// IGHM /// | IgM rheumatoid factor RF-TT9, variable heavy chain |
| | | 3507 | MGC27165 | |
| 1.1 | 215118_s_at | 283650 | MGC27165 | Hypothetical protein MGC27165 |
| 1.1 | 205267_at | 5450 | POU2AF1 | POU domain, class 2, associating factor 1 |
| 1.1 | 215121_x_at | 3535 /// 3538 | IGL@ /// IGLC2 | immunoglobulin lambda locus /// immunoglobulin |
| | | | | lambda constant 1 (Mcg marker) /// immunoglobulin |
| | | | | lambda constant 2 (Kern-Oz- marker) /// immunoglobulin lambda variable 2-14 |
| 1.1 | 223565_at | 51237 | PACAP | proapoptotic caspase adaptor protein |
| 1.1 | 211645_x_at | --- | --- | Immunoglobulin kappa constant /// Immunoglobulin |
| | | | | kappa constant /// Cationic anti-DNA autoantibody /// |
| | | | | Cationic anti-DNA autoantibody |
| 1.1 | 211798_x_at | 28831 | IGLJ3 | immunoglobulin lambda joining 3 |
| 1.1 | 215949_x_at | 3507 | IGHM | immunoglobulin heavy constant mu |
| 1.1 | 216853_x_at | --- | --- | Immunoglobulin lambda joining 3 |
| 1.1 | 221671_x_at | 3514 | IGKC | immunoglobulin kappa constant /// immunoglobulin |
| | | | | kappa variable 1-5 |
| 1.1 | 214973_x_at | 3495 | IGHD | immunoglobulin heavy constant delta |
| 1.1 | 216984_x_at | --- | --- | Immunoglobulin lambda variable 3-21 |
| 1.1 | 209138_x_at | 3538 | IGLC2 | Immunoglobulin lambda variable 3-21 |
| 1.1 | 212592_at for | 3512 | IGJ | Immunoglobulin J polypeptide, linker protein |
| | | | | immunoglobulin alpha and mu polypeptides |
| 1.2 | 207389_at | 2811 | GP1BA | glycoprotein Ib (platelet), alpha polypeptide |
| 1.2 | 215492_x_at | 171558 | PTCRA | pre T-cell antigen receptor alpha |
| 1.2 | 208601_s_at | 81027 | TUBB1 | tubulin, beta 1 /// tubulin, beta 1 |
| 1.2 | 229778_at | --- | --- | Hypothetical protein MGC10946 |
| 1.2 | 205128_x_at | 5742 | PTGS1 | prostaglandin-endoperoxide synthase 1 (prostaglandin |
| | | | | G/H synthase and cyclooxygenase) |
| 1.2 | 218999_at | 55281 | FLJ11000 | hypothetical protein FLJ11000 |
| 1.2 | 201540_at | 2273 | FHL1 | four and a half LIM domains 1 |
| 1.2 | 222892_s_at | 55287 | TMEM40 | transmembrane protein 40 |
| 1.2 | 212077_at | 800 | CALD1 | caldesmon 1 |
| 1.2 | 205442_at | 9848 | MFAP3L | --- |
| 1.2 | 207815_at | 5197 | PF4V1 | platelet factor 4 variant 1 |
| 1.2 | 37965_at | 29780 | PARVB | parvin, beta |
| 1.2 | 227473_at | --- | --- | Cortactin |
| 1.2 | 201980_s_at | 6251 | RSU1 | Ras suppressor protein 1 |
| 1.2 | 225354_s_at | 83699 | SH3BGRL2 | SH3 domain binding glutamic acid-rich protein like 2 |
| 1.2 | 222803_at 1 | 56952 | PRTFDC1 | phosphoribosyl transferase domain containing |
| 1.2 | 242094_at | --- | --- | Full length insert CDNA clone YR40C10 |
| 1.2 | 215111_s_at | 8848 | TGFB114 | TSC22 domain family 1 |
| 1.2 | 204042_at | 10810 | WASF3 | WAS protein family, member 3 |
| 1.2 | 235885_at | 64805 | P2RY12 | purinergic receptor P2Y, G-protein coupled, 12 |
| 1.2 | 202729_s_at | 4052 | LTBP1 | latent transforming growth factor beta binding protein 1 |
| 1.2 | 215813_s_at | 5742 | PTGS1 | prostaglandin-endoperoxide synthase 1 (prostaglandin |
| | | | | G/H synthase and cyclooxygenase) |
| 1.2 | 206493_at | 3674 | ITGA2B | integrin, alpha 2b (platelet glycoprotein complex, antigen CD41B) IIb of IIb/IIIa |
| 1.2 | 220094_s_at | 63933 | C6orf79 | chromosome 6 open reading frame 79 |
| 1.2 | 223809_at | 64407 | RGS18 | regulator of G-protein signalling 18 |
| 1.2 | 226018_at | 222166 | Ells1 | hypothetical protein Ells1 |
| 1.2 | 204628_s_at | 3690 | ITGB3 | integrin, beta 3 (platelet glycoprotein IIIa, antigen |
| | | | | CD61) |
| 1.2 | 228568_at | 145781 | Gup1 | GRINL1A complex upstream protein |
| 1.2 | 220336_s_at | 51206 | GP6 | glycoprotein VI (platelet) |
| 1.2 | 204069_at | 4211 | MEIS1 | Meis1, myeloid ecotropic viral integration site 1 |
| | | | | homolog (mouse) |
| 1.2 | 31874_at | 10634 | GA52L1 | growth arrest-specific 2 like 1 |
| 1.2 | 220496_at | 51266 | CLEC1B | C-type lectin domain family 1, member B |
| 1.2 | 219860_at | 80741 | LY6G5C | lymphocyte antigen 6 complex, locus G5C |
| 1.2 | 206254_at | 1950 | EGF | epidermal growth factor (beta-urogastrone) |
| 1.2 | 224764_at | 57584 | ARHGAP21 | Rho GTPase activating protein 21 |
| 1.2 | 209839_at | 26052 | DNM3 | dynamin 3 |
| 1.2 | 223777_at | 84771 | MGC13005 | hypothetical protein MGC13005 |
| 1.2 | 214020_x_at | --- | --- | Integrin, beta 5 |
| 1.2 | 240456_at | --- | --- | Hypothetical protein FLJ11795 |
| 1.2 | 230645_at | 257019 | FRMD3 | FERM domain containing 3 |
| 1.2 | 204838_s_at | 27030 | MLH3 | mutL homolog 3 (E. coli) |
| 1.2 | 215071_s_at | --- | --- | histone 1, H2ac |
| 1.2 | 224991_at | 80790 | CMIP | c-Maf-inducing protein |
| 1.2 | 201108_s_at | 7057 | THBS1 | thrombospondin 1 |
| 1.2 | 202555_s_at | 4638 | MYLK | myosin, light polypeptide kinase /// myosin, light |
| | | | | polypeptide kinase |
| 1.2 | 204115_at | 2791 | GNG11 | guanine nucleotide binding protein (G protein), gamma |
| 1.2 | 208527_x_at | 8344 | HIST1H2BE | 11 histone 1, H2be |
| 1.2 | 209911_x_at | 3017 | HIST1H2BD | histone 1, H2bd |
| 1.2 | 214146_s_at | 5473 | PPBP | pro-platelet basic protein (chemokine (C-X-C motif) |
| | | | | ligand 7) |
| 1.2 | 211026_s_at | 11343 | MGLL | monoglyceride lipase /// monoglyceride lipase |
| 1.2 | 200931_s_at | 7414 | VCL | vinculin |
| 1.2 | 48031_r_at | 10826 | C5orf4 | chromosome 5 open reading frame 4 |
| 1.2 | 230833_at | 84519 | ACRBP | acrosin binding protein |
| 1.2 | 37966_at | 29780 | PARVB | parvin, beta |
| 1.2 | 203817_at | 2983 | GUCY1B3 | --- |
| 1.2 | 217963_s_at | 27018 | NGFRAP1 | nerve growth factor receptor (TNFRSF16) associated |
| | | | | protein 1 |
| 1.2 | 216253_s_at | 29780 | PARVB | parvin, beta |
| 1.2 | 206283_s_at | 6886 | TAL1 | T-cell acute lymphocytic leukemia 1 |
| 1.2 | 230942_at | 116173 | CKLFSF5 | chemokine-like factor super family 5 |
| 1.2 | 209301_at | 760 | CA2 | carbonic anhydrase II |
| 1.2 | 205612_at | 22915 | MMRN1 | multimerin 1 |
| 1.2 | 206167_s_at | 395 | ARHGAP6 | Rho GTPase activating protein 6 |
| 1.2 | 218711_s_at | 8436 | SDPR | serum deprivation response (phosphatidylserine |
| | | | | binding protein) |
| 1.2 | 206049_at | 6403 | SELP | selectin P (granule membrane protein 140kDa, |
| | | | | antigen CD62) |
| 1.2 | 208791_at | 1191 | CLU | clusterin (complement lysis inhibitor, SP-40,40, sulfated glycoprotein 2, testosterone-repressed prostate message 2, apolipoprotein J) |
| 1.2 | 210757_x_at | 1601 | DAB2 | disabled homolog 2, mitogen-responsive phosphoprotein (Drosophila) |
| 1.2 | 208579_x_at | 54145 | H2BFS | H2B histone family, member S |
| 1.2 | 226188_at | --- | --- | HSPC159 protein |
| 1.2 | 221027_s_at | 81579 | PLA2G12A | phospholipase A2, group XIIA /// phospholipase A2, |
| | | | | group XIIA |
| 1.2 | 204993_at | 2781 | GNAZ | guanine nucleotide binding protein (G protein), alpha z |
| | | | | polypeptide |
| 1.2 | 242323_at | --- | --- | Full-length cDNA clone CS0DF027YF17 of Fetal brain |
| | | | | of Homo sapiens (human) |
| 1.2 | 203414_at | 23531 | MMD | monocyte to macrophage differentiation-associated |
| 1.2 | 241133_at | --- | --- | T cell receptor beta chain (TCRB) mRNA, TCRB-BV27 |
| | | | | J2-4 BC2 allele |
| 1.2 | 216956_s_at | 3674 | ITGA2B | integrin, alpha 2b (platelet glycoprotein IIb of IIb/IIIa |
| | | | | complex, antigen CD41B) |
| 1.2 | 218243_at | 80230 | RUFY1 | RUN and FYVE domain containing 1 |
| 1.2 | 207206_s_at | 239 | ALOX12 | arachidonate 12-lipoxygenase |
| 1.2 | 208546_x_at | 8345 | HIST1H2BH | histone 1, H2bh |
| 1.2 | 214974_x_at | 6374 | CXCL5 | chemokine (C-X-C motif) ligand 5 |
| 1.2 | 230574_at | 388480 | LOC388480 | --- |
| 1.2 | 200665_s_at | 6678 | SPARC | secreted protein, acidic, cysteine-rich (osteonectin) /// |
| | | | | secreted protein, acidic, cysteine-rich (osteonectin) |
| 1.2 | 212813_at | 83700 | JAM3 | junctional adhesion molecule 3 |
| 1.2 | 223717_s_at | 84519 | ACRBP | acrosin binding protein |
| 1.2 | 231181_at | --- | --- | Transcribed locus |
| 1.2 | 208501_at | --- | --- | growth factor independent 1B (potential regulator of |
| | | | | CDKN1A, translocated in CML) |
| 1.2 | 230740_at | 30845 | EHD3 | EH-domaₗn containing 3 |
| 1.2 | 201278_at | 1601 | DAB2 | Disabled homolog 2, mitogen-responsive phosphoprotein (Drosophila) |
| 1.2 | 201121_s_at | 10857 | PGRMC1 | progesterone receptor membrane component 1 |
| 1.2 | 203585_at | 7739 | ZNF185 | zinc finger protein 185 (LIM domain) |
| 1.2 | 34408_at | 6253 | RTN2 | reticulon 2 |
| 1.2 | 210387_at | 8339 | HIST1H2BG | histone 1, H2bg |
| 1.2 | 228618_at | 375033 | FLJ00193 | FLJ00193 protein |
| 1.2 | 209586_s_at | 58497 | PRUNE | prune homolog (Drosophila) |
| 1.2 | 221555_x_at | 8555 | CDC14B | CDC14 cell division cycle 14 homolog B (S. cerevisiae) |
| 1.2 | 225775_at | 340348 | MGC50844 | hypothetical protein MGC50844 |
| 1.2 | 214525_x_at | 27030 | MLH3 | mutL homolog 3 (E. coli) |
| 1.2 | 210986_s_at | 7168 | TPM1 | tropomyosin 1 (alpha) |
| 1.2 | 208792_s_at | 1191 | CLU | clusterin (complement lysis inhibitor, SP-40,40, |
| | | | | sulfated glycoprotein 2, testosterone-repressed |
| | | | | prostate message 2, apolipoprotein J) |
| 1.2 | 216323_x_at | --- | --- | alpha-tubulin isotype H2-alpha |
| 1.2 | 336_at | 6915 | TBXA2R | thromboxane A2 receptor |
| 1.2 | 204081_at | 4900 | NRGN | neurogranin (protein kinase C substrate, RC3) |
| 1.2 | 206655_s_at | 2812 | GP1BB | glycoprotein Ib (platelet), beta polypeptide |
| 1.2 | 215240_at | 3690 | ITGB3 | --- |
| 1.2 | 212573_at | 23052 | KIAA0830 | KIAA0830 protein |
| 1.2 | 238669_at | 5742 | PTGS1 | prostaglandin-endoperoxide synthase 1 (prostaglandin |
| | | | | G/H synthase and cyclooxygenase) |
| 1.2 | 229530_at | --- | --- | Transcribed locus |
| 1.2 | 201120_s_at | 10857 | PGRMC1 | progesterone receptor membrane component 1 |
| 1.2 | 206390_x_at | 5196 | PF4 | platelet factor 4 (chemokine (C-X-C motif) ligand 4) |
| 1.2 4 | 214039_s_at | 55353 | LAPTM4B | lysosomal associated protein transmembrane beta |
| 1.2 | 218935_at | 30845 | EHD3 | EH-domain containing 3 |
| 1.2 | 210987_x_at | 7168 | TPM1 | tropomyosin 1 (alpha) |
| 1.2 | 203680_at | 5577 | PRKAR2B | protein kinase, cAMP-dependent, regulatory, type II, |
| | | | | beta |
| 1.2 | 207808_s_at | 5627 | PROS1 | protein S (alpha) |
| 1.2 | 206698_at | 7504 | XK | Kell blood group precursor (McLeod phenotype) |
| 1.2 | 206110_at | 8357 | HIST1H3H | histone 1, H3h |
| 1.2 | 222717_at (phosphatidylserine | 8436 | SDPR | serum deprivation response |
| | | | | binding protein) |
| 1.2 | 230690_at | 81027 | TUBB1 | tubulin, beta 1 |
| 1.2 | 244523_at | 23531 | MMD | monocyte to macrophage differentiation-associated |
| 1.2 | 209806_at | 85236 | HIST1H2BK | histone 1, H2bk |
| 1.2 | 224823_at | 4638 | MYLK | myosin, light polypeptide kinase |
| 1.2 | 203305_at | 2162 | F13A1 | coagulation factor XIII, A1 polypeptide |
| 1.2 | 202708_s_at | 8349 | HIST2H2BE | histone 2, H2be |
| 1.2 | 204627_s_at antigen | 3690 | ITGB3 | integrin, beta 3 (platelet glycoprotein IIIa, |
| | | | | CD61) |
| 1.2 | 221556_at | 8555 | CDC14B | CDC14 cell division cycle 14 homolog B (S. cerevisiae) |
| 1.2 | 201280_s_at | 1601 | DAB2 | disabled homolog 2, mitogen-responsive phosphoprotein (Drosophila) |
| 1.2 | 205127_at | 5742 | PTGS1 | prostaglandin-endoperoxide synthase 1 (prostaglandin |
| | | | | G/H synthase and cyclooxygenase) |
| 1.2 | 201059_at | --- | --- | --- |
| 1.2 | 221211_s_at | 56911 | C21orf7 | chromosome 21 open reading frame 7 |
| 1.2 | 230535_s_at | --- | --- | Tubulin, beta 1 |
| 1.2 | 228195_at | 84281 | MGC13057 | Hypothetical protein MGC13057 |
| 1.2 | 208690_s_at | 9124 | PDLIM1 | PDZ and LIM domain 1 (elfin) |
| 1.3 | 213674_x_at | 3495 | IGHD | immunoglobulin heavy constant delta |
| 1.3 | 226122_at | 57480 | PLEKHG1 | pleckstrin homology domain containing, family G (with |
| | | | | RhoGef domain) member 1 |
| 1.3 | 44790_s_at | 80183 | C13orf18 | chromosome 13 open reading frame 18 |
| 1.3 | 226912_at | 254887 | ZDHHC23 | zinc finger, DHHC domain containing 23 |
| 1.3 | 226878_at | 3111 | HLA-DOA | major histocompatibility complex, class II, DO alpha |
| 1.3 | 228599_at | 931 | MS4A1 | Membrane-spanning 4-domains, subfamily A, member |
| 1.3 | 213891_s_at | 6925 | TCF4 | Transcription factor 4 |
| 1.3 | 239287_at | --- | --- | Transcribed locus, moderately similar to XP_370630.2 |
| | | | | protein phosphatase 1, regulatory (inhibitor) subunit |
| | | | | 14B [Homo sapiens] |
| 1.3 | 226150_at | 84513 | HTPAP | HTPAP protein |
| 1.3 | 218781_at | 79677 | SMC6L1 | SMC6 structural maintenance of chromosomes 6-like |
| | | | | 1 (yeast) |
| 1.3 | 212827_at | 3507 | IGHM | immunoglobulin heavy constant mu /// immunoglobulin heavy constant mu |
| 1.3 | 225081_s_at | 55536 | RAM2 | transcription factor RAM2 |
| 1.3 | 210356_x_at | 931 | MS4A1 | membrane-spanning 4-domains, subfamily A, member |
| 1.3 | 206896_s_at | 2788 | GNG7 | guanine nucleotide binding protein (G protein), gamma |
| | | | | 7 |
| 1.3 | 210448_s_at 5 | 5026 | P2RX5 | purinergic receptor P2X, ligand-gated ion channel, |
| 1.3 | 213772_s_at | 23062 | GGA2 | golgi associated, gamma adaptin ear containing, ARF |
| | | | | binding protein 2 |
| 1.3 | 243968_x_at | 115350 | FCRH1 | Fc receptor-like 1 |
| 1.3 | 212386_at | 6925 | TCF4 | Transcription factor 4 |
| 1.3 | 219667_s_at | 55024 | BANK1 | B-cell scaffold protein with ankyrin repeats 1 |
| 1.3 | 230648_at | 283663 | LOC283663 | hypothetical protein LOC283663 |
| 1.3 | 235372_at | 84824 | FREB | Fc receptor homolog expressed in B cells |
| 1.3 | 202759_s_at | 445815 | PALM2-AKAP2 | PALM2-AKAP2 protein |
| 1.3 | 39318_at | 8115 | TCL1A | T-cell leukemia/lymphoma 1A |
| 1.3 | 219073_s_at | 114884 | OSBPL10 | oxysterol binding protein-like 10 |
| 1.3 | 206255_at | 640 | BLK | B lymphoid tyrosine kinase |
| 1.3 | 227198_at | 3899 | LAF4 | Lymphoid nuclear protein related to AF4 |
| 1.3 | 203642_s_at | 22837 | COBLL1 | COBL-like 1 |
| 1.3 | 219497_s_at | 53335 | BCL11A | B-cell CLL/lymphoma 11A (zinc finger protein) |
| 1.3 | 230245_s_at | 283663 | LOC283663 | hypothetical protein LOC283663 |
| 1.3 | 228592_at | 931 | MS4A1 | Membrane-spanning 4-domains, subfamily A, member |
| 1.3 | 226550 at | --- | --- | --- |
| 1.3 x | 228831_s_at | 2788 | GNG7 | guanine nucleotide binding protein (G member gamma |
| | | | | 7 |
| 1.3 | 215925_s_at | 971 | CD72 | CD72 antigen |
| 1.3 | 204004_at | 5074 | PAWR | PRKC, apoptosis, WT1, regulator |
| 1.3 | 226384_at | 84513 | HTPAP | HTPAP protein |
| 1.3 | 206398_s_at | 930 | CD19 | CD19 antigen |
| 1.3 | 210889_s_at | 2213 | FCGR2B | Fc fragment of IgG, low affinity IIb, receptor (CD32) |
| 1.3 | 219471_at | 80183 | C13orf18 | chromosome 13 open reading frame 18 |
| 1.3 | 219498_s_at | 53335 | BCL11A | B-cell CLL/lymphoma 11A (zinc finger protein) |
| 1.3 | 202732_at | 11142 | PKIG | protein kinase (cAMP-dependent, catalytic) inhibitor |
| | | | | gamma |
| 1.3 | 220068_at | 29802 | VPREB3 | pre-B lymphocyte gene 3 |
| 1.3 | 205671_s_at | 3112 | HLA-DOB | major histocompatibility complex, class II, DO beta |
| 1.3 | 206759_at | 2208 | FCER2 | Fc fragment of IgE, low affinity II, receptor for (CD23A) |
| 1.3 | 238071_at | 158062 | LCN6 | lipocalin 6 |
| 1.3 | 223569_at | 84513 | HTPAP | HTPAP protein |
| 1.3 | 209995_s_at | 8115 | TCL1A | T-cell leukemia/lymphoma 1A /// T-cell leukemia/lymphoma 1A |
| 1.3 | 230877_at | 3495 | IGHD | immunoglobulin heavy constant delta |
| 1.3 | 206126_at | 643 | BLR1 | Burkitt lymphoma receptor 1, GTP binding (chemokine (C-X-C motif) receptor 5) protein |
| 1.3 | 235401_s_at | 84824 | FREB | Fc receptor homolog expressed in B cells |
| 1.3 | 202431_s_at | 4609 | MYC | v-myc myelocytomatosis viral oncogene homolog |
| 1.3 | 221239_s_at | 79368 | SPAP1 | (avian) Fc receptor-like 2 /// Fc receptor-like 2 |
| 1.3 | 220059_at | 26228 | BRDG1 | BCR downstream signaling 1 |
| 1.3 | 235982_at | 115350 | FCRH1 | Fc receptor-like 1 |
| 1.3 | 209583_s_at | 4345 | CD200 | CD200 antigen |
| 1.3 | 227533_at | --- | --- | Transcribed locus |
| 1.3 | 230509_at | 79856 | SNX22 | sorting nexin 22 |
| 1.3 | 221969_at | 5079 | PAX5 | Paired box gene 5 (B-cell lineage specific activator) |
| 1.3 | 207777_s_at | 11262 | SP140 | SP140 nuclear body protein |
| 1.3 | 205049_s_at | 973 | CD79A | CD79A antigen (immunoglobulin-associated alpha) /// |
| | | | | CD79A antigen (immunoglobulin-associated alpha) |
| 1.3 | 224735_at | 220002 | CYBASC3 | cytochrome b, ascorbate dependent 3 |
| 1.3 | 204581_at | 933 | CD22 | CD22 antigen |
| 1.3 | 223245_at | 55342 | STRBP | spermatid perinuclear RNA binding protein |
| 1.3 | 243780_at | --- | --- | CDNA FLJ46553 fis, clone THYMU3038879 |
| 1.3 | 205861_at | 6689 | SPIB | Spi-B transcription factor (Spi-1/PU.1 related) /// Spi- |
| | | | | B transcription factor (Spi-1/PU.1 related) |
| 1.3 | 230983_at | 199786 | BCNP1 | B-cell novel protein 1 |
| 1.3 | 38521_at | 933 | CD22 | CD22 antigen |
| 1.3 | 217418_x_at | 931 | MS4A1 | membrane-spanning 4-domains, subfamily A, member |
| 1.3 | 222915_s_at | 55024 | BANK1 | B-cell scaffold protein with ankyrin repeats 1 |
| 1.3 | 201689_s_at | 7163 | TPD52 | tumor protein D52 |
| 1.3 | 205297_s_at | 974 | CD79B | CD79B antigen (immunoglobulin-associated beta) |
| 1.3 | 227224_at | 55103 | RALGPS2 | Ral GEF with PH domain and SH3 binding motif 2 |
| 1.3 | 207655_s_at | 29760 | BLNK | B-cell linker |
| 1.3 | 217979_at | 27075 | TM4SF13 | tetraspanin 13 |
| 1.3 | 206478_at | 9834 | KIAA0125 | KIAA0125 |
| 1.3 | 222891_s_at | 53335 | BCL11A | B-cell CLL/lymphoma 11A (zinc finger protein) |
| 1.3 | 204208_at | 8732 | RNGTT | RNA guanylyltransferase and 5'-phosphatase |
| 1.3 | 238376_at | 23274 | KIAA0350 | KIAA0350 protein |
| 1.3 | 203221_at | 7088 | TLE1 | transducin-like enhancer of split 1 (E(sp1) homolog, |
| | | | | Drosophila) |
| 1.3 | 227646_at | 1879 | EBF | Early B-cell factor |
| 1.3 | 229513_at | 55342 | STRBP | Spermatid perinuclear RNA binding protein |
| 1.4 | 212430_at | 55544 | RNPC1 | RNA-binding region (RNP1, RRM) containing 1 /// |
| | | | | RNA-binding region (RNP1, RRM) containing 1 |
| 1.4 | 232044_at | 5930 | RBBP6 | retinoblastoma binding protein 6 |
| 1.4 | 212434_at | 80273 | GRPEL1 | GrpE-like 1, mitochondrial (E. coli) |
| 1.4 | 218319_at | 57162 | PELI1 | pellino homolog 1 (Drosophila) |
| 1.4 | 208632_at | 9921 | RNF10 | ring finger protein 10 /// ring finger protein 10 |
| 1.4 | 226650_at | 90637 | LOC90637 | hypothetical protein LOC90637 |
| 1.4 | 238633_at | 80314 | EPC1 | Enhancer of polycomb homolog 1 (Drosophila) |
| 1.4 | 233506_at | --- | --- | Full length insert cDNA clone ZB81B12 |
| 1.4 | 201745_at | 5756 | PTK9 | PTK9 protein tyrosine kinase 9 |
| 1.4 | 218399_s_at | 55038 | CDCA4 | cell division cycle associated 4 |
| 1.4 | 202861_at | 5187 | PER1 | period homolog 1 (Drosophila) |
| 1.4 | 215948_x_at | 9205 | ZNF237 | zinc finger protein 237 |
| 1.4 | 212665_at | 25976 | TIPARP | TCDD-inducible poly(ADP-ribose) polymerase |
| 1.4 | 220046_s_at | 57018 | CCNL1 | cyclin L1 |
| 1.4 | 218012_at | 64061 | TSPYL2 | TSPY-like 2 |
| 1.4 | 212241_at | 81488 | GRINL1A | glutamate receptor, ionotropic, N-methyl D-aspartate- |
| | | | | like 1A |
| 1.4 | 203321_s_at | 22850 | KIAA0863 | KIAA0863 protein |
| 1.4 | 36711_at | 23764 | MAFF | v-maf musculoaponeurotic fibrosarcoma oncogene |
| | | | | homolog F (avian) |
| 1.4 | 225199_at | 29035 | PRO0149 | PRO0149 protein |
| 1.4 | 200731_s_at | 7803 | PTP4A1 | protein tyrosine phosphatase type IVA, member 1 |
| 1.4 | 200870_at | 11171 | STRAP | serine/threonine kinase receptor associated protein |
| 1.4 | 227718_at | 5814 | PURB | purine-rich element binding protein B |
| 1.4 | 203708_at | 5142 | PDE4B | phosphodiesterase 4B, cAMP-specific (phosphodiesterase E4 dunce homolog, Drosophila) |
| 1.4 | 202776_at | 30836 | ERBP | estrogen receptor binding protein |
| 1.4 | 202558_s_at | 6782 | STCH | stress 70 protein chaperone, microsome-associated, |
| | | | | 60kDa |
| 1.4 | 205548_s_at | 10950 | BTG3 | BTG family, member 3 |
| 1.4 | 207332_s_at | 7037 | TFRC | transferrin receptor (p90, CD71) |
| 1.4 | 221821_s_at | 54934 | FLJ20436 | hypothetical protein FLJ20436 |
| 1.4 | 210836_x_at | 5144 | PDE4D | phosphodiesterase 4D, cAMP-specific (phosphodiesterase E3 dunce homolog, Drosophila) |
| 1.4 | 201751_at | 9929 | KIAA0063 | KIAA0063 gene product |
| 1.4 | 242356_at | 143187 | VTI1A | Vesicle transport through interaction with t-SNAREs |
| | | | | homolog 1A (yeast) |
| 1.4 | 225951_s_at | 440309 | --- | LOC440309 |
| 1.4 | 208078_s_at | 150094 | SNF1LK | SNF1-like kinase /// SNF1-like kinase |
| 1.4 | 206374_at | 1850 | DUSP8 | dual specificity phosphatase 8 |
| 1.4 | 208720_s_at | 9584 | RNPC2 | RNA-binding region (RNP1, RRM) containing 2 |
| 1.4 | 229106_at | 140735 | Dlc2 | dynein light chain 2 |
| 1.4 | 230134_s_at | 54542 | MNAB | membrane associated DNA binding protein |
| 1.4 | 205214_at | 9262 | STK17B | serine/threonine kinase 17b (apoptosis-inducing) |
| 1.4 | 202643_s_at 3 | 7128 | TNFAIP3 | tumor necrosis factor, alpha-induced protein |
| 1.4 | 227110_at | 3183 | HNRPC | heterogeneous nuclear ribonucleoprotein C (C1/C2) |
| 1.4 | 228962_at | 5144 | PDE4D | Phosphodiesterase 4D, cAMP-specific (phosphodiesterase E3 dunce homolog, Drosophila) |
| 1.4 | 226370_at | 80311 | KLHL15 | kelch-like 15 (Drosophila) |
| 1.4 | 203322_at | 22850 | KIAA0863 | KIAA0863 protein |
| 1.4 | 216834_at | 5996 | RGS1 | --- |
| 1.4 | 200779_at | 468 | ATF4 | activating transcription factor 4 (tax-responsive |
| | | | | enhancer element B67) |
| 1.4 | 242676_at | --- | --- | --- |
| 1.4 | 208622 s at | 7430 | VIL2 | villin 2 (ezrin) |
| 1.4 | 215722_s_at | 6627 | SNRPA1 | small nuclear ribonucleoprotein polypeptide A' |
| 1.4 | 210281_s_at | 7750 | ZNF198 | zinc finger protein 198 |
| 1.4 | 207630_s_at | 1390 | CREM | cAMP responsive element modulator |
| 1.4 | 201356_at | 10291 | SF3A1 | splicing factor 3a, subunit 1, 120kDa |
| 1.4 | 204440_at | 9308 | CD83 | CD83 antigen (activated B lymphocytes, immunoglobulin superfamily) |
| 1.4 | 222044_at | 63935 | c20orf67 | --- |
| 1.4 | 208810_at | 10049 | DNAJB6 | DnaJ (Hsp40) homolog, subfamily B, member 6 |
| 1.4 | 209020_at | 51526 | C20orf111 | chromosome 20 open reading frame 111 |
| 1.4 | 224454_at | 55500 | ETNK1 | ethanolamine kinase 1 /// ethanolamine kinase 1 |
| 1.4 | 210054_at | 79441 | C4orf15 | chromosome 4 open reading frame 15 |
| 1.4 | 209510_at | 11236 | RNF139 | ring finger protein 139 |
| 1.4 | 218496_at | 246243 | RNASEH1 | ribonuclease H1 |
| 1.4 | 203752_s_at | 3727 | JUND | jun D proto-oncogene |
| 1.4 | 200719_at | 6500 | SKP1A | S-phase kinase-associated protein 1A (p19A) |
| 1.4 | 241924_at | 4204 | MECP2 | Methyl CpG binding protein 2 (Rett syndrome) |
| 1.4 | 210110_x_at | 3189 | HNRPH3 | heterogeneous nuclear ribonucleoprotein H3 (2H9) |
| 1.4 | 213546_at | 222161 | DKFZp586I1420 | hypothetical protein DKFZp586I1420 |
| 1.4 | 228062_at | 266812 | NAP1L5 | nucleosome assembly protein 1-like 5 |
| 1.4 | 208840_s_at | 9908 | G3BP2 | Ras-GTPase activating protein SH3 domain- |
| | | | | 2 protein binding |
| 1.4 | 227932_at | 10425 | ARIH2 | --- |
| 1.4 | 233127_at | 55422 | ZNF331 | Zinc finger protein 331 |
| 1.4 | 238509_at | 8454 | CUL1 | Cullin 1 |
| 1.4 | 222669_s_at | 51119 | SBDS | Shwachman-Bodian-Diamond syndrome |
| 1.4 | 211423_s_at | 6309 | SC5DL | sterol-C5-desaturase (ERG3 delta-5-desaturase |
| | | | | homolog, fungal)-like |
| 1.4 | 205281_s_at | 5277 | PIGA | phosphatidylinositol glycan, class A (paroxysmal |
| | | | | nocturnal hemoglobinuria) /// phosphatidylinositol |
| | | | | glycan, class A (paroxysmal nocturnal hemoglobinuria) |
| 1.4 | 208881_x_at | 3422 | IDI1 | isopentenyl-diphosphate delta isomerase |
| 1.4 | 222045_s_at | 63935 | C20orf67 | chromosome 20 open reading frame 67 |
| 1.4 | 226206_at | 7975 | MAFK | v-maf musculoaponeurotic fibrosarcoma oncogene |
| | | | | homolog K (avian) |
| 1.4 | 231182_at | 7456 | WASPIP | Wiskott-Aldrich syndrome protein interacting protein |
| 1.4 | 220239_at | 55975 | KLHL7 | kelch-like 7 (Drosophila) |
| 1.4 | 202644_s_at | 7128 | TNFAIP3 | tumor necrosis factor, alpha-induced protein 3 |
| 1.4 | 65588_at | 388796 | LOC388796 | hypothetical LOC388796 |
| 1.4 | 219094_at | 25852 | ARMC8 | armadillo repeat containing 8 |
| 1.4 | 230133_at | 54542 | MNAB | Membrane associated DNA binding protein |
| 1.4 | 227613_at | 55422 | ZNF331 | zinc finger protein 331 |
| 1.4 | 209967_s_at | 1390 | CREM | cAMP responsive element modulator |
| 1.4 | 202021_x_at | 10209 | SUI1 | putative translation initiation factor |
| 1.4 | 208811_s_at | 10049 | DNAJB6 | DnaJ (Hsp40) homolog, subfamily B, member 6 |
| 1.4 | 204622_x_at | 4929 | NR4A2 | nuclear receptor subfamily 4, group A, member 2 |
| 1.4 | 60084_at | 1540 | CYLD | cylindromatosis (turban tumor syndrome) |
| 1.4 | 216248_s_at | 4929 | NR4A2 | nuclear receptor subfamily 4, group A, member 2 |
| 1.4 | 209694_at | 5805 | PTS | 6-pyruvoyltetrahydropterin synthase |
| 1.4 | 204244_s_at | 10926 | ASK | activator of S phase kinase |
| 1.4 | 221727_at 4 | 10923 | PC4 | activated RNA polymerase II transcription cofactor |
| | | | | /// similar to Activated RNA polymerase II transcriptional coactivator p15 (Positive cofactor 4) |
| | | | | (PC4) (p14) |
| 1.4 | 225857_s_at | 388796 | RNU71A | Hypothetical LOC388796 |
| 1.4 | 241985_at | 133746 | JMY | junction-mediating and regulatory protein |
| 1.4 | 229718_at | 90634 | CG018 | Hypothetical gene CG018 |
| 1.4 | 212130_x_at | 10209 | SUI1 | putative translation initiation factor |
| 1.4 | 209457_at | 1847 | DUSP5 | dual specificity phosphatase 5 |
| 1.4 | 228284_at | 7088 | TLE1 | Transducin-like enhancer of split 1 (E(sp1) homolog, |
| | | | | Drosophila) |
| 1.4 | 223296_at | 84275 | MGC4399 | mitochondrial carrier protein |
| 1.4 | 231085_s_at | --- | --- | --- |
| 1.4 | 200732_s_at | 7803 | PTP4A1 | protein tyrosine phosphatase type IVA, member 1 |
| 1.4 | 219228_at | 55422 | ZNF331 | zinc finger protein 331 |
| 1.4 | 230304_at | --- | --- | CDNA clone IMAGE:30332316, partial cds |
| 1.4 | 208931_s_at | 3609 | ILF3 | interleukin enhancer binding factor 3, 90kDa |
| 1.4 | 228955_at | --- | --- | Homo sapiens, clone IMAGE:4753714, mRNA |
| 1.4 | 213538_at | 6651 | SON | SON DNA binding protein |
| 1.4 | 239678_at | 11276 | AP1GBP1 | AP1 gamma subunit binding protein 1 |
| 1.4 | 214714_at | 84124 | ZNF394 | zinc finger protein 394 |
| 1.4 | 89948_at | 63935 | C20orf67 | --- |
| 1.4 | 208707_at | 1983 | EIF5 | eukaryotic translation initiation factor 5 |
| 1.4 | 228381_at | 80063 | ATF7IP2 | Activating transcription factor 7 interacting protein 2 |
| 1.4 | 228857_at | 285831 | LOC285831 | hypothetical protein LOC285831 |
| 1.4 | 226914_at | 81873 | ARPC5L | actin related protein 2/3 complex, subunit 5-like |
| 1.4 | 225493_at | 144438 | LOC144438 | hypothetical protein LOC144438 |
| 1.4 | 212227_x_at | 10209 | SUI1 | putative translation initiation factor |
| 1.4 | 211999_at | 3021 | H3F3B | H3 histone, family 3B (H3.3B) |
| 1.4 | 223584_s_at | 25948 | KBTBD2 | kelch repeat and BTB (POZ) domain containing 2 |
| 1.4 | 229428_at | --- | --- | CDNA FLJ40725 fis, clone TKIDN1000001, highly |
| | | | | similar to Translocase of inner mitochondrial |
| | | | | membrane 23 |
| 1.4 | 222142_at | 1540 | CYLD | cylindromatosis (turban tumor syndrome) |
| 1.4 | 210837_s_at | 5144 | PDE4D | phosphodiesterase 4D, cAMP-specific (phosphodiesterase E3 dunce homolog, Drosophila) |
| 1.4 | 227650_at | 51182 | HSPA14 | heat shock 70kDa protein 14 |
| 1.4 | 218936_s_at | 29080 | HSPC128 | HSPC128 protein |
| 1.4 | 202684_s_at | 8731 | RNMT | RNA (guanine-7-) methyltransferase |
| 1.4 | 206342_x_at | 3423 | IDS | iduronate 2-sulfatase (Hunter syndrome) |
| 1.4 | 224783_at | 283991 | MGC29814 | hypothetical protein MGC29814 |
| 1.4 | 226858_at | 1454 | CSNK1E | Casein kinase 1, epsilon |
| 1.4 | 41577_at | 26051 | PPP1R16B | protein phosphatase 1, regulatory (inhibitor) subunit |
| | | | | 16B |
| 1.4 | 204615_x_at | 3422 | IDI1 | isopentenyl-diphosphate delta isomerase |
| 1.4 | 223598_at | 5887 | RAD23B | RAD23 homolog B (S. cerevisiae) |
| 1.4 | 204491_at | 5144 | PDE4D | Phosphodiesterase 4D, cAMP-specific (phosphodiesterase E3 dunce homolog, Drosophila) |
| 1.4 | 233952_s_at | 49854 | ZNF295 | zinc finger protein 295 |
| 1.4 | 219016_at | 60493 | FLJ13149 | hypothetical protein FLJ13149 |
| 1.4 | 213134_x_at | 10950 | BTG3 | BTG family, member 3 |
| 1.5 | 208018_s_at | 3055 | HCK | hemopoietic cell kinase |
| 1.5 | 205382_s_at | 1675 | DF | D component of complement (adipsin) |
| 1.5 | 226728_at | 376497 | SLC27A1 | solute carrier family 27 (fatty acid transporter), |
| | | | | member 1 |
| 1.5 | 209901_x_at | 199 | AIF1 | allograft inflammatory factor 1 |
| 1.5 | 205859_at | 9450 | LY86 | lymphocyte antigen 86 |
| 1.5 | 219593_at | 51296 | SLC15A3 | solute carrier family 15, member 3 |
| 1.5 | 201743_at | 929 | CD14 | CD14 antigen /// CD14 antigen |
| 1.5 | 202943_s_at | 4668 | NAGA | N-acetylgalactosaminidase, alpha- |
| 1.5 | 201995_at | 2131 | EXT1 | exostoses (multiple) 1 |
| 1.5 | 207697_x_at | 10288 | LILRB2 | leukocyte immunoglobulin-like receptor, subfamily B |
| | | | | (with TM and ITIM domains), member 2 |
| 1.5 | 218473_s_at | 79709 | GLT25D1 | glycosyltransferase 25 domain containing 1 |
| 1.5 | 210146_x_at | 10288 /// 79168 | LILRB2 /// LILRB6 | leukocyte immunoglobulin-like receptor, subfamily B |
| | | | | (with TM and ITIM domains), member 2 |
| 1.5 | 209499_x_at | 407977 /// 8741 | TNFSF13 /// | tumor necrosis factor (ligand) superfamily, member 13 |
| | | | TNFSF12-TNFSF13 | /// tumor necrosis factor (ligand) |
| | | | | member 12-member 13 superfamily, |
| 1.5 | 207104_x_at | 10859 | LILRB1 | leukocyte immunoglobulin-like receptor, subfamily B |
| | | | | (with TM and ITIM domains), member 1 |
| 1.5 | 205789_at | 912 | CD1D | CD1D antigen, d polypeptide /// CD1D antigen, d |
| | | | | polypeptide |
| 1.5 | 202878_s_at | 22918 | C1QR1 | complement component 1, q subcomponent, receptor |
| 1.5 | 223405_at | 80896 | NPL | N-acetylneuraminate pyruvate lyase (dihydrodipicolinate synthase) |
| 1.5 | 215633_x_at | 7940 | LST1 | leukocyte specific transcript 1 |
| 1.5 x | 223501_at | 10673 | TNFSF13B | tumor necrosis factor (ligand) superfamily, receptor 13b |
| 1.5 | 204647_at | 9454 | HOMER3 | homer homolog 3 (Drosophila) |
| 1.5 | 210580_x_at | 6818 | SULT1A3 | sulfotransferase family, cytosolic, 1A, phenol- |
| | | | | preferring, member 3 |
| 1.5 | 200678_x_at | 2896 | GRN | granulin |
| 1.5 | 203501_at | 10404 | PGCP | plasma glutamate carboxypeptidase |
| 1.5 | 215051_x_at | 199 | AIF1 | allograft inflammatory factor 1 |
| 1.5 | 38487_at | 23166 | STAB1 | stabilin 1 |
| 1.5 | 200743_s_at | 1200 | TPP1 | tripeptidyl peptidase I |
| 1.5 | 217388_s_at | 8942 | KYNU | kynureninase (L-kynurenine hydrolase) |
| 1.5 | 211284_s_at | 2896 | GRN | granulin |
| 1.5 | 204924_at | 7097 | TLR2 | toll-like receptor 2 |
| 1.5 | 212737_at | 2760 | GM2A | GM2 ganglioside activator |
| 1.5 | 240095_at | --- | --- | Transcribed locus |
| 1.5 | 208982_at | 5175 | PECAM1 | Platelet/endothelial cell adhesion molecule (CD31antigen) |
| 1.5 | 201506_at | 7045 | TGFBI | transforming growth factor, beta-induced, 68kDa |
| 1.5 | 239135_at | 55313 | FLJ11151 | Hypothetical protein FLJ11151 |
| 1.5 | 211582_x_at | 7940 | LST1 | leukocyte specific transcript 1 |
| 1.5 | 203063_at | 9647 | PPM1F | protein phosphatase 1F (PP2C domain containing) |
| 1.5 | 200788_s_at | 8682 | PFA15 | phosphoprotein enriched in astrocytes 15 |
| 1.5 | 205686_s_at | 942 | CD86 | CD86 antigen (CD28 antigen ligand 2, B7-2 antigen) |
| 1.5 | 207610_s_at | 30817 | EMR2 | egf-like module containing, mucin-like, hormone |
| | | | | receptor-like 2 |
| 1.5 | 212334_at | 2799 | GNS | glucosamine (N-acetyl)-6-sulfatase (Sanfilippo |
| | | | | disease IIID) |
| 1.5 | 215049_x_at | 9332 | CD163 | CD163 antigen |
| 1.5 | 208146_s_at | 54504 | CPVL | carboxypeptidase, vitellogenic-like /// carboxypeptidase, vitellogenic-like |
| 1.5 | 213503_x_at | 302 | ANXA2 | annexin A2 |
| 1.5 | 202812_at | 2548 | GAA | glucosidase, alpha; acid (Pompe disease, glycogen storage disease type II) |
| 1.5 | 212993_at | --- | --- | MRNA; cDNA DKFZp667B1718 (from clone |
| 1.5 | 214746_s_at | 168544 | ZNF467 | zinc finger protein 467 |
| 1.5 | 217118_s_at | 23313 | C22orf9 | chromosome 22 open reading frame 9 |
| 1.5 | 212820_at | 23312 | DMXL2 | Dmx-like 2 |
| 1.5 | 58780_s_at | 55701 | FLJ10357 | hypothetical protein FLJ10357 |
| 1.5 | 204619_s_at | 1462 | CSPG2 | chondroitin sulfate proteoglycan 2 (versican) |
| 1.5 | 218240_at | 28511 | NKIRAS2 | NFKB inhibitor interacting Ras-like 2 |
| 1.5 | 201536_at | 1845 | DUSP3 | dual specificity phosphatase 3 (vaccinia phosphatase VH1-related) virus |
| 1.5 | 202795_x_at | 11078 | HRIHFB2122 | Tara-like protein |
| 1.5 | 200714_x_at | 10956 | OS-9 | amplified in osteosarcoma |
| 1.5 | 205076_s_at | 10903 | CRA | myotubularin related protein 11 |
| 1.5 | 236297_at | 84898 | PLXDC2 | Plexin domain containing 2 |
| 1.5 | 203561_at | 2212 | FCGR2A | Fc fragment of IgG, low affinity IIa, |
| | (CD32) | | | receptor |
| 1.5 | 200660_at | 6282 | S100A11 | S100 calcium binding protein All (calgizzarin) |
| 1.5 3 | 212681_at | 23136 | EPB41L3 | erythrocyte membrane protein band 4.1-like |
| 1.5 | 208890_s_at | 23654 | PLXNB2 | plexin B2 |
| 1.5 | 202944_at | 4668 | NAGA | N-acetylgalactosaminidase, alpha- |
| 1.5 | 210784_x_at B | 11025 | LILRB3 | leukocyte immunoglobulin-like receptor, subfamily |
| | | | | (with TM and ITIM domains), member 2 /// leukocyte |
| | | | | immunoglobulin-like receptor, subfamily B (with TM |
| | | | | and ITIM domains), member 3 |
| 1.5 | 203773_x_at | 644 | BLVRA | biliverdin reductase A |
| 1.5 | 204169_at | 3614 | IMPDH1 | IMP (inosine monophosphate) dehydrogenase 1 |
| 1.5 | 35820_at | 2760 | GM2A | GM2 ganglioside activator |
| 1.5 | 200838_at | 1508 | CTSB | cathepsin B |
| 1.5 | 204971_at | 1475 | CSTA | cystatin A (stefin A) |
| 1.5 | 206743_s_at | 432 | ASGR1 | asialoglycoprotein receptor 1 |
| 1.5 | 206420_at | 10261 | IGSF6 | immunoglobulin superfamily, member 6 |
| 1.5 | 201360_at | 1471 | CST3 | cystatin C (amyloid angiopathy and cerebral hemorrhage) |
| 1.5 | 208248_x_at | 334 | APLP2 | amyloid beta (A4) precursor-like protein 2 |
| 1.5 | 213095_x_at | 199 | AIF1 | allograft inflammatory factor 1 |
| 1.5 | 221802_s_at | 57698 | KIAA1598 | KIAA1598 |
| 1.5 | 209500_x_at | 407977 /// 8741 | TNFSF13 /// | tumor necrosis factor (ligand) superfamily, member 13 |
| | | | TNFSF12-TNFSF13 | /// tumor necrosis factor (ligand) superfamily, |
| | | | | member 12-member 13 |
| 1.5 | 210629_x_at | 7940 | LST1 | leukocyte specific transcript 1 |
| 1.5 | 208981_at | 5175 | PECAM1 | platelet/endothelial cell adhesion molecule (CD31 |
| | | | | antigen) |
| 1.5 | 202367_at | 1523 | CUTL1 | cut-like 1, CCAAT displacement protein (Drosophila) |
| 1.5 | 222218_s_at | 29992 | PILRA | paired immunoglobin-like type 2 receptor alpha |
| 1.5 | 217507_at | 6556 | SLC11A1 | Solute carrier family 11 (proton-coupled divalent metal |
| | | | | ion transporters), member 1 |
| 1.5 | 227276_at | 84898 | PLXDC2 | plexin domain containing 2 |
| 1.5 | 211135_x_at B | 11025 | LILRB3 | leukocyte immunoglobulin-like receptor, subfamily |
| | | | | (with TM and ITIM domains), member 2 /// |
| | | | | immunoglobulin-like receptor, subfamily B leukocyte (with TM |
| | | | | and ITIM domains), member 3 |
| 1.5 | 202902_s_at | 1520 | CTSS | cathepsin S |
| 1.5 | 37384_at | 9647 | PPM1F | protein phosphatase 1F (PP2C domain containing) |
| 1.5 | 224374_s_at | 84034 | EMILIN2 | elastin microfibril interfacer 2 /// elastin microfibril |
| | | | | interfacer 2 |
| 1.5 N- | 211732_x_at | 3176 | HNMT | histamine N-methyltransferase /// histamine |
| | | | | methyltransferase |
| 1.5 | 204254_s_at | 7421 | VDR | vitamin D (1,25- dihydroxyvitamin D3) receptor |
| 1.5 | 211729_x_at A | 644 | BLVRA | biliverdin reductase A /// biliverdin reductase |
| 1.5 | 210895_s_at | 942 | CD86 | CD86 antigen (CD28 antigen ligand 2, B7-2 antigen) |
| 1.5 | 207224_s_at | 27036 | SIGLEC7 | sialic acid binding Ig-like lectin 7 |
| 1.5 | 218217_at | 59342 | SCPEP1 | serine carboxypeptidase 1 |
| 1.5 | 209263_x_at | 7106 | TM4SF7 | tetraspanin 4 |
| 1.5 | 207872_s_at B | 11024 | LILRA1 | leukocyte immunoglobulin-like receptor, subfamily |
| | | | | (with TM and ITIM domains), member 1 /// |
| | | | | immunoglobulin-like receptor, subfamily A leukocyte |
| | | | | domain), member 1 (with TM |
| 1.5 | 221731_x_at | 1462 | CSPG2 | chondroitin sulfate proteoglycan 2 (versican) |
| 1.5 | 219788_at | 29992 | PILRA | paired Immunoglobin-like type 2 receptor alpha |
| 1.5 | 208702_x_at | 334 | APLP2 | amyloid beta (A4) precursor-like protein 2 |
| 1.5 | 210844_x_at | 1495 | CTNNA1 | catenin (cadherin-associated protein), alpha 1, |
| | | | | 102kDa |
| 1.5 | 64408_s_at | 91860 | CALML4 | calmodulin-like 4 |
| 1.5 | 201186_at | 4043 | LRPAP1 | low density lipoprotein receptor-related protein |
| | | | | associated protein 1 |
| 1.5 | 220162_s_at | 64170 | CARD9 | caspase recruitment domain family, member 9 |
| 1.5 | 204588_s_at | 9056 | SLC7A7 | solute carrier family 7 (cationic amino acid |
| | | | | transporter, y+ system), member 7 |
| 1.5 | 205936_s_at | 3101 | HK3 | hexokinase 3 (white cell) |
| 1.5 | 224724_at | 55959 | SULF2 | sulfatase 2 |
| 1.5 | 200764_s_at | 1495 | CTNNA1 | catenin (cadherin-associated protein), alpha 1, |
| | | | | 102kDa |
| 1.5 | 209949_at | 4688 | NCF2 | neutrophil cytosolic factor 2 (65kDa, autosomal chronic |
| | | | | granulomatous disease, 2) |
| 1.5 | 209124_at | 4615 | MYD88 | myeloid differentiation primary response gene (88) |
| 1.5 | 217865_at | 55819 | RNF130 | ring finger protein 130 |
| 1.5 | 227135_at | 27163 | ASAHL | --- |
| 1.5 | 202833_s_at | 5265 | SERPINA1 | serine (or cysteine) proteinase inhibitor, clade A |
| | | | | (alpha-1 antiproteinase, antitrypsin), member 1 |
| | | | | |
| 1.5 | 204393_s_at | 55 | ACPP | acid phosphatase, prostate |
| 1.5 | 205237_at 1 | 2219 | FCN1 | ficolin (collagen/fibrinogen domain containing) |
| 1.5 | 207111_at | 2015 | EMR1 | egf-like module containing, mucin-like, hormone |
| | | | | receptor-like 1 |
| 1.5 | 211336_x_at | 10859 | LILRB1 | leukocyte immunoglobulin-like receptor, subfamily B |
| | | | | (with TM and ITIM domains), member 1 |
| 1.5 | 204981_at | 5002 | SLC22A18 | solute carrier family 22 (organic cation transporter), |
| | | | | member 18 |
| 1.5 | 204495_s_at | --- | --- | DKFZP434H132 protein |
| 1.5 | 220091_at | 11182 | SLC2A6 | solute carrier family 2 (facilitated glucose transporter), |
| | | | | member 6 |
| 1.5 | 200765_x_at | 1495 | CTNNA1 | catenin (cadherin-associated protein), alpha 1, |
| | | | | 102kDa |
| 1.5 | 202030_at | 10295 | BCKDK | branched chain ketoacid dehydrogenase kinase |
| 1.5 | 205603_s_at | 1730 | DIAPH2 | diaphanous homolog 2 (Drosophila) |
| 1.5 | 216041_x_at | 2896 | GRN | granulin |
| 1.5 | 219358_s_at | 55803 | CENTA2 | centaurin, alpha 2 |
| 1.5 | 214181_x_at | 7940 | LST1 | leukocyte specific transcript 1 |
| 1.5 | 217897_at 6 | 53826 | FXYD6 | FXYD domain containing ion transport regulator |
| 1.5 | 212119_at | 23433 | RHOQ | ras homolog gene family, member Q |
| 1.5 | 217764_s_at | 11031 | RAB31 | RAB31, member RAS oncogene family |
| 1.5 | 218610_s_at | 55313 | FLJ11151 | hypothetical protein FLJ11151 |
| 1.5 | 210660_at A | 11024 | LILRA1 | leukocyte immunoglobulin-like receptor, subfamily |
| | | | | (with TM domain), member 1 /// leukocyte immunoglobulin-like receptor, subfamily A (with TM |
| | | | | domain), member 1 |
| 1.5 | 219259_at | 64218 | SEMA4A | sema domain, immunoglobulin domain (Ig), transmembrane domain (TM) and short cytoplasmic |
| | | | | domain, (semaphorin) 4A |
| 1.5 | 235252_at | 8844 | KSR | Kinase suppressor of ras |
| 1.5 8 | 217989_at | 51170 | DHRS8 | dehydrogenase/reductase (SDR family) member |
| 1.5 | 207677_s_at | 4689 | NCF4 | neutrophil cytosolic factor 4, 40kDa /// neutrophil |
| | | | | cytosolic factor 4, 40kDa |
| 1.5 | 213716_s_at | 6398 | SECTM1 | secreted and transmembrane 1 |
| 1.5 | 208450_at | 3957 | LGALS2 | lectin, galactoside-binding, soluble, 2 (galectin 2) /// |
| | | | | lectin, galactoside-binding, soluble, 2 |
| | | | | (galectin 2) |
| 1.5 | 211495_x_at | 407977 /// 8741 | TNFSF13 /// | tumor necrosis factor (ligand) superfamily, member 13 |
| | | | TNFSF12-TNFSF13 | /// tumor necrosis factor (ligand) superfamily, |
| | | | | member 12-member 13 |
| 1.5 | 223703_at | 83938 | C10orf11 | chromosome 10 open reading frame 11 |
| 1.5 | 218454_at | 79887 | FLJ22662 | hypothetical protein FLJ22662 |
| 1.5 | 213275_x_at | 1508 | CTSB | Cathepsin B |
| 1.5 | 210314_x_at | 407977 /// 8741 | TNFSF13 /// | tumor necrosis factor (ligand) superfamily, member 13 |
| | | | TNFSF12-TNFSF13 | /// tumor necrosis factor (ligand) superfamily, |
| | | | | member 12-member 13 |
| 1.5 | 207857_at | 11027 | LILRA2 | leukocyte immunoglobulin-like receptor, subfamily A |
| | | | | (with TM domain), member 2 /// leukocyte immunoglobulin-like receptor, subfamily A (with TM |
| | | | | domain), member 2 |
| 1.5 | 208130_s_at | 6916 | TBXAS1 | thromboxane A synthase 1 (platelet, cytochrome |
| | | | | P450, family 5, subfamily A) /// thromboxane A |
| | | | | synthase 1 (platelet, cytochrome P450, family 5, subfamily A) |
| 1.5 | 211776_s_at | 23136 | EPB41L3 | erythrocyte membrane protein band 4.1-like 3 /// |
| | | | | erythrocyte membrane protein band 4.1-like 3 |
| 1.5 | 211100_x_at | 11027 | LILRA2 | leukocyte immunoglobulin-like receptor, subfamily A |
| | | | | (with TM domain), member 2 |
| 1.5 | 203508_at | 7133 | TNFRSF1B | tumor necrosis factor receptor superfamily, member 1B |
| 1.6 | 220071_x_at | 55142 | C15orf25 | chromosome 15 open reading frame 25 |
| 1.6 | 241303_x_at | --- | --- | --- |
| 1.6 | 215269_at | 7109 | TMEM1 | transmembrane protein 1 |
| 1.6 | 238070_at | 9557 | CHD1L | Chromodomain helicase DNA binding protein 1-like |
| 1.6 | 231825_x_at | 55729 | ATF7IP | activating transcription factor 7 interacting protein |
| 1.6 | 244826_at | 23760 | PITPNB | Phosphatidylinositol transfer protein, beta |
| 1.6 | 233867_at | --- | --- | Hypothetical protein MGC40405 |
| 1.6 | 243981_at | 6789 | STK4 | serine/threonine kinase 4 |
| 1.6 | 233630_at | 8760 | CDS2 | CDP-diacylglycerol synthase (phosphatidate cytidylyltransferase) 2 |
| 1.6 | 244535_at | --- | --- | Forkhead box P1 |
| 1.6 | 244766_at | 23049 /// 283846 /// | SMG1 /// KIAA0220 | PI-3-kinase-related kinase SMG-1 /// PI-3-kinase- |
| | | 440345 | /// LOC440345 | related kinase SMG-1-like /// hLAT1-3TM /// similar to |
| | | | | PI-3-kinase-related kinase SMG-1 isoform 1; |
| | | | | lambda/iota protein kinase C-interacting protein; |
| | | | | phosphatidylinositol 3-kinase-related protein kinase /// |
| 1.6 | 244185_at | 10988 | METAP2 | Methionyl aminopeptidase 2 |
| 1.6 | 229206_at | --- | --- | CDNA FLJ13350 fis, clone OVARC1002143 |
| 1.6 | 237330_at | 253260 | AVO3 | Likely ortholog of mouse TORC2-specific protein |
| | | | | AVO3 (S. cerevisiae) |
| 1.6 | 242108_at | 53373 | TPCN1 | Two pore segment channel 1 |
| 1.6 | 244165_at | 54906 | C10orf18 | --- |
| 1.6 | 243561_at | 10138 | YAF2 | YY1 associated factor 2 |
| 1.6 | 215204_at | 26054 | SENP6 | SUMO1/sentrin specific protease 6 |
| 1.6 | 244145_at | 79443 | FYCO1 | FYVE and coiled-coil domain containing 1 |
| 1.6 | 239379_at | --- | --- | CDNA FLJ13876 fis, clone THYRO1001401 |
| 1.6 | 232333_at | 84441 | MAML2 | Mastermind-like 2 (Drosophila) |
| 1.6 | 242865_at | 27020 | SDFR1 | Stromal cell derived factor receptor 1 |
| 1.6 | 240665-at | 10659 | CUGBP2 | CUG triplet repeat, RNA binding protein 2 |
| 1.6 | 243030_at | --- | --- | Mitogen-activated protein kinase kinase kinase 1 |
| 1.6 | 241993_x_at | --- | --- | Forkhead box P1 |
| 1.6 | 242920_at | 23387 | KIAA0999 | KIAA0999 protein |
| 1.6 | 244633_at | 9063 | PIAS2 | Protein inhibitor of activated STAT, 2 |
| 1.6 | 206088_at | 440457 | LOC440457 | hypothetical LOC440457 |
| 1.6 | 215032_at | 6239 | RREB1 | ras responsive element binding protein 1 |
| 1.6 | 242068_at | 57448 | BIRC6 | Baculoviral IAP repeat-containing 6 (apollon) |
| 1.6 | 39313_at | 65125 | WNK1 | WNK lysine deficient protein kinase 1 |
| 1.6 | 238712_at | --- | --- | Transcribed locus |
| 1.6 | 244197_x_at | --- | --- | CCR4-NOT transcription complex, subunit 2 |
| 1.6 | 240594_at | 9425 | CDYL | Chromodomain protein, Y-like |
| 1.6 | 242110_at | 394 | ARHGAP5 | Rho GTPase activating protein 5 |
| 1.6 | 236685_at | --- | --- | Transcribed locus |
| 1.6 | 240600_at | 8546 | AP3B1 | Adaptor-related protein complex 3, beta 1 subunit |
| 1.6 | 222186_at | 54469 | ZA20D3 | Zinc finger, A20 domain containing 3 |
| 1.6 | 234923_at | 253959 | GARNL1 | GTPase activating Rap/RanGAP domain-like 1 |
| 1.6 | 242646_at | 51111 | SUV420H1 | Suppressor of variegation 4-20 homolog 1 (Drosophila) |
| 1.6 | 241905_at | 5286 | PIK3C2A | Phosphoinositide-3-kinase, class 2, alpha polypeptide |
| 1.6 | 244414_at | 84441 | MAML2 | Mastermind-like 2 (Drosophila) |
| 1.6 | 243450_at | 11214 | AKAP13 | A kinase (PRKA) anchor protein 13 |
| 1.6 | 227383_at | 440668 | --- | Similar to KIAA0454 protein |
| 1.6 | 207474_at | 54861 | SNRK | SNF related kinase |
| 1.6 | 233473_x_at | 9958 | USP15 | Ubiquitin specific protease 15 |
| 1.6 | 234032_at | --- | --- | PRO1550 |
| 1.6 | 242407_at | --- | --- | Arginine-glutamic acid dipeptide (RE) repeats |
| 1.6 | 228866_at | 29123 | ANKRD11 | Ankyrin repeat domain 11 |
| 1.6 | 239646_at | 51735 | RAPGEF6 | KIAA1961 gene |
| 1.6 | 242413_at | 50618 | ITSN2 | Intersectin 2 |
| 1.6 | 233690_at | 80215 | C21orf96 | Runt-related transcription factor 1 (acute myeloid |
| | | | | leukemia 1; aml1 oncogene) |
| 1.6 | 210556_at | 4775 | NFATC3 | nuclear factor of activated T-cells, |
| | | | | calcineurin-dependent 3 cytoplasmic, |
| 1.6 | 217653_x_at | --- | --- | Homo sapiens, Similar to ARF GTPase-activating |
| | | | | protein, clone IMAGE:5245118, mRNA |
| 1.6 | 239243_at | 27332 | ZNF638 | Zinc finger protein 638 |
| 1.6 | 222282_at | 167153 | PAPD4 | PAP associated domain containing 4 |
| 1.6 | 215375_x_at | --- | --- | CDNA FLJ13876 fis, clone THYRO1001401 |
| 1.6 | 229642_at | 8874 | ARHGEF7 | Rho guanine nucleotide exchange factor (GEF) 7 |
| 1.6 | 236472_at | 4898 | NRD1 | Nardilysin (N-arginine dibasic convertase) |
| 1.6 | 233056_x_at | 22839 | DLGAP4 | discs, large (Drosophila) homolog-associated protein |
| | | | | 4 |
| 1.6 | 243827_at | 60685 | TEX27 | Testis expressed sequence 27 |
| 1.6 | 235138_at | 23369 | PUM2 | Vacuolar protein sorting 35 (yeast) |
| 1.6 | 232744_x_at | 360030 | LOC360030 | Homeobox C14 |
| 1.6 | 239748_x_at | --- | --- | OCIA domain containing 1 |
| 1.6 | 241681_at | 4154 | MBNL1 | Muscleblind-like (Drosophila) |
| 1.6 | 222380_s_at | 391733 | --- | Similar to Microneme antigen |
| 1.6 | 239274_at | 8301 | PICALM | Phosphatidylinositol binding clathrin assembly protein |
| 1.6 | 217679_x_at | --- | --- | --- |
| 1.6 | 240499_at | 54462 | KIAA1128 | KIAA1128 |
| 1.6 | 207365_x_at | 9736 | USP34 | ubiquitin specific protease 34 |
| 1.6 | 231024_at | --- | --- | Similar to phosphoglucomutase 5 |
| 1.6 | 242225_at | 23041 | KIAA1040 | KIAA1040 protein |
| 1.6 | 238558_at | 4154 | MBNL1 | Muscleblind-like (Drosophila) |
| 1.6 | 239453_at | 23048 | FNBP1 | Formin binding protein 1 |
| 1.6 | 238883_at | 23389 | THRAP2 | Thyroid hormone receptor associated protein 2 |
| 1.6 | 242008_at | 23287 | AGTPBP1 | ATP/GTP binding protein 1 |
| 1.6 | 236437_at | 3912 | LAMB1 | Laminin, beta 1 |
| 1.6 | 215179_x_at | 5228 | PGF | Placental growth factor, vascular endothelial growth |
| | | | | factor-related protein |
| 1.6 | 235879_at | 4154 | MBNL1 | Muscleblind-like (Drosophila) |
| 1.6 | 232347_x_at | 84869 | CBR4 | Carbonic reductase 4 |
| 1.6 | 242688_at | 9320 | TRIP12 | Thyroid hormone receptor interactor 12 |
| 1.6 | 243589_at | 284058 | LOC284058 | hypothetical protein LOC284058 |
| 1.6 | 231205_at | --- | --- | --- |
| 1.6 | 214805_at | 1973 | EIF4A1 | Eukaryotic translation initiation factor isoform 1 4A, |
| 1.6 | 230415_at | 27086 | FOXP1 | Forkhead box P1 |
| 1.6 | 236561_at | 7046 | TGFBR1 | Transforming growth factor, beta receptor I (activin A |
| | | | | receptor type II-like kinase, 53kDa) |
| 1.6 | 241268_x_at | --- | --- | --- |
| 1.6 | 233302_at | 64919 | BCL11B | B-cell CLL/lymphoma 11B (zinc finger protein) |
| 1.6 | 221616_s_at | 5230 | PGK1 | CDNA FLJ23869 fis, clone LNG09860 |
| 1.6 | 239238_at | 6599 | SMARCC1 | SWI/SNF related, matrix associated, actin dependent |
| | | | | regulator of chromatin, subfamily c, |
| | | | | member 1 |
| 1.6 | 216682_s_at | --- | --- | family with sequence similarity 48, member A |
| 1.6 | 242737_at | 5890 | RAD51L1 | RAD51-like 1 (S. cerevisiae) |
| 1.6 | 235028_at | --- | --- | CDNA FLJ42313 fis, clone TRACH2019425 |
| 1.6 | 220918_at | 80215 | C21orf96 | chromosome 21 open reading frame 96 |
| 1.6 | 236621_at | 6232 | RPS27 | ribosomal protein S27 (metallopanstimulin 1) |
| 1.6 | 240008_at | 57492 | ARID1B | AT rich interactive domain 1B (SWI1-like) |
| 1.6 | 243640_x_at | --- | --- | CDC14 cell division cycle 14 homolog A (S. cerevisiae) |
| 1.6 | 235023_at | 54832 | VPS13C | Vacuolar protein sorting 13C (yeast) |
| 1.6 | 232012_at | 823 | CAPN1 | calpain 1, (mu/I) large subunit |
| 1.6 | 233824_at | --- | --- | CDNA: FLJ21428 fis, clone COL04203 |
| 1.6 | 231109_at | 10659 | CUGBP2 | CUG triplet repeat, RNA binding protein 2 |
| 1.6 | 244457_at | --- | --- | Family with sequence similarity 20, member C |
| 1.6 | 243524_at | 148867 | SLc30A7 | Solute carrier family 30 (zinc transporter), member 7 |
| 1.6 | 220467_at | 80100 | FLJ21272 | hypothetical protein FLJ21272 |
| 1.6 | 241798_at | 23244 | SCC-112 | SCC-112 protein |
| 1.6 | 222371_at | 8554 | PIAS1 | Protein inhibitor of activated STAT, 1 |
| 1.6 | 236924_at | 11146 | GLMN | Novel protein |
| 1.6 | 232528_at | 10477 | UBE2E3 | Ubiquitin-conjugating enzyme E2E 3 (UBC4/5 homolog, yeast) |
| 1.6 | 234278_at | 2060 | EPS15 | --- |
| 1.6 | 233595_at | 9736 | USP34 | Ubiquitin specific protease 34 |
| 1.6 | 240271_at | --- | --- | Myotubularin related protein 3 |
| 1.6 | 243286_at | 8454 | CUL1 | Cullin 1 |
| 1.6 | 232583_at | --- | --- | CDNA FLJ11435 fis, clone HEMBA1001208 |
| 1.6 | 81811_at | 391733 | --- | Similar to Microneme antigen |
| 1.6 | 239561_at | 23389 | THRAP2 | Thyroid hormone receptor associated protein 2 |
| 1.6 | 236545_at | 5530 | PPP3CA | Protein phosphatase 3 (formerly 2B), |
| | | | | subunit, alpha isoform (calcineurin A catalytic |
| | | | | alpha) |
| 1.6 | 242476_at | --- | --- | Transcribed locus, strongly similar to XP_520590.1 |
| | | | | similar to hypothetical protein FLJ30435 [Pan |
| | | | | troglodytes] |
| 1.6 | 242593_at | --- | --- | KIAA0143 protein |
| 1.6 | 235912_at | 57488 | CHR2SYT | Chr2 synaptotagmin |
| 1.6 | 235847_at | 60685 | TEX27 | Testis expressed sequence 27 |
| 1.6 | 239228_at | 1457 | CSNK2A1 | Casein kinase 2, alpha 1 polypeptide |
| 1.6 | 215577_at | 7324 | UBE2E1 | Ubiquitin-conjugating enzyme E2E 1 (UBC4/5 homolog, yeast) |
| 1.6 | 239049_at | --- | --- | --- |
| 1.6 | 233228_at | --- | --- | Zinc finger protein 407 |
| 1.6 | 208246_x_at | 7084 | TK2 | Thymidine kinase 2, mitochondrial |
| 1.6 | 243992_at | 7750 | ZNF198 | Zinc finger protein 198 |
| 1.6 | 216176_at | --- | --- | hepatocellular carcinoma-related HCRP1 |
| 1.6 | 215221_at | --- | --- | Forkhead box P1 |
| 1.6 | 230229_at | 26069 | DKFZP586B0319 | DKFZP586B0319 protein |
| 1.6 | 242241_x_at | --- | --- | --- |
| 1.6 | 215191_at | 22992 | FBXL11 | F-box and leucine-rich repeat protein 11 |
| 1.6 | 239388_at | 8635 | RNASET2 | Ribonuclease T2 |
| 1.6 | 232615_at | 388685 | FLJ39739 | FLJ39739 protein |
| 1.6 | 243993_at | 5128 | PCTK2 | PCTAIRE protein kinase 2 |
| 1.6 | 238783_at | 153396 | MGC33214 | hypothetical protein MGC33214 |
| 1.6 | 244010_at | 58508 | MLL3 | B melanoma antigen family, member 4 |
| 1.6 | 239937_at | 7756 | ZNF207 | Zinc finger protein 207 |
| 1.6 | 230387_at | 27032 | ATP2C1 | ATPase, Ca++ transporting, type 2C, member 1 |
| 1.6 | 234788_x_at | 80006 | FLJ13611 | Hypothetical protein FLJ13611 |
| 1.6 | 230332_at | --- | --- | PRO1550 |
| 1.6 | 229694_at | 55717 | WDR11 | WD repeat domain 11 |
| 1.7 | 211983_x_at | 71 | ACTG1 | actin, gamma 1 |
| 1.7 | 200763_s_at | 6176 | RPLP1 | ribosomal protein, large, P1 |
| 1.7 | 217807_s_at | 29997 | GLTSCR2 | glioma tumor suppressor candidate region gene 2 |
| 1.7 | 212284_x_at | 7178 | TPT1 | tumor protein, translationally-controlled 1 |
| 1.7 | 212537_x_at | 6139 | RPL17 | ribosomal protein L17 |
| 1.7 | 200809_x_at | 6136 | RPL12 | ribosomal protein L12 |
| 1.7 | 214003_x_at | 6224 | RPS20 | ribosomal protein S20 |
| 1.7 | 200725_x_at | 6134 | RPL10 | ribosomal protein L10 |
| 1.7 | 216520_s_at | 7178 | TPT1 | tumor protein, translationally-controlled 1 |
| 1.7 | 229563_s_at | 4736 | RPL10A | ribosomal protein L10a |
| 1.7 | 200716_x_at | 23521 | RPL13A | ribosomal protein L13a |
| 1.7 | 211073_x_at | 6122 | RPL3 | ribosomal protein L3 /// ribosomal protein L3 |
| 1.7 | 211710_x_at | 6124 | RPL4 | ribosomal protein L4 /// ribosomal protein L4 |
| 1.7 | 212433_x_at | 6187 | RPS2 | ribosomal protein S2 |
| 1.7 | 200741_s_at | 6232 | RPS27 | ribosomal protein S27 (metallopanstimulin 1) |
| 1.7 | 213801_x_at | 388524 /// 3921 | RPSA /// LOC388524 | ribosomal protein SA /// similar to Laminin receptor 1 |
| | | | | (p40) (34/67 laminin-resistance-similar to 40S ribosomal protein SA |
| | | | | kDa laminin receptor) (Colon carcinoma binding protein) (NEM/1CHD4) (Multidrug associated protein MGrl-Ag) |
| 1.7 | 200801_x_at | 60 | ACTB | actin, beta |
| 1.7 | 212869_x_at | 7178 | TPT1 | tumor protein, translationally-controlled 1 |
| 1.7 | 200099_s_at | 6189 | RPS3A | ribosomal protein S3A /// ribosomal protein S3A |
| 1.7 | 209134_s_at | 6194 | RPS6 | ribosomal protein S6 |
| 1.7 | 212270_x_at | 6139 | RPL17 | ribosomal protein L17 |
| 1.7 | 200823_x_at | 6159 | RPL29 | ribosomal protein L29 |
| 1.7 | 212581_x_at | 2597 | GAPD | glyceraldehyde-3-phosphate dehydrogenase |
| 1.7 | 201010_s_at | 10628 | TXNIP | thioredoxin interacting protein |
| 1.7 | 209140_x_at | 3106 | HLA-B | major histocompatibility complex, class I, B |
| 1.7 | 200674_s_at | 6161 | RPL32 | ribosomal protein L32 |
| 1.7 | 201257_x_at | 6189 | RPS3A | ribosomal protein S3A |
| 1.7 | 211943_x_at | 7178 | TPT1 | tumor protein, translationally-controlled 1 |
| 1.7 | 221700_s_at | 7311 | UBA52 | ubiquitin A-52 residue ribosomal protein fusion |
| | | | | product 1 /// ubiquitin A-52 residue |
| | | | | ribosomal protein |
| | | | | fusion product 1 |
| 1.7 | 208692_at | 6188 | RPS3 | ribosomal protein S3 |
| 1.7 | 212039_x_at | 6122 | RPL3 | ribosomal protein L3 |
| 1.7 | 214459_x_at | 3107 | HLA-C | major histocompatibility complex, class I, C |
| 1.7 | 200031_s_at | 6205 | RPS11 | ribosomal protein S11 /// ribosomal protein S11 |
| 1.7 | 214351_x_at | 6137 | RPL13 | ribosomal protein L13 |
| 1.7 | 201094_at | 6235 | RPS29 | ribosomal protein S29 |
| 1.7 | 213583_x_at | --- | --- | similar to eukaryotic translation elongation factor 1 |
| | | | | alpha 1-like 14 /// similar to eukaryotic translation |
| | | | | elongation factor 1 alpha 1; eukaryotic translation |
| | | | | elongation factor 1 alpha 1-like 14; CTCL tumor |
| | | | | antigen; translation elongation factor 1 alpha |
| 1.7 | 217740_x_at | 6130 | RPL7A | ribosomal protein L7a |
| 1.7 | 210646_x_at | 23521 | RPL13A | ribosomal protein L13a |
| 1.7 | 200926_at | 6228 | RPS23 | ribosomal protein S23 |
| 1.7 | 200021_at | 1072 | CFL1 | cofilin 1 (non-muscle) /// cofilin 1 (non-muscle) |
| 1.7 | 203107_x_at | 6187 | RPS2 | ribosomal protein S2 |
| 1.7 | 213084_x_at | 6147 | RPL23A | ribosomal protein L23a |
| 1.7 | 213214_x_at | 71 | ACTG1 | actin, gamma 1 |
| 1.7 | 212578_x_at | 6218 | RPS17 | ribosomal protein S17 |
| 1.7 | 211956_s_at | 10209 | SUI1 | putative translation initiation factor |
| 1.7 | 213969_x_at | 6159 | RPL29 | ribosomal protein L29 |
| 1.7 | 204102_s_at | 1938 | EEF2 | eukaryotic translation elongation factor 2 |
| 1.7 | 201049_s_at | 6222 | RPS18 | ribosomal protein S18 |
| 1.7 | 212790_x_at | 23521 | RPL13A | ribosomal protein L13a |
| 1.7 | 211995_x_at | 71 | ACTG1 | actin, gamma 1 |
| 1.7 | 208812_x_at | 3106 /// 3107 | HLA-B /// HLA-C | major histocompatibility complex, class I, C |
| 1.7 | 200012_x_at | 6144 | RPL21 | ribosomal protein L21 /// ribosomal protein L21 |
| 1.7 | 200905_x_at | 3133 | HLA-E | major histocompatibility complex, class I, E |
| 1.7 | 216231_s_at | 567 | B2M | beta-2-microglobulin |
| 1.7 | 212191_x_at | 6137 | RPL13 | ribosomal protein L13 |
| 1.7 | 201665_x_at | 6218 | RPS17 | ribosomal protein S17 |
| 1.7 | 208834_x_at | 6147 | RPL23A | ribosomal protein L23a |
| 1.7 | 203012_x_at | 6147 | RPL23A | ribosomal protein L23a |
| 1.7 | 211720_x_at | 6175 | RPLPO | ribosomal protein, large, P0 /// ribosomal large, P0 protein, |
| 1.7 | 201492_s_at | 6171 | RPL41 | ribosomal protein L41 |
| 1.7 | 200869_at | 6142 | RPL18A | ribosomal protein L18a |
| 1.7 | 216342_x_at | --- | --- | --- |
| 1.7 | 221775_x_at | 6146 | RPL22 | ribosomal protein L22 |
| 1.7 | 207783_x_at | --- | --- | --- |
| 1.7 | 213356_x_at | 3178 /// 441507 | HNRPA1 /// | heterogeneous nuclear ribonucleoprotein to Heterogeneous nuclear ribonucleoprotein /// similar A1 |
| | | | | (Helix-destabilizing protein) (Single-A1 |
| | | | | strand binding protein) (hnRNP core protein A1) (HDP) /// |
| | | | | Heterogeneous nuclear ribonucleoprotein A1 similar to |
| | | | | (Hel |
| 1.7 | 213890_x_at | 6217 | RPS16 | ribosomal protein S16 |
| 1.7 | 201429_s_at | 6168 | RPL37A | ribosomal protein L37a |
| 1.7 | 211972_x_at | 6175 | RPLPO | ribosomal protein, large, P0 |
| 1.7 | 208768_x_at | 6146 | RPL22 | ribosomal protein L22 |
| 1.7 | 213377_x_at | 6206 | RPS12 | ribosomal protein S12 |
| 1.7 | 201154_x_at | 6124 | RPL4 | ribosomal protein L4 |
| 1.7 | 212734_x_at | 6137 | RPL13 | ribosomal protein L13 |
| 1.7 | 213614_x_at | 1915 | EEF1A1 | eukaryotic translation elongation factor 1 alpha 1 |
| 1.7 | 208645_s_at | 6208 | RPS14 | ribosomal protein S14 |
| 1.7 | 211970_x_at | 71 | ACTG1 | actin, gamma 1 |
| 1.7 | 200029_at | 6143 | RPL19 | ribosomal protein L19 /// ribosomal protein L19 |
| 1.7 | 213867_x_at | 60 | ACTB | actin, beta |
| 1.7 | 200092_s_at | 6167 | RPL37 | ribosomal protein L37 /// ribosomal protein L37 |
| 1.7 | 203034_s_at | 6157 | RPL27A | ribosomal protein L27a /// similar to 60S ribosomal |
| | | | | protein L27a |
| 1.7 | 208695_s_at | 6170 | RPL39 | ribosomal protein L39 |
| 1.7 | 200026_at | 6164 | RPL34 | ribosomal protein L34 /// ribosomal protein L34 /// |
| | | | | similar to ribosomal protein L34; 60S ribosomal |
| | | | | protein L34 /// similar to ribosomal protein L34; 60S |
| | | | | ribosomal protein L34 |
| 1.7 | 215157_x_at | 26986 | PABPC1 | poly(A) binding protein, cytoplasmic 1 |
| L17 | 200038_s_at | 6139 | RPL17 | ribosomal protein L17 /// ribosomal protein 1.7 |
| 1.7 | 213932_x_at | 3105 | HLA-A | Major histocompatibility complex, class I, A |
| 1.7 | 216526_x_at | 3107 | HLA-C | major histocompatibility complex, class I, C |
| 1.7 | 211487_x_at | 6218 | RPS17 | ribosomal protein S17 |
| 1.7 | 200062_s_at | 6156 | RPL30 | ribosomal protein L30 /// ribosomal protein L30 |
| 1.7 | 208856_x_at | 6175 | RPLPO | ribosomal protein, large, P0 |
| 1.7 | 211942_x_at | 23521 | RPL13A | ribosomal protein L13a |
| 1.7 | 200032_s_at | 6133 | RPL9 | ribosomal protein L9 /// ribosomal protein L9 |
| 1.7 | 200817_x_at | 6204 | RPS10 | ribosomal protein S10 |
| 1.7 | 221607_x_at | 71 | ACTG1 | actin, gamma 1 |
| 1.7 | 201033_x_at | 6175 | RPLPO | ribosomal protein, large, P0 |
| 1.7 | 204892_x_at | 1915 | EEF1A1 | eukaryotic translation elongation factor 1 alpha 1 |
| 1.7 | 211940_x_at | 3020 | H3F3A | H3 histone, family 3A |
| 1.7 | 200963_x_at | 6160 | RPL31 | ribosomal protein L31 |
| 1.7 | 200689_x_at | 1937 | EEF1G | eukaryotic translation elongation factor 1 gamma |
| 1.7 | 200933_x_at | 6191 | RPS4X | ribosomal protein S4, x-linked |
| 1.7 | 200819_s_at | 6209 | RPS15 | ribosomal protein S15 |
| 1.7 | 200088_x_at | 6136 | RPL12 | ribosomal protein L12 /// ribosomal protein L12 |
| 1.7 | 211528_x_at | 3135 | HLA-G | HLA-G histocompatibility antigen, class I, G |
| 1.7 | 200061_s_at | 6229 | RPS24 | ribosomal protein S24 /// ribosomal protein S24 |
| 1.7 | 226131_s_at | 6217 | RPS16 | ribosomal protein S16 |
| 1.7 | 201217_x_at | 6122 | RPL3 | ribosomal protein L3 |
| 1.7 | 213477_x_at | 1915 | EEF1A1 | eukaryotic translation elongation factor 1 alpha 1 |
| 1.7 | 211296_x_at | 7316 | UBC | ubiquitin C |
| 1.7 | 212988_x_at | 71 | ACTG1 | actin, gamma 1 |
| 1.7 | 201891_s_at | 567 | B2M | beta-2-microglobulin |
| 1.7 | 206559_x_at | 1915 | EEF1A1 | eukaryotic translation elongation factor 1 alpha 1 |
| 1.7 | 214143_x_at | 153201 /// 6152 | RPL24 /// SLC36A2 | ribosomal protein L24 /// solute carrier family 36 |
| | | | | (proton/amino acid symporter), member 2 |
| 1.7 | 217733_s_at | 9168 | TMSB10 | thymosin, beta 10 |
| 1.7 | 213347_x_at | 6191 | RPS4X | ribosomal protein S4, X-linked |
| 1.7 | 211542_x_at | 6204 | RPS10 | ribosomal protein S10 |
| 1.7 | 208825_x_at | 6147 | RPL23A | ribosomal protein L23a |
| 1.7 | 200717_x_at | 6129 | RPL7 | ribosomal protein L7 |
| 1.7 | 211927_x_at | 1937 | EEF1G | eukaryotic translation elongation factor 1 gamma |
| 1.7 | 212391_x_at | 6189 | RPS3A | ribosomal protein S3A |
| 1.7 | 200018_at | 6207 | RPS13 | ribosomal protein S13 /// ribosomal protein S13 |
| 1.7 | 208929_x_at | 6137 | RPL13 | ribosomal protein L13 |
| 1.7 | 221798_x_at | --- | --- | ribosomal protein S2 /// similar to ribosomal protein |
| | | | | S2; 40S ribosomal protein S2 /// similar to |
| | | | | protein S2 ; 40S ribosomal protein S2 /// ribosomal |
| | | | | ribosomal protein S2; 40S ribosomal protein similar to |
| | | | | hypothetical gene supported by AB082925; S2 /// |
| | | | | BC0 |
| 1.7 | 221791_s_at | 51372 | HSPC016 | hypothetical protein HSPC016 |
| 1.7 | 201550_x_at | 71 | ACTG1 | actin, gamma 1 |
| 1.7 | 212788_x_at | 2512 | FTL | ferritin, light polypeptide |
| 1.7 | 208729_x_at | 3106 | HLA-B | major histocompatibility complex, class I, B |
| 1.7 | 211345_x_at | 1937 | EEF1G | eukaryotic translation elongation factor 1 gamma |
| 1.7 | 201254_x_at | 6194 | RPS6 | ribosomal protein S6 |
| 1.7 | 200781_s_at | 23204 /// 6210 | RPS15A /// ARL6IP | ribosomal protein S15a /// ADP-ribosylation 6 interacting protein factor-like |
| 1.7 | 202029_x_at | 6169 | RPL38 | ribosomal protein L38 |
| 1.7 | 208904_s_at | 6234 | RPS28 | ribosomal protein S28 |
| 1.8 | 224430_s_at | 25821 | MTO1 | mitochondrial translation optimization 1 homolog (S. |
| | | | | cerevisiae) /// mitochondrial translation optimization 1 |
| | | | | homolog (S. cerevisiae) |
| 1.8 | 202983_at | 6596 | SMARCA3 | SWI/SNF related, matrix associated, actin dependent |
| | | | | regulator of chromatin, subfamily a, member 3 |
| 1.8 | 228805_at | 375484 | FLJ44216 | FLJ44216 protein |
| 1.8 | 213626_at | 84869 | CBR4 | carbonic reductase 4 |
| 1.8 | 224748_at | 10238 | HAN11 | WD-repeat protein |
| 1.8 | 212405_s_at | 51603 | KIAA0859 | KIAA0859 |
| 1.8 | 209497_s_at | 83759 | RBM30 | RNA binding motif protein 30 |
| 1.8 | 229367_s_at | 474344 | GIMAP6 | GTPase, IMAP family member 6 |
| 1.8 | 207438_s_at | 10073 | RNUT1 | RNA, U transporter 1 |
| 1.8 | 226230_at | 57223 | KIAA1387 | KIAA1387 protein |
| 1.8 | 206734_at | 8690 | JRKL | Jerky homolog-like (mouse) |
| 1.8 | 201964_at | --- | --- | amyotrophic lateral sclerosis 4 |
| 1.8 | 226180_at | 134430 | WDR36 | WD repeat domain 36 |
| 1.8 | 218534_s_at | 55109 | AGGF1 | angiogenic factor with G patch and FHA domains 1 |
| 1.8 | 212872_s_at | 9477 | USP49 | Trf (TATA binding protein-related factor)-homolog (Drosophila) proximal |
| 1.8 | 218614_at | 55196 | FLJ10652 | hypothetical protein FLJ10652 |
| 1.8 | 235306_at | 155038 | GIMAP8 | GTPase, IMAP family member 8 |
| 1.8 | 53968_at | 80789 | KIAA1698 | KIAA1698 protein |
| 1.8 | 207513_s_at | 7743 | ZNF189 | zinc finger protein 189 |
| 1.8 | 226604_at | 160418 | SMILE | SMILE protein |
| 1.8 | 217907_at | 29074 | MRPL18 | mitochondrial ribosomal protein L18 |
| 1.8 | 214988_s_at | 6651 | SON | SON DNA binding protein |
| 1.8 | 214440_at | 9 | NAT1 | N-acetyltransferase 1 (arylamine N-acetyltransferase) |
| 1.8 | 216863_s_at | 22880 | ZCWCC1 | zinc finger, CW type with coiled-coil domain 1 |
| 1.8 | 229969_at | --- | --- | Transcribed locus, moderately similar to XP_508230.1 |
| | | | | zinc finger protein 195 [Pan troglodytes] |
| 1.8 | 201832_s_at | 8615 | VDP | vesicle docking protein p115 |
| 1.8 | 226387_at | 222194 | RSBN1L | round spermatid basic protein 1-like |
| 1.8 | 203159_at | 2744 | GLS | glutaminase |
| 1.8 | 226357_at | 10869 | USP19 | ubiquitin specific protease 19 |
| 1.8 | 234295_at | 51163 | DBR1 | debranching enzyme homolog 1 (S. cerevisiae) |
| 1.8 | 225433_at | 2957 | GTF2A1 | General transcription factor IIA, 1, 19/37kDa |
| 1.8 | 218626_at | 56478 | EIF4ENIF1 | eukaryotic translation initiation factor 4E nuclear |
| | | | | import factor 1 |
| 1.8 | 201270_x_at | 23386 | NUDCD3 | Nudc domain containing 3 |
| 1.8 | 235612_at | --- | --- | Transcribed locus, moderately similar to NP_858931.1 NFS1 nitrogen fixation 1 (S. |
| | | | | [Homo sapiens] cerevisiae) |
| 1.8 | 218716_x_at | 25821 | MTO1 | mitochondrial translation optimization 1 homolog (S. |
| | | | | cerevisiae) |
| 1.8 | 233665_x_at | 25821 | MTO1 | mitochondrial translation optimization 1 homolog (S.cerevisiae) |
| 1.8 | 219123_at | 7775 | ZNF232 | zinc finger protein 232 |
| 1.8 | 205140_at | 8790 | FPGT | fucose-1-phosphate guanylyltransferase |
| 1.8 | 204882_at | --- | --- | Rho GTPase activating protein 25 |
| 1.8 | 212740_at | 30849 | PIK3R4 | phosphoinositide-3-kinase, regulatory subunit 4, p150 |
| 1.8 | 228336_at | 114825 | KIAA1935 | KIAA1935 protein |
| 1.8 | 218138_at | 8195 | MKKS | McKusick-Kaufman syndrome |
| 1.8 | 223507_at | 10845 | CLPX | ClpX caseinolytic protease X homolog (E. coli) |
| 1.8 | 227335_at | 85362 | C20orf158 | chromosome 20 open reading frame 158 |
| 1.8 | 227626_at | 85315 | C6orf33 | chromosome 6 open reading frame 33 |
| 1.8 | 228963_at | --- | --- | MRNA; CDNA DKFZp434L201 (from clone |
| 1.8 | 227026_at | 54737 | HSMPP8 | M-phase phosphoprotein, mpp8 |
| 1.8 | 212402_at | 23091 | KIAA0853 | KIAA0853 |
| 1.8 | 224959_at | 1836 | SLC26A2 | solute carrier family 26 (sulfate transporter), member 2 |
| 1.8 | 204327_s_at | 7753 | ZNF202 | zinc finger protein 202 |
| 1.8 | 226159_at | 285636 | LOC285636 | hypothetical protein LOC285636 |
| 1.8 | 203694_s_at | 8449 | DHX16 | DEAH (Asp-Glu-Ala-His) box polypeptide 16 |
| 1.8 | 213405_at | 57403 | RAB22A | RAB22A, member RAS oncogene family |
| 1.8 | 221081_s_at | 79961 | FLJ22457 | hypothetical protein FLJ22457 |
| 1.8 | 239376_at | --- | --- | CDNA clone IMAGE:4333081, partial cds |
| 1.8 | 228453_at | 284267 | LOC284267 | hypothetical protein LOC284267 |
| 1.8 | 219128_at | 54980 | FLJ20558 | hypothetical protein FLJ20558 |
| 1.8 | 212632_at | 8417 | STX7 | Syntaxin 7 |
| 1.8 | 210053_at | 6877 | TAF5 | TAF5 RNA polymerase II, TATA box binding protein |
| | | | | (TBP)-associated factor, 100kDa |
| 1.8 | 209175_at | 11196 | SEC23IP | SEC23 interacting protein |
| 1.8 | 206332_s_at | 3428 | IFI16 | interferon, gamma-inducible protein 16 |
| 1.8 | 226242_at | 128061 | DKFZp547B1713 | hypothetical protein DKFZp547B1713 |
| 1.8 | 218303_x_at | 51315 | LOC51315 | hypothetical protein LOC51315 |
| 1.8 | 225988_at | 26091 | HERC4 | hect domain and RLD 4 |
| 1.8 | 238653_at | 9860 | LRIG2 | Leucine-rich repeats and immunoglobulin-like |
| | | | | domains 2 |
| 1.8 | 226958_s_at | 400569 | MGC88387 | similar to HSPC296 |
| 1.8 | 218056_at | 51283 | BFAR | bifunctional apoptosis regulator |
| 1.8 | 212170_at | 10137 | RBM12 | RNA binding motif protein 12 |
| 1.8 | 226032_at | 835 | CASP2 | caspase 2, apoptosis-related cysteine protease |
| | | | | (neural precursor cell expressed, developmentally |
| | | | | down-regulated 2) |
| 1.8 | 228916_at | 143884 | CWF19L2 | CWF19-like 2, cell cycle control (S. pombe) |
| 1.8 | 207338_s_at | 7752 | ZNF200 | zinc finger protein 200 |
| 1.8 | 202453_s_at 1, | 2965 | GTF2H1 | general transcription factor IIH, polypeptide 62kDa |
| 1.8 | 220992_s_at | 81627 | Clorf25 | chromosome 1 open reading frame 25 /// chromosome 1 open reading frame 25 |
| 1.8 | 230142_s_at | 1153 | CIRBP | cold inducible RNA binding protein |
| 1.8 | 219303_at | 79596 | C13orf7 | chromosome 13 open reading frame 7 |
| 1.8 | 225370_at | 90780 | PYGO2 | pygopus homolog 2 (Drosophila) |
| 1.8 | 228920_at | 339324 | LOC339324 | zinc finger protein 260 |
| 1.8 | 219913_s_at | 51340 | CRNKL1 | Crn, crooked neck-like 1 (Drosophila) |
| 1.8 | 226371_at | 5927 | JARID1A | Jumonji, AT rich interactive domain 1A (RBBP2-like) |
| 1.8 | 222028_at | 7596 | ZNF45 | zinc finger protein 45 |
| 1.8 | 222811_at | 55783 | FLJ11171 | hypothetical protein FLJ11171 |
| 1.8 | 209662_at | 1070 | CETN3 | centrin, EF-hand protein, 3 (CDC31 homolog, yeast) |
| 1.8 | 218104_at | 54881 | TEX10 | testis expressed sequence 10 |
| 1.8 | 201447_at | 7072 | TIAL | TIA1 cytotoxic granule-associated RNA binding |
| | | | | protein |
| 1.8 | 208121_s_at | | PTPRO | protein tyrosine phosphatase, receptor type, O 5800 /// |
| | | | | protein tyrosine phosphatase, receptor |
| | | | | type, O |
| 1.8 | 227426_at | 6654 | SOS1 | Son of sevenless homolog 1 (Drosophila) |
| 1.8 | 228041_at | 132949 | AASDH | 2-aminoadipic 6-semialdehyde dehydrogenase |
| 1.8 | 233759_s_at | 57223 | KIAA1387 | KIAA1387 protein |
| 1.8 | 219243_at | 55303 | GIMAP4 | GTPase, IMAP family member 4 |
| 1.8 | 202951_at | 11329 | STK38 | serine/threonine kinase 38 |
| 1.8 | 225816_at | 79960 | PHF17 | PHD finger protein 17 |
| 1.8 | 213838_at --- | | --- | nucleolar protein 7, 27kDa |
| 1.8 | 221865_at | 203197 | C9orf91 | chromosome 9 open reading frame 91 |
| 1.8 | 226109_at | 54149 | C21orf91 | --- |
| 1.8 | 223434_at | 2635 | GBP3 | guanylate binding protein 3 |
| 1.8 | 218242_s_at | 51111 | SUV420H1 | suppressor of variegation 4-20 homolog 1 (Drosophila) |
| 1.8 | 203143_s_at | 9674 | KIAA0040 | KIAA0040 |
| 1.8 | 201142_at | 1965 | EIF2S1 | Eukaryotic translation initiation factor 2, subunit 1 |
| | | | | alpha, 35kDa |
| 1.8 | 226481_at | 9730 | VprBP | Vpr-binding protein |
| 1.8 | 226031_at | 55610 | FLJ20097 | hypothetical protein FLJ20097 |
| 1.8 | 218371_s_at | 55269 | PSPC1 | paraspeckle component 1 |
| 1.8 (S. | 203427_at | 25842 | ASF1A | ASF1 anti-silencing function 1 homolog A |
| | | | | cerevislae) |
| 1.8 | 202322_s_at | 9453 | GGPS1 | geranylgeranyl diphosphate synthase 1 |
| 1.8 | 213127_s_at | 112950 | MED8 | mediator of RNA polymerase II transcription, subunit 8 |
| | | | | homolog (yeast) |
| 1.8 | 222754_at | 51095 | TRNT1 | tRNA nucleotidyl transferase, CCA-adding, 1 |
| 1.8 | 219777_at | 474344 | GIMAP6 | GTPase, IMAP family member 6 |
| 1.8 | 222613_at | 57102 | C12orf4 | chromosome 12 open reading frame 4 |
| 1.8 | 223351_at --- | | --- | lung cancer-related protein 8 |
| 1.8 | 231844_at | 157247 | MGC27345 | hypothetical protein MGC27345 |
| 1.8 | 204676_at | 25880 | DKFZP564K2062 | chromosome 16 open reading frame 51 |
| 1.8 | 224648_at | 65056 | DKFZp761C169 | vasculin |
| 1.8 | 218588_s_at | 10827 | C5orf3 | chromosome 5 open reading frame 3 |
| 1.8 | 216221_s_at | 23369 | PUM2 | pumilio homolog 2 (Drosophila) |
| 1.8 | 202227_s_at | 10902 | BRD8 | bromodomain containing 8 |
| 1.8 | 212116_at | 5987 | RFP | ret finger protein |
| 1.8 | 226821_at | 55183 | Rnf1 | PAP1 interacting factor homolog (yeast) |
| 1.8 | 235215_at | --- | --- | Transcribed locus, moderately similar to XP_498452.1 |
| | | | | hypothetical gene supported by NM_173697 [Homo sapiens] |
| 1.8 | 235152_at | --- | --- | Homo sapiens, clone IMAGE:5218412, mRNA |
| 1.8 | 227536_at | 23091 | KIAA0853 | KIAA0853 |
| 1.8 | 200854_at | 9611 | NCOR1 | nuclear receptor co-repressor 1 |
| 1.8 | 211758_x_at | 10190 | TXNDC9 | thioredoxin domain containing 9 /// thioredoxin domain |
| | | | | containing 9 |
| 1.8 | 202060_at | 9646 | SH2BP1 | SH2 domain binding protein 1 (tetratricopeptide repeat containing) |
| 1.8 | 226867_at | 55667 | C9orf55 | chromosome 9 open reading frame 55 |
| 1.8 | 218455_at | 9054 | NFS1 | NFS1 nitrogen fixation 1 (S. cerevisiae) |
| 1.8 | 228029_at | 170960 | KIAA1982 | KIAA1982 protein |
| 1.8 | 212378_at | 2618 | GART | phosphoribosylglycinamide formyltransferase, |
| | | | | phosphoribosylglycinamide synthetase, phosphoribosylaminoimidazole synthetase |
| 1.8 | 224880_at | 5898 | RALA | v-ral simian leukemia viral oncogene homolog A (ras related) |
| 1.8 | 210541_s_at | 5987 | RFP | ret finger protein |
| 1.8 | 203614_at | 9724 | UTP14C | UTP14, U3 small nucleolar ribonucleoprotein, |
| | | | | homolog C (yeast) |
| 1.8 | 225290_at | --- | --- | CDNA clone IMAGE:5261903, partial cds |
| 1.8 | 236401_at | --- | --- | --- |
| 1.8 | 228071_at | 168537 | GIMAP7 | GTPase, IMAP family member 7 |
| 1.8 | 212222_at | 23198 | PSME4 | proteasome (prosome, macropain) activator subunit 4 |
| 1.8 | 209828_s_at | 3603 | IL16 | interleukin 16 (lymphocyte chemoattractant factor) |
| 1.8 | 203611_at | 7014 | TERF2 | telomeric repeat binding factor 2 |
| 1.8 | 219467_at | 54826 | FLJ20125 | hypothetical protein FLJ20125 |
| 1.8 | 212453_at | 26128 | KIAA1279 | KIAA1279 |
| 1.8 | 203584_at | 9694 | KIAA0103 | KIAA0103 |
| 1.8 | 218968_s_at | 55734 | ZFP64 | zinc finger protein 64 homolog (mouse) |
| 1.8 | 227102_at | 23087 | TRIM35 | tripartite motif-containing 35 |
| 1.8 | 226480_at | --- | --- | Dimethyladenosine transferase |
| 1.8 | 222691_at | 51000 | SLC35B3 | solute carrier family 35, member B3 |
| 1.8 | 228167_at | 89857 | KLHL6 | kelch-like 6 (Drosophila) |
| 1.8 | 202184_s_at | 55746 | NUP133 | nucleoporin 133kDa |
| 1.8 | 41329_at | 57147 | PACE-1 | SCY1-like 3 (S. cerevisiae) |
| 1.8 | 223297_at | 83607 | MGC4268 | hypothetical protein MGC4268 |
| 1.8 | 219130_at | 54482 | FLJ10287 | hypothetical protein FLJ10287 |
| 1.8 | 219146_at | 79736 | FLJ22729 | hypothetical protein FLJ22729 |
| 1.8 | 64418_at | --- | --- | CDNA FLJ34482 fis, clone HLUNG2004067 |
| 1.8 | 238793_at | 91151 | TIGD7 | tigger transposable element derived 7 |
| 1.8 | 229167_at | 5813 | PURA | Purine-rich element binding protein A |
| 1.8 | 214129_at | 9659 | PDE4DIP | Phosphodiesterase 4D interacting protein (myomegalin) |
| 1.8 | 225300_at | 90417 | C15orf23 | chromosome 15 open reading frame 23 |
| 1.8 | 226020_s_at | 115209 /// 1600 | DAB1 /// OMA1 | disabled homolog 1 (Drosophila) /// OMA1 homolog, |
| | | | | zinc metallopeptidase (S. cerevisiae) |

**Table 2**

| module ID | affy ID | Entrez ID | Gene Symbol | Gene Title |
|---|---|---|---|---|
| 2.1 | 206785_s_at | 3821 /// 3822 | KLRC1 /// KLRC2 | killer cell lectin-like receptor subfamily C, member 1 /// killer cell lectin-like receptor subfamily C, member 2 |
| 2.1 | 206267_s_at | 4145 | MATK | megakaryocyte-associated tyrosine kinase |
| 2.1 | 211597_s_at | 84525 | HOP | homeodomain-only protein /// homeodomain-only protein |
| 2.1 | 205171_at | 5775 | PTPN4 | protein tyrosine phosphatase, non-receptor |
| 2.1 | 204160_s_at | 22875 | ENPP4 | type 4 (megakaryocyte) ectonucleotide |
| | | | | pyrophosphatase/phosphodiesterase 4 (putative function) |
| 2.1 | 210006_at | 25864 | DKFZP564O243 | abhydrolase domain containing 14A |
| 2.1 | 218927_s_at | 55501 | CHST12 | carbohydrate (chondroitin 4) sulfotransferase 12 |
| 2.1 | 210288_at | 10219 | KLRG1 | killer cell lectin-like receptor subfamily G, member 1 |
| 2.1 | 205821_at | 22914 | KLRK1 | killer cell lectin-like receptor subfamily K, member 1 |
| 2.1 | 204731_at | 7049 | TGFBR3 | transforming growth factor, beta receptor III (betaglycan, 300kDa) |
| 2.1 | 215894_at | 5729 | PTGDR | prostaglandin D2 receptor (DP) |
| 2.1 | 207734_at | 54900 | LAX | hypothetical protein FLJ20340 |
| 2.1 | 206666_at | 3003 | GZMK | granzyme K (serine protease, granzyme 3; tryptase II) /// granzyme K (serine protease, granzyme 3; tryptase II) |
| 2.1 | 212599_at | 26053 | AUTS2 | autism susceptibility candidate 2 |
| 2.1 | 204960_at | 5790 | PTPRCAP | protein tyrosine phosphatase, receptor type, C-associated protein |
| 2.1 | 210606_x_at | 3824 | KLRD1 | killer cell lectin-like receptor subfamily D, member 1 |
| 2.1 | 232914_s_at | 54843 | SYTL2 | synaptotagmin-like 2 |
| 2.1 | 210164_at | 3002 | GZMB | granzyme B (granzyme 2, cytotoxic T-lymphocyte-associated serine esterase 1) /// granzyme B (granzyme 2, cytotoxic T-lymphocyte-associated serine esterase 1) |
| 2.1 | 219541_at | 54923 | FLJ20406 | hypothetical protein FLJ20406 |
| 2.1 | 227946_at | 114881 | OSBPL7 | oxysterol binding protein-like 7 |
| 2.1 | 210140_at | 8530 | CST7 | cystatin F (leukocystatin) |
| 2.1 | 207840_at | 11126 | CD160 | CD160 antigen |
| 2.1 | 212070_at | 9289 | GPR56 | G protein-coupled receptor 56 |
| 2.1 | 204655_at | --- | --- | --- |
| 2.1 | 214995_s_at | 200316 /// 60489 | APOBEC3G /// APOBEC3F | apolipoprotein B mRNA editing enzyme, catalytic polypeptide-like 3G /// apolipoprotein B mRNA editing enzyme, catalytic polypeptide-like 3F |
| 2.1 | 218764_at | 5583 | PRKCH | protein kinase C, eta |
| 2.1 | 225105_at | 387882 | LOC387882 | hypothetical protein |
| 2.1 | 215332_s_at | 926 | CD8B1 | CD8 antigen, beta polypeptide 1 (p37) |
| 2.1 | 216191_s_at | 6955 /// 6964 | TRA@ /// TRD@ | T cell receptor alpha locus /// T cell receptor delta locus |
| 2.1 | 220646_s_at | 51348 | KLRF1 | killer cell lectin-like receptor subfamily F, member 1 |
| 2.1 | 206366_x_at | 6846 | XCL2 | chemokine (C motif) ligand 2 |
| 2.1 | 231241_at | --- | --- | Schlafen 5 |
| 2.1 | 241803_s_at | 441423 | LOC441423 | LOC441423 |
| 2.1 | 231776_at | 8320 | EOMES | eomesodermin homolog (Xenopus laevis) |
| 2.1 | 1405_1_at | 6352 | CCL5 | chemokine (C-C motif) ligand 5 |
| 2.1 | 205495_s_at | 10578 | GNLY | granulysin /// granulysin |
| 2.1 | 213906_at | 4603 | MYBL1 | v-myb myeloblastosis viral oncogene homolog (avian)-like 1 |
| 2.1 | 207979_s_at | 926 | CD8B1 | CD8 antigen, beta polypeptide 1 (p37) |
| 2.1 | 228774_at | 84131 | C9orf81 | chromosome 9 open reading frame 81 |
| 2.1 | 202761_s_at | 23224 | SYNE2 | spectrin repeat containing, nuclear envelope 2 |
| 2.1 | 205232_s_at | 5051 | PAFAH2 | platelet-activating factor acetylhydrolase 2, 40kDa |
| 2.1 | 228240_at | --- | --- | Full-length cDNA clone CS0DM002YA18 of Fetal liver of Homo sapiens (human) |
| 2.1 | 216033_s_at | 2534 | FYN | FYN oncogene related to SRC, FGR, YES |
| 2.1 | 213658_at | 374655 | DKFZp547K1113 | Hypothetical protein DKFZp547K1113 |
| 2.1 | 37145_at | 10578 | GNLY | granulysin |
| 2.1 | 214617_at | 5551 | PRF1 | perform 1 (pore forming protein) /// perform 1 (pore forming protein) |
| 2.1 | 219529_at | 9022 | CLIC3 | chloride intracellular channel 3 |
| 2.1 | 207795_s_at | 3824 | KLRD1 | killer cell lectin-like receptor subfamily D, member 1 |
| 2.1 | 236265_at | --- | --- | Homo sapiens, clone IMAGE:3887266, mRNA |
| 2.1 | 225688_s_at | 90102 | PHLDB2 | pleckstrin homology-like domain, family B, member 2 |
| 2.1 | 214567_s_at | 6375 /// 6846 | XCL1 /// XCL2 | chemokine (C motif) ligand 1 /// chemokine (C motif) ligand 2 |
| 2.1 | 236782_at | 154075 | SAMD3 | sterile alpha motif domain containing 3 |
| 2.1 | 230753_at | 197135 | LOC197135 | Similar to RIKEN cDNA 4930424G05 |
| 2.1 | 230464_at | 53637 | EDG8 | endothelial differentiation, sphingolipid G-protein-coupled receptor, 8 |
| 2.1 | 220684_at | 30009 | TBX21 | T-box 21 |
| 2.1 | 225706_at | 113263 | GLCCI1 | glucocorticold induced transcript 1 |
| 2.1 | 211144_x_at | 445347 /// 6983 | TRGV9 /// TARP | T cell receptor gamma constant 2 |
| 2.1 | 214450_at | 1521 | CTSW | cathepsin W (lymphopain) /// cathepsin W (lymphopain) |
| 2.1 | 210972_x_at | 6955 | TRA@ | T cell receptor alpha locus /// T cell receptor alpha constant |
| 2.1 | 223836_at | 83888 | KSP37 | Ksp37 protein |
| 2.1 | 207509_s_at | 3904 | LAIR2 | leukocyte-associated Ig-like receptor 2 |
| 2.1 | 223530_at | 11022 | TDRKH | tudor and KH domain containing |
| 2.1 | 205758_at | 925 | CD8A | CD8 antigen, alpha polypeptide (p32) /// CD8 antigen, alpha polypeptide (p32) |
| 2.1 | 228658_at | 150271 | LOC150271 | hypothetical protein LOC150271 |
| 2.1 | 204070_at | 5920 | RARRES3 | retinoic acid receptor responder (tazarotene induced) 3 |
| 2.1 | 221267_s_at | 81926 | C19orf27 | chromosome 19 open reading frame 27 /// chromosome 19 open reading frame 27 |
| 2.1 | 210763_x_at | 259197 | NCR3 | natural cytotoxicity triggering receptor 3 |
| 2.1 | 204103_at | 6351 | CCL4 | chemokine (C-C motif) ligand 4 |
| 2.1 | 203562_at | 9638 | FE21 | fasciculation and elongation protein zeta 1 (zygin I) |
| 2.1 | 223259_at | 94103 | ORMDL3 | ORM1-like 3 (S. cerevisiae) |
| 2.1 | 205488_at | 3001 | GZMA | granzyme A (granzyme 1, cytotoxic T-lymphocyte-associated serine esterase 3) /// granzyme A (granzyme 1, cytotoxic T-lymphocyte-associated serine esterase 3) |
| 2.1 | 217143_s_at | 6955 /// 6964 | TRA@ /// TRD@ | T cell receptor alpha locus /// T cell receptor delta locus |
| 2.1 | 202931_x_at | 274 | BIN1 | bridging integrator 1 |
| 2.1 | 218638_s_at | 10417 | SPON2 | spondin 2, extracellular matrix protein |
| 2.1 | 207351_s_at | 9047 | SH2D2A | SH2 domain protein 2A |
| 2.1 | 213830_at | 6964 | TRD@ | T cell receptor delta locus /// T-cell receptor-delta mRNA, 3' end. |
| 2.1 | 207723_s_at | 3823 | KLRC3 | killer cell lectin-like receptor subfamily C, member 3 |
| 2.1 | 206118_at | 6775 | STAT4 | signal transducer and activator of transcription 4 |
| 2.1 | 205291_at | 3560 | IL2RB | Interleukln 2 receptor, beta /// interleukin 2 receptor, beta |
| 2.1 | 209883_at | 23127 | GLT25D2 | glycosyltransferase 25 domain containing 2 |
| 2.1 | 215806_x_at | 445347 /// 6983 | TRGV9 /// TARP | T cell receptor gamma constant 2 /// T cell receptor gamma variable 9 /// similar to T-cell receptor gamma chain C region PT-gamma-1/2 /// TCR gamma alternate reading frame protein |
| 2.1 | 235919_at | --- | --- | Transcribed locus |
| 2.1 | 225525_at | 85379 | KIAA1671 | KIAA1671 protein |
| 2.1 | 205831_at | 914 | CD2 | CD2 antigen (p50), sheep red blood cell receptor /// CD2 antigen (p50), sheep red blood cell receptor |
| 2.1 | 203713_s_at | 3993 | LLGL2 | lethal glant larvae homolog 2 (Drosophila) |
| 2.1 | 219304_s_at | 80310 | PDGFD | platelet derived growth factor D |
| 2.1 | 213915_at | 4818 | NKG7 | natural killer cell group 7 sequence |
| 2.1 | 216920_s_at | 445347 /// 6983 | TRGV9 /// TARP | T cell receptor gamma constant 2 /// T cell receptor gamma variable 9 /// similar to T-cell receptor gamma chain C region PT-gamma-1/2 /// TCR gamma alternate reading frame protein |
| 2.1 | 226003_at | 55605 | KIF21A | kinesin family member 21A |
| 2.1 | 210321_at | 2999 | GZMH | granzyme H (cathepsin G-like 2, protein h-CCPX) /// granzyme H (cathepsin G-like 2, protein h-CCPX) |
| 2.1 | 226625_at | 7049 | TGFBR3 | Transforming growth factor, beta receptor III (betaglycan, 300kDa) |
| 2.1 | 211685_s_at | 83988 | NCALD | neurocalcin delta /// neurocalcin delta |
| 2.1 | 47069_at | 23779 | ARHGAP8 | proline rich protein 5 |
| 2.1 | 234165_at | 5729 | PTGDR | prostaglandin D2 receptor (DP) |
| 2.1 | 209813_x_at | 445347 /// 6983 | TRGV9 /// TARP | T cell receptor gamma constant 2 /// T cell receptor gamma constant 2 /// T cell receptor gamma variable 9 /// T cell receptor gamma variable 9 /// similar to T-cell receptor gamma chain C region PT-gamma-1/2 /// similar to T-cell receptor gamma chain C r |
| 2.2 | 209275_s_at | 1201 | CLN3 | ceroid-lipofuscinosis, neuronal 3, juvenile (Batten, Spielmeyer-Vogt disease) |
| 2.2 | 203936_s_at | 4318 | MMP9 | matrix metalloproteinase 9 (gelatinase B, 92kDa gelatinase, 92kDa type IV collagenase) |
| 2.2 | 202018_s_at | 4057 | LTF | lactotransferrin |
| 2.2 | 216379_x_at | 934 | CD24 | CD24 antigen (small cell lung carcinoma cluster 4 antigen) |
| 2.2 | 209771_x_at | 934 | CD24 | CD24 antigen (small cell lung carcinoma cluster 4 antigen) |
| 2.2 | 211883_x_at | 634 | CEACAM1 | carcinoembryonic antigen-related cell adhesion molecule 1 (biliary glycoprotein) |
| 2.2 | 208651_x_at | 934 | CD24 | CD24 antigen (small cell lung carcinoma cluster 4 antigen) |
| 2.2 | 207205_at | 1089 | CEACAM4 | carcinoembryonic antigen-related cell adhesion molecule 4 |
| 2.2 | 205513_at | 6947 | TCN1 | transcobalamin I (vitamin B12 binding protein, R binder family) |
| 2.2 | 223670_s_at | 55363 | HEMGN | hemogen |
| 2.2 | 209211_at | 688 | KLF5 | Kruppel-like factor 5 (intestinal) |
| 2.2 | 211657_at | 4680 | CEACAM6 | carcinoembryonic antigen-related cell adhesion molecule 6 (non-specific cross reacting antigen) /// carcinoembryonic antigen-related cell adhesion molecule 6 (non-specific cross reacting antigen) |
| 2.2 | 204351_at | 6286 | S100P | S100 calcium binding protein P |
| 2.2 | 219281_at | 4482 | MSRA | methionine sulfoxide reductase A |
| 2.2 | 210244_at | 820 | CAMP | cathelicidin antimicrobial peptide |
| 2.2 | 212531_at | 3934 | LCN2 | lipocalin 2 (oncogene 24p3) |
| 2.2 | 221479_s_at | 665 | BNIP3L | BCL2/adenovirus E1B 19kDa interacting protein 3-like /// BCL2/adenovirus E1B 19kDa interacting protein 3-like |
| 2.2 | 224889_at | 2309 | FOXO3A | forkhead box O3A |
| 2.2 | 221765_at | 7357 | UGCG | --- |
| 2.2 | 231688_at | --- | --- | Transcribed locus |
| 2.2 | 201161_s_at | 8531 | CSDA | cold shock domain protein A |
| 2.2 | 227171_at | --- | --- | --- |
| 2.2 | 201160_s_at | 8531 | CSDA | cold shock domain protein A |
| 2.2 | 206871_at | 1991 | ELA2 | elastase 2, neutrophil |
| 2.2 | 220570_at | 56729 | RETN | resistin |
| 2.2 | 206177_s_at | 383 | ARG1 | arginase, liver |
| 2.2 | 206697_s_at | 3240 | HP | haptoglobin |
| 2.2 | 203021_at | 6590 | SLPI | secretory leukocyte protease inhibitor (antileukoproteinase) |
| 2.2 | 210004_at | 4973 | OLR1 | oxidised low density lipoprotein (lectin-like) receptor 1 |
| 2.2 | 203502_at | 669 | BPGM | 2,3-bisphosphoglycerate mutase /// 2,3-bisphosphoglycerate mutase |
| 2.2 | 202252_at | 5872 | RAB13 | RAB13, member RAS oncogene family |
| 2.2 | 205557_at | 671 | BPI | bactericidal/permeability-increasing protein |
| 2.2 | 235816_s_at | 266747 | Rgr | Ral-GDS related protein Rgr |
| 2.2 | 213515_x_at | 3048 | HBG2 | hemoglobin, gamma A /// hemoglobin, gamma A /// hemoglobin, gamma G /// hemoglobin, gamma G |
| 2.2 | 209369_at | 306 | ANXA3 | annexin A3 |
| 2.2 | 210395_x_at | 4635 | MYL4 | myosin, light polypeptide 4, alkali; atrial, embryonic |
| 2.2 | 207269_at | 1669 | DEFA4 | defensin, alpha 4, corticostatin |
| 2.2 | 216054_x_at | 4635 | MYL4 | myosin, light polypeptide 4, alkali; atrial, embryonic |
| 2.2 | 266_s_at | 934 | CD24 | CD24 antigen (small cell lung carcinoma cluster 4 antigen) |
| 2.2 | 204881_s_at | 7357 | UGCG | UDP-glucose ceramide glucosyltransferase |
| 2.2 | 206834_at | 3045 | HBD | hemoglobin, delta /// hemoglobin, delta |
| 2.2 | 205033_s_at | 1667 /// 1668 | DEFA1 /// DEFA3 | defensin, alpha 1, myeloid-related sequence /// defensin, alpha 3, neutrophil-specific |
| 2.2 | 208650_s_at | 934 | CD24 | CD24 antigen (small cell lung carcinoma cluster 4 antigen) |
| 2.2 | 212768_s_at | 10562 | OLFM4 | olfactomedin 4 |
| 2.2 | 210254_at | 932 | MS4A3 | membrane-spanning 4-domains, subfamily A, member 3 (hematopoietic cell-specific) |
| 2.2 | 206676_at | 1088 | CEACAM8 | carcinoembryonic antigen-related cell adhesion molecule 8 |
| 2.2 | 212987_at | 26268 | FBX09 | F-box protein 9 |
| 2.2 | 203116_s_at | 2235 | FECH | ferrochelatase (protoporphyria) |
| 2.2 | 219295_s_at | 26577 | PCOLCE2 | procollagen C-endopeptidase enhancer 2 |
| 2.3 | 215499_at | 5606 | MAP2K3 | mitogen-activated protein kinase kinase 3 /// mitogen-activated protein kinase kinase 3 |
| 2.3 | 223649_s_at | 51629 | CGI-69 | CGI-69 protein |
| 2.3 | 235684_s_at | 143686 | SESN3 | sestrin 3 |
| 2.3 | 202947_s_at | 2995 | GYPC | glycophorin C (Gerbich blood group) |
| 2.3 | 211781_x_at | --- | --- | --- |
| 2.3 | 241881_at | 343171 | OR2W3 | olfactory receptor, family 2, subfamily W, member 3 |
| 2.3 | 202468_s_at | 8727 | CTNNAL1 | catenin (cadherin-associated protein), alpha-like 1 |
| 2.3 | 231274_s_at | --- | --- | Mitochondrial solute carrier protein |
| 2.3 | 224057_s_at | 51078 | THAP4 | THAP domain containing 4 |
| 2.3 | 242335_at | --- | --- | FP15737 |
| 2.3 | 225319_s_at | 84923 | FLJ14775 | hypothetical protein FLJ14775 |
| 2.3 | 211040_x_at | 51512 | GTSE1 | G-2 and S-phase expressed 1 /// G-2 and S-phase expressed 1 |
| 2.3 | 226686_at | 493856 | LOC493856 | similar to RIKEN cDNA 1500009M05 gene |
| 2.3 | 212312_at | 598 | BCL2L1 | BCL2-like 1 /// BCL2-like 1 |
| 2.3 | 55705_at | 91300 | C19orf22 | chromosome 19 open reading frame 22 |
| 2.3 | 211699_x_at | 3039 /// 3040 | HBA1 /// HBA2 | hemoglobin, alpha 1 /// hemoglobin, alpha 1 /// hemoglobin, alpha 2 /// hemoglobm, alpha 2 |
| 2.3 | 217274_x_at | 4635 | MYL4 | myosin, light polypeptide 4, alkali; atrial, embryonic |
| 2.3 | 234611_at | 3964 | LGALS8 | Lectin, galactoside-binding, soluble, 8 (galectin 8) |
| 2.3 | 206665_s_at | 598 | BCL2L1 | BCL2-like 1 |
| 2.3 | 222730_s_at | 51201 | ZDHHC2 | zinc finger, DHHC domain containing 2 |
| 2.3 | 208353_x_at | 286 | ANK1 | ankyrin 1, erythrocytic |
| 2.3 | 215498_s_at | 5606 | MAP2K3 | mntogen-actmated protein kinase kinase 3 /// mitogen-activated protein kinase kinase 3 |
| 2.3 | 201052_s_at | 9491 | PSMF1 | proteasome (prosome, macropain) inhibitor subunit 1 (PI31) |
| 2.3 | 201141_at | 10457 | GPNMB | glycoprotein (transmembrane) nmb |
| 2.3 | 202364_at | 4601 | MXI1 | MAX interactor 1 /// MAX interactor 1 |
| 2.3 | 222528_s_at | 51312 | MSCP | mitochondrial solute carrier protein |
| 2.3 | 201039_s_at | 5886 | RAD23A | RAD23 homolog A (S. cerevislae) |
| 2.3 | 220757_s_at | 80700 | UBXD1 | UBX domain containing 1 |
| 2.3 | 204466_s_at | 6622 | SNCA | synuclein, alpha (non A4 component of amylold precursor) /// synuclein, alpha (non A4 component of amyloid precursor) |
| 2.3 | 211475_s_at | 573 | BAG1 | BCL2-associated athanogene |
| 2.3 | 230572_at | 152641 | FLJ30277 | hypothetical protein FLJ30277 |
| 2.3 | 209890_at | 10098 | TM4SF9 | tetraspanin 5 /// tetraspanin 5 |
| 2.3 | 211546_x_at | 6622 | SNCA | synuclein, alpha (non A4 component of amyloid precursor) |
| 2.3 | 241898_at | --- | --- | Transcribed locus, moderately similar to XP_517655.1 similar to KIAA0825 protein [Pan troglodytes] |
| 2.3 | 237819_at | 64764 | CREB3L2 | CAMP responsive element binding protein 3-like 2 |
| 2.3 | 221675_s_at | 56994 | CHPT1 | choline phosphotransferase 1 |
| 2.3 | 205305_at | 2267 | FGL1 | fibrinogen-like 1 |
| 2.3 | 235683_at | 143686 | SESN3 | sestrin 3 |
| 2.3 | 221425_s_at | 81689 | HBLD2 | HESB like domain containing 2 /// HESB like domain containing 2 |
| 2.3 | 221920_s_at | 51312 | MSCP | mitochondrial solute carrier protein |
| 2.3 | 216938_x_at | 1813 | DRD2 | dopamine receptor D2 |
| 2.3 | 201178_at | 25793 | FBX07 | F-box protein 7 |
| 2.3 | 204187_at | 2766 | GMPR | guanosine monophosphate reductase /// guanosine monophosphate reductase |
| 2.3 | 241143_at | --- | --- | Transcribed locus |
| 2.3 | 209046_s_at | 11345 | GABARAPL2 | GABA(A) receptor-associated protein-like 2 |
| 2.3 | 207667_s_at | 5606 | MAP2K3 | mitogen-activated protein kinase kinase 3 |
| 2.3 | 212479_s_at | 64795 | FLJ13910 | hypothetical protein FLJ13910 |
| 2.3 | 211630_s_at | 2937 | GSS | glutathione synthetase /// glutathione synthetase |
| 2.3 | 231933_at | 220972 | 8-Mar | membrane-associated ring finger (C3HC4) 8 |
| 2.3 | 205856_at | 6563 | SLC14Al | solute carrier family 14 (urea transporter), member 1 (Kldd blood group) |
| 2.3 | 229351_at | 29964 | C6orf49 | Chromosome 6 open reading frame 49 |
| 2.3 | 222529_at | 51312 | MSCP | mitochondrial solute carrier protein |
| 2.3 | 219672_at | 51327 | ERAF | erythroid associated factor |
| 2.3 | 224693_at | 116151 | C20orf108 | chromosome 20 open reading frame 108 |
| 2.3 | 240919_at | --- | --- | Transcribed locus, strongly similar to XP_509281.1 similar to SNRPF protein [Pan troglodytes] |
| 2.3 | 214273_x_at | 8131 | C16orf35 | chromosome 16 open reading frame 35 |
| 2.3 | 204659_s_at | 2671 | GFER | growth factor, augmenter of liver regeneration (ERV1 homolog, S. cerevisiae) |
| 2.3 | 202387_at | 573 | BAG1 | BCL2-associated athanogene /// BCL2-associated athanogene |
| 2.3 | 203040_s_at | 3145 | HMBS | hydroxymethylbilane synthase |
| 2.3 | 207827_x_at | 6622 | SNCA | synuclein, alpha (non A4 component of amyloid precursor) |
| 2.3 | 226179_at | --- | --- | FP15737 |
| 2.3 | 223012_at | 80700 | UBXD1 | UBX domain containing 1 |
| 2.3 | 223266_at | 55437 | ALS2CR2 | amyotrophnc lateral sclerosis 2 (Juvenile) chromosome region, candidate 2 |
| 2.3 | 204848_x_at | 3047 /// 3048 | HBG1 /// HBG2 | hemoglobin, gamma A /// hemoglobin,gamma A |
| 2.3 | 201912_s_at | 2935 | GSPT1 | G1 to S phase transition 1 /// G1 to S phase transition 1 |
| 2.3 | 209845_at | 23608 | MKRN1 | makorin, ring finger protein, 1 |
| 2.3 | 230208_at | --- | --- | Transcribed locus |
| 2.3 | 202448_s_at | 10444 | C9orf60 | chromosome 9 open reading frame 60 |
| 2.3 | 243024_at | --- | --- | --- |
| 2.3 | 240336_at | 445449 | HBM | hemoglobin mu chain |
| 2.3 | 215684_s_at | 84164 | ASCC2 | activating signal cointegrator 1 complex subunit 2 |
| 2.3 | 236750_at | 9369 | NRXN3 | Neurexin 3 |
| 2.3 | 235110_at | 11145 | HRASLS3 | HRAS-like suppressor 3 |
| 2.3 | 228361_at | 1870 | E2F2 | E2F transcription factor 2 |
| 2.3 | 203892_at | 10406 | WFDC2 | WAP four-disulfide core domain 2 |
| 2.3 | 203059_s_at | 9060 | PAPSS2 | 3'-phosphoadenosme 5'-phosphosulfate synthase 2 |
| 2.3 | 203966_s_at | 5494 | PPM1A | protein phosphatase 1A (formerly 2C), alpha isoform /// protein phosphatase 1A (formerly 2C), magnesium-dependent, alpha isoform |
| 2.3 | 217748_at | 51094 | ADIPOR1 | adiponectin receptor 1 /// adiponectin receptor 1 |
| 2.3 | 223548_at | 54823 | Clorf26 | chromosome 1 open reading frame 26 |
| 2.3 | 204505_s_at | 2039 | EPB49 | erythrocyte membrane protein band 4.9 (dematin) |
| 2.3 | 223124_s_at | 57095 | HT014 | HT014 |
| 2.3 | 212512_s_at | 10498 | CARM1 | coactivator-associated arginine methyltransferase 1 |
| 2.3 | 219458_s_at | 63899 | NSUN3 | NOL1/NOP2/SUn domain family, member 3 |
| 2.3 | 221824_s_at | 220972 | 8-Mar | membrane-associated ring finger (C3HC4) 8 |
| 2.3 | 241907_at | 2719 | GPC3 | Glypican 3 |
| 2.3 | 221932_s_at | 51218 | C14orf87 | chromosome 14 open reading frame 87 |
| 2.3 | 238124_at | 127294 | MYOM3 | Myomesin family, member 3 |
| 2.3 | 218030_at | 28964 | GIT1 | G protein-coupled receptor kinase interactor 1 |
| 2.3 | 203661_s_at | 7111 | TMOD1 | tropomodulin 1 |
| 2.3 | 200075_s_at | 2987 | GUK1 | guanylate kinase 1 /// guanylate kinase 1 |
| 2.3 | 209018_s_at | 65018 | PINK1 | PTEN induced putative kinase 1 |
| 2.3 | 202568_s_at | 4140 | MARK3 | MAP/microtubule affinity-regulating kinase 3 |
| 2.3 | 201285_at | 23608 | MKRN1 | makorin, ring finger protein, 1 /// makorin, ring finger protein, 1 |
| 2.3 | 211590_x_at | 6915 | TBXA2R | thromboxane A2 receptor |
| 2.3 | 206302_s_at | 11163 | NUDT4 | nudix (nucleoside diphosphate linked molety X)-type motif 4 |
| 2.3 | 205012_s_at | 3029 | HAGH | hydroxyacylglutathione hydrolase |
| 2.3 | 227185_at | --- | --- | Homo sapiens, clone IMAGE:3632546, mRNA |
| 2.3 | 205570_at | 5305 | PIP5K2A | phosphatidylinositol-4-phosphate-5-kinase, type II, alpha |
| 2.3 | 226009_at | 25911 | DPCD | deleted In a mouse model of primary ciliary dyskinesia |
| 2.3 | 218847_at | 10644 | IMP-2 | IGF-II mRNA-binding protein 2 |
| 2.3 | 202130_at | 8780 | RIOK3 | RIO kinase 3 (yeast) /// RIO kinase 3 (yeast) |
| 2.3 | 231078_at | 51312 | MSCP | --- |
| 2.3 | 212340_at | 286451 | MGC21416 | hypothetical protein MGC21416 |
| 2.3 | 215047_at | 25893 | TRIM58 | tripartite motif-containing 58 |
| 2.3 | 202974_at | 4354 | MPP1 | membrane protein, palmitoylated 1, 55kDa |
| 2.3 | 78383_at | 10210 | TOPORS | Topoisomerase I binding, arginine/serine-rich |
| 2.3 | 223179_at | 83719 | YPEL3 | yippee-like 3 (Drosophila) |
| 2.3 | 205592_at | 6521 | SLC4Al | Solute carrier family 4, anion exchanger, member 1 (erythrocyte membrane protein band 3, Diego blood group) |
| 2.3 | 204131_s_at | 2309 | FOXO3A | forkhead box O3A |
| 2.3 | 220466_at | 80071 | FLJ13215 | coiled-coil domain containing 15 |
| 2.3 | 241644_at | 81893 | LAT1-3TM | LAT1-3TM protein |
| 2.3 | 212445_s_at | 23327 | NEDD4L | neural precursor cell expressed, developmentally down-regulated 4-like |
| 2.3 | 204467_s_at | 6622 | SNCA | synuclein, alpha (non A4 component of amyloid precursor) /// synuclein, alpha (non A4 component of amyloid precursor) |
| 2.3 | 235095_at | 146439 | LOC146439 | hypothetical LOC146439 |
| 2.3 | 202131_s_at | 8780 | RIOK3 | RIO kinase 3 (yeast) /// RIO kinase 3 (yeast) |
| 2.3 | 242841_at | --- | --- | Full length insert CDNA clone YS02G11 |
| 2.3 | 217499_x_at | --- | --- | PREDICTED: Homo sapiens olfactory receptor, family 7, subfamnly E, member 31 pseudogene (OR7E31P), mRNA |
| 2.3 | 237163_x_at | --- | --- | --- |
| 2.3 | 217362_x_at | 3128 | HLA-DRB6 | major histocompatibility complex, class II, DR beta 6 (pseudogene) |
| 2.3 | 217144_at | 7314 | UBB | ubiquitin B |
| 2.3 | 232579_at | 80853 | KIAA1718 | KIAA1718 protein |
| 2.3 | 238757_at | 80174 | DRF1 | Dbf4-related factor 1 |
| 2.3 | 207801_s_at | 9921 | RNF10 | ring finger protein 10 |
| 2.3 | 230999_at | 399972 | --- | Hypothetical gene supported by AK096370 |
| 2.3 | 205950_s_at | 759 | CA1 | carbonic anhydrase I |
| 2.3 | 224690_at | 116151 | C20orf108 | chromosome 20 open reading frame 108 |
| 2.3 | 203115_at | 2235 | FECH | ferrochelatase (protoporphyria) |
| 2.3 | 224789_at | 25853 | WDR40A | WD repeat domain 40A |
| 2.3 | 225074_at | 84932 | RAB2B | RAB2B, member RAS oncogene family |
| 2.3 | 234011_at | --- | --- | hypothetical protein MGC16384 |
| 2.3 | 222446_s_at | 25825 | BACE2 | beta-site APP-cleaving enzyme 2 |
| 2.3 | 214433_s_at | 8991 | SELENBP1 | selenium binding protein 1 /// selenium binding protein 1 |
| 2.3 | 236667_at | 144404 | LOC144404 | hypothetical LOC144404 |
| 2.3 | 208916_at | 6510 | SLC1A5 | solute carrier family 1 (neutral amino acid transporter), member 5 |
| 2.3 | 204419_x_at | 3048 | HBG2 | hemoglobin, gamma A /// hemoglobin, gamma A /// hemoglobin, gamma G /// hemoglobin, gamma G |
| 2.3 | 200896_x_at | 3068 | HDGF | hepatoma-derived growth factor (high-mobility group protein 1-like) |
| 2.3 | 220659_s_at | 55262 | FLJ10925 | hypothetical protein FLJ10925 |
| 2.3 | 223409 at | --- | --- | --- |
| 2.3 | 213843_x_at | 6535 | SLC6A8 | solute carrier family 6 (neurotransmitter transporter, creatine), member 8 |
| 2.3 | 212829_at | --- | --- | CDNA FLJ13267 fis, clone OVARC1000964 /// CDNA FLJ13267 fis, clone OVARC1000964 |
| 2.3 | 210504_at | 10661 | KLF1 | Kruppel-like factor 1 (erythroid) |
| 2.3 | 221748_s_at | 7145 | TNS | tensin /// tensin |
| 2.3 | 218136_s_at | 51312 | MSCP | mitochondrial solute carrier protein |
| 2.3 | 215438_x_at | 2935 | GSPT1 | G1 to S phase transition 1 |
| 2.3 | 226811_at | 54855 | FAM46C | family with sequence similarity 46, member C |
| 2.3 | 228897_at | 91319 | DERL3 | Derl-like domain family, member 3 |
| 2.3 | 212540_at | 997 | CDC34 | cell division cycle 34 |
| 2.3 | 210088_x_at | 4635 | MYL4 | myosin, light polypeptide 4, alkali; atrial, embryonic |
| 2.4 | 208746_x_at | 10632 | ATP5L | ATP synthase, H+ transporting, mitochondrial F0 complex, subunit g |
| 2.4 | 201230_s_at | 10425 | ARIH2 | ariadne homolog 2 (Drosophila) |
| 2.4 | 235134_at | --- | --- | CDNA FLJ30156 fis, clone BRACE2000487 |
| 2.4 | 225220_at | --- | --- | CDNA clone IMAGE:4184613, partial cds |
| 2.4 | 221475_s_at | 6138 | RPL15 | ribosomal protein L15 |
| 2.4 | 221476_s_at | 6138 | RPL15 | ribosomal protein L15 |
| 2.4 | 214097_at | 6227 | RPS21 | ribosomal protein S21 |
| 2.4 | 208656_s_at | 10983 | CCNI | cyclin I |
| 2.4 | 212042_x_at | 6129 | RPL7 | ribosomal protein L7 |
| 2.4 | 225190_x_at | 6165 | RPL35A | ribosomal protein L35a |
| 2.4 | 217256_x_at | --- | --- | --- |
| 2.4 | 213581_at | 5134 | PDCD2 | programmed cell death 2 |
| 2.4 | 221494_x_at | 27335 | EIF3S12 | eukaryotic translation initiation factor 3, subunit 12 |
| 2.4 | 200024_at | --- | --- | --- |
| 2.4 | 201338_x_at | 2971 | GTF3A | general transcription factor IIIA |
| 2.4 | 220755_s_at | 50854 | C6orf48 | chromosome 6 open reading frame 48 |
| 2.4 | 213041_s_at | 513 | ATP5D | ATP synthase, H+ transporting, mitochondrial F1 complex, delta subunit |
| 2.4 | 200063_s_at | 4869 | IVPM1 | nucleophosmin (nucleolar phosphoprotein B23, numatrin) /// nucleophosmin (nucleolar phosphoprotein B23, numatrin) |
| 2.4 | 213846_at | 1350 | cox7c | cytochrome c oxidase subunit VIIc |
| 2.4 | 201993_x_at | 9987 | HNRPDL | heterogeneous nuclear ribonucleoprotein D-like |
| 2.4 | 216421_at | --- | --- | --- |
| 2.4 | 200834_s_at | 6227 | RPS21 | ribosomal protein S21 |
| 2.4 | 208635_x_at | 4666 | NACA | nascent-polypeptide-associated complex alpha polypeptide |
| 2.4 | 216032_s_at | 51614 | SDBCAG84 | serologically defined breast cancer antigen 84 |
| 2.4 | 200818_at | 539 | ATP50 | ATP synthase, H+ transporting, mitochondrial F1 complex, O subunit (oligomycin sensitivity conferring protein) |
| 2.4 | 217340_at | --- | --- | --- |
| 2.4 | 225547_at | --- | --- | U87HG mRNA, complete sequence |
| 2.4 | 212017_at | 130074 | LOC130074 | hypothetical protein LOC130074 |
| 2.4 | 222099_s_at | 26065 | C19orf13 | family with sequence similarity 61, member A |
| 2.4 | 217906_at | 23588 | KLHDC2 | kelch domain containing 2 |
| 2.4 | 208752_x_at | 4673 | NAP1L1 | nucleosome assembly protein 1-like 1 |
| 2.4 | 213080_x_at | 6125 | RPL5 | ribosomal protein L5 |
| 2.4 | 201922_at | 10412 | TINP1 | TGF beta-inducible nuclear protein 1 |
| 2.4 | 202365_at | 84747 | MGC5139 | hypothetical protein MGC5139 |
| 2.4 | 200093_s_at | 3094 | HINT1 | histidine triad nucleotide binding protein 1 /// histidine triad nucleotide binding protein 1 |
| 2.4 | 200034_s_at | 6128 | RPL6 | ribosomal protein L6 /// ribosomal protein L6 |
| 2.4 | 228049_x_at | --- | --- | Transcribed locus, weakly similar to XP_347139.1 LOC363435 [Rattus norvegicus] |
| 2.4 | 200631_s_at | 6418 | SET | SET translocation (myeloid leukemia-associated) |
| 2.4 | 205849_s_at | 7381 | UQCRB | ubiquinol-cytochrome c reductase binding protein |
| 2.4 | 217988_at | 57820 | CCNB1IP1 | cyclin B1 interacting protein 1 |
| 2.4 | 203818_s_at | 10946 | SF3A3 | splicing factor 3a, subunit 3, 60kDa |
| 2.4 | 201272_at | 231 | AKR1B1 | aldo-keto reductase family 1, member B1 (aldose reductase) |
| 2.4 | 200936_at | 6132 | RPL8 | ribosomal protein L8 |
| 2.4 | 224763_at | 6167 | RPL37 | ribosomal protein L37 |
| 2.4 | 214527_s_at | 10084 | PQBP1 | polyglutamine binding protein 1 |
| 2.4 | 220960_x_at | 6146 | RPL22 | ribosomal protein L22 |
| 2.4 | 208826_x_at | 3094 | HINT1 | histidine triad nucleotide binding protein 1 |
| 2.4 | 201592_at | 8667 | EIF3S3 | eukaryotic translation initiation factor 3, subunit 3 gamma, 40kDa |
| 2.4 | 227878_s_at | 84266 | SPATA11 | spermatogenesis associated 11 |
| 2.4 | 200036_s_at | 4736 | RPL10A | ribosomal protein L10a /// ribosomal protein L10a |
| 2.4 | 216570_x_at | --- | --- | --- |
| 2.4 | 210501_x_at | 27335 | EIF3S12 | eukaryotic translation initiation factor 3, subunit 12 |
| 2.4 | 213892_s_at | 353 | APRT | adenine phosphoribosyltransferase |
| 2.4 | 208646_at | 6208 | RPS14 | PRO2640 |
| 2.4 | 201812_s_at | 201725 /// 54543 | TOMM7 /// LOC201725 | translocase of outer mitochondrial membrane 7 homolog (yeast) /// hypothetical protein LOC201725 |
| 2.4 | 200909_s_at | 6181 | RPLP2 | ribosomal protein, large P2 |
| 2.4 | 213414_s_at | 6223 | RPS19 | ribosomal protein S19 |
| 2.4 | 208117_s_at | 81887 | LAS1L | LAS1-like (S. cerevisiae) /// LAS1-like (S. cerevisiae) |
| 2.4 | 200826_at | 6633 | SNRPD2 | small nuclear ribonucleoprotein D2 polypeptide 16.5kDa |
| 2.4 | 219155_at | 26207 | PITPNC1 | phosphatidylinositol transfer protein, cytoplasmic 1 |
| 2.4 | 208697_s_at | 3646 | EIF3S6 | eukaryotic translation initiation factor 3, subunit 6 48kDa |
| 2.4 | 236208_at | 4338 | MOCS2 | Molybdenum cofactor synthesis 2 |
| 2.4 | 200094_s_at | 1938 | EEF2 | eukaryotic translation elongation factor 2 /// eukaryotic translation elongation factor 2 |
| 2.4 | 213941_x_at | 6201 | RPS7 | ribosomal protein S7 |
| 2.4 | 202408_s_at | 26121 | PRPF31 | PRP31 pre-mRNA processing factor 31 homolog (yeast) |
| 2.4 | 200002_at | 11224 | RPL35 | ribosomal protein L35 /// ribosomal protein L35 |
| 2.4 | 217969_at | 738 | Cllorf2 | chromosome 11 open reading frame2 |
| 2.4 | 218188_s_at | 26517 | TIMM13 | translocase of inner mitochondrial membrane 13 homolog (yeast) |
| 2.4 | 217379_at | --- | --- | --- |
| 2.4 | 210212_x_at | 4515 | MTCP1 | mature T-cell proliferation 1 |
| 2.4 | 213687_s_at | 6165 | RPL35A | ribosomal protein L35a |
| 2.4 | 212197_x_at | 23164 | M-RIP | myosin phosphatase-Rho interacting protein |
| 2.4 | 220753_s_at | 51084 | CRYL1 | crystallin, lambda 1 |
| 2.4 | 200937_s_at | 6125 | RPL5 | ribosomal protein L5 |
| 2.4 | 211623_s_at | 2091 | FBL | fibrillarin /// fibrillarin |
| 2.4 | 200651_at | 10399 | GNB2L1 | guanine nucleotide binding protein (G protein), beta polypeptide 2-like 1 |
| 2.4 | 201345_s_at | 7322 | UBE2D2 | ubiquitin-conjugating enzyme E2D 2 (UBC4/5 homolog, yeast) |
| 2.4 | 214317_x_at | 6203 | RPS9 | Ribosomal protein S9 |
| 2.4 | 211666_x_at | 6122 | RPL3 | ribosomal protein L3 /// ribosomal protein L3 |
| 2.4 | 200074_s_at | 9045 | RPL14 | ribosomal protein L14 /// ribosomal protein L14 |
| 2.4 | 200081_s_at | 6194 | RPS6 | ribosomal protein S6 /// ribosomal protein S6 |
| 2.4 | 210453_x_at | 10632 | ATP5L | ATP synthase, H+ transporting, mitochondrial FO complex, subunit g |
| 2.4 | 200082_s_at | 6201 | RPS7 | ribosomal protein S7 /// ribosomal protein S7 |
| 2.4 | 218253_s_at | 1939 | LGTN | ligatin |
| 2.4 | 221548_s_at | 80895 | ILKAP | integrin-linked kinase-associated serine/threonine phosphatase 2C |
| 2.4 | 212933_x_at | 6137 | RPL13 | ribosomal protein L13 |
| 2.4 | 203113_s_at | 1936 | EEF1D | eukaryotic translation elongation factor 1 delta (guanine nucleotide exchange protein) |
| 2.4 | 200858_s_at | 6202 | RPS8 | ribosomal protein S8 |
| 2.4 | 216862_s_at | 4515 | MTCP1 | mature T-cell proliferation 1 |
| 2.4 | 234875_at | --- | --- | --- |
| 2.4 | 212955_s_at | 5438 | POLR2I | polymerase (RNA) II (DNA directed) polypeptide I, 14.5kDa |
| 2.4 | 212716_s_at | 27335 | EIF3S12 | eukaryotic translation initiation factor 3, subunit 12 |
| 2.4 | 213166_x_at | --- | --- | hypothetical protein FLJ14346 |
| 2.4 | 221726_at | 6146 | RPL22 | ribosomal protein L22 |
| 2.4 | 209104_s_at | 55651 | NOLA2 | nucleolar protein family A, member 2 (H/ACA small nucleolar RNPs) |
| 2.4 | 211939_x_at | 689 | BTF3 | basic transcription factor 3 |
| 2.4 | 240806_at | 6138 | RPL15 | Ribosomal protein L15 |
| 2.4 | 225029_at | --- | --- | CDNA clone IMAGE:4686928, partial cds |
| 2.4 | 204772_sat | 7270 | TTF1 | transcription termination factor, RNA polymerase I |
| 2.4 | 212114_at | 387869 | --- | Similar to microtubule-associated proteins 1A/1B light chain 3 |
| 2.4 | 200013_at | 6152 | RPL24 | ribosomal protein L24 /// ribosomal protein L24 |
| 2.4 | 210825_s_at | 5037 | PBP | prostatic binding protein |
| 2.4 | 219817_at | --- | --- | apoptosis-related protein PNAS-1 |
| 2.4 | 209538_at | 7580 | ZNF32 | zinc finger protein 32 (KOX 30) |
| 2.4 | 200705_s_at | 1933 | EEF1B2 | eukaryotic translation elongation factor 1 beta 2 |
| 2.4 | 207132_x_at | 5204 | PFDN5 | prefoldin 5 |
| 2.4 | 238026_at | --- | --- | Ribosomal protein L35a |
| 2.4 | 216505_x_at | --- | --- | --- |
| 2.4 | 218495_at | 8409 | UXT | ubiquitously-expressed transcript |
| 2.4 | 214042_s_at | 6146 | RPL22 | ribosomal protein L22 |
| 2.4 | 218421_at | 64781 | CERK | ceramide kinase |
| 2.4 | 214167_s_at | 6175 | RPLPO | ribosomal protein, large, P0 |
| 2.4 | 200017_at | 6233 | RPS27A | ribosomal protein S27a /// ribosomal protein S27a |
| 2.4 | 200023_s_at | 8665 | EIF3S5 | eukaryotic translation initiation factor 3, subunit 5 epsilon, 47kDa /// eukaryotic translation initiation factor 3, subunit 5 epsilon, |
| | | | | 47kDa |
| 2.4 | 200715_x_at | 23521 | RPL13A | ribosomal protein L13a |
| 2.4 | 214271_x_at | 6136 | RPL12 | ribosomal protein L12 |
| 2.4 | 202649_x_at | 6223 | RPS19 | ribosomal protein S19 |
| 2.4 | 221519_at | 6468 | SHFM3 | F-box and WD-40 domain protein 4 |
| 2.4 | 201258_at | 6217 | RPS16 | ribosomal protein S16 |
| 2.4 | 226336_at | 5478 | PPIA | --- |
| 2.4 | 211724_x_at | 54468 | FLJ20323 | hypothetical protein FLJ20323 /// hypothetical protein FLJ20323 |
| 2.4 | 212995_x_at | 80097 | FLJ14346 | hypothetical protein FLJ14346 |
| 2.4 | 210792_x_at | 10572 | SIVA | CD27-binding (Siva) protein |
| 2.4 | 217747_s_at | 6203 | RPS9 | ribosomal protein S9 |
| 2.4 | 200089_s_at | 6124 | RPL4 | ribosomal protein L4 /// ribosomal protein L4 |
| 2.4 | 207721_x_at | 3094 | HINT1 | histidine triad nucleotide binding protein 1 |
| 2.4 | 219762_s_at | 25873 | RPL36 | ribosomal protein L36 |
| 2.4 | 201406_at | 6173 | RPL36A | ribosomal protein L36a |
| 2.4 | 208517_x_at | 689 | BTF3 | basic transcription factor 3 |
| 2.4 | 200949_x_at | 6224 | RPS20 | ribosomal protein S20 |
| 2.4 | 224574_at | 124944 | MGC49942 | hypothetical protein MGC49942 /// similar to DNA segment, Chr 11, Brigham & Womens Genetics 0434 expressed |
| 2.4 | 214173_x_at | 8725 | C19orf2 | chromosome 19 open reading frame 2 |
| 2.4 | 225312_at | 170622 | COMMD6 | COMM domain containing 6 |
| 2.5 | 205144_at | 84179 | FLJ22269 | hypothetical protein FLJ22269 |
| 2.5 | 207308_at | 6579 | SLC01A2 | solute carrier organic anion transporter family, member 1A2 |
| 2.5 | 201205_at | --- | --- | ribosome binding protein 1 homolog 180kDa (dog) |
| 2.5 | 237619_at | 222826 | C6orf146 | chromosome 6 open reading frame 146 |
| 2.5 | 204813_at | 5602 | MAPK10 | mitogen-activated protein kinase 10 |
| 2.5 | 49327_at | 60626 | RIC-8 | Likely ortholog of mouse synembryn |
| 2.5 | 210247_at | 6854 | SYN2 | synapsin II |
| 2.5 | 207951_at | 1447 | CSN2 | casein beta |
| 2.5 | 243515_at | --- | --- | Chromosome 22 open reading frame 23 |
| 2.5 | 207665_at | 8747 | ADAM21 | a disintegrin and metalloproteinase domain 21 |
| 2.5 | 212729_at | 1741 | DLG3 | discs, large homolog 3 (neuroendocrine-dlg, Drosophila) |
| 2.5 | 208448_x_at | 3449 | IFNA16 | interferon, alpha 16 |
| 2.5 | 209436_at | 10418 | SPON1 | spondin 1, extracellular matrix protein |
| 2.5 | 216159_s_at | --- | --- | CDNA FLJ13695 fis, clone PLACE2000124 |
| 2.5 | 211362_s_at | 5275 | SERPINB13 | serine (or cysteine) proteinase inhibitor, clade B (ovalbumin), member 13 |
| 2.5 | 215366_at | 23161 | SNX13 | sorting nexin 13 |
| 2.5 | 213900_at | 9413 | C9orf61 | chromosome 9 open reading frame 61 |
| 2.5 | 213929_at | --- | --- | Homo sapiens, Similar to likely ortholog of yeast ARV1, clone IMAGE:4733238, mRNA |
| 2.5 | 242346_x_at | --- | --- | --- |
| 2.5 | 215126_at | --- | --- | CDNA FLJ42949 fis, clone BRSTN2006583 |
| 2.5 | 234956_at | --- | --- | hypothetical protein LOC93444 |
| 2.5 | 205949_at | 759 | CA1 | carbonic anhydrase I |
| 2.5 | 224099_at | 90134 | KCNH7 | potassium voltage-gated channel, subfamily H (eag-related), member 7 |
| 2.5 | 215557_at | 79966 | SCD4 | Stearoyl-CoA desaturase 4 |
| 2.5 | 58367_s_at | 79744 | ZNF419 | zinc finger protein 419 |
| 2.5 | 211515_s_at | 25778 | RIPK5 | receptor interacting protein kinase 5 |
| 2.5 | 220244_at | 29931 | LOH3CR2A | loss of heterozygosity, 3, chromosomal region 2, gene A |
| 2.5 | 212425_at | 9522 | SCAMP1 | Secretory carrier membrane protein 1 |
| 2.5 | 205166_at | 726 | CAPN5 | calpain 5 |
| 2.5 | 216739_at | --- | --- | CDNA: FLJ20874 fis, clone ADKA02818 |
| 2.5 | 203788_s_at | 10512 | SEMA3C | sema domain, immunoglobulin domain (Ig), short basic domain, secreted, (semaphorin) 3C |
| 2.5 | 214087_s_at | 4604 | MYBPC1 | myosin binding protein C, slow type |
| 2.5 | 217269_s_at | 5651 | PRSS7 | protease, serine, 7 (enterokinase) |
| 2.5 | 221038_at | --- | --- | Src-associated protein SAW |
| 2.5 | 216213_at | --- | --- | NIMA (never In mitosis gene a)-related kinase 1 |
| 2.5 | 232405_at | 9871 | SEC24D | SEC24 related gene family, member D (S. cerevisiae) |
| 2.5 | 210816_s_at | 1534 | CYB561 | cytochrome b-561 |
| 2.5 | 205729_at | 9180 | OSMR | oncostatin M receptor |
| 2.5 | 207919_at | 417 | ART1 | ADP-ribosyltransferase 1 |
| 2.5 | 208486_at | 1816 | DRD5 | dopamine receptor D5 |
| 2.5 | 208035_at | 2916 | GRM6 | glutamate receptor, metabotropic 6 |
| 2.5 | 234509_at | --- | --- | MRNA; cDNA DKFZp564B206 (from clone DKFZp564B206) |
| 2.5 | 202975_s_at | 22836 | RHOBTB3 | Rho-related BTB domain containing 3 |
| 2.5 | 205189_s_at | 2176 | FANCC | Fanconi anemia, complementation group C |
| 2.5 | 207684_at | 6911 | TBX6 | T-box 6 |
| 2.5 | 219535_at | 30811 | HUNK | hormonally upregulated Neu-associated kinase |
| 2.5 | 205347_s_at | 11013 | TMSNB | thymosin-like 8 |
| 2.5 | 208039_at | --- | --- | --- |
| 2.5 | 205636_at | --- | --- | SH3-domain GRB2-like 3 |
| 2.5 | 206306_at | 6263 | RYR3 | ryanodine receptor 3 |
| 2.5 | 208578_at | 6336 | SCN10A | sodium channel, voltage-gated, type X, alpha |
| 2.5 | 240795_at | --- | --- | Homo sapiens, clone IMAGE:5288566, mRNA |
| 2.5 | 206940_s_at | 5457 | POU4F1 | POU domain, class 4, transcription factor 1 |
| 2.5 | 215775_at | 7057 | THBS1 | Thrombospondin 1 |
| 2.5 | 207408_at | 9389 | SLC22A14 | solute carrier family 22 (organic cation transporter), member 14 |
| 2.5 | 204602_at | 22943 | DKK1 | dickkopf homolog 1 (Xenopus laevis) |
| 2.5 | 226211_at | 55384 | MEG3 | maternally expressed 3 |
| 2.5 | 206377_at | 2295 | FOXF2 | forkhead box F2 |
| 2.5 | 160020_at | 4323 | MMP14 | matrix metalloproteinase 14 (membrane-inserted) |
| 2.5 | 207041_at | 10747 | MASP2 | mannan-binding lectin serine protease 2 |
| 2.5 | 207176_s_at | 941 | CD80 | CD80 antigen (CD28 antigen ligand 1, B7-1 antigen) |
| 2.5 | 204987_at | 3698 | ITIH2 | inter-alpha (globulin) inhibitor H2 |
| 2.5 | 212558_at | 10252 | SPRY1 | sprouty homolog 1, antagonist of FGF signaling (Drosophila) |
| 2.5 | 203666_at | 6387 | CXCL12 | chemokine (C-X-C motif) ligand 12 (stromal cell-derived factor 1) |
| 2.5 | 220853_at | 79712 | GTDC1 | glycosyltransferase-like domain containing 1 |
| 2.5 | 220509_at | 64062 | C13orf10 | chromosome 13 open reading frame 10 |
| 2.5 | 206533_at | 1138 | CHRNA5 | cholinergic receptor, nicotinic, alpha polypeptide 5 |
| 2.5 | 242207_at | 64768 | C9orf12 | Chromosome 9 open reading frame 12 |
| 2.5 | 217558_at | 1559 | CYP2C9 | cytochrome P450, family 2, subfamily C, polypeptide 9 |
| 2.5 | 216678_at | --- | --- | WD repeat domain 10 |
| 2.5 | 218692_at | 55638 | FLJ20366 | hypothetical protein FLJ20366 |
| 2.5 | 232193_at | 2952 | GSTT1 | Glutathione S-transferase theta 1 |
| 2.5 | 221453_at | 57818 | G6PC2 | glucose-6-phosphatase, catalytic, 2 |
| 2.5 | 210271_at | 4761 | NEUROD2 | neurogenic differentiation 2 |
| 2.5 | 217637_at | --- | --- | Full length insert CDNA clone ZE05A03 |
| 2.5 | 207152_at | 4915 | NTRK2 | neurotrophic tyrosine kinase, receptor, type 2 |
| 2.5 | 234534_at | --- | --- | Monoamine oxidase A |
| 2.5 | 214734_at | 23086 | SLAC2-B | SLAC2-B |
| 2.5 | 216368_s_at | --- | --- | collagen, type IV, alpha 3 (Goodpasture antigen) |
| 2.5 | 65521_at | 51619 | UBE2D4 | ubiquitin-conjugating enzyme E2D 4 (putative) |
| 2.5 | 234206_at | --- | --- | CDNA: FLJ21271 fis, clone COL01751 |
| 2.5 | 214222_at | 56171 | DNAH7 | dynein, axonemal, heavy polypeptide 7 |
| 2.5 | 216809_at | --- | --- | cylicin, basic protein of sperm head cytoskeleton 1 |
| 2.5 | 233977_at | 80000 | KIAA1772 | KIAA1772 |
| 2.5 | 59705_at | 51540 | SCLY | selenocysteine lyase |
| 2.5 | 236861_at | --- | --- | Ngg1 interacting factor 3 like 1 binding protein 1 |
| 2.5 | 32811_at | 4641 | MYO1C | myosin IC |
| 2.5 | 1598_g_at | 2621 | GA56 | growth arrest-specific 6 |
| 2.5 | 206212_at | 1358 | CPA2 | carboxypeptidase A2 (pancreatic) |
| 2.5 | 241016_at | 8452 | CUL3 | Cullin 3 |
| 2.5 | 221460_at | 4993 | OR2C1 | olfactory receptor, family 2, subfamily C, member 1 |
| 2.5 | 242743_at | 3566 | IL4R | Interleukin 4 receptor |
| 2.5 | 211916_s_at | 4640 | MY01A | myosin IA /// myosin IA |
| 2.5 | 243427_at | 23348 | DOCK9 | Dedicator of cytokinesis 9 |
| 2.5 | 219941_at | 55266 | TMEM19 | transmembrane protein 19 |
| 2.5 | 206704_at | 1184 | CLCN5 | chloride channel 5 (nephrolithiasis 2, X-linked, Dent disease) |
| 2.5 | 214775_at | 23138 | N4BP3 | Nedd4 binding protein 3 |
| 2.5 | 220944_at | 57115 | PGLYRP4 | peptidoglycan recognition protein 4 |
| 2.5 | 214923_at | 51382 | ATP6V1D | ATPase, H+ transporting, lysosomal 34kDa, V1 subunit D |
| 2.5 | 220158_at | 56891 | LGALS14 | lectin, galactoside-binding, soluble, 14 |
| 2.5 | 58900_at | 222070 | LOC222070 | hypothetical protein LOC222070 |
| 2.5 | 33768_at | 1762 | DMWD | Dystrophia myotonica-containing WD repeat motif |
| 2.5 | 71933_at | 7475 | WNT6 | wingless-type MMTV integration site family, member 6 |
| 2.5 | 243424_at | 55553 | SOX6 | SRY (sex determining region Y)-box 6 |
| 2.5 | 220446_s_at | 10164 | CHST4 | carbohydrate (N-acetylglucosamine 6-O) sulfotransferase 4 |
| 2.5 | 215756_at | 440708 | --- | hypothetical gene supported by AK024293 |
| 2.5 | 206263_at | 2329 | FMO4 | flavin containing monooxygenase 4 |
| 2.5 | 210436_at | 10694 | CCT8 | chaperonin containing TCP1, subunit 8 (theta) |
| 2.5 | 205847_at | 64063 | PRSS22 | protease, serine, 22 |
| 2.5 | 237114_at | 26091 | HERC4 | CUB and Sushi multiple domains 1 |
| 2.5 | 234685_x_at | 85286 | KRTAP4-9 | keratin associated protein 4-9 |
| 2.5 | 221170_at | 59340 | HRH4 | histamine receptor H4 |
| 2.5 | 205328_at | 9071 | CLDN10 | claudin 10 |
| 2.5 | 220375_s_at | --- | --- | --- |
| 2.5 | 207303_at | 5137 | PDE1C | phosphodnesterase 1C, calmodulin-dependent 70kDa |
| 2.5 | 240638_at | 10154 | PLXNC1 | Plexin C1 |
| 2.5 | 214462_at | 9306 | SOCS6 | suppressor of cytokine signaling 6 |
| 2.5 | 221738_at | 57148 | KIAA1219 | KIAA1219 protein /// KIAA1219 protein |
| 2.5 | 230503_at | --- | --- | Transcribed locus |
| 2.5 | 205357_s_at | 185 | AGTR1 | anglotensin II receptor, type 1 |
| 2.5 | 211572_s_at | 9962 | SLC23A2 | solute carrier family 23 (nucleobase transporters), member 2 |
| 2.5 | 215511_at | --- | --- | transcription factor 20 (AR1) |
| 2.5 | 211332_x_at | 3077 | HFE | hemochromatosis |
| 2.5 | 216049_at | 22836 | RHOBTB3 | Rho-related BTB domain containing 3 |
| 2.5 | 44783_s_at | 23462 | HEY1 | hairy/enhancer-of-split related with YRPW motif 1 |
| 2.5 | 224997_x_at | 283120 | H19 | H19, imprinted maternally expressed untranslated mRNA |
| 2.5 | 202488_s_at | 5349 | FXYD3 | FXYD domain containing ion transport regulator 3 |
| 2.5 | 228228_at | 147906 | MGC15476 | thymus expressed gene 3-like |
| 2.5 | 216540_at | --- | --- | T cell receptor alpha chain (TCRA) mRNA, TCRA-AV1S2 J32 AC allele |
| 2.5 | 239949_at | 55258 | FLJ10916 | hypothetical protein FLJ10916 |
| 2.5 | 236825_at | 8419 | BFSP2 | Beaded filament structural protein 2, phakinin |
| 2.5 | 207096_at | 6291 | SAA4 | serum amyloid A4, constitutive |
| 2.5 | 212580_at | 831 | CAST | Type 1 tumor necrosis factor receptor shedding aminopeptidase regulator |
| 2.5 | 207466_at | 51083 | GAL | galanin |
| 2.5 | 217477_at | --- | --- | phosphatidylinositol-4-phosphate 5-kinase, type I, beta |
| 2.5 | 217020_at | 5915 | RARB | retinoic acid receptor, beta |
| 2.5 | 234239_at | --- | --- | CDNA: FLJ21668 fis, clone COL08982 |
| 2.5 | 225299_at | 4645 | MY05B | myosin VB |
| 2.5 | 243485_at | --- | --- | --- |
| 2.5 | 233544_at | --- | --- | Guanine nucleotide binding protein-like 1 |
| 2.5 | 216979_at | 8013 | NR4A3 | nuclear receptor subfamily 4, group A, member 3 |
| 2.5 | 220580_at | 80114 | BICC1 | bicaudal C homolog 1 (Drosophila) |
| 2.5 | 220591_s_at | 80258 | EFHC2 | EF-hand domain (C-terminal) containing 2 |
| 2.5 | 219923_at | 80263 | TRIM45 | tripartite motif-containing 45 |
| 2.5 | 214417_s_at | --- | --- | Fetuin B |
| 2.5 | 212477_at | --- | --- | centaurin, beta 2 |
| 2.5 | 239295_at | 135295 | SRrp35 | Serine-arginine repressor protein (35 kDa) |
| 2.5 | 211364_at | 4507 | MTAP | methylthioadenosine phosphorylase |
| 2.5 | 220195_at | 55777 | MBD5 | methyl-CpG binding domain protein 5 |
| 2.5 | 220082_at | 54866 | PPP1R14D | protein phosphatase 1, regulatory (inhibitor) subunit 14D |
| 2.5 | 230949_at | 151295 | SLC23A3 | solute carrier family 23 (nucleobase transporters), member 3 |
| 2.5 | 218211_s_at | 79083 | MLPH | melanophilin |
| 2.5 | 209822_s_at | 7436 | VLDLR | very low density lipoprotein receptor |
| 2.5 | 220115_s_at | 1008 | CDH10 | cadherin 10, type 2 (T2-cadherin) |
| 2.5 | 206525_at | 2569 | GABRR1 | gamma-aminobutyric acid (GABA) receptor, rho 1 |
| 2.5 | 205359_at | 9472 | AKAP6 | A kinase (PRKA) anchor protein 6 |
| 2.5 | 235419_at | --- | --- | Transcribed locus |
| 2.5 | 210864_x_at | 3077 | HFE | hemochromatosis |
| 2.5 | 234490_at | --- | --- | Myosin IF |
| 2.5 | 223481_s_at | --- | --- | --- |
| 2.5 | 237553_at | --- | --- | Transcribed locus |
| 2.5 | 215766_at | 2938 | GSTA1 | Glutathione S-transferase A1 |
| 2.5 | 244658_at | --- | --- | --- |
| 2.5 | 230533_at | 23613 | PRKCBP1 | protein kinase C binding protein 1 |
| 2.5 | 215916_at | 1145 | CHRNE | cholinergic receptor, nicotinic, epsilon polypeptide |
| 2.5 | 215608_at | --- | --- | --- |
| 2.5 | 221461_at | 50835 | TAS2R9 | taste receptor, type 2, member 9 |
| 2.5 | 240501_at | --- | --- | Transcribed locus, weakly similar to XP_510104.1 similar to hypothetical protein FLJ25224 [Pan troglodytes] |
| 2.5 | 202454_s_at | 2065 | ERBB3 | v-erb-b2 erythroblastic leukemia viral oncogene homolog 3 (avian) |
| 2.5 | 211079_s_at | 1859 | DYRK1A | dual-specificity tyrosine-(Y)-phosphorylation regulated kinase 1A /// dual-specificity tyrosine-(Y)-phosphorylation regulated kinase 1A |
| 2.5 | 204665_at | --- | --- | hypothetical protein FLJ21168 |
| 2.5 | 219635_at | 80095 | ZNF606 | zinc finger protein 606 |
| 2.5 | 211863_x_at | 3077 | HFE | hemochromatosis |
| 2.5 | 243326_at | 64651 | AXUD1 | AXIN1 up-regulated 1 |
| 2.5 | 237354_at | 23042 | KIAA0251 | KIAA0251 protein |
| 2.5 | 220032_at | 79974 | FLJ21986 | hypothetical protein FLJ21986 |
| 2.5 | 207780_at | 1539 | CYLC2 | cylicin, basic protein of sperm head cytoskeleton 2 |
| 2.5 | 205855_at | 10168 | ZNF197 | zinc finger protein 197 |
| 2.5 | 35150_at | 958 | CD40 | CD40 antigen (TNF receptor superfamily member 5) |
| 2.5 | 216318_at | --- | --- | --- |
| 2.5 | 216292_at | --- | --- | Hypothetical protein FLJ11996 |
| 2.5 | 233984_at | 22863 | KIAA0831 | KIAA0831 |
| 2.5 | 208076_at | 8360 | HIST1H4D | histone 1, H4d |
| 2.5 | 242150_at | --- | --- | Transcribed locus |
| 2.5 | 33323_r_at | 2810 | SFN | stratifin |
| 2.5 | 215246_at | 51574 | HDCMA18P | HDCMA18P protein |
| 2.5 | 38447_at | 156 | ADRBK1 | adrenergic, beta, receptor kinase 1 |
| 2.5 | 221444_at | 50833 | TAS2R16 | taste receptor, type 2, member 16 |
| 2.5 | 215479_at | 57556 | SEMA6A | Sema domain, transmembrane domain (TM), and cytoplasmic domain, (semaphorin) 6A |
| 2.5 | 220460_at | 53919 | SLCO1C1 | solute carrier organic anion transporter family, member 1C1 |
| 2.5 | 91816_f_at | 399664 | RKHD1 | ring finger and KH domain containing 1 |
| 2.5 | 36907_at | 4598 | MVK | mevalonate kinase (mevalonic aciduria) |
| 2.5 | 240299_at | --- | --- | Testis nuclear RNA-binding protein |
| 2.5 | 207347_at | 2074 | ERCC6 | excision repair cross-complementing rodent repair deficiency, complementation group 6 |
| 2.5 | 202485_s_at | 8932 | MBD2 | methyl-CpG binding domain protein 2 |
| 2.5 | 221016_s_at | 83439 | TCF7L1 | transcription factor 7-like 1 (T-cell specific, HMG-box) /// transcription factor 7-like 1 (T-cell specific, HMG-box) |
| 2.5 | 215057_at | --- | --- | Clone 161455-2-3 B cell expressed mRNA from chromosome X. |
| 2.5 | 233726_at | --- | --- | Dmx-like 1 |
| 2.5 | 204955_at | 8406 | SRPX | sush1-repeat-containing protein, X-linked |
| 2.5 | 231043_at | 200373 | LOC200373 | Similar to hypothetical protein |
| 2.5 | 234176_at | --- | --- | 5-azacytidine induced 2 |
| 2.5 | 214888_at | 824 | CAPN2 | calpain 2, (m/II) large subunit |
| 2.5 | 244710_at | 136332 | FLJ32786 | hypothetical protein FLJ32786 |
| 2.5 | 205991_s_at | 5396 | PRRX1 | paired related homeobox 1 |
| 2.5 | 216091_s_at | 8945 | BTRC | beta-transducin repeat containing |
| 2.5 | 213344_s_at | --- | --- | H2A histone family, member X |
| 2.5 | 213386_at | --- | --- | MRNA full length insert CDNA clone EUROIMAGE 1585492 |
| 2.5 | 242533_at | --- | --- | Hypothetical protein FLJ12681 |
| 2.5 | 209758_s_at | 8076 | MFAP5 | microfibrillar associated protein 5 |
| 2.5 | 207859_s_at | 1142 | CHRNB3 | cholinergic receptor, nicotinic, beta polypeptide 3 |
| 2.5 | 239983_at | 169026 | SLC30A8 | --- |
| 2.5 | 203363_s_at | 9776 | KIAA0652 | KIAA0652 gene product |
| 2.5 | 206094_x_at | 54575 /// 54576 /// 54577 /// 54578 /// 54579 /// 54600 54657 /// 54657 /// 54658 /// 54659 | UGT1A10 /// UGT1A8 /// UGT1A7 /// UGT1A6 UGT1A6 /// UGT1A5 /// UGT1A9 /// UGT1A4 /// | UDP glycosyltransferase 1 family, polypeptide A6 |
| 2.5 | 205520_at | 6801 | STRN | striatin, calmodulin binding protein |
| 2.5 | 218966_at | 55930 | MY05C | myosin VC |
| 2.5 | 243584_at | --- | --- | Transcribed locus, weakly similar to NP_060190.1 signal-transducing adaptor protein-2 [Homo sapiens] |
| 2.5 | 212699_at | 192683 | SCAMPS | secretory carrier membrane protein 5 |
| 2.5 | 203074_at | 244 | ANXA8 | annexin A8 |
| 2.5 | 216408_at | 81697 | OR2B2 | olfactory receptor, family 2, subfamily B, member 2 |
| 2.5 | 121_at | 7849 | PAX8 | paired box gene 8 |
| 2.5 | 211328_x_at | 3077 | HFE | hemochromatosis |
| 2.5 | 213496_at | 9890 | LPPR4 | plasticity related gene 1 |
| 2.5 | 205601_s_at | 3215 | HOXB5 | homeo box B5 |
| 2.5 | 220287_at | 56999 | ADAMTS9 | a disintegrin-like and metalloprotease (reprolysin type) with thrombospondin type 1 motif, 9 |
| 2.5 | 219398_at | 63924 | CIDEC | cell death-inducing DFFA-like effector c |
| 2.5 | 214759_at | --- | --- | Wilms tumor 1 associated protein |
| 2.5 | 205927_s_at | 1510 | CTSE | cathepsin E |
| 2.5 | 208472_at | 64375 | ZNFN1A4 | zinc finger protein, subfamily 1A, 4 (Eos) |
| 2.5 | 214772_at | 25758 | G2 | G2 protein |
| 2.5 | 236605_at | 27335 | EIF3S12 | eukaryotic translation initiation factor 3, subunit 12 |
| 2.5 | 206022_at | 4693 | NDP | Norrie disease (pseudoglioma) |
| 2.5 | 51774_s_at | 222070 | LOC222070 | hypothetical protein LOC222070 |
| 2.5 | 221019_s_at | 81035 | COLEC12 | collectin member 12 /// collectin sub-family member 12 |
| 2.5 | 242253_at | 400708 | --- | --- |
| 2.5 | 211471_s_at | 9609 | RAB36 | RAB36, member RAS oncogene family |
| 2.5 | 216730_at | --- | --- | CDNA: FLJ20908 fis, clone ADSE00417 |
| 2.5 | 217117_x_at | 57876 | MUC3B | mucin 3A, intestinal |
| 2.5 | 1494_f_at | 1548 | CYP2A6 | cytochrome P450, family 2, subfamily A, polypeptide 6 |
| 2.5 | 211504_x_at | 9475 | ROCK2 | Rho-associated, coiled-coil containing protein kinase 2 |
| 2.5 | 215883_at | 401210 | --- | Hypothetical gene supported by AK022326 |
| 2.5 | 236927_at | --- | --- | Transcribed locus |
| 2.5 | 233469_at | 387590 | psiTPTE22 | TPTE pseudogene |
| 2.5 | 207687_at | 3626 | INHBC | inhibin, beta C |
| 2.5 | 220826_at | 55264 | C21orf77 | chromosome 21 open reading frame 77 |
| 2.5 | 244151_at | 285733 | LOC285733 | hypothetical protein LOC285733 |
| 2.5 | 207052_at | 26762 | HAVCR1 | hepatitis A virus cellular receptor 1 |
| 2.5 | 205150_s_at | 9865 | KIAA0644 | KIAA0644 gene product |
| 2.5 | 44702_at | 85360 | 7h3 | hypothetical protein FLJ13511 |
| 2.5 | 222835_at | 79875 | FLJ13710 | Hypothetical protein FLJ13710 |
| 2.5 | 207520_at | 6738 | SSA2 | Sjogren syndrome antigen A2 (60kDa, ribonucleoprotein autoantigen SS-A/Ro) |
| 2.5 | 221464_at | 4991 | OR1D2 | olfactory receptor, family 1, subfamily D, member 2 |
| 2.5 | 214419_s_at | --- | --- | Cytochrome P450, family 2, subfamily C, polypeptide 9 |
| 2.5 | 209863_s_at | 8626 | TP73L | tumor protein p73-like |
| 2.5 | 41856_at | 219699 | UNC5B | Unc-5 homolog B (C. elegans) |
| 2.5 | 240385_at | 2627 | GATA6 | GATA binding protein 6 |
| 2.5 | 241174_at | 23431 | AP4E1 | Adaptor-related protein complex 4, epsilon 1 subunit |
| 2.5 | 210796_x_at | 946 | SIGLEC6 | sialic acid binding Ig-like lectin 6 |
| 2.5 | 203864_s_at | 88 | ACTN2 | actinin, alpha 2 |
| 2.5 | 213307_at | 22941 | SHANK2 | SH3 and multiple ankyrin repeat domains 2 |
| 2.5 | 202011_at | 7082 | TJP1 | tight junction protein 1 (zona occludens 1) |
| 2.5 | 228747_at | 55176 | SEC61A2 | Sec61 alpha 2 subunit (S. cerevisiae) |
| 2.5 | 216025_x_at | --- | --- | --- |
| 2.5 | 220236_at | 55066 | PDPR | pyruvate dehydrogenase phosphatase regulatory subunit |
| 2.5 | 236776_at | --- | --- | CDNA FLJ42077 fis, clone SYNOV2019280 |
| 2.5 | 220957_at | 64693 | CTAGE1 | cutaneous T-cell lymphoma-associated antigen 1 |
| 2.5 | 243403_x_at | 1368 | CPM | carboxypeptidase M |
| 2.5 | 216203_at | 9517 | SPTLC2 | serme long chain base subunit 2 |
| 2.5 | 220167_s_at | 24150 | TP53TG3 | TP53TG3 protein |
| 2.5 | 207908_at | 3849 | KRT2A | keratin 2A (epidermal ichthyosis bullosa of Siemens) |
| 2.5 | 219489_s_at | 64359 | NXN | nucleoredoxin |
| 2.5 | 205741_s_at | 1837 | DTNA | dystrobrevin, alpha |
| 2.5 | 212372_at | 4628 | MYH10 | myosin, heavy polypeptide 10, non-muscle |
| 2.5 | 214421_x_at | 1559 | CYP2C9 | cytochrome P450, family 2, subfamily C, polypeptide 9 |
| 2.5 | 218554_s_at | 55870 | ASH1L | ashl (absent, small, or homeotic)-like (Drosophila) |
| 2.5 | 208360_s_at | --- | --- | --- |
| 2.5 | 214569_at | 3442 | IFNA5 | interferon, alpha 5 |
| 2.5 | 32540_at | --- | --- | Protein phosphatase 3 (formerly 2B), catalytic subunit, gamma isoform (calcineurin A gamma) |
| 2.5 | 205294_at | 10458 | BAIAP2 | BAI1-associated protein 2 |
| 2.5 | 210923_at | 6512 | SLC1A7 | solute carrier family 1 (glutamate transporter), member 7 |
| 2.5 | 202995_s_at | 2192 | FBLN1 | fibulin 1 |
| 2.5 | 227524_at | --- | --- | Transcribed locus |
| 2.5 | 212157_at | 6383 | SDC2 | syndecan 2 (heparan sulfate proteoglycan 1, cell surface-associated, fibroglycan) |
| 2.5 | 210695_s_at | 51741 | WWOX | WW domain containing oxidoreductase |
| 2.5 | 220288_at | 51168 | MYO15A | myosin XVA |
| 2.5 | 220663_at | 11141 | IL1RAPL1 | interleukin 1 receptor accessory protein-like 1 |
| 2.5 | 243100_at | 93034 | NT5C1B | 5'-nucleotidase, cytosolic IB |
| 2.5 | 219287_at | 27345 | KCNMB4 | potassium large conductance calcium-activated channel, subfamily M, beta member 4 |
| 2.5 | 206907_at | 8744 | TNFSF9 | tumor necrosis factor (ligand) superfamily, member 9 |
| 2.5 | 221339_at | 442194 | OR10C1 | olfactory receptor, family 10, subfamily C, member 1 |
| 2.5 | 207969_x_at | 56 | ACRV1 | acrosomal vesicle protein 1 |
| 2.5 | 222853_at | 23767 | FLRT3 | fibronectin leucine rich transmembrane protein 3 |
| 2.5 | 234143_at | --- | --- | CDNA: FLJ22813 fis, clone KAIA2964 |
| 2.5 | 214294_at | 57235 | KIAA0485 | KIAA0485 protein |
| 2.5 | 204762_s_at | 2775 | GNAO1 | guanine nucleotide binding protein (G protein), alpha activating activity polypeptide O |
| 2.5 | 237125_at | --- | --- | --- |
| 2.5 | 217397_at | --- | --- | T cell receptor V-alpha 24 (TCRA) |
| 2.5 | 206353_at | 1339 | COX6A2 | cytochrome c oxidase subunit VIa polypeptide 2 |
| 2.5 | 240895_at | --- | --- | Transcribed locus |
| 2.5 | 203899_s_at | 27297 | RCP9 | calcitonin gene-related peptide-receptor component protein |
| 2.5 | 220366_at | 64100 | ELSPBP1 | epididymal sperm binding protein 1 |
| 2.5 | 221466_at | 5030 | P2RY4 | pyrimidinergic receptor P2Y, G-protein coupled, 4 |
| 2.5 | 221394_at | 9287 | GPR58 | trace amine associated receptor 2 |
| 2.5 | 208217_at | 2570 | GABRR2 | gamma-aminobutyric acid (GABA) receptor, rho 2 |
| 2.5 | 234670_at | --- | --- | Rho GTPase activating protein 6 |
| 2.5 | 235860_at | 56888 | KCMF1 | Potassium channel modulatory factor 1 |
| 2.5 | 59433_at | 286434 | LOC286434 | hypothetical protein LOC286434 |
| 2.5 | 239612_at | 3222 | HOXC5 | Hypothetical gene supported by AK022887; AK056417 |
| 2.5 | 216437_at | --- | --- | Enhancer of polycomb homolog 1 (Drosophila) |
| 2.5 | 221670_s_at | 8022 | LHX3 | LIM homeobox 3 |
| 2.5 | 213974_at | 57188 | ADAMTSL3 | ADAMTS-like 3 |
| 2.5 | 209766_at | 10935 | PRDX3 | peroxiredoxin 3 |
| 2.5 | 203187_at | 1793 | DOCK1 | dedicator of cytokinesis 1 |
| 2.5 | 233731_at | --- | --- | Phospholipase C-like 3 |
| 2.5 | 214610_at | 1584 | CYP11B1 | cytochrome P450, family 11, subfamily B, polypeptide 1 |
| 2.5 | 210739_x_at | 8671 | SLC4A4 | solute carrier family 4, sodium bicarbonate cotransporter, member 4 |
| 2.6 | 238066_at | 116362 | RBP7 | retinol binding protein 7, cellular |
| 2.6 | 202934_at | 3099 | HK2 | hexokinase 2 |
| 2.6 | 217762_s_at | 11031 | RAB31 | RAB31, member RAS oncogene family |
| 2.6 | 208704_x_at | 334 | APLP2 | amyloid beta (A4) precursor-like protein 2 |
| 2.6 | 208594_x_at | 79168 | LILRB6 | leukocyte immunoglobulin-like receptor, subfamily B (with TM and ITIM domains), member 6 |
| 2.6 | 208949_s_at | 3958 /// 81625 | LGALS3 /// GALIG | lectin, galactoside-binding, soluble, 3 (galectin 3) /// galectin-3 internal gene |
| 2.6 | 201484_at | 6827 | SUPT4H1 | suppressor of Ty 4 homolog 1 (S. cerevisiae) |
| 2.6 | 202197_at | 8897 | MTMR3 | myotubularin related protein 3 |
| 2.6 | 208438_s_at | 2268 | FGR | Gardner-Rasheed feline sarcoma viral (v-fgr) oncogene homolog |
| 2.6 | 224674_at | 80727 | TTYH3 | tweety homolog 3 (Drosophila) |
| 2.6 | 213792_s_at | 3643 | INSR | insulin receptor |
| 2.6 | 209288_s_at | 10602 | CDC42EP3 | CDC42 effector protein (Rho GTPase binding) 3 |
| 2.6 | 236292_at | 55819 | RNF130 | Ring finger protein 130 |
| 2.6 | 200958_s_at | 6386 | SDCBP | syndecan binding protein (syntenin) |
| 2.6 | 210401_at | 5023 | P2RX1 | purinergic receptor P2X, ligand-gated ion channel, 1 |
| 2.6 | 229770_at | 144423 | FLJ31978 | hypothetical protein FLJ31978 |
| 2.6 | 213590_at | 9121 | SLC16A5 | solute carrier family 16 (monocarboxylic acid transporters), member 5 |
| 2.6 | 223482_at | 83862 | TMPIT | transmembrane protein induced by tumor necrosis factor alpha |
| 2.6 | 223553_s_at | 79930 | DOK3 | docking protein 3 |
| 2.6 | 221541_at | 83716 | LCRISP2 | cysteine-rich secretory protein LCCL domain containing 2 |
| 2.6 | 226577_at | 5663 | PSEN1 | Presenilin 1 (Alzheimer disease 3) |
| 2.6 | 202917_s_at | 6279 | S100A8 | S100 calcium binding protein A8 (calgranulin A) |
| 2.6 | 203645_s_at | 9332 | CD163 | CD163 antigen |
| 2.6 | 205119_s_at | 2357 | FPR1 | formyl peptide receptor 1 /// formyl peptide receptor 1 |
| 2.6 | 209467_s_at | 8569 | MKNK1 | MAP kinase interacting serine/threonine kinase 1 |
| 2.6 | 205640_at | 221 | ALDH3B1 | aldehyde dehydrogenase 3 family, member B1 |
| 2.6 | 204227_s_at | 7084 | TK2 | thymidine kinase 2, mitochondrial |
| 2.6 | 201003_x_at | --- | --- | --- |
| 2.6 | 214084_x_at | 4687 | NCF1 | --- |
| 2.6 | 214574_x_at | 7940 | LST1 | leukocyte specific transcript 1 |
| 2.6 | 229699_at | --- | --- | CDNA FLJ45384 fis, clone BRHIP3021987 |
| 2.6 | 213006_at | 1052 | CEBPD | CCAAT/enhancer binding protein (C/EBP), delta |
| 2.6 | 230263_s_at | 80005 | DOCKS | dedicator of cytokinesis 5 |
| 2.6 | 200701_at | 10577 | NPC2 | Niemann-Pick disease, type C2 |
| 2.6 | 209367_at | 6813 | STXBP2 | syntaxin binding protein 2 |
| 2.6 | 207643_s_at | 7132 | TNFRSF1A | tumor necrosis factor receptor superfamily, member 1A |
| 2.6 | 205627_at | 978 | CDA | cytidine deaminase |
| 2.6 | 226683_at | 112574 | SNAG1 | Sorting nexin associated golgi protein 1 |
| 2.6 | 230207_s_at | --- | --- | Dedicator of cytokinesis 5 |
| 2.6 | 211133_x_at | 11025 | LILRB3 | leukocyte immunoglobulin-like receptor, subfamily B (with TM and ITIM domains), member 2 /// leukocyte immunoglobulin-like receptor, subfamily B (with TM and ITIM domains), member 3 |
| 2.6 | 224652_at | 219771 | C10orf9 | chromosome 10 open reading frame 9 |
| 2.6 | 241742_at | 84106 | PPAM-1 | PPAM-1 protein |
| 2.6 | 226026_at | 84925 | DIRC2 | disrupted in renal carcinoma 2 |
| 2.6 | 220005_at | 53829 | P2RY13 | purinergic receptor P2Y, G-protein coupled, 13 /// purinergic receptor P2Y, G-protein coupled, 13 |
| 2.6 | 212506_at | 8301 | PICALM | phosphatidylinositol binding clathrin assembly protein |
| 2.6 | 205922_at | --- | --- | --- |
| 2.6 | 200839_s_at | 1508 | CTSB | cathepsin B |
| 2.6 | 227649_s_at | 23380 | SRGAP2 | SLIT-ROBO Rho GTPase activating protein 2 |
| 2.6 | 217931_at | 10695 | TNRC5 | trinucleotide repeat containing 5 |
| 2.6 | 229101_at | 150166 | LOC150166 | Hypothetical protein LOC150166 |
| 2.6 | 211576_s_at | 6573 | SLC19A1 | solute carrier family 19 (folate transporter), member 1 |
| 2.6 | 235593_at | 9839 | ZFHX1B | zinc finger homeobox 1b |
| 2.6 | 225883_at | 89849 | FLJ00012 | FLJ00012 protein |
| 2.6 | 219053_s_at | 55048 | FLJ20847 | vacuolar protein sorting 37C (yeast) |
| 2.6 | 202838_at | 2517 | FUCA1 | fucosidase, alpha-L- 1, tissue |
| 2.6 | 228325_at | 23514 | KIAA0146 | KIAA0146 protein |
| 2.6 | 211404_s_at | 334 | APLP2 | amyloid beta (A4) precursor-like protein 2 |
| 2.6 | 228499_at | 5210 | PFKFB4 | 6-phosphofructo-2-kinase/fructose-2,6-biphosphatase 4 |
| 2.6 | 204961_s_at | 4687 | NCF1 | neutrophil cytosolic factor 1 (47kDa, chronic granulomatous disease, autosomal 1) |
| 2.6 | 203005_at | 4055 | LTBR | lymphotoxin beta receptor (TNFR superfamily, member 3) |
| 2.6 | 210225_x_at | 11025 | LILRB3 | leukocyte immunoglobulin-like receptor, subfamily B (with TM and ITIM domains), member 2 /// leukocyte immunoglobulin-like receptor, subfamily B (with TM and ITIM domains), member 2 /// leukocyte immunoglobulin-like receptor, subfamily B (with |
| 2.6 | 211101_x_at | 11027 | LILRA2 | leukocyte immunoglobulin-like receptor, subfamily A (with TM domain), member 2 |
| 2.6 | 226066_at | 4286 | MITF | microphthalmia-associated transcription factor |
| 2.6 | 243099_at | 150372 | NFAM1 | NFAT activating protein with ITAM motif 1 |
| 2.6 | 205068_s_at | 23092 | ARHGAP26 | Rho GTPase activating protein 26 |
| 2.6 | 202201_at | 645 | BLVRB | biliverdin reductase B (flavin reductase (NADPH)) |
| 2.6 | 209286_at | 10602 | CDC42EP3 | CDC42 effector protein (Rho GTPase binding) 3 |
| 2.6 | 218773_s_at | 22921 | MSRB2 | methionine sulfoxide reductase B2 |
| 2.6 | 225373_at | 64115 | PP2135 | chromosome 10 open reading frame 54 |
| 2.6 | 227367_at | 28232 | SLCO3A1 | Solute carrier organic anion transporter family, member 3A1 |
| 2.6 | 201425_at | 217 | ALDH2 | aldehyde dehydrogenase 2 family (mitochondrial) |
| 2.6 | 222934_s_at | 26253 | CLEC4E | C-type lectin domain family 4, member E |
| 2.6 | 218627_at | 55332 | FLJ11259 | hypothetical protein FLJ11259 |
| 2.6 | 210785_s_at | 9473 | Clorf38 | chromosome 1 open reading frame 38 |
| 2.6 | 211581_x_at | 7940 | LST1 | leukocyte specific transcript 1 |
| 2.6 | 219132_at | 57161 | PELI2 | pellino homolog 2 (Drosophila) |
| 2.6 | 211474_s_at | 5269 | SERPINB6 | serine (or cysteine) proteinase inhibitor, clade B (ovalbumin), member 6 |
| 2.6 | 234942_s_at | 116092 | DNTTIP1 | deoxynucleotidyltransferase, terminal, interacting protein 1 |
| 2.6 | 209607_x_at | 6818 | SULT1A3 | sulfotransferase family, cytosolic, 1A, phenol-preferring, member 3 |
| 2.6 | 223440_at | 80262 | Lin10 | lin-10 protein homolog |
| 2.6 | 221036_s_at | 83464 | APH1B | anterior pharynx defective 1 homolog B (C. elegans) /// anterior pharynx defective 1 homolog B (C. elegans) |
| 2.6 | 227721_at | 27151 | CPAMD8 | C3 and PZP-like, alpha-2-macroglobulin domain containing 8 |
| 2.6 | 210422_x_at | 6556 | SLC11A1 | solute carrier family 11 (proton-coupled divalent metal ion transporters), member 1 |
| 2.6 | 229510_at | 84689 | NYD-SP21 | testes development-related NYD-SP21 |
| 2.6 | 227129_x_at | 388312 /// 399844 | LOC388312 /// FLJ45445 | hypothetical protein LOC284701 /// hypothetical gene supported by AK128780 /// FLJ45445 protein /// hypothetical gene supported by AK097080; AL117642; BC047304; BC054485 |
| 2.6 | 207765_s_at | 80256 | KIAA1539 | KIAA1539 |
| 2.6 | 223502_s_at | 10673 | TNFSF13B | tumor necrosis factor (ligand) superfamily, member 13b |
| 2.6 | 202155_s_at | 8021 | NUP214 | nucleoporin 214kDa |
| 2.6 | 225056_at | 57568 | SIPA1L2 | signal-induced proliferation-associated 1 like 2 |
| 2.6 | 203814_s_at | 4835 | NQO2 | NAD(P)H dehydrogenase, quinone 2 |
| 2.6 | 223591_at | 84282 | RNF135 | ring finger protein 135 |
| 2.6 | 204232_at | 2207 | FCER1G | Fc fragment of IgE, high affinity I, receptor for; gamma polypeptide |
| 2.6 | 244756_at | 338692 | LOC338692 | hypothetical protein LOC338692 |
| 2.6 | 231579_s_at | 7077 | TIMP2 | tissue inhibitor of metalloproteinase 2 |
| 2.6 | 210663_s_at | 8942 | KYNU | kynureninase (L-kynurenine hydrolase) |
| 2.6 | 227111_at | 403341 | ZBTB34 | zinc finger and BTB domain containing 34 |
| 2.6 | 227948_at | 121512 | FGD4 | FYVE, RhoGEF and PH domain containing 4 |
| 2.6 | 204053_x_at | 5728 | PTEN | phosphatase and tensin homolog (mutated in multiple advanced cancers 1) |
| 2.6 | 223280_x_at | 64231 | MS4A6A | membrane-spanning 4-domains, subfamily A, member 6A |
| 2.6 | 209179_s_at | 79143 | LENG4 | leukocyte receptor cluster (LRC) member 4 |
| 2.6 | 224573_at | 440400 | MGC71993 | similar to DNA segment, Chr 11, Brigham & Womens Genetics 0434 expressed |
| 2.6 | 218191_s_at | 55788 | C6orf209 | chromosome 6 open reading frame 209 |
| 2.6 | 210340_s_at | 1438 | CSF2RA | colony stimulating factor 2 receptor, alpha, low-affinity (granulocyte-macrophage) |
| 2.6 | 225685_at | 10602 | CDC42EP3 | CDC42 effector protein (Rho GTPase binding) 3 |
| 2.6 | 213702_x_at | 427 | ASAH1 | N-acylsphingosine amidohydrolase (acid ceramidase) 1 |
| 2.6 | 202436_s_at | 1545 | CYP1B1 | cytochrome P450, family 1, subfamily B, polypeptide 1 |
| 2.6 | 202192_s_at | 8522 | GAS7 | growth arrest-specific 7 |
| 2.6 | 213198_at | 91 | ACVR1B | activin A receptor, type IB |
| 2.6 | 225665_at | 51776 | ZAK | sterile alpha motif and leucine zipper containing kinase AZK |
| 2.6 | 224480_s_at | 84803 | MGC11324 | hypothetical protein MGC11324 /// hypothetical protein MGC11324 |
| 2.6 | 202897_at | 140885 | PTPNS1 | protein tyrosine phosphatase, non-receptor type substrate 1 |
| 2.6 | 211429_s_at | 5265 | SERPINA1 | serine (or cysteine) proteinase inhibitor, clade A (alpha-1 antiproteinase, antitrypsin), member 1 |
| 2.6 | 211102_s_at | 11027 | LILRA2 | leukocyte immunoglobulin-like receptor, subfamily A (with TM domain), member 2 |
| 2.6 | 221840_at | 5791 | PTPRE | protein tyrosine phosphatase, receptor type, E |
| 2.6 | 209696_at | 2203 | FBP1 | fructose-1,6-bisphosphatase 1 |
| 2.6 | 203604_at | 9658 | ZNF516 | zinc finger protein 516 |
| 2.6 | 220066_at | 64127 | CARD15 | caspase recruitment domain family, member |
| 2.6 | 204446_s_at | 240 | ALOX5 | arachidonate 5-lipoxygenase |
| 2.6 | 200677_at | 754 | PTTG1IP | pituitary tumor-transforming 1 interacting protein |
| 2.6 | 202803_s_at | 3689 | ITGB2 | integrin, beta 2 (antigen CD18 (p95), lymphocyte function-associated antigen 1; macrophage antigen 1 (mac-1) beta subunit) |
| 2.6 | 226169_at | 81846 | SBF2 | SET binding factor 2 |
| 2.6 | 222689_at | 55331 | PHCA | phytoceramidase, alkaline |
| 2.6 | 229164_s_at | 80325 | ABTB1 | ankyrin repeat and BTB (POZ) domain containing 1 |
| 2.6 | 208919_s_at | 65220 | FLJ13052 | NAD kinase |
| 2.6 | 201422_at | 10437 | IFI30 | --- |
| 2.6 | 213187_x_at | 2512 | FTL | ferritin, light polypeptide |
| 2.6 | 223392_s_at | 57616 | ZNF537 | zinc finger protein 537 |
| 2.6 | 205568_at | 366 | AQP9 | aquaporin 9 |
| 2.6 | 223303_at | 83706 | URP2 | UNC-112 related protein 2 |
| 2.6 | 227929_at | --- | --- | Homo sapiens, clone IMAGE:5277945, mRNA |
| 2.6 | 209370_s_at | 6452 | SH3BP2 | SH3-domain binding protein 2 |
| 2.6 | 218132_s_at | 79042 | LENG5 | tRNA splicing endonuclease 34 homolog (SEN34, S. cerevisiae) |
| 2.6 | 206380_s_at | 5199 | PFC | properdin P factor, complement |
| 2.6 | 226354_at | 114294 | LACTB | lactamase, beta |
| 2.6 | 200645_at | 11337 | GABARAP | GABA(A) receptor-associated protein |
| 2.6 | 202388_at | 5997 | RGS2 | regulator of G-protein signalling 2, 24kDa |
| 2.6 | 223376_s_at | 25798 | BRI3 | brain protein I3 |
| 2.6 | 211661_x_at | 5724 | PTAFR | platelet-activating factor receptor /// platelet-activating factor receptor |
| 2.6 | 224560_at | 7077 | TIMP2 | tissue inhibitor of metalloproteinase 2 |
| 2.6 | 204122_at | 7305 | TYROBP | TYRO protein tyrosine kinase binding protein |
| 2.6 | 202100_at | 5899 | RALB | v-ral simian leukemia viral oncogene homolog B (ras related; GTP binding protein) |
| 2.6 | 206855_s_at | 8692 | HYAL2 | hyaluronoglucosaminidase 2 |
| 2.6 | 211067_s_at | 8522 | GAS7 | growth arrest-specific 7 /// growth arrest-specific 7 |
| 2.6 | 203535_at | 6280 | S100A9 | S100 calcium binding protein A9 (calgranulin B) |
| 2.6 | 210044_s_at | 4066 | LYL1 | lymphoblastic leukemia derived sequence 1 |
| 2.6 | 201126_s_at | 4245 | MGAT1 | mannosyl (alpha-1,3-)-glycoprotein beta-1,2-N-acetylglucosaminyltransferase |
| 2.6 | 208540_x_at | --- | --- | --- |
| 2.6 | 223519_at | --- | --- | --- |
| 2.6 | 228176_at | 1903 | EDG3 | endothelial differentiation, sphingolipid G-protein-coupled receptor, 3 |
| 2.6 | 227013_at | 26524 | LATS2 | LATS, large tumor suppressor, homolog 2 (Drosophila) |
| 2.6 | 201336_at | 9341 | VAMP3 | vesicle-associated membrane protein 3 (cellubrevin) |
| 2.6 | 201875_s_at | 9019 | MPZL1 | myelin protein zero-like 1 |
| 2.6 | 48106_at | --- | --- | hypothetical protein FLJ20489 |
| 2.6 | 214875_x_at | 334 | APLP2 | amyloid beta (A4) precursor-like protein 2 |
| 2.6 | 224341_x_at | --- | --- | toll-like receptor 4 /// toll-like receptor 4 |
| 2.6 | 204204_at | 1318 | SLC31A2 | solute carrier family 31 (copper transporters), member 2 |
| 2.6 | 222105_s_at | 28511 | NKIRAS2 | NFKB inhibitor interacting Ras-like 2 |
| 2.6 | 203041_s_at | 3920 | LAMP2 | lysosomal-associated membrane protein 2 |
| 2.6 | 201642_at | 3460 | IFNGR2 | interferon gamma receptor 2 (interferon gamma transducer 1) |
| 2.6 | 212041_at | 9114 | ATP6V0D1 | ATPase, H+ transporting, lysosomal 38kDa, V0 subunit d isoform 1 |
| 2.6 | 207571_x_at | 9473 | C1orf38 | chromosome 1 open reading frame 38 |
| 2.6 | 201200_at | 8804 | CREG1 | cellular repressor of E1A-stimulated genes 1 |
| 2.6 | 202426_s_at | 6256 | RXRA | retinoid X receptor, alpha |
| 2.6 | 203167_at | 7077 | TIMP2 | tissue inhibitor of metalloproteinase 2 |
| 2.6 | 208703_s_at | 334 | APLP2 | amyloid beta (A4) precursor-like protein 2 |
| 2.7 | 222207_x_at | --- | --- | CDNA: FLJ20949 fis, clone ADSE01902 |
| 2.7 | 216229_x_at | --- | --- | HLA complex group 2 pseudogene 7 |
| 2.7 | 234562_x_at | 152189 | CKLFSF8 | Chemokine-like factor super family 8 |
| 2.7 | 204842_x_at | 5576 | PRKAR2A | protein kinase, cAMP-dependent, regulatory, type II, alpha |
| 2.7 | 56919_at | 57599 | WDR48 | WD repeat domain 48 |
| 2.7 | 220720_x_at | 80097 | FLJ14346 | hypothetical protein FLJ14346 |
| 2.7 | 214902_x_at | 401105 | FLJ42393 | FLJ42393 protein |
| 2.7 | 233270_x_at | 5108 | PCM1 | Pericentriolar material 1 |
| 2.7 | 236715_x_at | --- | --- | uveal autoantigen with coiled-coil domains and ankyrin repeats |
| 2.7 | 206551_x_at | 54800 | DRE1 | DRE1 protein |
| 2.7 | 234762_x_at | 57486 | NLN | Neurolysin (metallopeptidase M3 family) |
| 2.7 | 215978_x_at | --- | --- | hypothetical protein LOC152719 |
| 2.7 | 223662_x_at | 83479 | DKFZP564B1023 | DEAD (Asp-Glu-Ala-Asp) box polypeptide 59 |
| 2.7 | 243442_x_at | --- | --- | Ring finger protein 138 pseudogene 1 |
| 2.7 | 223697_x_at | 84267 | C9orf64 | chromosome 9 open reading frame 64 |
| 2.7 | 208238_x_at | --- | --- | --- |
| 2.7 | 215212_at | --- | --- | CDNA FLJ12091 fis, clone HEMBB1002582 |
| 2.7 | 233041_x_at | 114781 | BTBD9 | BTB (POZ) domain containing 9 |
| 2.7 | 241686_x_at | --- | --- | Transcribed locus |
| 2.7 | 234949_at | 284802 | MGC72104 | Similar to FRG1 protein (FSHD region gene 1 protein) |
| 2.7 | 233193_x_at | 92105 | MGC16733 | hypothetical gene MGC16733 similar to CG12113 |
| 2.7 | 220215_at | 79862 | FLJ12606 | zinc finger protein 669 |
| 2.7 | 238913_at | --- | --- | Cleavage and polyadenylation specific factor 6, 68kDa |
| 2.7 | 215383_x_at | 51324 | SPG21 | spastic paraplegia 21 (autosomal recessive, Mast syndrome) |
| 2.7 | 215588_x_at | 8780 | RIOK3 | RIO kinase 3 (yeast) |
| 2.7 | 208082_x_at | --- | --- | --- |
| 2.7 | 234020_x_at | --- | --- | CDNA clone IMAGE:4698949, partial cds |
| 2.7 | 208137_x_at | 81856 | ZNF611 | --- |
| 2.7 | 233912_x_at | 255520 | ELMOD2 | ELMO domain containing 2 |
| 2.7 | 242578_x_at | 6581 | SLC22A3 | Solute carrier family 22 (extraneuronal monoamine transporter), member 3 |
| 2.7 | 216858_x_at | --- | --- | --- |
| 2.7 | 217579_x_at | --- | --- | ADP-ribosylation factor-like 6 interacting protein 2 |
| 2.7 | 220796_x_at | 79939 | SLC35E1 | solute carrier family 35, member E1 |
| 2.7 | 242403_at | 7323 | UBE2D3 | Ubiquitin-conjugating enzyme E2D 3 (UBC4/5 homolog, yeast) |
| 2.7 | 224667_x_at | 119504 | C10orf104 | chromosome 10 open reading frame 104 |
| 2.7 | 241223_x_at | --- | --- | --- |
| 2.7 | 214722_at | 388677 | NOTCH2NL | Notch homolog 2 (Drosophila) N-terminal like |
| 2.7 | 210679_x_at | --- | --- | --- |
| 2.7 | 243509_at | 256021 | LOC256021 | Hypothetical protein LOC256021 |
| 2.7 | 233775_x_at | 51710 | ZNF44 | Similar to hypothetical protein FLJ38281 |
| 2.7 | 205370_x_at | 1629 | DBT | dihydrolipoamide branched chain transacylase E2 |
| 2.7 | 232169_x_at | 4728 | NDUFS8 | NADH dehydrogenase (ubiquinone) Fe-S protein 8, 23kDa (NADH-coenzyme Q |
| 2.7 | 233702_x_at | --- | --- | CDNA: FLJ20946 fis, clone ADSE1819 |
| 2.7 | 231770_x_at | 51057 | LOC51057 | hypothetical protein LOC51057 |
| 2.7 | 206792_x_at | 5143 | PDE4C | phosphodiesterase 4C, cAMP-specific (phosphodiesterase E1 dunce homolog, Drosophila) |
| 2.7 | 222133_s_at | 51105 | PHF20L1 | PHD finger protein 20-like 1 |
| 2.7 | 216187_x_at | --- | --- | Kinesin 2 60/70kDa |
| 2.7 | 214715_x_at | 90338 | ZNF160 | zinc finger protein 160 |
| 2.7 | 207953_at | --- | --- | neuronal thread protein AD7c-NTP |
| 2.7 | 232597_x_at | 9169 | SFRS2IP | Splicing factor, arginine/serine-rich 2, interacting protein |
| 2.7 | 219392_x_at | 55771 | FLJ11029 | hypothetical protein FLJ11029 |
| 2.7 | 216614_at | --- | --- | Family with sequence similarity 20, member C |
| 2.7 | 215373_x_at | 80047 | FLJ12151 | hypothetical protein FLJ12151 |
| 2.7 | 237475_x_at | --- | --- | CH5400 |
| 2.7 | 215208_x_at | 6165 | RPL35A | Ribosomal protein L35a |
| 2.7 | 215600_x_at | 285231 | FBXW12 | F-box and WD-40 domain protein 12 |
| 2.7 | 204387_x_at | 78988 | MRP63 | mitochondrial ribosomal protein 63 |
| 2.7 | 214594_x_at | 5205 | ATP8B1 | ATPase, Class I, type 8B, member 1 |
| 2.7 | 205383_s_at | 26137 | ZBTB20 | zinc finger and BTB domain containing 20 |
| 2.7 | 215483_at | 10142 | AKAP9 | A kinase (PRKA) anchor protein (yotiao) 9 |
| 2.7 | 235327_x_at | 165324 | UBXD4 | UBX domain containing 4 |
| 2.7 | 234981_x_at | 134147 | LOC134147 | similar to mouse 2310016A09Rik gene |
| 2.7 | 215604_x_at | --- | --- | Ubiquitin-conjugating enzyme E2D 2 (UBC4/5 homolog, yeast) |
| 2.7 | 213936_x_at | 6439 | SFTPB | surfactant, pulmonary-associated protein B |
| 2.7 | 207730_x_at | 114782 | KIAA1881 | Hepatoma-derived growth factor-related protein 2 |
| 2.7 | 217713_x_at | --- | --- | Hypothetical protein DKFZp566N034 |
| 2.7 | 214707_x_at | 7840 | ALMS1 | Alstrom syndrome 1 |
| 2.7 1 | 211452_x_at | --- | --- | leucine rich repeat (in FLII) interacting protein |
| | | | | |
| 2.7 | 215123_at | --- | --- | --- |
| 2.7 | 216813_at | --- | --- | --- |
| 2.7 | 233319_x_at | 65979 | PHACTR4 | Phosphatase and actin regulator 4 |
| 2.8 | 204612_at | 5569 | PKIA | protein kinase (cAMP-dependent, catalytic) inhibitor alpha |
| 2.8 | 230489_at | 921 | CD5 | CD5 antigen (p56-62) |
| 2.8 | 226433_at | 114804 | RNF157 | ring finger protein 157 |
| 2.8 | 213958_at | 923 | CD6 | CD6 antigen /// CD6 antigen |
| 2.8 | 218918_at | 57134 | MAN1C1 | mannosidase, alpha, class 1C, member 1 |
| 2.8 | 227867_at | 129293 | LOC129293 | hypothetical protein LOC129293 |
| 2.8 | 213093_at | 5578 | PRKCA | protein kinase C, alpha |
| 2.8 | 218648_at | 64784 | TORC3 | --- |
| 2.8 | 225478_at | 9258 | MFHAS1 | Malignant fibrous histiocytoma amplified sequence 1 |
| 2.8 | 210915_x_at | 28639 | TRBC1 | T cell receptor beta constant 1 |
| 2.8 | 226452_at | 5163 | PDK1 | pyruvate dehydrogenase kinase, isoenzyme 1 |
| 2.8 | 205254_x_at | 6932 | TCF7 | transcription factor 7 (T-cell specific, HMG-box) |
| 2.8 | 216945_x_at | 23178 | PASK | PAS domain containing serine/threonine kinase |
| 2.8 | 203580_s_at | 9057 | SLC7A6 | solute carrier family 7 (cationic amino acid transporter, y+ system), member 6 |
| 2.8 | 213564_x_at | 3945 | LDHB | lactate dehydrogenase B |
| 2.8 | 219315_s_at | 79652 | C16orf30 | chromosome 16 open reading frame 30 |
| 2.8 | 227934_at | 3841 | KPNA5 | Karyopherin alpha 5 (importin alpha 6) |
| 2.8 | 205006_s_at | 9397 | NMT2 | N-myristoyltransferase 2 |
| 2.8 | 216262_s_at | 60436 | TGIF2 | TGFB-induced factor 2 (TALE family homeobox) |
| 2.8 | 204777_s_at | 4118 | MAL | mal, T-cell differentiation protein |
| 2.8 | 241871_at | 814 | CAMK4 | calcium/calmodulin-dependent protein kinase IV |
| 2.8 | 211339_s_at | 3702 | ITK | IL2-inducible T-cell kinase |
| 2.8 | 201656_at | 3655 | ITGA6 | integrin, alpha 6 |
| 2.8 | 222696_at | 8313 | AXIN2 | axin 2 (conductin, axil) |
| 2.8 | 203413_at | 4753 | NELL2 | NEL-like 2 (chicken) /// NEL-like 2 (chicken) |
| 2.8 | 229064_s_at | --- | --- | --- |
| 2.8 | 222307_at | 282997 | LOC282997 | hypothetical protein LOC282997 |
| 2.8 | 227262_at | 145864 | HAPLN3 | hyaluronan and proteoglycan link protein 3 |
| 2.8 | 213039_at | 23370 | ARHGEF18 | rho/rac guanine nucleotide exchange factor (GEF) 18 |
| 2.8 | 220999_s_at | 26999 | CYFIP2 | cytoplasmic FMR1 interacting protein 2 /// cytoplasmic FMR1 interacting protein 2 |
| 2.8 | 227686_at | 92106 | MGC15763 | hypothetical protein BC008322 |
| 2.8 | 205590_at | 10125 | RASGRP1 | RAS guanyl releasing protein 1 (calcium and DAG-regulated) |
| 2.8 | 229544_at | --- | --- | MRNA; cDNA DKFZp564C0762 (from clone DKFZp564C0762) |
| 2.8 | 203717_at | 1803 | DPP4 | dipeptidylpeptidase 4 (CD26, adenosine deaminase complexing protein 2) |
| 2.8 | 227449_at | 2043 | EPHA4 | EPH receptor A4 |
| 2.8 | 206337_at | 1236 | CCR7 | chemokine (C-C motif) receptor 7 /// chemokine (C-C motif) receptor 7 |
| 2.8 | 219724_s_at | 9840 | KIAA0748 | KIAA0748 gene product |
| 2.8 | 228879_at | --- | --- | Transcribed locus, weakly similar to XP_331555.1 Neurospora crassa NCU09163.1 gene |
| 2.8 | 203387_s_at | 9882 | TBC1D4 | TBC1 domain family, member 4 |
| 2.8 | 210202_s_at | 274 | BIN1 | bridging integrator 1 |
| 2.8 | 221558_s_at | 51176 | LEF1 | lymphoid enhancer-binding factor 1 |
| 2.8 | 205790-at | 8631 | SCAP1 | src family associated phosphoprotein 1 |
| 2.8 | 225314_at | 132299 | OCIAD2 | OCIA domain containing 2 |
| 2.8 | 212414_s_at | 23157 | 6-Sep | septin 6 /// cytokine-like nuclear factor n-pac |
| 2.8 | 229029_at | --- | --- | Transcribed locus, weakly similar to XP_499236.1 LOC442377 [Homo sapiens] |
| 2.8 | 204453_at | 7637 | ZNF84 | zinc finger protein 84 (HPF2) |
| 2.8 | 218932_at | 54680 | FLJ20729 | hypothetical protein FLJ20729 |
| 2.8 | 219765_at | 79673 | FLJ12586 | zinc finger protein 329 |
| 2.8 | 210707_x_at | 5389 | PMS2L11 | postmeiotic segregation increased 2-like 11 |
| 2.8 | 206545_at | 940 | CD28 | CD28 antigen (Tp44) |
| 2.8 | 39248_at | 360 | AQP3 | aquaporin 3 |
| 2.8 | 201030_x_at | 3945 | LDHB | lactate dehydrogenase B |
| 2.8 | 213295_at | 1540 | CYLD | Cylindromatosis (turban tumor syndrome) |
| 2.8 | 211902_x_at | 6955 | TRA@ | T cell receptor alpha locus |
| 2.8 | 204019_s_at | 26751 | SH3YL1 | SH3 domain containing, Ysc84-like 1 (S. cerevisiae) |
| 2.8 | 210607_at | 2323 | FLT3LG | fms-related tyrosine kinase 3 ligand |
| 2.8 | 223401_at | 56985 | MDS006 | x 006 protein |
| 2.8 | 221601_s_at | 9214 | TOSO | Fas apoptotic inhibitory molecule /// Fas apoptotic inhibitory molecule |
| 2.8 | 212549_at | 6777 | STAT5B | signal transducer and activator of transcription 5B |
| 2.8 | 209604_s_at | 2625 | GATA3 | GATA binding protein 3 |
| 2.8 | 212400_at | 399665 | EEIG1 | chromosome 9 open reading frame 132 |
| 2.8 | 209504_s_at | 58473 | PLEKHB1 | pleckstrin homology domain containing, family B (evectins) member 1 |
| 2.8 | 216232_s_at | 10985 | GCN1L1 | GCN1 general control of amino-acid synthesis 1-like 1 (yeast) |
| 2.8 | 225864_at | 157638 | NSE2 | breast cancer membrane protein 101 |
| 2.8 | 228988_at | 7552 | ZNF6 | zinc finger protein 6 (CMPX1) |
| 2.8 | 207339_s_at | 4050 | LTB | lymphotoxin beta (TNF superfamily, member 3) |
| 2.8 | 232001_at | 439949 | LOC439949 | hypothetical gene supported by AY007155 |
| 2.8 | 203386_at | 9882 | TBC1D4 | TBC1 domain family, member 4 |
| 2.8 | 229264_at | 440667 | LOC440667 | Poly (ADP-ribose) polymerase family, member 8 |
| 2.8 | 221602_s_at | 9214 | TOSO | Fas apoptotic inhibitory molecule /// Fas apoptotic inhibitory molecule |
| 2.8 | 212642_s_at | 3097 | HIVEP2 | human immunodeficiency virus type I enhancer binding protein 2 |
| 2.8 | 57082_at | 26119 | ARH | low density lipoprotein receptor adaptor protein 1 |
| 2.8 | 213534_s_at | 23178 | PASK | PAS domain containing serine/threonine kinase |
| 2.8 | 213193_x_at | 28639 | TRBC1 | T cell receptor beta constant 1 /// T cell receptor beta constant 1 |
| 2.8 | 223162_s_at | 57189 | LCHN | LCHN protein |
| 2.8 | 228109_at | 5924 | RASGRF2 | Ras protein-specific guanine nucleotide-releasing factor 2 |
| 2.8 | 203385_at | 1606 | DGKA | diacylglycerol kinase, alpha 80kDa |
| 2.8 | 211272_s_at | 1606 | DGKA | diacylglycerol kinase, alpha 80kDa |
| 2.8 | 218510_x_at | 54463 | FLJ20152 | hypothetical protein FLJ20152 |
| 2.8 | 201313_at | 2026 | ENO2 | enolase 2 (gamma, neuronal) |
| 2.8 | 206492_at | 2272 | FHIT | fragile histidine triad gene |
| 2.8 | 236826_at | 158219 | C9orf52 | Chromosome 9 open reading frame 52 |
| 2.8 | 212771_at | 221061 | C10orf38 | chromosome 10 open reading frame 38 |
| 2.8 | 239122_at | --- | --- | Interleukin 24 |
| 2.8 | 212096_s_at | 57509 | MTUS1 | mitochondrial tumor suppressor 1 |
| 2.8 | 218532_s_at | 54463 | FLJ20152 | hypothetical protein FLJ20152 |
| 2.8 | 47560_at | 22859 | LPHN1 | latrophilin 1 |
| 2.8 | 214081_at | 57125 | PLXDC1 | plexin domain containing 1 |
| 2.8 | 202969_at | 8445 | DYRK2 | Dual-specificity tyrosine-(Y)-phosphorylation regulated kinase 2 |
| 2.8 | 220485_s_at | 55423 | SIRPB2 | signal-regulatory protein beta 2 |
| 2.8 | 244798_at | --- | --- | --- |
| 2.8 | 215967_s_at | 4063 | LY9 | lymphocyte antigen 9 |
| 2.8 | 238823_at | 91010 | FMNL3 | formin-like 3 |
| 2.8 | 212641_at | 3097 | HIVEP2 | human immunodeficiency virus type I enhancer binding protein 2 |
| 2.8 | 221648_s_at | 51029 | PNAS-4 | CGI-146 protein |
| 2.8 | 214177_s_at | 57326 | PBXIP1 | pre-B-cell leukemia transcription factor interacting protein 1 |
| 2.8 | 219734_at | 54847 | SIDT1 | SID1 transmembrane family, member 1 |
| 2.8 | 201522_x_at | 6638 /// 8926 | SNRPN /// SIVURF | small nuclear ribonucleoprotein polypeptide N /// SNRPN upstream reading frame |
| 2.8 | 221712_s_at | 54663 | FLJ10439 | hypothetical protein FLJ10439 /// hypothetical protein FLJ10439 |
| 2.8 | 210948_s_at | 51176 | LEF1 | lymphoid enhancer-binding factor 1 |
| 2.8 | 226272_at | --- | --- | Full length insert cDNA clone ZD79H10 |
| 2.8 | 213340_s_at | 57212 | KIAA0495 | KIAA0495 |
| 2.8 | 213971_s_at | 23512 | SUZ12 | suppressor of zeste 12 homolog (Drosophila) |
| 2.8 | 212413_at | 23157 | 6-Sep | septin 6 |
| 2.8 | 211796_s_at | 28639 | TRBC1 | T cell receptor beta constant 1 |
| 2.8 | 228960_at | 79664 | NARG2 | NMDA receptor regulated 2 |
| 2.8 | 228570_at | 121551 | BTBD11 | BTB (POZ) domain containing 11 |
| 2.8 | 200965_s_at | 3983 | ABLIM1 | actin binding LIM protein 1 |
| 2.8 | 219700_at | 57125 | PLXDC1 | plexin domain containing 1 |
| 2.8 | 205005_s_at | 9397 | NMT2 | N-myristoyltransferase 2 |
| 2.8 | 209337_at | 11168 | PSIP1 | PC4 and SFRS1 interacting protein 1 |
| 2.8 | 228046_at | 152485 | LOC152485 | Hypothetical protein LOC152485 |
| 2.8 | 242947_at | --- | --- | KIAA0962 protein |
| 2.8 | 206042_x_at | 6638 /// 8926 | SNRPN /// SNURF | small nuclear ribonucleoprotein polypeptide N /// SNRPN upstream reading frame |
| 2.8 | 205255_x_at | 6932 | TCF7 | transcription factor 7 (T-cell specific, HMG-box) |
| 2.8 | 227072_at | 25914 | RTTN | rotatin |
| 2.8 | 218877_s_at | 60487 | C6orf75 | chromosome 6 open reading frame 75 |
| 2.8 | 202746_at | 9452 | ITM2A | integral membrane protein 2A |
| 2.8 | 202968_s_at | 8445 | DYRK2 | dual-specificity tyrosine-(Y)-phosphorylation regulated kinase 2 |
| 2.8 | 203408_s_at | 6304 | SATB1 | special AT-rich sequence binding protein 1 (binds to nuclear matrix/scaffold-associating DNA's) |
| 2.8 | 218667_at | 64219 | PJA1 | praja 1 |
| 2.8 | 219528_s_at | 64919 | BCL11B | B-cell CLL/lymphoma 11B (zinc finger protein) |
| 2.8 | 212313_at | 91782 | MGC29816 | CHMP family, member 7 |
| 2.8 | 217950_at | 51070 | NOSIP | nitric oxide synthase interacting protein |
| 2.8 | 202747_s_at | 9452 | ITM2A | integral membrane protein 2A |
| 2.8 | 218259_at | 57496 | MKL2 | MKL/myocardin-like 2 |
| 2.8 | 213540_at | 7923 | HSD17B8 | hydroxysteroid (17-beta) dehydrogenase 8 |
| 2.8 | 235457_at | 84441 | MAML2 | mastermind-like 2 (Drosophila) |
| 2.8 | 218437_s_at | 54585 | LZTFL1 | leucine zipper transcription factor-like 1 |
| 2.8 | 219922_s_at | 4054 | LTBP3 | latent transforming growth factor beta binding protein 3 |
| 2.8 | 224838_at | 27086 | FOXP1 | forkhead box P1 |
| 2.8 | 218517_at | --- | --- | --- |
| 2.8 | 206761_at | 10225 | CD96 | CD96 antigen |
| 2.8 | 214049_x_at | 924 | CD7 | CD7 antigen (p41) |
| 2.8 | 217729_s_at | 166 | AES | amino-terminal enhancer of split |
| 2.8 | 220054_at | 51561 | IL23A | interleukin 23, alpha subunit p19 |
| 2.8 | 224196_x_at | 51611 | CGI-30 | CGI-30 protein |
| 2.8 | 239401_at | --- | --- | Transcribed locus |
| 2.8 | 205376_at | 8821 | INPP4B | inositol polyphosphate-4-phosphatase, type II, 105kDa |
| 2.8 | 204951_at | 399 | RHOH | ras homolog gene family, member H |
| 2.8 | 214482_at | 7597 | ZNF46 | zinc finger protein 46 (KUP) |
| 2.9 | 200751_s_at | 3183 | HNRPC | heterogeneous nuclear ribonucleoprotein C (C1/C2) |
| 2.9 | 214697_s_at | 9991 | ROD1 | ROD1 regulator of differentiation 1 (S. pombe) |
| 2.9 | 204716_at | 8030 | CCDC6 | coiled-coil domain containing 6 |
| 2.9 | 202604_x_at | 102 | ADAM10 | a disintegrin and metalloproteinase domain 10 |
| 2.9 | 216266_s_at | 10565 | ARFGEF1 | ADP-ribosylation factor guanine nucleotide-exchange factor 1 (brefeldin A-inhibited) |
| 2.9 | 232591_s_at | 55754 | TMEM30A | transmembrane protein 30A |
| 2.9 | 201835_s_at | 5564 | PRKAB1 | protein kinase, AMP-activated, beta 1 non-catalytic subunit |
| 2.9 | 212409_s_at | 26092 | LAP1B | lamina-associated polypeptide 1B |
| 2.9 | 215739_s_at | 10426 | TUBGCP3 | tubulin, gamma complex associated protein 3 |
| 2.9 | 200604_s_at | 5573 | PRKAR1A | protein kinase, cAMP-dependent, regulatory, type I, alpha (tissue specific extinguisher 1) |
| 2.9 | 201399_s_at | 23471 | TPAM1 | translocation associated membrane protein 1 |
| 2.9 | 209055_s_at | 988 | CDC5L | CDC5 cell division cycle 5-like (S. pombe) |
| 2.9 | 214657_s_at | 283131 | TncRNA | Trophoblast-derived noncoding RNA |
| 2.9 | 220386_s_at | 27436 | EML4 | echinoderm microtubule associated protein like 4 |
| 2.9 | 234299_s_at | 51199 | NIN | ninein (GSK3B interacting protein) |
| 2.9 | 213548_s_at | 55573 | H41 | hypothetical protein H41 |
| 2.9 | 201946_s_at | 10576 | CCT2 | chaperonin containing TCP1, subunit 2 (beta) |
| 2.9 | 205732_s_at | 10499 | NCOA2 | nuclear receptor coactivator 2 |
| 2.9 | 208351_s_at | 5594 | MAPK1 | mitogen-activated protein kinase 1 |
| 2.9 | 76897_s_at | 23307 | KIAA0674 | KIAA0674 |
| 2.9 | 201211_s_at | 1654 | DDX3X | DEAD (Asp-Glu-Ala-Asp) box polypeptide 3, X-linked |
| 2.9 | 239170_at | 10096 | ACTR3 | ARP3 actin-related protein 3 homolog (yeast) |
| 2.9 | 221220_s_at | 55681 | SCYL2 | SCY1-like 2 (S. cerevisiae) |
| 2.9 | 224149_x_at | --- | --- | sarcolemma associated protein |
| 2.9 | 219868_s_at | 51479 | ANKFY1 | ankyrin repeat and FYVE domain containing 1 |
| 2.9 | 203357_s_at | 23473 | CAPN7 | calpain 7 |
| 2.9 | 221638_s_at | 8675 | STX16 | syntaxin 16 |
| 2.9 | 200641_s_at | 7534 | YWHAZ | tyrosine 3-monooxygenase/tryptophan 5-monooxygenase activation protein, zeta polypeptide |
| 2.9 | 201504_s_at | 7247 | TSN | translin |
| 2.9 | 202784_s_at | 23530 | NNT | nicotinamide nucleotide transhydrogenase |
| 2.9 | 224642_at | 84248 | FYTTD1 | forty-two-three domain containing 1 |
| 2.9 | 206562_s_at | 1452 | CSNK1A1 | casein kinase 1, alpha 1 |
| 2.9 | 212107_s_at | 1660 | DHX9 | DEAH (Asp-Glu-Ala-His) box polypeptide 9 |
| 2.9 | 210148_at | 10114 | HIPK3 | homeodomain interacting protein kinase 3 |
| 2.9 | 223271_s_at | 51496 | HSPC129 | hypothetical protein HSPC129 |
| 2.9 | 214513_s_at | 1385 | CREB1 | CAMP responsive element binding protein 1 |
| 2.9 | 215357_s_at | 84271 | POLDIP3 | polymerase (DNA-directed), delta interacting protein 3 |
| 2.9 | 212720_at | 10914 | PAPOLA | poly(A) polymerase alpha |
| 2.9 | 200987_x_at | 10197 | PSME3 | proteasome (prosome, macropain) activator subunit 3 (PA28 gamma; Ki) |
| 2.9 | 210257_x_at | 8450 | CUL4B | cullin 4B |
| 2.9 | 225408_at | --- | --- | Myelin basic protein |
| 2.9 | 222522_x_at | 55173 | MRPS10 | mitochondrial ribosomal protein S10 |
| 2.9 | 204109_s_at | 4800 | NFYA | nuclear transcription factor Y, alpha |
| 2.9 | 205446_s_at | 1386 | ATF2 | activating transcription factor 2 |
| 2.9 | 201259_s_at | 6856 | SYPL | synaptophysin-like protein |
| 2.9 | 201635_s_at | 8087 | FXR1 | fragile X mental retardation, autosomal homolog 1 |
| 2.9 | 201228_s_at | 10425 | ARIH2 | ariadne homolog 2 (Drosophila) |
| 2.9 | 206184_at | 1399 | CRKL | v-crk sarcoma virus CT10 oncogene homolog (avian)-like |
| 2.9 | 228810_at | 151195 | FLJ40432 | hypothetical protein FLJ40432 |
| 2.9 | 233878_s_at | 22803 | XRN2 | 5'-3' exoribonuclease 2 |
| 2.9 | 221643_s_at | 473 | RERE | arginine-glutamic acid dipeptide (RE) repeats |
| 2.9 | 206788_s_at | 865 | CBFB | core-binding factor, beta subunit |
| 2.9 | 220477_s_at | 29058 | C20orf30 | chromosome 20 open reading frame 30 |
| 2.9 | 215207_x_at | 116150 | C6orf68 | YDD19 protein /// chromosome 6 open reading frame 68 /// similar to hypothetical protein, MGC:7199 |
| 2.9 | 221194_s_at | 51136 | LOC51136 | PTD016 protein |
| 2.9 | 200722_s_at | 4076 | M11S1 | membrane component, chromosome 11, surface marker 1 |
| 2.9 | 212225_at | 10209 | SUI1 | putative translation initiation factor |
| 2.9 | 210639_s_at | 9474 | APG5L | APG5 autophagy 5-like (S. cerevisiae) |
| 2.9 | 216901_s_at | 10320 | ZNFN1A1 | zinc finger protein, subfamily 1A, 1 (Ikaros) |
| 2.9 | 215997_s_at | 8450 | CUL4B | cullin 4B |
| 2.9 | 210935_s_at | 9948 | WDR1 | WD repeat domain 1 |
| 2.9 | 232048_at | 143684 | MGC33371 | hypothetical protein MGC33371 |
| 2.9 | 202089_s_at | 25800 | SLC39A6 | solute carrier family 39 (zinc transporter), member 6 |
| 2.9 | 210971_s_at | 406 | ARNTL | aryl hydrocarbon receptor nuclear translocator-like |
| 2.9 | 208097_s_at | 81542 | TXNDC | thioredoxin domain containing /// thioredoxin domain containing |
| 2.9 | 207686_s_at | 841 | CASP8 | caspase 8, apoptosis-related cysteine protease |
| 2.9 | 202101_s_at | 5899 | RALB | v-ral simian leukemia viral oncogene homolog B (ras related; GTP binding protein) |
| 2.9 | 224455_s_at | 83440 | ADPGK | ADP-dependent glucokinase /// ADP-dependent glucokinase |
| 2.9 | 200727_s_at | 10097 | ACTR2 | ARP2 actin-related protein 2 homolog (yeast) |
| 2.9 | 238644_at | 114803 | KIAA1915 | KIAA1915 protein |
| 2.9 | 212595_s_at | 9802 | DAZAP2 | DAZ associated protein 2 |
| 2.9 | 210286_s_at | 9497 | SLC4A7 | solute carrier family 4, sodium bicarbonate cotransporter, member 7 |
| 2.9 | 212008_at | 23190 | UBXD2 | UBX domain containing 2 |
| 2.9 | 235234_at | 219988 | FLJ36874 | hypothetical protein FLJ36874 |
| 2.9 | 214336_s_at | 1314 | COPA | coatomer protein complex, subunit alpha |
| 2.9 | 215512_at | 10299 | 6-Mar | membrane-associated ring finger (C3HC4) 6 |
| 2.9 | 238816_at | 5663 | PSEN1 | Presenilin 1 (Alzheimer disease 3) |
| 2.9 | 221268_s_at | 81537 | SGPP1 | sphingosine-1-phosphate phosphatase 1 /// sphingosine-1-phosphate phosphatase 1 |
| 2.9 | 224678_at | 57148 | KIAA1219 | KIAA1219 protein |
| 2.9 | 216252_x_at | 355 | FAS | Fas (TNF receptor superfamily, member 6) |
| 2.9 | 210077_s_at | 6430 | SFRS5 | splicing factor, arginine/serine-rich 5 |
| 2.9 | 222660_s_at | 152006 | RNF38 | ring finger protein 38 |
| 2.9 | 200796_s_at | 4170 | MCL1 | myeloid cell leukemia sequence 1 (BCL2-related) |
| 2.9 | 224407_s_at | 51765 | MST4 | Mst3 and SOK1-related kinase /// Mst3 and SOK1-related kinase |
| 2.9 | 222385_x_at | 29927 | SEC61A1 | Sec61 alpha 1 subunit (S. cerevisiae) |
| 2.9 | 208853_s_at | 821 | CANX | calnexin |
| 2.9 | 222611_s_at | 55269 | PSPC1 | paraspeckle component 1 |
| 2.9 | 235475_at | 27230 | SERP1 | Stress-associated endoplasmic reticulum protein 1 |
| 2.9 | 201775_s_at | 9813 | KIAA0494 | KIAA0494 gene product |
| 2.9 | 223765_s_at | 55709 | KBTBD4 | kelch repeat and BTB (POZ) domain containing 4 |
| 2.9 | 210235_s_at | 8500 | PPFIA1 | protein tyrosine phosphatase, receptor type, f polypeptide (PTPRF), interacting protein (liprin), alpha 1 |
| 2.9 | 204426_at | 10959 | RNP24 | coated vesicle membrane protein |
| 2.9 | 201044_x_at | 1843 | DUSP1 | dual specificity phosphatase 1 |
| 2.9 | 238801_at | 155435 | LOC155435 | hypothetical protein LOC155435 |
| 2.9 | 214959_s_at | 8539 | API5 | apoptosis inhibitor 5 |
| 2.9 | 223016_x_at | 9406 | ZNF265 | zinc finger protein 265 |
| 2.9 | 208698_s_at | 4841 | NONO | non-POU domain containing, octamer-binding |
| 2.9 | 200778_s_at | 4735 | 2-Sep | septin 2 |
| 2.9 | 218036_x_at | 51068 | NMD3 | NMD3 homolog (S. cerevisiae) |
| 2.9 | 208721_s_at | 51433 | ANAPC5 | anaphase promoting complex subunit 5 |
| 2.9 | 216607_s_at | 1595 | CYP51A1 | cytochrome P450, family 51, subfamily A, polypeptide 1 |
| 2.9 | 241984_at | 1112 | CHES1 | checkpoint suppressor 1 |
| 2.9 | 204152_s_at | 4242 | MFNG | manic fringe homolog (Drosophila) |
| 2.9 | 206113_s_at | 5868 | RAB5A | RAB5A, member RAS oncogene family |
| 2.9 | 205885_s_at | 3676 | ITGA4 | integrin, alpha 4 (antigen CD49D, alpha 4 subunit of VLA-4 receptor) |
| 2.9 | 216205_s_at | 9927 | MFN2 | mitofusin 2 |
| 2.9 | 203676_at | 2799 | GNS | glucosamine (IV-acetyl)-6-sulfatase (Sanfilippo disease IIID) |
| 2.9 | 217097_s_at | 57157 | PHTF2 | putative homeodomain transcription factor 2 |
| 2.9 | 235331_x_at | 84333 | PCGF5 | polycomb group ring finger 5 |
| 2.9 | 216294_s_at | 84162 | KIAA1109 | KIAA1109 |
| 2.9 | 205408_at | 8028 | MLLT10 | myeloid/lymphoid or mixed-lineage leukemia (trithorax homolog, Drosophila); translocated to, 10 |
| 2.9 | 211090_s_at | 8899 | PRPF4B | PRP4 pre-mRNA processing factor 4 homolog B (yeast) /// PRP4 pre-mRNA processing factor 4 homolog B (yeast) |
| 2.9 | 205401_at | 8540 | AGPS | alkylglycerone phosphate synthase |
| 2.9 | 214786_at | 4214 | MAP3K1 | mitogen-activated protein kinase kinase kinase 1 |
| 2.9 | 224311_s_at | 51719 | CAB39 | calcium binding protein 39 |
| 2.9 | 209231_s_at | 84516 | MGC3248 | dynactin 4 |
| 2.9 | 207223_s_at | 9991 | ROD1 | ROD1 regulator of differentiation 1 (S. pombe) |
| 2.9 | 202213_s_at | 8450 | CUL4B | cullin 4B |
| 2.9 | 222502_s_at | 51569 | Ufm1 | ubiquitin-fold modifier 1 |
| 2.9 | 207164_s_at | 10472 | ZNF238 | zinc finger protein 238 |
| 2.9 | 225140_at | 51274 | KLF3 | Kruppel-like factor 3 (basic) |
| 2.9 | 233230_s_at | 57606 | KIAA1458 | KIAA1458 protein |
| 2.9 | 211016_x_at | 3308 | HSPA4 | heat shock 70kDa protein 4 |
| 2.9 | 216976_s_at | 6259 | RYK | RYK receptor-like tyrosine kinase |
| 2.9 | 210317_s_at | 7531 | YWHAE | tyrosine 3-monooxygenase/tryptophan 5-monooxygenase activation protein, epsilon polypeptide |
| 2.9 | 221423_s_at | 81555 | SMAP-5 | golgi membrane protein SB140 /// golgi membrane protein SB140 |
| 2.9 | 232229_at | 23064 | ALS4 | amyotrophic lateral sclerosis 4 |
| 2.9 | 212105_s_at | 1660 | DHX9 | DEAH (Asp-Glu-Ala-His) box polypeptide 9 |
| 2.9 | 215236_s_at | 8301 | PICALM | phosphatidylinositol binding clathrin assembly protein |
| 2.9 | 222846_at | 51762 | RAB8B | RAB8B, member RAS oncogene family |
| 2.9 | 214975_s_at | 8776 | MTMR1 | myotubularin related protein 1 |
| 2.9 | 217294_s_at | 2023 | ENO1 | enolase 1, (alpha) |
| 2.9 | 228801_at | 94101 | ORMDL1 | ORM1-like 1 (S. cerevisiae) |
| 2.9 | 212514_x_at | 1654 | DDX3X | DEAD (Asp-Glu-Ala-Asp) box polypeptide 3, X-linked |
| 2.9 | 210284_s_at | 23118 | MAP3K7IP2 | mitogen-activated protein kinase kinase kinase 7 interacting protein 2 |
| 2.9 | 222527_s_at | 55696 | RBM22 | RNA binding motif protein 22 |
| 2.9 | 201777_s_at | 9813 | KIAA0494 | KIAA0494 gene product |
| 2.9 | 205026_at | 6777 | STAT5B | signal transducer and activator of transcription 5B |
| 2.9 | 209895_at | 5781 | PTPN11 | protein tyrosine phosphatase, non-receptor type 11 (Noonan syndrome 1) |
| 2.9 | 211537_x_at | 6885 | MAP3K7 | mitogen-activated protein kinase kinase kinase 7 |
| 2.9 | 223701_s_at | 55031 | USP47 | ubiquitin specific protease 47 |
| 2.9 | 202647_s_at | 4893 | NRAS | neuroblastoma RAS viral (v-ras) oncogene homolog |
| 2.9 | 201337_s_at | 9341 | VAMP3 | vesicle-associated membrane protein 3 (cellubrevin) |
| 2.9 | 207549_x_at | 4179 | MCP | membrane cofactor protein (CD46, trophoblast-lymphocyte cross-reactive antigen) |
| 2.9 | 212065_s_at | 9736 | USP34 | ubiquitin specific protease 34 |
| 2.9 | 207724_s_at | 6683 | SPG4 | spastin |
| 2.9 | 218884_s_at | 60558 | FLJ13220 | hypothetical protein FLJ13220 |
| 2.9 | 211825_s_at | 2313 | FLI1 | Friend leukemia virus integration 1 |
| 2.9 | 208852_s_at | 821 | CANX | calnexin |
| 2.9 | 227214_at | 57120 | GOPC | golgi associated PDZ and coiled-coil motif containing |
| 2.9 | 216100_s_at | 26092 | LAP1B | lamina-associated polypeptide 1B |
| 2.9 | 219679_s_at | 51322 | WAC | WW domain containing adaptor with coiled-coil |
| 2.9 | 222562_s_at | 80351 | TNKS2 | tankyrase, TRF1-interacting ankyrin-related ADP-ribose polymerase 2 |
| 2.9 | 221082_s_at | 57446 | NDRG3 | NDRG family member 3 |
| 2.9 | 210892_s_at | 2969 | GTF2I | general transcription factor II, 1 |
| 2.9 | 211665_s_at | 6655 | SOS2 | son of sevenless homolog 2 (Drosophila) /// son of sevenless homolog 2 (Drosophila) |
| 2.9 | 217310_s_at | 22887 | FOXJ3 | forkhead box J3 |
| 2.9 | 201299_s_at | 55233 | MOBK1B | MOB1, Mps One Binder kinase activator-like 1B (yeast) |
| 2.9 | 201815_s_at | 9779 | TBC1D5 | TBC1 domain family, member 5 |
| 2.1 | 223592_s_at | 84282 | RNF135 | ring finger protein 135 |
| 2.1 | 225280_x_at | 414 | ARSD | arylsulfatase D |
| 2.1 | 217853_at | 64759 | TENS1 | tensin-like SH2 domain containing 1 |
| 2.1 | 221841_s_at | 9314 | KLF4 | Kruppel-like factor 4 (gut) |
| 2.1 | 227792_at | --- | --- | CDNA: FLJ22994 fis, clone KAT11918 |
| 2.1 | 221539_at | 1978 | EIF4EBP1 | eukaryotic translation initiation factor 4E binding protein 1 |
| 2.1 | 230741_at | --- | --- | Full length insert cDNA clone YX74D05 |
| 2.1 | 212792_at | 23333 | KIAA0877 | KIAA0877 protein |
| 2.1 | 207761_s_at | 25840 | DKFZP586A0522 | DKFZP586A0522 protein |
| 2.1 | 203104_at | 1436 | CSF1R | colony stimulating factor 1 receptor, formerly McDonough feline sarcoma viral (v-fms) oncogene homolog /// colony stimulating factor 1 receptor, formerly McDonough feline sarcoma viral (v-fms) oncogene homolog |
| 2.1 | 229383_at | 55016 | MARCH-I | Membrane-associated ring finger (C3HC4) 1 |
| 2.1 | 204112_s_at | 3176 | HNMT | histamine N-methyltransferase |
| 2.1 | 227749_at | --- | --- | Transcribed locus |
| 2.1 | 221656_s_at | 55160 | FLJ10521 | hypothetical protein FLJ10521 |
| 2.1 | 228234_at | 353376 | TICAM2 | toll-like receptor adaptor molecule 2 |
| 2.1 | 226818_at | 219972 | MPEG1 | macrophage expressed gene 1 |
| 2.1 | 205726_at | 1730 | DIAPH2 | diaphanous homolog 2 (Drosophila) |
| 2.1 | 228094_at | 120425 | AMICA1 | adhesion molecule, interacts with CXADR antigen 1 |
| 2.1 | 200699_at | 11014 | KDELR2 | KDEL (Lys-Asp-Glu-Leu) endoplasmic reticulum protein retention receptor 2 |
| 2.1 | 230131_x_at | 414 | ARSD | arylsulfatase D |
| 2.1 | 208818_s_at | 1312 | COMT | catechol-O-methyltransferase |
| 2.1 | 222881_at | 10855 | HPSE | heparanase |
| 2.1 | 211864_s_at | 26509 | FER1L3 | fer-1-like 3, myoferlin (C. elegans) |
| 2.1 | 227647_at | 10008 | KCNE3 | potassium voltage-gated channel, Isk-related family, member 3 |
| 2.1 | 235054_at | 131870 | NUDT16 | nudix (nucleoside diphosphate linked moiety X)-type motif 16 |
| 2.1 | 229937_x_at | 10859 | LILRB1 | Leukocyte immunoglobulin-like receptor, subfamily B (with TM and ITIM domains), member 1 |
| 2.1 | 226841_at | 219972 | MPEG1 | macrophage expressed gene 1 |
| 2.1 | 223922_x_at | 64231 | MS4A6A | membrane-spanning 4-domains, subfamily A, member 6A |
| 2.1 | 225731_at | 57182 | KIAA1223 | KIAA1223 protein |
| 2.1 | 203922_s_at | 1536 | CYBB | cytochrome b-245, beta polypeptide (chronic granulomatous disease) |
| 2.1 | 226853_at | 55589 | BMP2K | BMP2 inducible kinase |
| 2.1 | 219666_at | 64231 | MS4A6A | membrane-spanning 4-domains, subfamily A, member 6A |
| 2.1 | 227889_at | 54947 | FLJ20481 | hypothetical protein FLJ20481 |
| 2.1 | 226459_at | 118788 | PIK3AP1 | phosphoinositide-3-kinase adaptor protein 1 |
| 2.1 | 229390_at | 441168 | LOC441168 | similar to RIKEN cDNA A630077B13 gene; RIKEN cDNA 2810048G17 |
| 2.1 | 224983_at | 950 | SCARB2 | scavenger receptor class B, member 2 |
| 2.1 | 235964_x_at | --- | --- | SAM domain and HD domain 1 |
| 2.1 | 209933_s_at | 11314 | CD300A | CD300A antigen |
| 2.1 | 228532_at | 128346 | MGC24133 | hypothetical protein MGC24133 |
| 2.1 | 222217_s_at | 11000 | SLC27A3 | solute carrier family 27 (fatty acid transporter), member 3 |
| 2.1 | 208370_s_at | 1827 | DSCR1 | Down syndrome critical region gene 1 |
| 2.1 | 223344_s_at | 58475 | MS4A7 | membrane-spanning 4-domains, subfamily A, member 7 |
| 2.1 | 229756_at | --- | --- | Transcribed locus, strongly similar to XP_515285.1 similar to inhibitor of DNA binding 2; inhibitor of differentiation 2; DNA-binding protein inhibitor ID2; helix-loop-helix protein ID2 |
| | | | | [Pan troglodytes] |
| 2.1 | 227265_at | --- | --- | MRNA; cDNA DKFZp686N07104 (from clone DKFZp686N07104) |
| 2.1 | 205685_at | 942 | CD86 | CD86 antigen (CD28 antigen ligand 2, B7-2 antigen) |
| 2.1 | 205992_s_at | 3600 | IL15 | interleukin 15 |
| 2.1 | 223498_at | 92521 | HCMOGT-1 | sperm antigen HCMOGT-1 |
| 2.1 | 238513_at | 79056 | PRRG4 | Proline rich Gla (G-carboxyglutamic acid) 4 (transmembrane) |
| 2.1 | 213379_at | 27235 | CL640 | para-hydroxybenzoate-polyprenyltransferase, mitochondrial |
| 2.1 | 228771_at | 157 | ADRBK2 | adrenergic, beta, receptor kinase 2 |
| 2.1 | 232383_at | 22797 | TFEC | transcription factor EC |
| 2.1 | 235385_at | 55016 | MARCH-I | Membrane-associated ring finger (C3HC4) 1 |
| 2.1 | 226507_at | 5058 | PAK1 | p21/Cdc42/Rac1-activated kinase 1 (STE20 homolog, yeast) |
| 2.1 | 241929_at | 948 | CD36 | CD36 antigen (collagen type I receptor, thrombospondin receptor) |
| 2.1 | 232617_at | 1520 | CTSS | cathepsin S |
| 2.1 | 227716_at | 91544 | SOC | socius |
| 2.1 | 222756_s_at | --- | --- | arrestin, beta 1 |
| 2.1 | 31826_at | 23307 | KIAA0674 | KIAA0674 |
| 2.1 | 214030_at | 131544 | DKFZp667G2110 | hypothetical protein DKFZp667G2110 |
| 2.1 | 230264_s_at | 8905 | AP1S2 | adaptor-related protein complex 1, sigma 2 subunit |
| 2.1 | 224358_s_at | 58475 | MS4A7 | membrane-spanning 4-domains, subfamily A, member 7 /// membrane-spanning 4-domains, subfamily A, member 7 |
| 2.1 | 201944_at | 3074 | HEXB | hexosaminidase B (beta polypeptide) |
| 2.1 | 223343_at | 58475 | MS4A7 | membrane-spanning 4-domains, subfamily A, member 7 |
| 2.1 | 229810_at | --- | --- | Transcribed locus, moderately similar to XP_517655.1 similar to KIAA0825 protein [Pan troglodytes] |
| 2.1 | 225171_at | 93663 | ARHGAP18 | Rho GTPase activating protein 18 |
| 2.1 | 241344_at | 2720 | GLB1 | Galactosidase, beta 1 |
| 2.1 | 224702_at | --- | --- | hypothetical protein MGC23909 |
| 2.1 | 205504_at | 695 | BTK | Bruton agammaglobulinemia tyrosine kinase |
| 2.1 | 204546_at | 9764 | KIAA0513 | KIAA0513 |
| 2.1 | 226137_at | 9324 | HMGN3 | Homo sapiens, clone IMAGE:5288537, mRNA |
| 2.1 | 224356_x_at | 64231 | MS4A6A | membrane-spanning 4-domains, subfamily A, member 6A /// membrane-spanning 4-domains, subfamily A, member 6A |
| 2.1 | 235529_x_at | --- | --- | SAM domain and HD domain 1 |
| 2.1 | 204834_at | 10875 | FGL2 | fibrinogen-like 2 |
| 2.1 | 211794_at | 2533 | FYB | FYN binding protein (FYB-120/130) |
| 2.1 | 211366_x_at | 834 | CASP1 | caspase 1, apoptosis-related cysteine protease (interleukin 1, beta, convertase) |
| 2.1 | 214058_at | --- | --- | v-myc myelocytomatosis viral oncogene homolog 1, lung carcinoma derived (avian) |
| 2.1 | 215000_s_at | 9637 | FEZ2 | fasciculation and elongation protein zeta 2 (zygin II) |
| 2.1 | 208407_s_at | 1500 | CTNND1 | catenin (cadherin-associated protein), delta 1 |
| 2.1 | 201576_s_at | 2720 | GLB1 | galactosidase, beta 1 |
| 2.1 | 238025_at | 197259 | MLKL | mixed lineage kinase domain-like |
| 2.1 | 208923_at | 23191 | CYFIP1 | cytoplasmic FMR1 interacting protein 1 |
| 2.1 | 235518_at | 6546 | SLC8A1 | solute carrier family 8 (sodium/calcium exchanger), member 1 |
| 2.1 | 241773_at | 1902 | EDG2 | --- |
| 2.1 | 213508_at | 171546 | C14orf147 | chromosome 14 open reading frame 147 |
| 2.1 | 227618_at | 392490 | FLJ44635 | TPT1-like protein |
| 2.1 | 228766_at | 948 | CD36 | CD36 antigen (collagen type I receptor, thrombospondin receptor) |
| 2.1 | 230550_at | 64231 | MS4A6A | membrane-spanning 4-domains, subfamily A, member 6A |
| 2.1 | 206087_x_at | 3077 | HFE | hemochromatosis |
| 2.1 | 222912_at | 408 | ARRB1 | arrestin, beta 1 |
| 2.1 | 206011_at | 834 | CASP1 | caspase 1, apoptosis-related cysteine protease (interleukin 1, beta, convertase) |
| 2.1 | 209970_x_at | 834 | CASP1 | caspase 1, apoptosis-related cysteine protease (interleukin 1, beta, convertase) |
| 2.1 | 236923_x_at | --- | --- | CD36 antigen (collagen type I receptor, thrombospondin receptor) |
| 2.1 | 211367_s_at | 834 | CASP1 | caspase 1, apoptosis-related cysteine protease (interleukin 1, beta, convertase) |
| 2.1 | 225944_at | 57486 | NLN | neurolysin (metallopeptidase M3 family) |
| 2.1 | 226136_at | --- | --- | --- |
| 2.1 | 226810_at | 79940 | C6orf155 | Chromosome 6 open reading frame 155 |
| 2.1 | 226850_at | 285362 | SUMF1 | sulfatase modifying factor 1 |
| 2.1 | 56256_at | 51092 | SIDT2 | SID1 transmembrane family, member 2 |
| 2.1 | 208056_s_at | 863 | CBFA2T3 | core-binding factor, runt domain, alpha subunit 2; translocated to, 3 |
| 2.1 | 225710_at | --- | --- | CDNA FLJ34013 fis, clone FCBBF2002111 |
| 2.1 | 219806_s_at | 56935 | FN5 | FN5 protein |
| 2.1 | 243969_at | 123041 | SLC24A4 | solute carrier family 24 (sodium/potassium/calcium exchanger), member 4 |
| 2.1 | 212663_at | 23307 | KIAA0674 | KIAA0674 |
| 2.1 | 223204_at | 51313 | DKFZp434L142 | hypothetical protein DKFZp434L142 |
| 2.1 | 213566_at | 6039 | RIVASE6 | ribonuclease, RNase A family, k6 /// ribonuclease, RNase A family, k6 |
| 2.1 | 211330_s_at | 3077 | HFE | hemochromatosis |
| 2.11 | 242829_x_at | --- | --- | --- |
| 2.11 | 242232_at | 149401 | LOC149401 | Hypothetical protein LOC149401 |
| 2.11 | 233819_s_at | 26046 | ZNF294 | zinc finger protein 294 |
| 2.11 | 239392_s_at | 57645 | POGK | Pogo transposable element with KRAB domain |
| 2.11 | 201901_s_at | 7528 | YY1 | YY1 transcription factor |
| 2.11 | 227039_at | 11214 | AKAP13 | A kinase (PRKA) anchor protein 13 |
| 2.11 | 210461_s_at | 3983 | ABLIM1 | actin binding LIM protein 1 |
| 2.11 | 236841_at | 440897 | --- | CXYorf1-related protein |
| 2.11 | 229586_at | 80205 | CHD9 | chromodomain helicase DNA binding protein 9 |
| 2.11 | 200702_s_at | 57062 | DDX24 | DEAD (Asp-Glu-Ala-Asp) box polypeptide 24 |
| 2.11 | 203319_s_at | 7707 | ZNF148 | zinc finger protein 148 (pHZ-52) |
| 2.11 | 206636_at | 5922 | RASA2 | RAS p21 protein activator 2 |
| 2.11 | 241860_at | 9262 | STK17B | serine/threonine kinase 17b (apoptosis-inducing) |
| 2.11 | 201593_s_at | 55854 | LEREPO4 | likely ortholog of mouse immediate early response, erythropoietin 4 |
| 2.11 | 243768_at | 26054 | SENP6 | SUMO1/sentrin specific protease 6 |
| 2.11 | 238346_s_at | 96764 | NCOA6IP | nuclear receptor coactivator 6 interacting protein |
| 2.11 | 223161_at | 57189 | LCHN | LCHN protein |
| 2.11 | 211085_s_at | 6789 | STK4 | serine/threonine kinase 4 /// serine/threonine kinase 4 |
| 2.11 | 201810_s_at | 9467 | SH3BP5 | SH3-domain binding protein 5 (BTK-associated) |
| 2.11 | 221006_s_at | 81609 | SNX27 | sorting nexin family member 27 /// sorting nexin family member 27 |
| 2.11 | 203176_s_at | 7019 | TFAM | transcription factor A, mitochondrial |
| 2.11 | 211993_at | 65125 | WNK1 | WNK lysine deficient protein kinase 1 /// WNK lysine deficient protein kinase 1 |
| 2.11 | 223217_s_at | 64332 | NFKBIZ | nuclear factor of kappa light polypeptide gene enhancer in B-cells inhibitor, zeta |
| 2.11 | 203742_s_at | 6996 | TDG | thymine-DNA glycosylase |
| 2.11 | 212291_at | 204851 | HIPK1 | homeodomain interacting protein kinase 1 |
| 2.11 | 222600_s_at | 55236 | FLJ10808 | hypothetical protein FLJ10808 |
| 2.11 | 218859_s_at | 51575 | C20orf6 | chromosome 20 open reading frame 6 |
| 2.11 | 212634_at | 23376 | KIAA0776 | KIAA0776 |
| 2.11 | 228545_at | 7707 | ZNF148 | Zinc finger protein 148 (pHZ-52) |
| 2.11 | 243985_at | 2958 | GTF2A2 | General transcription factor IIA, 2, 12kDa |
| 2.11 | 244546_at | 54205 | CYCS | cytochrome c, somatic |
| 2.11 | 203181_x_at | 6733 | SRPK2 | SFRS protein kinase 2 |
| 2.11 | 236524_at | 83941 | BBP | Beta-amyloid binding protein precursor |
| 2.11 | 208993_s_at | 9360 | PPIG | peptidyl-prolyl isomerase G (cyclophilin G) |
| 2.11 | 227740_at | 127933 | UHMK1 | U2AF homology motif (UHM) kinase 1 |
| 2.11 | 219158_s_at | 80155 | NARG1 | NMDA receptor regulated 1 |
| 2.11 | 221753_at | 54434 | SSH1 | slingshot homolog 1 (Drosophila) |
| 2.11 | 228674_s_at | 27436 | EML4 | echinoderm microtubule associated protein like 4 |
| 2.11 | 212027_at | 58517 | RBM25 | RNA binding motif protein 25 |
| 2.11 | 240314_at | --- | --- | Nuclear receptor co-repressor 1 |
| 2.11 | 238536_at | 389753 | --- | Similar to phosphoglucomutase 5 |
| 2.11 | 223038_s_at | 58516 | C12orf14 | family with sequence similarity 60, member A |
| 2.11 | 209750_at | 9975 | NR1D2 | nuclear receptor subfamily 1, group D, member 2 |
| 2.11 | 238563_at | 10006 | ABI1 | Abl-interactor 1 |
| 2.11 | 235003_at | 127933 | UHMK1 | U2AF homology motif (UHM) kinase 1 |
| 2.11 | 238013_at | 59339 | PLEKHA2 | Pleckstrin homology domain containing, family A (phosphoinositide binding specific) member 2 |
| 2.11 | 222158_s_at | 51029 | PNAS-4 | CGI-146 protein |
| 2.11 | 243293_at | --- | --- | CDNA clone IMAGE:3346533, containing frame-shift errors |
| 2.11 | 201602_s_at | 4659 | PPP1R12A | protein phosphatase 1, regulatory (inhibitor) subunit 12A |
| 2.11 | 232311_at | 567 | B2M | Beta-2-microglobulin |
| 2.11 | 212451_at | 9728 | KIAA0256 | KIAA0256 gene product |
| 2.11 | 233964_at | --- | --- | Hypothetical protein FLJ14753 |
| 2.11 | 241242_at | 23196 | C9orf10 | Chromosome 9 open reading frame 10 |
| 2.11 | 201996_s_at | 23013 | SPEN | spen homolog, transcriptional regulator (Drosophila) |
| 2.11 | 222850_s_at | 79982 | FLJ14281 | hypothetical protein FLJ14281 |
| 2.11 | 210282_at | 7750 | ZNF198 | zinc finger protein 198 |
| 2.11 | 229353_s_at | 64710 | NUCKS | nuclear ubiquitous casein kinase and cyclin-dependent kinase substrate |
| 2.11 | 208003_s_at | 10725 | NFAT5 | nuclear factor of activated T-cells 5, tonicity-responsive |
| 2.11 | 206928_at | 7678 | ZNF124 | zinc finger protein 124 (HZF-16) |
| 2.11 | 214055_x_at | 23215 | BAT2D1 | BAT2 domain containing 1 |
| 2.11 | 209088_s_at | 29855 | UBN1 | ubinuclein 1 |
| 2.11 | 212742_at | 27246 | ZNF364 | zinc finger protein 364 |
| 2.11 | 240908_at | 391733 | --- | Hypothetical gene supported by AK055127; BC053586; BC067863 |
| 2.11 | 232489_at | 54482 | FLJ10287 | hypothetical protein FLJ10287 |
| 2.11 | 226975_at | 55599 | RNP | RNA-binding region (RNP1, RRM) containing 3 |
| 2.11 | 213015_at | 56987 | BBX | Bobby sox homolog (Drosophila) |
| 2.11 | 224676_at | 222068 | TMED4 | transmembrane emp24 protein transport domain containing 4 |
| 2.11 | 203627_at | 3480 | IGF1R | Insulin-like growth factor 1 receptor |
| 2.11 | 202124_s_at | 66008 | ALS2CR3 | amyotrophic lateral sclerosis 2 (juvenile) chromosome region, candidate 3 |
| 2.11 | 238130_at | 84901 | NFATC2IP | nuclear factor of activated T-cells, cytoplasmic, calcineurin-dependent 2 interacting protein |
| 2.11 | 210943_s_at | 1130 | LYST | lysosomal trafficking regulator |
| 2.11 | 242261_at | 3658 | IREB2 | Iron-responsive element binding protein 2 |
| 2.11 | 222413_s_at | 58508 | MLL3 | myeloid/lymphoid or mixed-lineage leukemia 3 |
| 2.11 | 229193_at | 51747 | CROP | Cisplatin resistance-associated overexpressed protein |
| 2.11 | 224851_at | 1021 | CDK6 | cyclin-dependent kinase 6 |
| 2.11 | 231920_s_at | 53944 | CSNK1G1 | casein kinase 1, gamma 1 |
| 2.11 | 210172_at | 7536 | SF1 | splicing factor 1 |
| 2.11 | 229398_at | 22931 | RAB18 | RAB18, member RAS oncogene family |
| 2.11 | 244869_at | 801 | CALM1 | Calmodulin 1 (phosphorylase kinase, delta) |
| 2.11 | 239692_at | 80895 | ILKAP | Integrin-linked kinase-associated serine/threonine phosphatase 2C |
| 2.11 | 212926_at | 23137 | SMC5L1 | SMC5 structural maintenance of chromosomes 5-like 1 (yeast) |
| 2.11 | 230970_at | 85464 | SSH2 | Slingshot homolog 2 (Drosophila) |
| 2.11 | 244396_at | 10146 | G3BP | Ras-GTPase-activating protein SH3-domain-binding protein |
| 2.11 | 215338_s_at | 4820 | NKTR | natural killer-tumor recognition sequence |
| 2.11 | 212587_s_at | 5788 | PTPRC | protein tyrosine phosphatase, receptor type, C |
| 2.11 | 227223_at | 9584 | RNPC2 | RNA-binding region (RNP1, RRM) containing 2 |
| 2.11 | 237176_at | 3936 | LCP1 | Lymphocyte cytosolic protein 1 (L-plastin) |
| 2.11 | 221830_at | 5911 | RAP2A | RAP2A, member of RAS oncogene family |
| 2.11 | 225507_at | 25957 | C6orf111 | chromosome 6 open reading frame 111 |
| 2.11 | 211944_at | 23215 | BAT2D1 | BAT2 domain containing 1 |
| 2.11 | 226675_s_at | 378938 | MALAT1 | metastasis associated lung adenocarcinoma transcript 1 (non-coding RNA) |
| 2.11 | 208835_s_at | 51747 | CROP | cisplatin resistance-associated overexpressed protein |
| 2.11 | 235376_at | 375341 | FLJ43654 | FLJ43654 protein |
| 2.11 | 225827_at | 27161 | EIF2C2 | eukaryotic translation initiation factor 2C, 2 |
| 2.11 | 233219_at | 4289 | MKLN1 | Muskelin 1, intracellular mediator containing kelch motifs |
| 2.11 | 230028_at | 22889 | KIAA0907 | KIAA0907 protein |
| 2.11 | 212240_s_at | 5295 | PIK3R1 | regulatory subunit 1 (p85 alpha) |
| 2.11 | 235213_at | 3707 | ITPKB | CDNA clone IMAGE:6201773 |
| 2.11 | 212007_at | 23190 | UBXD2 | UBX domain containing 2 |
| 2.11 | 227931_at | --- | --- | MRNA; cDNA DKFZp686D22106 (from clone DKFZp686D22106) |
| 2.11 | 214352_s_at | 3845 | KRAS | v-Ki-ras2 Kirsten rat sarcoma viral oncogene homolog |
| 2.11 | 201183_s_at | 1108 | CHD4 | chromodomain helicase DNA binding protein 4 |
| 2.11 | 230375_at | 25957 | C6orf111 | chromosome 6 open reading frame 111 |
| 2.11 | 215342_s_at | 9910 | RABGAP1L | RAB GTPase activating protein 1-like |
| 2.11 | 213729_at | 55660 | FNBP3 | formin binding protein 3 |
| 2.11 | 229841_at | --- | --- | Eukaryotic translation initiation factor 2C, 2 |
| 2.11 | 239571_at | 4205 | MEF2A | MADS box transcription enhancer factor 2, polypeptide A (myocyte enhancer factor 2A) |
| 2.11 | 213517_at | 5094 | PCBP2 | Poly(rC) binding protein 2 |
| 2.11 | 243329_at | 10336 | RNF3 | Polycomb group ring finger 3 |
| 2.11 | 235716_at | --- | --- | Transformer-2 alpha |
| 2.11 | 208610_s_at | 23524 | SRRM2 | serine/arginine repetitive matrix 2 |
| 2.11 | 212177_at | 25957 | C6orf111 | chromosome 6 open reading frame 111 |
| 2.11 | 224939_at | 57532 | 182-FIP | 82-kD FMRP Interacting Protein |
| 2.11 | 202412_s_at | 7398 | USP1 | ubiquitin specific protease 1 |
| 2.11 | 220739_s_at | 26505 | CNNM3 | cyclin M3 |
| 2.11 | 223134_at | 56987 | BBX | bobby sox homolog (Drosophila) |
| 2.11 | 229982_at | 79832 | FLJ21924 | hypothetical protein FLJ21924 |
| 2.11 | 239597_at | 255967 | PAN3 | PABP1-dependent poly A-specific ribonuclease subunit PAN3 |
| 2.11 | 207700_s_at | 8202 | NCOA3 | nuclear receptor coactivator 3 |
| 2.11 | 232356_at | 23167 | KIAA0143 | KIAA0143 protein |
| 2.11 | 213311_s_at | 22980 | KIAA1049 | KIAA1049 protein |
| 2.11 | 228628_at | 440608 | --- | Similar to Formin binding protein 2 (srGAP2) |
| 2.11 | 244677_at | --- | --- | --- |
| 2.11 | 230213_at | 79002 | MGC2803 | hypothetical protein MGC2803 |
| 2.11 | 201867_s_at | 6907 | TBL1X | transducin (beta)-like 1X-linked |
| 2.11 | 243751_at | --- | --- | --- |
| 2.11 | 229571_at | 805 | CALM2 | Calmodulin 2 (phosphorylase kinase, delta) |
| 2.11 | 242903_at | 3459 | IFNGR1 | Interferon gamma receptor 1 |
| 2.11 | 235567_at | --- | --- | CDNA FLJ31407 fis, clone NT2NE2000137 |
| 2.11 | 209678_s_at | 5584 | PRKCI | protein kinase C, iota |
| 2.11 | 219757_s_at | 54916 | C14orf101 | chromosome 14 open reading frame 101 |
| 2.11 | 211948_x_at | 23215 | BAT2D1 | BAT2 domain containing 1 |
| 2.11 | 242429_at | 163081 | ZNF567 | zinc finger protein 567 |
| 2.11 | 235954_at | 8125 | ANP32A | Acidic (leucine-rich) nuclear phosphoprotein 32 family, member A |
| 2.11 | 202972_s_at | 10144 | FAM13A1 | family with sequence similarity 13, member A1 |
| 2.11 | 202971_s_at | 8445 | DYRK2 | dual-specificity tyrosine-(Y)-phosphorylation regulated kinase 2 |
| 2.11 | 212629_s_at | 5586 | PKN2 | protein kinase N2 |
| 2.11 | 236327_at | --- | --- | Choline/ethanolamine phosphotransferase 1 |
| 2.11 | 230669_at | 5922 | RASA2 | --- |
| 2.11 | 229389_at | 89849 | FLJ00012 | FLJ00012 protein |
| 2.11 | 222544_s_at | 54904 | WHSC1L1 | Wolf-Hirschhorn syndrome candidate 1-lnke 1 |
| 2.11 | 230735_at | 3455 | IFNAR2 | Interferon (alpha, beta and omega) receptor 2 |
| 2.11 | 201730_s_at | 7175 | TPR | translocated promoter region (to activated MET oncogene) |
| 2.11 | 222540_s_at | 51773 | HBXAP | hepatitis B virus x associated protein |
| 2.11 | 235409_at | 23269 | MGA | MAX gene associated |
| 2.11 | 202323_s_at | 64746 | ACBD3 | acyl-Coenzyme A binding domain containing 3 |
| 2.11 | 222787_s_at | 54664 | FLJ11273 | hypothetical protein FLJ11273 |
| 2.11 | 227454_at | 57551 | TAOK1 | TAO kinase 1 |
| 2.11 | 234151_at | 10659 | CUGBP2 | CUG triplet repeat, RNA binding protein 2 |
| 2.11 | 225640_at | 401504 | LOC401504 | hypothetical gene supported by AK091718 |
| 2.11 | 224631_at | 80829 | ZFP91 | zinc finger protein 91 homolog (mouse) |
| 2.11 | 236254_at | 157680 | COH1 | vacuolar protein sorting 13B (yeast) |
| 2.11 | 235984_at | --- | --- | --- |
| 2.11 | 208859_s_at | 546 | ATRX | alpha thalassemia/mental retardation syndrome X-linked (RAD54 homolog, S. cerevisiae) |
| 2.11 | 224250_s_at | 79048 | SECISBP2 | SECIS binding protein 2 |
| 2.11 | 238706_at | 167153 | PAPD4 | PAP associated domain containing 4 |
| 2.11 | 230180_at | 10521 | DDX17 | DEAD (Asp-Glu-Ala-Asp) box polypeptide 17 |
| 2.11 | 203318_s_at | 7707 | ZNF148 | zinc finger protein 148 (pHZ-52) |
| 2.11 | 211503_s_at | 51552 | RAB14 | RAB14, member RAS oncogene family |
| 2.11 | 213850_s_at | 9169 | SFRS2IP | splicing factor, arginine/serine-rich 2, interacting protein |
| 2.11 | 213998_s_at | 10521 | DDX17 | DEAD (Asp-Glu-Ala-Asp) box polypeptide 17 |
| 2.11 | 210180_s_at | 6434 | SFRS10 | splicing factor, arginine/serine-rich 10 (transformer 2 homolog, Drosophila) |
| 2.11 | 202379_s_at | 4820 | NKTR | natural killer-tumor recognition sequence |
| 2.11 | 234326_at | --- | --- | --- |
| 2.11 | 209127_s_at | 9733 | SART3 | squamous cell carcinoma antigen recognised by T cells 3 |
| 2.11 | 241403_at | 57396 | CLK4 | CDC-like kinase 4 |
| 2.11 | 205798_at | 3575 | IL7R | interleukin 7 receptor /// interleukin 7 receptor |
| 2.11 | 240695_at | 10044 | SH2D3C | SH2 domain containing 3C |
| 2.11 | 201918_at | 55186 | FLJ10618 | Hypothetical protein FLJ10618 |
| 2.11 | 237813_at | 5094 | PCBP2 | poly(rC) binding protein 2 /// similar to mCBP |
| 2.11 | 201846_s_at | 23429 | RYBP | RING1 and YY1 binding protein |
| 2.11 | 201182_s_at | 1108 | CHD4 | chromodomain helicase DNA binding protein 4 |
| 2.11 | 209060_x_at | 8202 | NCOA3 | nuclear receptor coactivator 3 |
| 2.11 | 242617_at | 283578 | LOC283578 | Transmembrane emp24 domain containing 8 |
| 2.11 | 232097_at | 9878 | C14orf92 | chromosome 14 open reading frame 92 |
| 2.11 | 227062_at | 283131 | TncRNA | trophoblast-derived noncoding RNA |

**Table 3**

| module ID | affy ID | Entrez ID | Gene Symbol | Gene Title |
|---|---|---|---|---|
| 3.1 | 204415_at | 2537 | G1P3 | interferon, alpha-inducible protein (clone IFI-6-16) |
| 3.1 | 229450_at | 3437 | IFIT3 | interferon-induced protein with tetratricopeptide repeats 3 |
| 3.1 | 204994_at | 4600 | MX2 | myxovirus (influenza virus) resistance 2 (mouse) |
| 3.1 | 218400_at | 4940 | OAS3 | 2'-5'-oligoadenylate synthetase 3, 100kDa |
| 3.1 | 219863_at | 51191 | HERC5 | hect domain and RLD 5 |
| 3.1 | 204747_at | 3437 | IFIT3 | interferon-induced protein with tetratricopeptide repeats 3 |
| 3.1 | 226702_at | 129607 | LOC129607 | hypothetical protein LOC129607 |
| 3.1 | 202086_at | 4599 | MX1 | myxovirus (influenza virus) resistance 1, interferon-inducible protein p78 (mouse) /// myxovirus (influenza virus) resistance 1, interferon-inducible protein p78 (mouse) |
| 3.1 | 205483_s_at | 9636 | G1P2 | interferon, alpha-inducible protein (clone IFI-15K) |
| 3.1 | 226757_at | 3433 | IFIT2 | interferon-induced protein with tetratricopeptide repeats 2 |
| 3.1 | 208436_s_at | 3665 | IRF7 | interferon regulatory factor 7 |
| 3.1 | 214453_s_at | 10561 | IFI44 | interferon-induced protein 44 |
| 3.1 | 212285_s_at | 375790 | AGRN | agrin |
| 3.1 | 201641_at | 684 | BST2 | bone marrow stromal cell antigen 2 |
| 3.1 | 209417_s_at | 3430 | IFI35 | interferon-induced protein 35 |
| 3.1 | 224701_at | 54625 | PARP14 | poly (ADP-ribose) polymerase family, member 14 |
| 3.1 | 204972_at | 4939 | OAS2 | 2'-5'-oligoadenylate synthetase 2, 69/71kDa |
| 3.1 | 228617_at | 54739 | HSXIAPAF1 | XIAP associated factor-1 |
| 3.1 | 202270_at | 2633 | GBP1 | guanylate binding protein 1, interferon-inducible, 67kDa /// guanylate binding protein 1, interferon-inducible, 67kDa |
| 3.1 | 228531_at | 54809 | SAMD9 | sterile alpha motif domain containing 9 |
| 3.1 | 230036_at | 219285 | C7orf6 | chromosome 7 open reading frame 6 |
| 3.1 | 236285_at | 113730 | MGC16635 | Hypothetical protein BC009980 |
| 3.1 | 242625_at | 91543 | RSAD2 | radical S-adenosyl methionine domain containing 2 |
| 3.1 | 200986_at | 710 | SERPING1 | serine (or cysteine) proteinase inhibitor, clade G (Cl inhibitor), member 1, (angioedema, hereditary) |
| 3.1 | 213294_at | --- | --- | Hypothetical protein FLJ38348 |
| 3.1 | 201649_at | 9246 | UBE2L6 | ubiquitin-conjugating enzyme E2L 6 |
| 3.1 | 218543_s_at | 64761 | ZC3HDC1 | zinc finger CCCH type domain containing 1 |
| 3.1 | 203882_at | 10379 | ISGF3G | interferon-stimulated transcription factor 3, gamma 48kDa |
| 3.1 | 206133_at | 54739 | HSXIAPAF1 | XIAP associated factor-1 |
| 3.1 | 242234_at | 54739 | HSXIAPAF1 | XIAP associated factor-1 |
| 3.1 | 225291_at | 87178 | PNPT1 | polyribonucleotide nucleotidyltransferase 1 |
| 3.1 | 227807_at | 83666 | PARP9 | Poly (ADP-ribose) polymerase family, member 9 |
| 3.1 | 227609_at | 94240 | EPSTI1 | epithelial stromal interaction 1 (breast) |
| 3.1 | 209969_s_at | 6772 | STAT1 | signal transducer and activator of transcription 1, 91kDa |
| 3.1 | 209762_x_at | 3431 | SP110 | SP110 nuclear body protein |
| 3.1 | 221816_s_at | 51131 | PHF11 | PHD finger protein 11 |
| 3.1 | 228607_at | 4939 | OAS2 | 2'-5'-oligoadenylate synthetase 2, 69/71kDa |
| 3.1 | 219209_at | 64135 | IFIH1 | interferon induced with helicase C domain 1 |
| 3.1 | 204439_at | 10964 | IFI44L | interferon-induced protein 44-like |
| 3.1 | 204211_x_at | 5610 | EIF2AK2 | eukaryotic translation initiation factor 2-alpha kinase 2 |
| 3.1 | 210985_s_at | 6672 | SP100 | nuclear antigen Sp100 |
| 3.1 | 209761_s_at | 3431 | SP110 | SP110 nuclear body protein |
| 3.1 | 231769_at | 26270 | FBXO6 | F-box protein 6 |
| 3.1 | 44673_at | 6614 | SN | sialoadhesin |
| 3.1 | 222986_s_at | 51246 | SCOTIN | scotin |
| 3.1 | 205552_s_at | 4938 | OAS1 | 2',5'-oligoadenylate synthetase 1, 40/46kDa |
| 3.1 | 225415_at | 151636 | DTX3L | deltex 3-like (Drosophila) |
| 3.1 | 202688_at | 8743 | TNFSF10 | tumor necrosis factor (ligand) superfamily, member 10 /// tumor necrosis factor (ligand) superfamily, member 10 |
| 3.1 | 226603_at | 219285 | C7orf6 | chromosome 7 open reading frame 6 |
| 3.1 | 213361_at | 23424 | TDRD7 | tudor domain containing 7 |
| 3.1 | 222793_at | 23586 | DDX58 | DEAD (Asp-Glu-Ala-Asp) box polypeptide 58 |
| 3.1 | 200887_s_at | 6772 | STAT1 | signal transducer and activator of transcription 1, 91kDa |
| 3.1 | 223220_s_at | 83666 | PARP9 | poly (ADP-ribose) polymerase family, member 9 |
| 3.1 | 219356_s_at | 51510 | SNF7DC2 | chromatin modifying protein 5 |
| 3.1 | 242020_s_at | 81030 | ZBP1 | Z-DNA binding protein 1 |
| 3.1 | 201601_x_at | 8519 | IFITM1 | interferon induced transmembrane protein 1 (9-27) |
| 3.1 | 204224_s_at | 2643 | GCH1 | GTP cyclohydrolase 1 (dopa-responsive dystonia) |
| 3.1 | 217933_s_at | 51056 | LAP3 | leucine aminopeptidase 3 |
| 3.1 | 200042_at | 51493 | HSPC117 | hypothetical protein HSPC117 /// hypothetical protein HSPC117 |
| 3.1 | 202446_s_at | 5359 | PLSCR1 | phospholipid scramblase 1 |
| 3.1 | 224806_at | 440448 | --- | LOC440448 |
| 3.1 | 201315_x_at | 10581 | IFITM2 | interferon induced transmembrane protein 2 (1-8D) |
| 3.1 | 212203_x_at | 10410 | IFITM3 | interferon induced transmembrane protein 3 (1-8U) |
| 3.1 | 213293_s_at | 10346 | TRIM22 | tripartite motif-containing 22 |
| 3.1 | 208012_x_at | 3431 | SP110 | SP110 nuclear body protein |
| 3.1 | 201786_s_at | 103 | ADAR | adenosine deaminase, RNA-specific |
| 3.1 | 218986_s_at | 55601 | FLJ20035 | hypothetical protein FLJ20035 |
| 3.1 | 35254_at | 10906 | FLN29 | FLN29 gene product |
| 3.1 | 202269_x_at | 2633 | GBP1 | guanylate binding protein 1, interferon-inducible, 67kDa /// guanylate binding protein 1, interferon-inducible, 67kDa |
| 3.1 | 231577_s_at | 2633 | GBP1 | guanylate binding protein 1, interferon-inducible, 67kDa |
| 3.1 | 223298_s_at | 51251 | NT5C3 | 5'-nucleotidase, cytosolic III |
| 3.1 | 219691_at | 54809 | SAMD9 | sterile alpha motif domain containing 9 |
| 3.1 | 222154_s_at | 26010 | DNAPTP6 | DNA polymerase-transactivated protein 6 |
| 3.1 | 228152_s_at | 91351 | FLJ31033 | hypothetical protein FLJ31033 |
| 3.1 | 239979_at | 94240 | EPSTI1 | Epithelial stromal interaction 1 (breast) |
| 3.1 | 202145_at | 4061 | LY6E | lymphocyte antigen 6 complex, locus E |
| 3.1 | 212380_at | 23070 | KIAA0082 | KIAA0082 |
| 3.1 | 230314_at | 440424 | --- | Similar to hypothetical protein 628 |
| 3.1 | 235276_at | 94240 | EPSTI1 | --- |
| 3.1 | 200923_at | 3959 | LGALS3BP | lectin, galactoside-binding, soluble, 3 binding protein |
| 3.1 | 225636_at | 6773 | STAT2 | signal transducer and activator of transcription 2, 113kDa |
| 3.1 | 205660_at | 8638 | OASL | 2'-5'-oligoadenylate synthetase-like |
| 3.1 | 210797_s_at | 8638 | OASL | 2'-5'-oligoadenylate synthetase-like |
| 3.1 | 216565_x_at | 391020 | LOC391020 | similar to Interferon-induced transmembrane protein 3 (Interferon-inducible protein 1-8U) |
| 3.1 | 202307_s_at | 6890 | TAP1 | transporter 1, ATP-binding cassette, sub-family B (MDR/TAP) |
| 3.1 | 218943_s_at | 23586 | DDX58 | DEAD (Asp-Glu-Ala-Asp) box polypeptide 58 |
| 3.1 | 202869_at | 4938 | OAS1 | 2',5'-oligoadenylate synthetase 1, 40/46kDa |
| 3.1 | 204533_at | 3627 | CXCL10 | chemokine (C-X-C motif) ligand 10 |
| 3.1 | 53720_at | 55337 | FLJ11286 | hypothetical protein FLJ11286 |
| 3.1 | 210705_s_at | 85363 | TRIMS | tripartite motif-containing 5 |
| 3.1 | 238439_at | 118932 | ANKRD22 | ankyrin repeat domain 22 |
| 3.1 | 34689_at | 11277 | TREX1 | three prime repair exonuclease 1 |
| 3.1 | 235061_at | 152926 | PPM1K | protein phosphatase 1K (PP2C domain containing) |
| 3.1 | 241801_at | 80055 | PGAP1 | --- |
| 3.1 | 203964_at | 9111 | NMI | N-myc (and STAT) interactor |
| 3.1 | 214933_at | 773 | CACNA1A | calcium channel, voltage-dependent, P/Q type, alpha 1A subunit |
| 3.1 | 208581_x_at | 4501 | MT1X | metallothionein 1X |
| 3.1 | 202687_s_at | 8743 | TNFSF10 | tumor necrosis factor (ligand) superfamily, member 10 /// tumor necrosis factor (ligand) superfamily, member 10 |
| 3.1 | 218085_at | 51510 | SNF7DC2 | chromatin modifying protein 5 |
| 3.1 | 202430_s_at | 5359 | PLSCR1 | phospholipid scramblase 1 |
| 3.1 | 225344_at | 135112 | NCOA7 | nuclear receptor coactivator 7 |
| 3.1 | 205126_at | 7444 | VRK2 | vaccinia related kinase 2 |
| 3.1 | 214022_s_at | 8519 | IFITM1 | interferon induced transmembrane protein 1 (9-27) |
| 3.1 | 235508_at | 5371 | PML | Promyelocytic leukemia |
| 3.1 | 225973_at | 6891 | TAP2 | transporter 2, ATP-binding cassette, sub-family B (MDR/TAP) |
| 3.1 | 225929_s_at | 57674 | C17orf27 | chromosome 17 open reading frame 27 |
| 3.1 | 219357_at | 9567 | GTPBP1 | GTP binding protein 1 |
| 3.1 | 225076_s_at | 57169 | KIAA1404 | KIAA1404 protein |
| 3.1 | 241812_at | 26010 | DNAPTP6 | DNA polymerase-transactivated protein 6 |
| 3.1 | 238327_at | 440836 | LOC440836 | similar to MGC52679 protein |
| 3.1 | 221680_s_at | 51513 | ETV7 | ets variant gene 7 (TEL2 oncogene) |
| 3.1 | 219439_at | 56913 | ClGALT1 | core 1 synthase, glycoprotein-N-acetylgalactosamine 3-beta-galactosyltransferase, 1 |
| 3.1 | 232517_s_at | 85441 | PRIC285 | peroxisomal proliferator-activated receptor A interacting complex 285 |
| 3.1 | 219014_at | 51316 | PLAC8 | placenta-specific 8 |
| 3.1 | 214059_at | 10561 | IFI44 | Interferon-induced protein 44 |
| 3.1 | 219352_at | 55008 | HERC6 | hect domain and RLD 6 |
| 3.1 | 212845_at | 23034 | SAMD4 | sterile alpha motif domain containing 4 |
| 3.1 | 207375_s_at | 3601 | IL15RA | interleukin 15 receptor, alpha |
| 3.1 | 232375_at | 6772 | STAT1 | Signal transducer and activator of transcription 1, 91kDa |
| 3.1 | 218429_s_at | 55337 | FLJ11286 | hypothetical protein FLJ11286 |
| 3.1 | 208819_at | 4218 | RAB8A | RAB8A, member RAS oncogene family |
| 3.1 | 239988_at | 55008 | HERC6 | Hect domain and RLD 6 |
| 3.2 | 215087_at | 56905 | DKFZP434H132 | DKFZP434H132 protein |
| 3.2 | 243371_at | --- | --- | --- |
| 3.2 | 202284_s_at | 1026 | CDKN1A | cyclin-dependent kinase inhibitor 1A (p21, Cip1) |
| 3.2 | 46270_at | 51271 | UBAP1 | ubiquitin associated protein 1 |
| 3.2 | 235407_at | 402538 | --- | --- |
| 3.2 | 209803_s_at | 7262 | PHLDA2 | pleckstrin homology-like domain, family A, member 2 |
| 3.2 | 203045_at | 4814 | NINJ1 | ninjurin 1 |
| 3.2 | 217739_s_at | 10135 | PBEF1 | pre-B-cell colony enhancing factor 1 |
| 3.2 | 222088_s_at | 6515 | SLC2A3 | solute carrier family 2 (facilitated glucose transporter), member 3 /// solute carrier family 2 (facilitated glucose transporter), member 14 |
| 3.2 | 212171_x_at | 7422 | VEGF | vascular endothelial growth factor |
| 3.2 | 227060_at | 84957 | TNFRSF19L | tumor necrosis factor receptor superfamily, member 19-like |
| 3.2 | 225612_s_at | 84002 | B3GNT5 | UDP-GlcNAc:betaGal beta-1,3-N-acetylglucosaminyltransferase 5 |
| 3.2 | 225884_s_at | 64412 | ZNF336 | zinc finger protein 336 |
| 3.2 | 227991_x_at | 23099 | ZNF297B | Zinc finger protein 297B |
| 3.2 | 239474_at | 6533 | SLC6A6 | Solute carrier family 6 (neurotransmitter transporter, taurine), member 6 |
| 3.2 | 202924_s_at | 5326 | PLAGL2 | pleiomorphic adenoma gene-like 2 |
| 3.2 | 217738_at | 10135 | PBEF1 | pre-B-cell colony enhancing factor 1 |
| 3.2 | 210845_s_at | 5329 | PLAUR | plasminogen activator, urokinase receptor |
| 3.2 | 210512_s_at | 7422 | VEGF | vascular endothelial growth factor |
| 3.2 | 202497_x_at | 6515 | SLC2A3 | solute carrier family 2 (facilitated glucose transporter), member 3 |
| 3.2 | 210264_at | 2859 | GPR35 | G protein-coupled receptor 35 |
| 3.2 | 223309_x_at | 50640 | IPLA2 (GAMMA) | intracellular membrane-associated calcium-independent phospholipase A2 gamma |
| 3.2 | 41387_ r_at | 23135 | JMJD3 | jumonji domain containing 3 |
| 3.2 | 235479_at | 132864 | CPEB2 | cytoplasmic polyadenylation element binding protein 2 |
| 3.2 | 218033_s_at | 8303 | SNN | stannin |
| 3.2 | 201490_s_at | 10105 | PPIF | peptidylprolyl isomerase F (cyclophilin F) |
| 3.2 | 219257_s_at | 8877 | SPHK1 | sphingosine kinase 1 |
| 3.2 | 221654_s_at | 9960 | USP3 | ubiquitin specific protease 3 |
| 3.2 | 201489_at | 10105 | PPIF | peptidylprolyl isomerase F (cyclophilin F) |
| 3.2 | 206472_s_at | 7090 | TLE3 | transducin-like enhancer of split 3 (E(sp1) homolog, Drosophila) |
| 3.2 | 212769_at | 7090 | TLE3 | transducin-like enhancer of split 3 (E(sp1) homolog, Drosophila) |
| 3.2 | 212723_at | 23210 | PTDSR | phosphatidylserine receptor |
| 3.2 | 212770_at | 7090 | TLE3 | transducin-like enhancer of split 3 (E(sp1) homolog, Drosophila) |
| 3.2 | 230052_s_at | 84807 | TA-NFKBH | T-cell activation NFKB-like protein |
| 3.2 | 204182_s_at | 23099 | ZNF297B | zinc finger protein 297B |
| 3.2 | 200711_s_at | 6500 | SKP1A | S-phase kinase-associated protein 1A (p19A) |
| 3.2 | 203927_at | 4794 | NFKBIE | nuclear factor of kappa light polypeptide gene enhancer in B-cells inhibitor, epsilon |
| 3.2 | 216236_s_at | 6515 | SLC2A3 | solute carrier family 2 (facilitated glucose transporter), member 3 /// solute carrier family 2 (facilitated glucose transporter), member 14 |
| 3.2 | 236223_s_at | --- | --- | Transcribed locus |
| 3.2 | 213146_at | 23135 | JMJD3 | jumonji domain containing 3 |
| 3.2 | 224572_s_at | 359948 | IRF2BP2 | interferon regulatory factor 2 binding protein 2 |
| 3.2 | 209545_s_at | 8767 | RIPK2 | receptor-interacting serine-threonine kinase 2 |
| 3.2 | 202859_x_at | 3576 | IL8 | interleukin 8 |
| 3.2 | 213524_s_at | 50486 | G0S2 | putative lymphocyte G0/G1 switch gene |
| 3.2 | 202464_s_at | 5209 | PFKFB3 | 6-phosphofructo-2-kinase/fructose-2,6-biphosphatase 3 |
| 3.2 | 203887_s_at | 7056 | THBD | thrombomodulin |
| 3.2 | 35617_at | 5598 | MAPK7 | mitogen-activated protein kinase 7 |
| 3.2 | 218881_s_at | 2355 | FOSL2 | FOS-like antigen 2 |
| 3.2 | 207674_at | 2204 | FCAR | Fc fragment of IgA, receptor for |
| 3.2 | 202047_s_at | 23466 | CBX6 | chromobox homolog 6 |
| 3.2 | 224835_at | 56261 | KIAA1434 | hypothetical protein KIAA1434 |
| 3.2 | 227080_at | 90874 | MGC45731 | hypothetical protein MGC45731 |
| 3.2 | 216015_s_at | 114548 | CIAS1 | cold autoinflammatory syndrome 1 |
| 3.2 | 225955_at | 284207 | METRNL | meteorin, glial cell differentiation regulator-like |
| 3.2 | 238011_at | 29035 | PRO0149 | PRO0149 protein |
| 3.2 | 202498_s_at | 6515 | SLC2A3 | solute carrier family 2 (facilitated glucose transporter), member 3 |
| 3.2 | 223394_at | 29950 | SERTAD1 | SERTA domain containing 1 |
| 3.2 | 226952_at | 85403 | EAF1 | ELL associated factor 1 |
| 3.2 | 209536_s_at | 30844 | EHD4 | EH-domain containing 4 |
| 3.2 | 201626_at | 3638 | INSIG1 | insulin induced gene 1 |
| 3.2 | 229221_at | 960 | CD44 | --- |
| 3.2 | 204181_s_at | 23099 | ZNF297B | zinc finger protein 297B |
| 3.2 | 64488_at | --- | --- | CDNA FLJ38849 fis, clone MESAN2008936 |
| 3.2 | 209076_s_at | 56270 | WDR45L | WDR45-like |
| 3.2 | 209345_s_at | 55361 | PI4KII | phosphatidylinositol 4-kinase type II |
| 3.2 | 222815_at | 51132 | RNF12 | ring finger protein 12 |
| 3.2 | 216260_at | 23405 | DICER1 | Dicer1, Dcr-1 homolog (Drosophila) |
| 3.2 | 219081_at | 54882 | ANKHD1 | ankyrin repeat and KH domain containing 1 |
| 3.2 | 211458_s_at | 23710 | GABARAPL1 | GABA(A) receptor-associated protein like 1 /// GABA(A) receptors associated protein like 3 |
| 3.2 | 214230_at | 998 | CDC42 | cell division cycle 42 (GTP binding protein, 25kDa) |
| 3.2 | 214696_at | 84981 | MGC14376 | hypothetical protein MGC14376 |
| 3.2 | 38037_at | 1839 | HBEGF | heparin-binding EGF-like growth factor |
| 3.2 | 208869_s_at | 23710 | GABARAPL1 | GABA(A) receptor-associated protein like 1 |
| 3.2 | 228846_at | 4084 | MAD | MAX dimerization protein 1 |
| 3.2 | 228340_at | 7090 | TLE3 | transducin-like enhancer of split 3 (E(sp1) homolog, Drosophila) |
| 3.2 | 214108_at | 4149 | MAX | MYC associated factor X |
| 3.2 | 201460_at | 9261 | MAPKAPK2 | mitogen-activated protein kinase-activated protein kinase 2 |
| 3.2 | 210190_at | 8676 | STX11 | syntaxin 11 |
| 3.2 | 202637_s_at | 3383 | ICAM1 | intercellular adhesion molecule 1 (CD54), human rhinovirus receptor |
| 3.2 | 205220_at | 8843 | GPR109B | G protein-coupled receptor 109B /// G protein-coupled receptor 109B |
| 3.2 | 217202_s_at | 2752 | GLUL | glutamate-ammonia ligase (glutamine synthase) |
| 3.2 | 220740_s_at | 9990 | SLC12A6 | solute carrier family 12 (potassium/chloride transporters), member 6 |
| 3.2 | 202807_s_at | 10043 | TOM1 | target of mybl (chicken) |
| 3.2 | 204494_s_at | 56905 | DKFZP434H132 | DKFZP434H132 protein |
| 3.2 | 207993_s_at | 11261 | CHP | calcium binding protein P22 |
| 3.2 | 214211_at | 2495 | FTH1 | ferritin, heavy polypeptide 1 |
| 3.2 | 203370_s_at | 9260 | PDLIM7 | PDZ and LIM domain 7 (enigma) |
| 3.2 | 219434_at | 54210 | TREM1 | triggering receptor expressed on myeloid cells 1 |
| 3.2 | 210582_s_at | 3985 | LIMK2 | LIM domain kinase 2 |
| 3.2 | 202656_s_at | 9792 | SERTAD2 | SERTA domain containing 2 |
| 3.2 | 211924_s_at | 5329 | PLAUR | plasminogen activator, urokinase receptor /// plasminogen activator, urokinase receptor |
| 3.2 | 223186_at | 387521 | Kua | ubiquitin-conjugating enzyme variant Kua |
| 3.2 | 205681_at | 597 | BCL2A1 | BCL2-related protein A1 |
| 3.2 | 224828_at | 80315 | CPEB4 | cytoplasmic polyadenylation element binding protein 4 |
| 3.2 | 218023_s_at | 51307 | FAM53C | family with sequence similarity 53, member C |
| 3.2 | 208092_s_at | 81553 | FAM49A | family with sequence similarity 49, member A /// family with sequence similarity 49, member A |
| 3.2 | 205349_at | 2769 | GNA15 | guanine nucleotide binding protein (G protein), alpha 15 (Gq class) |
| 3.2 | 219622_at | 55647 | RAB20 | RAB20, member RAS oncogene family |
| 3.2 | 200868_s_at | 55905 | ZNF313 | zinc finger protein 313 |
| 3.2 | 218611_at | 51278 | IER5 | immediate early response 5 |
| 3.2 | 201170_s_at | 8553 | BHLHB2 | basic helix-loop-helix domain containing, class B, 2 |
| 3.2 | 217996_at | 22822 | PHLDA1 | pleckstrin homology-like domain, family A, member 1 |
| 3.2 | 212666_at | 57154 | SMURF1 | SMAD specific E3 ubiquitin protein ligase 1 /// LOC442601 |
| 3.2 | 39402_at | 3553 | IL1B | interleukin 1, beta |
| 3.2 | 204908_s_at | 602 | BCL3 | B-cell CLL/lymphoma 3 |
| 3.2 | 212470_at | 9043 | SPAG9 | sperm associated antigen 9 |
| 3.2 | 201670_s_at | 4082 | MARCKS | myristoylated alanine-rich protein kinase C substrate |
| 3.2 | 243931_at | 965 | CD58 | CD58 antigen, (lymphocyte function-associated antigen 3) |
| 3.2 | 203120_at | 7159 | TP53BP2 | tumor protein p53 binding protein, 2 |
| 3.2 | 200730_s_at | 7803 | PTP4A1 | protein tyrosine phosphatase type IVA, member 1 |
| 3.2 | 203094_at | 9587 | MAD2L1BP | MAD2L1 binding protein |
| 3.2 | 203297_s_at | 3720 | JARID2 | Jumonji, AT rich interactive domain 2 |
| 3.2 | 224164_at | 7170 | TPM3 | tropomyosin 3 |
| 3.2 | 201574_at | 2107 | ETF1 | eukaryotic translation termination factor 1 |
| 3.2 | 200989_at | 3091 | HIF1A | hypoxia-inducible factor 1, alpha subunit (basic helix-loop-helix transcription factor) |
| 3.2 | 219397_at | 80219 | FLJ13448 | hypothetical protein FLJ13448 |
| 3.2 | 91682_at | 54512 | EXOSC4 | --- |
| 3.2 | 210513_s_at | 7422 | VEGF | vascular endothelial growth factor |
| 3.2 | 219500_at | 23529 | CLC | cardiotrophin-like cytokine factor 1 |
| 3.2 | 221490_at | 51271 | UBAP1 | ubiquitin associated protein 1 |
| 3.2 | 236224_at | --- | --- | Transcribed locus |
| 3.2 | 243296_at | 10135 | PBEF1 | Pre-B-cell colony enhancing factor 1 |
| 3.2 | 37028_at | 23645 | PPP1R15A | protein phosphatase 1, regulatory (inhibitor) subunit 15A |
| 3.2 | 225557_at | 64651 | AXUD1 | AXIN1 up-regulated 1 |
| 3.2 | 209304_x_at | 4616 | GADD45B | growth arrest and DNA-damage-inducible, beta |
| 3.2 | 202147_s_at | 3475 | IFRD1 | interferon-related developmental regulator 1 |
| 3.2 | 244025_at | --- | --- | --- |
| 3.2 | 202672_s_at | 467 | ATF3 | activating transcription factor 3 |
| 3.2 | 210916_s_at | 960 | CD44 | CD44 antigen (homing function and Indian blood group system) |
| 3.2 | 210365_at | 861 | RUNX1 | runt-related transcription factor 1 (acute myeloid leukemia 1; aml1 oncogene) |
| 3.2 | 219382_at | 29946 | SERTAD3 | SERTA domain containing 3 |
| 3.2 | 210592_s_at | 6303 | SAT | spermidine/spermine Nl-acetyltransferase |
| 3.2 | 236899_at | --- | --- | --- |
| 3.2 | 226095_s_at | 146517 | LOC146517 | hypothetical protein LOC146517 |
| 3.2 | 201329_s_at | 2114 | ETS2 | v-ets erythroblastosis virus E26 oncogene homolog 2 (avian) |
| 3.2 | 225262_at | 2355 | FOSL2 | FOS-like antigen 2 |
| 3.2 | 224826_at | 56261 | KIAA1434 | hypothetical protein KIAA1434 |
| 3.2 | 201110_s_at | 7057 | THBS1 | thrombospondin 1 |
| 3.2 | 209099_x_at | 182 | JAG1 | Jagged 1 (Alagille syndrome) |
| 3.2 | 205020_s_at | 10124 | ARL4A | ADP-ribosylation factor-like 4A |
| 3.2 | 216268_s_at | 182 | JAG1 | Jagged 1 (Alagille syndrome) |
| 3.2 | 220712_at | --- | --- | --- |
| 3.2 | 218880_at | 2355 | FOSL2 | FOS-like antigen 2 |
| 3.2 | 221858_at | 23232 | TBC1D12 | TBC1 domain family, member 12 |
| 3.2 | 218559_s_at | 9935 | MAFB | v-maf musculoaponeurotic fibrosarcoma oncogene homolog B (avian) |
| 3.2 | 238893_at | 338758 | LOC338758 | hypothetical protein LOC338758 |
| 3.2 | 239486_at | --- | --- | Transcribed locus |
| 3.2 | 219557_s_at | 56675 | NRIP3 | nuclear receptor interacting protein 3 |
| 3.2 | 205807_s_at | 7286 | TUFT1 | tuftelin 1 |
| 3.2 | 230170_at | 5008 | OSM | oncostatin M |
| 3.2 | 203888_at | 7056 | THBD | thrombomodulin |
| 3.2 | 211506_s_at | 3576 | IL8 | interleukin 8 |
| 3.2 | 204614_at | 5055 | SERPINB2 | serine (or cysteine) proteinase inhibitor, clade B (ovalbumin), member 2 |
| 3.2 | 218145_at | 57761 | TRIB3 | tribbles homolog 3 (Drosophila) |
| 3.2 | 202241_at | 10221 | TRIB1 | tribbles homolog 1 (Drosophila) |
| 3.2 | 200663_at | 967 | CD63 | CD63 antigen (melanoma 1 antigen) |
| 3.2 | 201858_s_at | 5552 | PRG1 | proteoglycan 1, secretory granule |
| 3.2 | 224570_s_at | 359948 | IRF2BP2 | interferon regulatory factor 2 binding protein 2 |
| 3.2 | 208093_s_at | 81565 | NDEL1 | nudE nuclear distribution gene E homolog like 1 (A. nidulans) /// nudE nuclear distribution gene E homolog like 1 (A. nidulans) |
| 3.2 | 224831_at | 80315 | CPEB4 | cytoplasmic polyadenylation element binding protein 4 |
| 3.2 | 211962_s_at | 677 | ZFP36L1 | zinc finger protein 36, C3H type-like 1 |
| 3.2 | 204206_at | 4335 | MNT | MAX binding protein |
| 3.2 | 203313_s_at | 7050 | TGIF | TGFB-induced factor (TALE family homeobox) |
| 3.2 | 217253_at | --- | --- | (clone B3B3E13) Huntington's disease candidate region mRNA fragment. |
| 3.2 | 222874_s_at | 2055 | CLN8 | ceroid-lipofuscinosis, neuronal 8 (epilepsy, progressive with mental retardation) |
| 3.2 | 217741_s_at | 7763 | ZA20D2 | zinc finger, A20 domain containing 2 |
| 3.2 | 207075_at | 114548 | CIAS1 | cold autoinflammatory syndrome 1 |
| 3.2 | 220088_at | 728 | C5R1 | complement component 5 receptor 1 (C5a ligand) |
| 3.2 | 223454_at | 58191 | CXCL16 | chemokine (C-X-C motif) ligand 16 |
| 3.2 | 36994_at | 527 | ATP6VOC | ATPase, H+ transporting, lysosomal 16kDa, V0 subunit c |
| 3.2 | 209808_x_at | 3621 | ING1 | inhibitor of growth family, member 1 |
| 3.2 | 228918_at | 124935 | SLC43A2 | Solute carrier family 43, member 2 |
| 3.2 | 230748_at | 9120 | SLC16A6 | solute carrier family 16 (monocarboxylic acid transporters), member 6 /// similar to solute carrier family 16, member 6; monocarboxylate transporter 6 |
| 3.2 | 200798_x_at | 4170 | MCL1 | myeloid cell leukemia sequence 1 (BCL2-related) |
| 3.2 | 218013_x_at | 51164 | DCTN4 | dynactin 4 (p62) |
| 3.2 | 206748_s_at | 9043 | SPAG9 | sperm associated antigen 9 |
| 3.2 | 243463_s_at | --- | --- | Transcribed locus |
| 3.2 | 227534_at | 195827 | C9orf21 | chromosome 9 open reading frame 21 |
| 3.2 | 201627_s_at | 3638 | INSIG1 | insulin induced gene 1 |
| 3.2 | 242035_at | --- | --- | Ring finger protein 32 |
| 3.2 | 207121_s_at | 5597 | MAPK6 | mitogen-activated protein kinase 6 |
| 3.2 | 219657_s_at | 51274 | KLF3 | Kruppel-like factor 3 (basic) |
| 3.2 | 204093_at | 902 | CCNH | cyclin H |
| 3.2 | 201543_s_at | 56681 | SARA1 | SARla gene homolog 1 (S. cerevisiae) |
| 3.2 | 225154_at | 94056 | SYAP1 | synapse associated protein 1, SAP47 homolog (Drosophila) |
| 3.2 | 225142_at | 80853 | KIAA1718 | KIAA1718 protein |
| 3.2 | 203003_at | 4209 | MEF2D | MADS box transcription enhancer factor 2, polypeptide D (myocyte enhancer factor 2D) |
| 3.2 | 233647_s_at | 81602 | CDADC1 | cytidine and dCMP deaminase domain containing 1 |
| 3.2 | 218077_s_at | 51304 | ZDHHC3 | zinc finger, DHHC domain containing 3 |
| 3.2 | 221582_at | 92815 | HIST3H2A | histone 3, H2a |
| 3.2 | 201055_s_at | 10949 | HNRPA0 | heterogeneous nuclear ribonucleoprotein A0 |
| 3.2 | 239494_at | 55294 | FBXW7 | F-box and WD-40 domain protein 7 (archipelago homolog, Drosophila) |
| 3.2 | 204370_at | 10978 | HEAB | ATP/GTP-binding protein |
| 3.2 | 217682_at | 29035 | PRO0149 | PRO0149 protein |
| 3.2 2 | 230082_at | 9209 | LRRFIP2 | Leucine rich repeat (in FLII) interacting protein |
| 3.2 | 201573_s_at | 2107 | ETF1 | eukaryotic translation termination factor 1 |
| 3.2 | 240459_at | 91746 | YT521 | splicing factor YT521-B |
| 3.2 | 219774_at | 54520 | FLJ10996 | hypothetical protein FLJ10996 |
| 3.2 | 240038_at | 22936 | ELL2 | Elongation factor, RNA polymerase II, 2 |
| 3.2 | 244492_at | 6503 | SLA | Src-like-adaptor |
| 3.2 | 208937_s_at | 3397 | ID1 | inhibitor of DNA binding 1, dominant negative helix-loop-helix protein |
| 3.2 | 226489_at | 57458 | TMCC3 | transmembrane and coiled-coil domains 3 |
| 3.2 | 204258_at | 1105 | CHD1 | chromodomain helicase DNA binding protein 1 |
| 3.2 | 225024_at | 58490 | C20orf77 | chromosome 20 open reading frame 77 |
| 3.2 | 201625_s_at | 3638 | INSIG1 | insulin induced gene 1 |
| 3.2 | 201548_s_at | 10765 | JARID1B | Jumonji, AT rich interactive domain 1B (RBP2-like) |
| 3.2 | 209191_at | 84617 | TUBB6 | tubulin, beta 6 |
| 3.2 | 213300_at | 23130 | KIAA0404 | KIAA0404 protein |
| 3.2 | 45749_at | 79567 | FLJ13725 | hypothetical protein FLJ13725 |
| 3.2 | 203411_s_at | 4000 | LMNA | lamin A/C |
| 3.2 | 212086_x_at | 4000 | LMNA | lamin A/C |
| 3.2 | 224692_at | 84919 | PPP1R15B | protein phosphatase 1, regulatory (inhibitor) subunit 15B |
| 3.2 | 210542_s_at | 28232 | SLCO3A1 | solute carrier organic anion transporter family, member 3A1 |
| 3.2 | 212432_at | 80273 | GRPEL1 | GrpE-like 1, mitochondrial (E. coli) |
| 3.2 | 201236_s_at | 7832 | BTG2 | BTG family, member 2 |
| 3.2 | 223602_at | 84749 | USP30 | ubiquitin specific protease 30 |
| 3.2 | 201531_at | 7538 | ZFP36 | zinc finger protein 36, C3H type, homolog (mouse) |
| 3.2 | 202333_s_at | 7320 | UBE2B | ubiquitin-conjugating enzyme E2B (RAD6 homolog) |
| 3.2 | 242230_at | 6310 | ATXN1 | ataxin 1 |
| 3.2 | 227572_at | 84749 | USP30 | Ubiquitin specific protease 30 |
| 3.2 | 227680_at | 284695 | ZNF326 | zinc finger protein 326 |
| 3.2 | 214553_s_at | 10776 | ARPP-19 | cyclic AMP phosphoprotein, 19 kD |
| 3.2 | 224920_x_at | 91663 | MYADM | myeloid-associated differentiation marker |
| 3.2 | 203505_at | 19 | ABCA1 | ATP-binding cassette, sub-family A (ABC1), member 1 |
| 3.2 | 36564_at | 127544 | IBRDC3 | IBR domain containing 3 |
| 3.2 | 227356_at | --- | --- | CDNA: FLJ22198 fis, clone HRC01218 |
| 3.2 | 202638_s_at | 3383 | ICAM1 | intercellular adhesion molecule 1 (CD54), human rhinovirus receptor |
| 3.2 | 230492_s_at | 56261 | KIAA1434 | hypothetical protein KIAA1434 |
| 3.2 | 222408_s_at | 51646 | YPEL5 | yippee-like 5 (Drosophila) |
| 3.2 | 218062_x_at | 23580 | CDC42EP4 | CDC42 effector protein (Rho GTPase binding) 4 |
| 3.2 | 214721_x_at | 23580 | CDC42EP4 | CDC42 effector protein (Rho GTPase binding) 4 |
| 3.2 | 225754_at | 164 | AP1G1 | adaptor-related protein complex 1, gamma 1 subunit |
| 3.2 | 202657_s_at | 9792 | SERTAD2 | SERTA domain containing 2 |
| 3.2 | 211559_s_at | 901 | CCNG2 | cyclin G2 |
| 3.2 | 201943_s_at | 1362 | CPD | carboxypeptidase D |
| 3.2 | 213138_at | 10865 | ARID5A | AT rich interactive domain 5A (MRF1-like) |
| 3.2 | 219409_at | 79753 | SNIP1 | Smad nuclear interacting protein |
| 3.2 | 221763_at | 221037 | JMJD1C | jumonji domain containing 1C |
| 3.2 | 217839_at | 10342 | TFG | TRK-fused gene |
| 3.2 | 220127_s_at | 54850 | FBXL12 | F-box and leucine-rich repeat protein 12 |
| 3.2 | 212947_at | 23315 | SLC9A8 | solute carrier family 9 (sodium/hydrogen exchanger), isoform 8 |
| 3.2 | 206115_at | 1960 | EGR3 | early growth response 3 |
| 3.2 | 201324_at | 2012 | EMP1 | epithelial membrane protein 1 |
| 3.2 | 211139_s_at | 4664 | NAB1 | NGFI-A binding protein 1 (EGR1 binding protein 1) |
| 3.2 | 238719_at | 5515 | PPP2CA | Protein phosphatase 2 (formerly 2A), catalytic subunit, alpha isoform |
| 3.2 | 206036_s_at | 5966 | REL | v-rel reticuloendotheliosis viral oncogene homolog (avian) |
| 3.2 | 235242_at | --- | --- | CDNA FLJ41375 fis, clone BRCAN2007700 |
| 3.2 | 203885_at | 23011 | RAB21 | RAB21, member RAS oncogene family |
| 3.2 | 218215_s_at | 7376 | NR1H2 | nuclear receptor subfamily 1, group H, member 2 |
| 3.2 | 218379_at | 10179 | RBM7 | RNA binding motif protein 7 |
| 3.2 | 201881_s_at | 25820 | ARIH1 | ariadne homolog, enzyme E2 binding protein, 1 (Drosophila) |
| 3.2 | 204243_at | 6018 | RLF | rearranged L-myc fusion sequence |
| 3.2 | 222706_at | 54883 | FLJ20291 | hypothetical protein FLJ20291 |
| 3.2 | 212146_at | 23207 | PLEKHM2 | pleckstrin homology domain containing, family M (with RUN domain) member 2 |
| 3.2 | 201410_at | 55041 | PLEKHB2 | Pleckstrin homology domain containing, family B (evectins) member 2 |
| 3.2 | 201631_s_at | 8870 | IER3 | immediate early response 3 |
| 3.2 | 215501_s_at | 11221 | DUSP10 | dual specificity phosphatase 10 |
| 3.2 | 202129_s_at | 8780 | RIOK3 | RIO kinase 3 (yeast) /// RIO kinase 3 (yeast) |
| 3.2 | 217591_at | 6498 | SKIL | SKI-like |
| 3.2 | 239598_s_at | 54947 | FLJ20481 | hypothetical protein FLJ20481 |
| 3.2 | 225659_at | 339745 | LOC339745 | hypothetical protein LOC339745 |
| 3.2 | 225498_at | 128866 | C20orf178 | chromatin modifying protein 4B |
| 3.2 | 225673_at | 91663 | MYADM | myeloid-associated differentiation marker |
| 3.2 | 203471_s_at | 5341 | PLEK | pleckstrin |
| 3.2 | 219681_s_at | 80223 | RAB11FIP1 | RAB11 family interacting protein 1 (class I) |
| 3.2 | 202014_at | 23645 | PPP1R15A | protein phosphatase 1, regulatory (inhibitor) subunit 15A |
| 3.2 | 213191_at | 148022 | TRIF | TIR domain containing adaptor inducing interferon-beta |
| 3.2 | 212722_s_at | 23210 | PTDSR | phosphatidylserine receptor |
| 3.2 | 225582_at | 85450 | KIAA1754 | KIAA1754 |
| 3.2 | 204490_s_at | 960 | CD44 | CD44 antigen (homing function and Indian blood group system) |
| 3.2 | 203749_s_at | 5914 | RARA | retinoic acid receptor, alpha |
| 3.2 | 204564_at | 10336 | PCGF3 | polycomb group ring finger 3 |
| 3.2 | 204334_at | 8609 | KLF7 | Kruppel-like factor 7 (ubiquitous) |
| 3.2 | 202071_at | 6385 | SDC4 | syndecan 4 (amphiglycan, ryudocan) |
| 3.2 | 203140_at | 604 | BCL6 | B-cell CLL/lymphoma 6 (zinc finger protein 51) /// B-cell CLL/lymphoma 6 (zinc finger protein 51) |
| 3.2 | 242931_at | --- | --- | Transcribed locus |
| 3.2 | 208785_s_at | 81631 | MAP1LC3B | microtubule-associated protein 1 light chain 3 beta |
| 3.2 | 226275_at | 4084 | MAD | MAX dimerization protein 1 |
| 3.2 | 200919_at | 1912 | PHC2 | polyhomeotic-like 2 (Drosophila) |
| 3.2 | 224891_at | 2309 | FOXO3A | forkhead box O3A |
| 3.2 | 200797_s_at | 4170 | MCL1 | myeloid cell leukemia sequence 1 (BCL2-related) |
| 3.2 | 208786_s_at | 81631 | MAP1LC3B | microtubule-associated protein 1 light chain 3 beta |
| 3.2 | 203234_at | 7378 | UPP1 | uridine phosphorylase 1 |
| 3.2 | 205767_at | 2069 | EREG | epiregulin |
| 3.2 | 203455_s_at | 6303 | SAT | spermidine/spermine N1-acetyltransferase |
| 3.2 | 215001_s_at | 2752 | GLUL | glutamate-ammonia ligase (glutamine synthase) |
| 3.2 | 240432_x_at | --- | --- | Transcribed locus, weakly similar to XP-548293.1 similar to Flotillin-2 (Reggie-1) (REG-1) [Canis familiaris] |
| 3.2 | 203574_at | 4783 | NFIL3 | nuclear factor, interleukin 3 regulated |
| 3.2 | 231990_at | 9958 | USP15 | ubiquitin specific protease 15 |
| 3.2 | 239124_at | 5306 | PITPNA | Phosphatidylinositol transfer protein, alpha |
| 3.2 | 221432_s_at | 81894 | SLC25A28 | solute carrier family 25, member 28 /// solute carrier family 25, member 28 |
| 3.2 | 218032_at | 8303 | SNN | stannin |
| 3.2 | 227195_at | 84858 | ZNF503 | zinc finger protein 503 |
| 3.2 | 201739_at | 6446 | SGK | serum/glucocorticoid regulated kinase |
| 3.2 | 223460_at | 84254 | CAMKK1 | calcium/calmodulin-dependent protein kinase kinase 1, alpha |
| 3.2 | 222670_s_at | 9935 | MAFB | v-maf musculoaponeurotic fibrosarcoma oncogene homolog B (avian) |
| 3.2 | 204180_s_at | 23099 | ZNF297B | zinc finger protein 297B |
| 3.2 | 222816_s_at | 54877 | ZCCHC2 | zinc finger, CCHC domain containing 2 |
| 3.2 | 202082_s_at | 6397 | SEC14L1 | SEC14-like 1 (S. cerevisiae) |
| 3.2 | 205114_s_at | 414062 /// 6348 /// 6349 | CCL3 /// CCL3L1 /// MGC12815 | chemokine (C-C motif) ligand 3 /// chemokine (C-C motif) ligand 3-like 1 /// chemokine (C-C motif) ligand 3-like, centromeric |
| 3.2 | 241027_at | 4976 | OPA1 | optic atrophy 1 (autosomal dominant) |
| 3.2 | 218631_at | 60370 | AVPI1 | arginine vasopressin-induced 1 |
| 3.2 | 212458_at | 200734 | SPRED2 | sprouty-related, EVH1 domain containing 2 |
| 3.2 | 212817_at | 25822 | DNAJB5 | DnaJ (Hsp40) homolog, subfamily B, member |
| 3.2 | 217997_at | 22822 | PHLDA1 | pleckstrin homology-like domain, family A, member 1 |
| 3.2 | 231756_at | 57829 | ZP4 | zona pellucida glycoprotein 4 |
| 3.2 | 209838_at | 9318 | COPS2 | COP9 constitutive photomorphogenic homolog subunit 2 (Arabidopsis) |
| 3.2 | 209706_at | 4824 | NKX3-1 | NK3 transcription factor related, locus 1 (Drosophila) |
| 3.2 | 225954_s_at | 90007 | MIDN | midnolin |
| 3.2 | 41386_1_at | 23135 | JMJD3 | jumonji domain containing 3 |
| 3.2 | 208922_s_at | 10482 | NXF1 | nuclear RNA export factor 1 |
| 3.2 | 200632_s_at | 10397 | NDRG1 | N-myc downstream regulated gene 1 |
| 3.2 | 31845_at | 2000 | ELF4 | E74-like factor 4 (ets domain transcription factor) |
| 3.2 | 242176_at | --- | --- | --- |
| 3.2 | 211865_s_at | 51343 | FZR1 | fizzy/cell division cycle 20 related 1 (Drosophila) |
| 3.2 | 223195_s_at | 83667 | SESN2 | sestrin 2 |
| 3.2 | 205409_at | 2355 | FOSL2 | FOS-like antigen 2 |
| 3.2 | 204095_s_at | 8178 | ELL | elongation factor RNA polymerase II |
| 3.2 | 224836_at | 58476 | TP53INP2 | tumor protein p53 inducible nuclear protein 2 |
| 3.2 | 237502_at | 54675 | C20orf155 | Chromosome 20 open reading frame 155 |
| 3.2 | 242397_at | 4973 | OLR1 | oxidised low density lipoprotein (lectin-like) receptor 1 |
| 3.2 | 203821_at | 1839 | HBEGF | heparin-binding EGF-like growth factor |
| 3.3 | 218450_at | 50865 | HEBP1 | heme binding protein 1 |
| 3.3 | 203615_x_at | 6817 | SULT1A1 | sulfotransferase family, cytosolic, 1A, phenol-preferring, member 1 |
| 3.3 | 215299_x_at | 6817 | SULT1A1 | sulfotransferase family, cytosolic, 1A, phenol-preferring, member 1 |
| 3.3 | 207122_x_at | 6799 | SULT1A2 | sulfotransferase family, cytosolic, 1A, phenol-preferring, member 2 |
| 3.3 | 211385_x_at | 6799 | SULT1A2 | sulfotransferase family, cytosolic, 1A, phenol-preferring, member 2 |
| 3.3 | 202096_s_at | 706 | BZRP | benzodiazapine receptor (peripheral) |
| 3.3 | 204099_at | 6604 | SMARCD3 | SWI/SNF related, matrix associated, actin dependent regulator of chromatin, subfamily d, member 3 |
| 3.3 | 208699_x_at | 7086 | TKT | transketolase (Wernicke-Korsakoff syndrome) |
| 3.3 | 208700_s_at | 7086 | TKT | transketolase (Wernicke-Korsakoff syndrome) |
| 3.3 | 209473_at | 953 | ENTPD1 | ectonucleoside triphosphate diphosphohydrolase 1 |
| 3.3 | 218660_at | 8291 | DYSF | dysferlin, limb girdle muscular dystrophy 2B (autosomal recessive) |
| 3.3 | 219549_s_at | 10313 | RTN3 | reticulon 3 |
| 3.3 | 201463_s_at | 6888 | TALDO1 | transaldolase 1 |
| 3.3 | 205786_s_at | 3684 | ITGAM | integrin, alpha M (complement component receptor 3, alpha; also known as CD11b (p170), macrophage antigen alpha polypeptide) |
| | | | | /// integrin, alpha M (complement component receptor 3, alpha; also known as CD11b (p170), macrophage antigen alpha polypeptide) |
| 3.3 | 215952_s_at | 4946 | OAZ1 | ornithine decarboxylase antizyme 1 |
| 3.3 | 219938_s_at | 9050 | PSTPIP2 | proline-serine-threonine phosphatase interacting protein 2 |
| 3.3 | 204214_s_at | 10981 | RAB32 | RAB32, member RAS oncogene family |
| 3.3 | 200824_at | 2950 | GSTP1 | glutathione S-transferase pi |
| 3.3 | 213572_s_at | 1992 | SERPINB1 | serine (or cysteine) proteinase inhibitor, clade B (ovalbumin), member 1 |
| 3.3 | 213397_x_at | 6038 | RNASE4 | ribonuclease, RNase A family, 4 |
| 3.3 | 215399_s_at | 10956 | OS-9 | amplified in osteosarcoma |
| 3.3 | 202990_at | 5836 | PYGL | phosphorylase, glycogen; liver (Hers disease, glycogen storage disease type VI) |
| 3.3 | 222688_at | 55331 | PHCA | phytoceramidase, alkaline |
| 3.3 | 206584_at | 23643 | LY96 | lymphocyte antigen 96 |
| 3.3 | 200975_at | 5538 | PPT1 | palmitoyl-protein thioesterase 1 (ceroid-lipofuscinosis, neuronal 1, infantile) |
| 3.3 | 204620_s_at | 1462 | CSPG2 | chondroitin sulfate proteoglycan 2 (versican) |
| 3.3 | 200827_at | 5351 | PLOD1 | procollagen-lysine 1, 2-oxoglutarate 5-dioxygenase 1 |
| 3.3 | 203299_s_at | 8905 | AP1S2 | adaptor-related protein complex 1, sigma 2 subunit |
| 3.3 | 205147_x_at | 4689 | NCF4 | neutrophil cytosolic factor 4, 40kDa |
| 3.3 | 225129_at | 221184 | CPNE2 | copine II |
| 3.3 | 201118_at | 5226 | PGD | phosphogluconate dehydrogenase /// phosphogluconate dehydrogenase |
| 3.3 | 217977_at | 51734 | SEPX1 | selenoprotein X, 1 |
| 3.3 | 220001_at | 23569 | PADI4 | peptidyl arginine deiminase, type IV |
| 3.3 | 207467_x_at | 831 | CAST | calpastatin |
| 3.3 | 208908_s_at | 831 | CAST | calpastatin |
| 3.3 | 208771_s_at | 4048 | LTA4H | leukotriene A4 hydrolase |
| 3.3 | 218383_at | 54930 | C14orf94 | chromosome 14 open reading frame 94 |
| 3.3 | 204714_s_at | 2153 | F5 | coagulation factor V (proaccelerin, labile factor) |
| 3.3 | 203912_s_at | 1774 | DNASE1L1 | deoxyribonuclease I-like 1 |
| 3.3 | 207168_s_at | 9555 | H2AFY | H2A histone family, member Y |
| 3.3 | 210715_s_at | 10653 | SPINT2 | serine protease inhibitor, Kunitz type, 2 |
| 3.3 | 201290_at | 23478 | SEC11L1 | SEC11-like 1 (S. cerevisiae) |
| 3.3 | 222409_at | 23603 | CORO1C | coronin, actin binding protein, 1C |
| 3.3 | 209522_s_at | 1384 | CRAT | carnitine acetyltransferase |
| 3.3 | 203591_s_at | 1441 | CSF3R | colony stimulating factor 3 receptor (granulocyte) /// colony stimulating factor 3 receptor (granulocyte) |
| 3.3 | 225941_at | 317649 | EIF4E3 | eukaryotic translation initiation factor 4E member 3 |
| 3.3 | 225796_at | 54899 | PXK | PX domain containing serine/threonine kinase |
| 3.3 | 220990_s_at | 81671 | VMP1 | likely ortholog of rat vacuole membrane protein 1 /// likely ortholog of rat vacuole membrane protein 1 |
| 3.3 | 229940_at | 84193 | C14orf154 | chromosome 14 open reading frame 154 |
| 3.3 | 226865_at | --- | --- | MRNA; cDNA DKFZp564O0862 (from clone DKFZp564O0862) |
| 3.3 | 227038_at | 166929 | MGC26963 | hypothetical protein MGC26963 |
| 3.3 | 203799_at | 9936 | CD302 | CD302 antigen |
| 3.3 | 238455_at | 84898 | PLXDC2 | Plexin domain containing 2 |
| 3.3 | 205174_s_at | 25797 | QPCT | glutaminyl-peptide cyclotransferase (glutaminyl cyclase) |
| 3.3 | 221210_s_at | 80896 | NPL | N-acetylneuraminate pyruvate lyase (dihydrodipicolinate synthase) /// N-acetylneuraminate pyruvate lyase (dihydrodipicolinate synthase) |
| 3.3 | 229228_at | --- | --- | cAMP responsive element binding protein 5 |
| 3.3 | 228220_at | 115548 | FCHO2 | FCH domain only 2 |
| 3.3 | 204860_s_at | 4671 | BIRC1 | baculoviral IAP repeat-containing 1 |
| 3.3 | 206643_at | 3034 | HAL | histidine ammonia-lyase |
| 3.3 | 205230_at | 22895 | RPH3A | rabphilin 3A homolog (mouse) |
| 3.3 | 227961_at | 1508 | CTSB | Cathepsin B |
| 3.3 | 209005_at | 26234 | FBXL5 | F-box and leucine-rich repeat protein 5 |
| 3.3 | 218673_s_at | 10533 | APG7L | APG7 autophagy 7-like (S. cerevisiae) |
| 3.3 | 212421_at | 23313 | C22orf9 | chromosome 22 open reading frame 9 |
| 3.3 | 213241_at | 10154 | PLXNC1 | NY-REN-58 antigen |
| 3.3 | 221666_s_at | 29108 | PYCARD | PYD and CARD domain containing |
| 3.3 | 212625_at | 8677 | STX10 | syntaxin 10 |
| 3.3 | 235678_at | 2760 | GM2A | GM2 ganglioside activator |
| 3.3 | 221581_s_at | 7462 | WBSCR5 | Williams-Beuren syndrome chromosome |
| 3.3 | 244313_at | --- | --- | Transcribed locus |
| 3.3 | 221492_s_at | 64422 | APG3L | APG3 autophagy 3-like (S. cerevisiae) |
| 3.3 | 209615_s_at | 5058 | PAK1 | p21/Cdc42/Rac1-activated kinase 1 (STE20 homolog, yeast) |
| 3.3 | 206130_s_at | 433 | ASGR2 | asialoglycoprotein receptor 2 |
| 3.3 | 205158_at | 6038 | RNASE4 | ribonuclease, RNase A family, 4 |
| 3.3 | 211742_s_at | 2124 | EVI2B | ecotropic viral integration site 2B /// ecotropic viral integration site 2B |
| 3.3 | 231736_x_at | 4257 | MGST1 | microsomal glutathione S-transferase 1 |
| 3.3 | 201887_at | 3597 | IL13RA1 | interleukin 13 receptor, alpha 1 |
| 3.3 | 211571_s_at | 1462 | CSPG2 | chondroitin sulfate proteoglycan 2 (versican) |
| 3.3 | 225372_at | 64115 | PP2135 | chromosome 10 open reading frame 54 |
| 3.3 | 228285_at | 122402 | TDRD9 | tudor domain containing 9 |
| 3.3 | 214665_s_at | 11261 | CHP | calcium binding protein P22 |
| 3.3 | 213733_at | 4542 | MYO1F | myosin IF |
| 3.3 | 223158_s_at | 10783 | NEK6 | NIMA (never in mitosis gene a)-related kinase 6 |
| 3.3 | 207809_s_at | 537 | ATP6AP1 | ATPase, H+ transporting, lysosomal accessory protein 1 |
| 3.3 | 227131_at | 4215 | MAP3K3 | mitogen-activated protein kinase kinase kinase 3 |
| 3.3 | 218072_at | 29099 | COMMD9 | COMM domain containing 9 |
| 3.3 | 203042_at | 3920 | LAMP2 | lysosomal-associated membrane protein 2 |
| 3.3 | 212807_s_at | 6272 | SORT1 | sortilin 1 |
| 3.3 | 210423_s_at | 6556 | SLC11A1 | solute carrier family 11 (proton-coupled divalent metal ion transporters), member 1 |
| 3.3 | 225059_at | 57085 | AGTRAP | angiotensin II receptor-associated protein |
| 3.3 | 50221_at | 7942 | TFEB | transcription factor EB |
| 3.3 | 201888_s_at | 3597 | IL13RA1 | interleukin 13 receptor, alpha 1 |
| 3.3 | 231029_at | --- | --- | Transcribed locus |
| 3.3 | 238765_at | 9550 | ATP6V1G1 | ATPase, H+ transporting, lysosomal 13kDa, V1 subunit G isoform 1 |
| 3.3 | 234978_at | 120103 | SLC36A4 | solute carrier family 36 (proton/amino acid symporter), member 4 |
| 3.3 | 201312_s_at | 6451 | SH3BGRL | SH3 domain binding glutamic acid-rich protein like |
| 3.3 | 202901_x_at | 1520 | CTSS | cathepsin S |
| 3.3 | 202449_s_at | 6256 | RXRA | retinoid X receptor, alpha |
| 3.3 | 219947_at | 50856 | CLEC4A | C-type lectin domain family 4, member A |
| 3.3 | 226817_at | 1824 | DSC2 | desmocollin 2 |
| 3.3 | 235696_at | --- | --- | Homo sapiens, clone IMAGE:4837650, mRNA, partial cds |
| 3.3 | 241370_at | 286052 | LOC286052 | hypothetical protein LOC286052 |
| 3.3 | 212268_at | 1992 | SERPINB1 | serine (or cysteine) proteinase inhibitor, clade B (ovalbumin), member 1 |
| 3.3 | 200999_s_at | 10970 | CKAP4 | cytoskeleton-associated protein 4 |
| 3.3 | 211275_s_at | 2992 | GYG | glycogenin |
| 3.3 | 205863_at | 6283 | S100A12 | S100 calcium binding protein A12 (calgranulin C) /// S100 calcium binding protein A12 (calgranulin C) |
| 3.3 | 202671_s_at | 8566 | PDXK | pyridoxal (pyridoxine, vitamin B6) kinase |
| 3.3 | 210386_s_at | 4580 | MTX1 | metaxin 1 |
| 3.3 | 209238_at | 6809 | STX3A | syntaxin 3A |
| 3.3 | 221156_x_at | 9236 | CCPG1 | cell cycle progression 1 |
| 3.3 | 221523_s_at | 58528 | RRAGD | Ras-related GTP binding D |
| 3.3 | 201971_s_at | 523 | ATP6V1A | ATPase, H+ transporting, lysosomal 70kDa, V1 subunit A |
| 3.3 | 212043_at | 10618 | TGOLN2 | trans-golgi network protein 2 |
| 3.3 | 202371_at | 79921 | TCEAL4 | transcription elongation factor A (SII)-like 4 |
| 3.3 | 210427_x_at | 302 | ANXA2 | annexin A2 |
| 3.3 | 235174_s_at | --- | --- | CDNA clone IMAGE:5286843, partial cds |
| 3.3 | 226364_at | 3092 | HIP1 | Huntingtin interacting protein 1 |
| 3.3 | 213817_at | --- | --- | CDNA FLJ13601 fis, clone PLACE1010069 |
| 3.3 | 235798_at | --- | --- | Transcribed locus, weakly similar to XP_517655.1 similar to KIAA0825 protein [Pan troglodytes] |
| 3.3 | 234985_at | 143458 | LOC143458 | --- |
| 3.3 | 201007_at | 3032 | HADHB | hydroxyacyl-Coenzyme A dehydrogenase/3-ketoacyl-Coenzyme A thiolase/enoyl-Coenzyme A hydratase (trifunctional protein), beta subunit |
| 3.3 | 227776_at | --- | --- | Transcribed locus, weakly similar to NP_775735.1 1(3)mbt-like 4 (Drosophila) [Homo sapiens] |
| 3.3 | 214151_s_at | 9488 | PIGB | phosphatidylinositol glycan, class B |
| 3.3 | 244650_at | --- | --- | CDNA FLJ43660 fis, clone SYNOV4004823 |
| 3.3 | 225603_s_at | 286144 | LOC286144 | Hypothetical protein LOC286144 |
| 3.3 | 238778_at | 143098 | MPP7 | membrane protein, palmitoylated 7 (MAGUK p55 subfamily member 7) |
| 3.3 | 221864_at | 93129 | MGC13024 | hypothetical protein MGC13024 |
| 3.3 | 230322_at | 150372 | NFAM1 | NFAT activating protein with ITAM motif 1 |
| 3.3 | 208270_s_at | 6051 | RNPEP | arginyl aminopeptidase (aminopeptidase B) |
| 3.3 | 202888_s_at | 290 | ANPEP | alanyl (membrane) aminopeptidase (aminopeptidase N, aminopeptidase M, microsomal aminopeptidase, CD13, p150) |
| 3.3 | 203184_at | 2201 | FBN2 | fibrillin 2 (congenital contractural arachnodactyly) |
| 3.3 | 208736_at | 10094 | ARPC3 | actin related protein 2/3 complex, subunit 3, 21kDa |
| 3.3 | 223168_at | 58480 | RHOU | ras homolog gene family, member U |
| 3.3 | 201350_at | 2319 | FLOT2 | flotillin 2 |
| 3.3 | 217868_s_at | 51108 | DREV1 | DORA reverse strand protein 1 |
| 3.3 | 208074_s_at | 1175 | AP2S1 | adaptor-related protein complex 2, sigma 1 subunit |
| 3.3 2 | 218364_at | 9209 | LRRFIP2 | leucine rich repeat (in FLII) interacting protein |
| 3.3 | 211047_x_at | --- | --- | --- |
| 3.3 | 218109_s_at | 64747 | MFSD1 | major facilitator superfamily domain containing 1 |
| 3.3 | 200602_at | 351 | APP | amyloid beta (A4) precursor protein (protease nexin-II, Alzheimer disease) |
| 3.3 | 222687_s_at | 55331 | PHCA | phytoceramidase, alkaline |
| 3.3 | 209154_at | 30851 | TAX1BP3 | Tax1 (human T-cell leukemia virus type I) binding protein 3 |
| 3.3 | 200736_s_at | 2876 | GPX1 | glutathione peroxidase 1 |
| 3.3 | 212882_at | 23276 | KLHL18 | kelch-like 18 (Drosophila) |
| 3.3 | 228083_at | 93589 | CACNA2D4 | calcium channel, voltage-dependent, alpha 2/delta subunit 4 |
| 3.3 | 200821_at | 3920 | LAMP2 | lysosomal-associated membrane protein 2 |
| 3.3 | 209575_at | 3588 | IL10RB | Interleukin 10 receptor, beta |
| 3.3 | 204500_s_at | 23287 | AGTPBP1 | ATP/GTP binding protein 1 |
| 3.3 | 222833_at | 54947 | FLJ20481 | hypothetical protein FLJ20481 |
| 3.3 | 225658_at | 339745 | LOC339745 | hypothetical protein LOC339745 |
| 3.3 | 225272_at | 112483 | SAT2 | spermidine/spermine Nl-acetyltransferase 2 |
| 3.3 | 217743_s_at | 55754 | TMEM30A | transmembrane protein 30A |
| 3.3 | 204361_s_at | 8935 | SCAP2 | src family associated phosphoprotein 2 |
| 3.3 | 201972_at | 523 | ATP6V1A | ATPase, H+ transporting, lysosomal 70kDa, V1 subunit A |
| 3.3 | 221498_at | 81609 | SNX27 | sorting nexin family member 27 |
| 3.3 | 209004_s_at | 26234 | FBXL5 | F-box and leucine-rich repeat protein 5 |
| 3.3 | 218404_at | 29887 | SNX10 | sorting nexin 10 |
| 3.3 | 214152_at | 9488 | PIGB | phosphatidylinositol glycan, class B |
| 3.3 | 202545_at | 5580 | PRKCD | protein kinase C, delta |
| 3.3 | 235072_s_at | --- | --- | Transcribed locus |
| 3.3 | 228365_at | 144402 | CPNE8 | copine VIII |
| 3.3 | 212830_at | 1955 | EGFL5 | EGF-like-domain, multiple 5 |
| 3.3 | 214629_x_at | 57142 | RTN4 | reticulon 4 |
| 3.3 | 204362_at | 8935 | SCAP2 | src family associated phosphoprotein 2 |
| 3.3 | 230435_at | 375190 | FLJ30851 | FLJ30851 protein |
| 3.3 | 201238_s_at | 830 | CAPZA2 | capping protein (actin filament) muscle Z-line, alpha 2 |
| 3.3 | 211004_s_at | 221 | ALDH3B1 | aldehyde dehydrogenase 3 family, member B1 |
| 3.3 | 203789_s_at | 10512 | SEMA3C | sema domain, immunoglobulin domain (Ig), short basic domain, secreted, (semaphorin) 3C |
| 3.3 | 218831_s_at | 2217 | FCGRT | Fc fragment of IgG, receptor, transporter, alpha |
| 3.3 | 239089_at | --- | --- | Transcribed locus |
| 3.3 | 212335_at | 2799 | GNS | glucosamine (N-acetyl)-6-sulfatase (Sanfilippo disease IIID) |
| 3.3 | 224918_x_at | 4257 | MGST1 | microsomal glutathione S-transferase 1 |
| 3.3 | 200785_s_at | 4035 | LRP1 | low density lipoprotein-related protein 1 (alpha-2-macroglobulin receptor) |
| 3.3 | 222447_at | 51108 | DREV1 | DORA reverse strand protein 1 |
| 3.3 | 225001_at | 9545 | RAB3D | RAB3D, member RAS oncogene family |
| 3.3 | 234312_s_at | 55902 | ACAS2 | acetyl-Coenzyme A synthetase 2 (ADP |
| 3.3 | 215749_s_at | 64689 | GORASP1 | golgi reassembly stacking protein 1, 65kDa |
| 3.3 | 236553_at | --- | --- | Transcribed locus |
| 3.3 | 219892_at | 53346 | TM6SF1 | transmembrane 6 superfamily member 1 |
| 3.3 | 205931_s_at | 9586 | CREB5 | cAMP responsive element binding protein 5 |
| 3.3 | 242794_at | 55534 | MAML3 | Mastermind-like 3 (Drosophila) |
| 3.3 | 223120_at | 2519 | FUCA2 | fucosidase, alpha-L- 2, plasma |
| 3.3 | 214500_at | 9555 | H2AFY | H2A histone family, member Y |
| 3.3 | 205639_at | 313 | AOAH | acyloxyacyl hydrolase (neutrophil) |
| 3.3 | 210968_s_at | 57142 | RTN4 | reticulon 4 |
| 3.3 | 207545_s_at | 8650 | NUMB | numb homolog (Drosophila) |
| 3.3 | 203185_at | 9770 | RASSF2 | Ras association (RalGDS/AF-6) domain family 2 |
| 3.3 | 218035_s_at | 54502 | FLJ20273 | RNA-binding protein |
| 3.3 | 208454_s_at | 10404 | PGCP | plasma glutamate carboxypeptidase |
| 3.3 | 205844_at | 8876 | VNN1 | vanin 1 /// vanin 1 |
| 3.3 | 202788_at | 7867 | MAPKAPK3 | mitogen-activated protein kinase-activated protein kinase 3 |
| 3.3 | 224796_at | 50807 | DDEF1 | development and differentiation enhancing factor 1 |
| 3.3 | 204168_at | 4258 | MGST2 | microsomal glutathione S-transferase 2 |
| 3.3 | 210609_s_at | 9540 | TP53I3 | tumor protein p53 inducible protein 3 |
| 3.3 | 216338_s_at | 25844 | C6orf109 | chromosome 6 open reading frame 109 |
| 3.3 | 222437_s_at | 51652 | VPS24 | vacuolar protein sorting 24 (yeast) |
| 3.3 | 238996_x_at | 226 | ALDOA | aldolase A, fructose-bisphosphate |
| 3.3 | 217995_at | 58472 | SQRDL | sulfide quinone reductase_like (yeast) |
| 3.3 | 204034_at | 23474 | ETHE1 | ethylmalonic encephalopathy 1 |
| 3.3 | 201470_at | 9446 | GSTO1 | glutathione S-transferase omega 1 |
| 3.3 | 213011_s_at | 7167 | TPI1 | triosephosphate isomerase 1 |
| 3.3 | 201379_s_at | 7165 | TPD52L2 | tumor protein D52-like 2 |
| 3.3 | 203175_at | 391 | RHOG | ras homolog gene family, member G (rho G) |
| 3.3 | 217947_at | 54918 | CKLFSF6 | chemokine-like factor super family 6 |
| 3.3 | 200941_at | 3281 | HSBP1 | heat shock factor binding protein 1 |
| 3.3 | 218945_at | 79091 | MGC2654 | hypothetical protein MGC2654 |
| 3.3 | 203665_at | 3162 | HMOX1 | heme oxygenase (decycling) 1 |
| 3.3 | 204301_at | 9920 | KIAA0711 | KIAA0711 gene product |
| 3.3 | 227069_at | --- | --- | FP6778 |
| 3.3 | 204445_s_at | 240 | ALOX5 | arachidonate 5-lipoxygenase |
| 3.3 | 226707_at | 93100 | PP3856 | similar to CG3714 gene product |
| 3.3 | 227379_at | 154141 | OACT1 | O-acyltransferase (membrane bound) domain containing 1 |
| 3.3 | 211178_s_at | 9051 | PSTPIP1 | proline-serine-threonine phosphatase interacting protein 1 |
| 3.3 | 204526_s_at | 11138 | TBC1D8 | TBC1 domain family, member 8 (with GRAM domain) |
| 3.3 | 226885_at | --- | --- | Transcribed locus, moderately similar to XP_525783.1 similar to KIAA1155 protein [Pan troglodytes] |
| 3.3 | 217910_x_at | 6945 | MLX | MAX-like protein X |
| 3.3 | 218950_at | 64411 | CENTD3 | centaurin, delta 3 |
| 3.3 | 220371_s_at | 56996 | SLC12A9 | solute carrier family 12 (potassium/chloride transporters), member 9 |
| 3.3 | 224912_at | 57217 | TTC7A | tetratricopeptide repeat domain 7A |
| 3.3 | 228120_at | 26523 | EIF2C1 | Eukaryotic translation initiation factor 2C, 1 |
| 3.3 | 217837_s_at | 51652 | VPS24 | vacuolar protein sorting 24 (yeast) |
| 3.3 | 212112_s_at | 23673 | STX12 | syntaxin 12 |
| 3.3 | 202377_at | 3953 /// 54741 | LEPR /// LEPROT | leptin receptor /// leptin receptor overlapping transcript |
| 3.3 | 212606_at | 23001 | WDFY3 | WD repeat and FYVE domain containing 3 |
| 3.3 | 226849_at | --- | --- | KIAA1608 |
| 3.3 | 204158_s_at | 10312 | TCIRG1 | T-cell, immune regulator 1, ATPase, H+ transporting, lysosomal V0 protein a isoform 3 |
| 3.3 | 223000_s_at | 50848 | F11R | F11 receptor |
| 3.3 | 204249_s_at | 4005 | LMO2 | LIM domain only 2 (rhombotin-like 1) |
| 3.3 | 212252_at | 10645 | CAMKK2 | calcium/calmodulin-dependent protein kinase kinase 2, beta |
| 3.3 | 219256_s_at | 54436 | SH3TC1 | SH3 domain and tetratricopeptide repeats 1 |
| 3.3 | 226212_s_at | 3643 | INSR | Insulin receptor |
| 3.3 | 208926_at | 4758 | NEU1 | sialidase 1 (lysosomal sialidase) |
| 3.3 | 200808_s_at | 7791 | ZYX | zyxin |
| 3.3 | 215706_x_at | 7791 | ZYX | zyxin |
| 3.3 | 218606_at | 55625 | ZDHHC7 | zinc finger, DHHC domain containing 7 |
| 3.3 | 203126_at | 3613 | IMPA2 | inositol(myo)-1(or 4)-monophosphatase 2 |
| 3.3 | 226274_at | 158563 | LOC158563 | hypothetical protein LOC158563 |
| 3.3 | 204336_s_at | 10287 | RGS19 | regulator of G-protein signalling 19 |
| 3.3 | 226607_at | 25943 | C20orf194 | chromosome 20 open reading frame 194 |
| 3.3 | 206295_at | 3606 | IL18 | interleukin 18 (interferon-gamma-inducing factor) |
| 3.3 | 239084_at | 9342 | SNAP29 | Synaptosomal-associated protein, 29kDa |
| 3.3 | 225350_s_at | 79699 | FLJ13456 | hypothetical protein FLJ13456 |
| 3.3 | 219161_s_at | 51192 | CKLF | chemokine-like factor |
| 3.3 | 209393_s_at | 9470 | EIF4E2 | eukaryotic translation initiation factor 4E member 2 |
| 3.3 | 201676_x_at | 5682 | PSMA1 | proteasome (prosome, macropain) subunit, alpha type, 1 |
| 3.3 | 211746_x_at | 5682 | PSMA1 | proteasome (prosome, macropain) subunit, alpha type, 1 /// proteasome (prosome, macropain) subunit, alpha type, 1 |
| 3.3 | 218189_s_at | 54187 | NANS | N-acetylneuraminic acid synthase (sialic acid synthase) |
| 3.3 | 202626_s_at | 4067 | LYN | v-yes-1 Yamaguchi sarcoma viral related oncogene homolog /// v-yes-1 Yamaguchi sarcoma viral related oncogene homolog |
| 3.3 | 206100_at | 1368 | CPM | carboxypeptidase M |
| 3.3 | 225899_x_at | 388312 /// 399844 /// 440470 | LOC388312 /// FLJ45445 /// LOC440470 | hypothetical protein LOC284701 /// hypothetical gene supported by AK128780 /// FLJ45445 protein /// hypothetical gene supported by AK093729; BX647918 /// similar to hypothetical protein LOC349114 /// gene supported by AK097080; |
| 3.3 | 222143_s_at | 64419 | FLJ22405 | hypothetical protein FLJ22405 |
| 3.3 | 220034_at | 11213 | IRAK3 | interleukin-1 receptor-associated kinase 3 |
| 3.3 | 218387_s_at | 25796 | PGLS | 6-phosphogluconolactonase |
| 3.3 | 221059_s_at | 23406 | COTL1 | coactosin-like 1 (Dictyostelium) |
| 3.3 | 224647_at | --- | --- | --- |
| 3.3 | 212636_at | 9444 | QKI | quaking homolog, KH domain RNA binding (mouse) |
| 3.3 | 214085_x_at | --- | --- | GLI pathogenesis-related 1 (glioma) |
| 3.3 | 203139_at | 1612 | DAPK1 | death-associated protein kinase 1 |
| 3.3 | 210980_s_at | 427 | ASAH1 | N-acylsphingosine amidohydrolase (acid ceramidase) 1 |
| 3.3 | 219165_at | 64236 | PDLIM2 | PDZ and LIM domain 2 (mystique) |
| 3.3 | 218998_at | 54942 | FLJ20457 | hypothetical protein FLJ20457 |
| 3.3 | 230100_x_at | 5058 | PAK1 | p21/Cdc42/Rac1-activated kinase 1 (STE20 homolog, yeast) |
| 3.3 | 215646_s_at | 1462 | CSPG2 | chondroitin sulfate proteoglycan 2 (versican) /// chondroitin sulfate proteoglycan 2 (versican) |
| 3.3 | 201380_at | 10491 | CRTAP | cartilage associated protein |
| 3.3 | 228949_at | 79971 | FLJ23091 | putative NFkB activating protein 373 |
| 3.3 | 201311_s_at | 6451 | SH3BGRL | SH3 domain binding glutamic acid-rich protein like |
| 3.3 | 206488_s_at | 948 | CD36 | CD36 antigen (collagen type I receptor, thrombospondin receptor) |
| 3.3 | 202787_s_at | 7867 | MAPKAPK3 | mitogen-activated protein kinase-activated protein kinase 3 |
| 3.3 | 214765_s_at | 27163 | ASAHL | N-acylsphingosine amidohydrolase (acid ceramidase)-like |
| 3.3 | 226656_at | 10491 | CRTAP | cartilage associated protein |
| 3.3 | 219496_at | 65124 | C2orf26 | chromosome 2 open reading frame 26 |
| 3.3 | 218388_at | 25796 | PGLS | 6-phosphogluconolactonase |
| 3.3 | 221188_s_at | 27141 | CIDEB | cell death-inducing DFFA-like effector b |
| 3.3 | 202185_at | 8985 | PLOD3 | procollagen-lysine, 2-oxoglutarate 5-dioxygenase 3 |
| 3.3 | 218017_s_at | 138050 | FLJ32731 | hypothetical protein FLJ32731 |
| 3.3 | 229635_at | --- | --- | CDNA clone IMAGE:4800262, partial cds |
| 3.4 | 211967_at | 114908 | PORIMIN | pro-oncosis receptor inducing membrane injury gene |
| 3.4 | 212006_at | 23190 | UBXD2 | UBX domain containing 2 |
| 3.4 | 200686_s_at | 9295 | SFRS11 | splicing factor, arginine/serine-rich 11 |
| 3.4 | 202396_at | 10915 | TCERG1 | transcription elongation regulator 1 |
| 3.4 | 200685_at | 9295 | SFRS11 | splicing factor, arginine/serine-rich 11 |
| 3.4 | 202853_s_at | 6259 | RYK | RYK receptor-like tyrosine kinase |
| 3.4 | 218386_x_at | 10600 | USP16 | ubiquitin specific protease 16 |
| 3.4 | 209704_at | 22823 | M96 | Likely ortholog of mouse metal response element binding transcription factor 2 |
| 3.4 | 214700_x_at | 55183 | RIF1 | RAP1 interacting factor homolog (yeast) |
| 3.4 | 213704_at | 5876 | RABGGTB | Rab geranylgeranyltransferase, beta subunit |
| 3.4 | 209654_at | 23379 | KIAA0947 | KIAA0947 protein |
| 3.4 | 212450_at | 9728 | KIAA0256 | KIAA0256 gene product |
| 3.4 | 205596_s_at | 64750 | SMURF2 | SMAD specific E3 ubiquitin protein ligase 2 |
| 3.4 | 202693_s_at | 9263 | STK17A | serine/threonine kinase 17a (apoptosis-inducing) |
| 3.4 | 212601_at | 23140 | ZZEF1 | zinc finger, ZZ type with EF hand domain 1 |
| 3.4 | 212779_at | 84162 | KIAA1109 | KIAA1109 |
| 3.4 | 218053_at | 55660 | FNBP3 | formin binding protein 3 |
| 3.4 | 211946_s_at | 23215 | BAT2D1 | BAT2 domain containing 1 |
| 3.4 | 212721_at | 140890 | SFRS12 | splicing factor, arginine/serine-rich 12 |
| 3.4 | 212569_at | 23347 | KIAA0650 | structural maintenance of chromosomes flexible hinge domain containing 1 |
| 3.4 | 207483_s_at | 55832 | TIP120A | TBP-interacting protein |
| 3.4 | 212060_at | 23350 | SR140 | U2-associated SR140 protein |
| 3.4 | 221493_at | 7259 | TSPYL1 | TSPY-like 1 |
| 3.4 | 212231_at | 23014 | FBXO21 | F-box protein 21 |
| 3.4 | 213212_x_at | 161527 | LOC161527 | hypothetical protein LOC161527 |
| 3.4 | 206095_s_at | 10772 | FUSIP1 | FUS interacting protein (serine-arginine rich) 1 |
| 3.4 | 214709_s_at | 10130 /// 3895 | KTN1 /// TXNDC7 | kinectin 1 (kinesin receptor) /// protein isomerase-associated 6 disulfide |
| 3.4 | 201486_at | 5955 | RCN2 | reticulocalbin 2, EF-hand calcium binding domain |
| 3.4 | 217466_x_at | 6187 | RPS2 | ribosomal protein S2 |
| 3.4 | 209187_at | 1810 | DR1 | down-regulator of transcription 1, TBP-binding (negative cofactor 2) |
| 3.4 | 221751_at | 79646 | PANK3 | Pantothenate kinase 3 |
| 3.4 | 203347_s_at | 22823 | M96 | likely ortholog of mouse metal response element binding transcription factor 2 |
| 3.4 | 212058_at | 23350 | SR140 | U2-associated SR140 protein |
| 3.4 | 209214_s_at | 2130 | EWSR1 | Ewing sarcoma breakpoint region 1 |
| 3.4 | 215038_s_at | 29072 | HYPB | huntingtin interacting protein B |
| 3.4 | 211285_s_at | 7337 | UBE3A | ubiquitin protein ligase E3A (human papilloma virus E6-associated protein, Angelman syndrome) |
| 3.4 | 218306_s_at | 8925 | HERC1 | hect (homologous to the E6-AP (UBE3A) carboxyl terminus) domain and RCC1 (CHC1)-like domain (RLD) 1 |
| 3.4 | 218528_s_at | 152006 | RNF38 | ring finger protein 38 |
| 3.4 | 213000_at | 23515 | ZCWCC3 | zinc finger, CW type with coiled-coil domain 3 |
| 3.4 | 218098_at | 10564 | ARFGEF2 | ADP-ribosylation factor guanine nucleotide-exchange factor 2 (brefeldin A-inhibited) |
| 3.4 | 213106_at | 10396 | ATP8A1 | ATPase, aminophospholipid transporter (APLT), Class I, type 8A, member 1 |
| 3.4 | 208127_s_at | 9655 | SOCS5 | suppressor of cytokine signaling 5 |
| 3.4 | 220099_s_at | 51631 | LUC7L2 | LUC7-like 2 (S. cerevisiae) |
| 3.4 | 202034_x_at | 9821 | RB1CC1 | RB1-inducible coiled-coil 1 |
| 3.4 | 204009_s_at | 3845 | KRAS | v-Ki-ras2 Kirsten rat sarcoma viral oncogene homolog |
| 3.4 | 209674_at | 1407 | CRY1 | cryptochrome 1 (photolyase-like) |
| 3.4 | 202097_at | 9972 | NUP153 | nucleoporin 153kDa |
| 3.4 | 222204_s_at | 54700 | RRN3 | RRN3 RNA polymerase I transcription factor homolog (yeast) |
| 3.4 | 201713_s_at | 5903 | RANBP2 | RAN binding protein 2 |
| 3.4 | 203480_s_at | 54726 | HSHIN1 | HIV-1 induced protein HIN-1 |
| 3.4 | 205288_at | 8556 | CDC14A | CDC14 cell division cycle 14 homolog A (S. cerevisiae) |
| 3.4 | 204512_at | 3096 | HIVEP1 | human immunodeficiency virus type I enhancer binding protein 1 |
| 3.4 | 200639_s_at | 7534 | YWHAZ | tyrosine 3-monooxygenase/tryptophan 5-monooxygenase activation protein, zeta polypeptide |
| 3.4 | 222119_s_at | 80204 | FBXO11 | F-box protein 11 |
| 3.4 | 209027_s_at | 10006 | ABI1 | abl-interactor 1 |
| 3.4 | 201304_at | 4698 | NDUFA5 | NADH dehydrogenase (ubiquinone) 1 alpha subcomplex, 5, 13kDa |
| 3.4 | 209085_x_at | 5981 | RFC1 | replication factor C (activator 1) 1, 145kDa |
| 3.4 | 209157_at | 10294 | DNAJA2 | DnaJ (Hsp40) homolog, subfamily A, member |
| 3.4 | 212239_at | 5295 | PIK3R1 | phosphoinositide-3-kinase, regulatory subunit 1 (p85 alpha) |
| 3.4 | 213225_at | 5495 | PPM1B | protein phosphatase 1B (formerly 2C), magnesium-dependent, beta isoform |
| 3.4 | 201260_s_at | 6856 | SYPL | synaptophysin-like protein |
| 3.4 | 202259_s_at | 10443 | PFAAP5 | phosphonoformate immuno-associated protein 5 |
| 3.4 | 206695_x_at | 7594 | ZNF43 | zinc finger protein 43 (HTF6) |
| 3.4 | 204809_at | 10845 | CLPX | ClpX caseinolytic protease X homolog (E. coli) |
| 3.4 | 209095_at | 1738 | DLD | dihydrolipoamide dehydrogenase (E3 component of pyruvate dehydrogenase complex, 2-oxo-glutarate complex, branched chain keto acid dehydrogenase complex) |
| 3.4 | 218239_s_at | 23560 | GTPBP4 | GTP binding protein 4 |
| 3.4 | 200626_s_at | 9782 | MATR3 | matrin 3 |
| 3.4 | 202160_at | 1387 | CREBBP | CREB binding protei (Rubinsteln-Taybi syndrome) |
| 3.4 | 203078_at | 8453 | CUL2 | cullin 2 |
| 3.4 | 212781_at | 5930 | RBBP6 | retinoblastoma binding protein 6 |
| 3.4 | 202541_at | 9255 | SCYE1 | small inducible cytokine subfamily E, member 1 (endothelial monocyte-activating) |
| 3.4 | 208828_at | 54107 | POLE3 | polymerase (DNA directed), epsilon 3 (p17 subunit) |
| 3.4 | 208051_s_at | 10605 | PAIP1 | poly(A) binding protein interacting protein 1 |
| 3.4 | 212928_at | 23270 | TSPYL4 | TSPY-like 4 |
| 3.4 | 203250_at | 22828 | RBM16 | RNA binding motif protein 16 |
| 3.4 | 221020_s_at | 81034 | MFTC | mitochondrial folate transporter/carrier /// mitochondrial folate transporter/carrier |
| 3.4 | 220018_at | 79872 | CBLL1 | Cas-Br-M (murine) ecotropic retroviral transforming sequence-like 1 |
| 3.4 | 202798_at | 10427 | SEC24B | SEC24 related gene family, member B (S. cerevisiae) |
| 3.4 | 221381_s_at | 10933 /// 10934 | MORF4L1 /// | mortality factor 4 like 1 /// mortality factor 4 |
| 3.4 | 212265_at | 9444 | QKI | quaking homolog, KH domain RNA binding (mouse) |
| 3.4 | 213918_s_at | 25836 | NIPBL | Nipped-B homolog (Drosophila) |
| 3.4 | 217851_s_at | 51012 | C20orf45 | chromosome 20 open reading frame 45 |
| 3.4 | 202171_at | 7716 | ZNF161 | zinc finger protein 161 |
| 3.4 | 203898_at | 27297 | RCP9 | calcitonin gene-related peptide-receptor component protein |
| 3.4 | 218172_s_at | 79139 | DERL1 | Der1-like domain family, member 1 |
| 3.4 | 209112_at | 1027 | CDKN1B | cyclin-dependent kinase inhibitor 1B (p27, Klp1) |
| 3.4 | 217816_s_at | 57092 | PCNP | PEST-containing nuclear protein |
| 3.4 | 201769_at | 9685 | ENTH | enthoprotin |
| 3.4 | 218878_s_at | 23411 | SIRT1 | sirtuin (silent mating type information regulation 2 homolog) 1 (S. cerevisiae) |
| 3.4 | 203378_at | 51585 | PCF11 | pre-mRNA cleavage complex II protein Pcfll |
| 3.4 | 204847_at | 27107 | ZBTB11 | zinc finger and BTB domain containing 11 |
| 3.4 | 203303_at | 6990 | TCTE1L | t-complex-associated-testis-expressed 1-like |
| 3.4 | 219029_at | 64417 | FLJ21657 | hypothetical protein FLJ21657 |
| 3.4 | 202265_at | 648 | PCGF4 | polycomb group ring finger 4 |
| 3.4 | 205251_at | 8864 | PER2 | period homolog 2 (Drosophila) |
| 3.4 | 212615_at | 80205 | CHD9 | chromodomain helicase DNA binding protein 9 |
| 3.4 | 221230_s_at | 51742 | ARID4B | AT rich interactive domain 4B (RBP1- like) /// AT rich interactive domain 4B (RBP1- like) |
| 3.4 | 203403_s_at | 6049 | RNF6 | ring finger protein (C3H2C3 type) 6 |
| 3.4 | 205664_at | 22944 | KIN | KIN, antigenic determinant of recA protein homolog (mouse) |
| 3.4 | 212579_at | 23347 | KIAA0650 | structural maintenance of chromosomes flexible hinge domain containing 1 |
| 3.4 | 203301_s_at | 9988 | DMTF1 | cyclin D binding myb-like transcription factor 1 |
| 3.4 | 209028_s_at | 10006 | ABI1 | abl-interactor 1 |
| 3.4 | 209666_s_at | 1147 | CHUK | conserved helix-loop-helix ubiquitous kinase |
| 3.4 | 212787_at | 56252 | YLPM1 | YLP motif containing 1 |
| 3.4 | 217833_at | 10492 | SYNCRIP | CDNA FLJ31626 fis, clone NT2RI2003317 |
| 3.4 | 203624_at | 8227 | DXYS155E | DNA segment on chromosome X and Y (unique) 155 expressed sequence |
| 3.4 | 218082_s_at | 7342 | UBP1 | upstream binding protein 1 (LBP-1a) |
| 3.4 | 217828_at | 79811 | FLJ13213 | modulator of estrogen induced transcription |
| 3.4 | 214855_s_at | 253959 | GARNL1 | GTPase activating Rap/RanGAP domain-like 1 |
| 3.4 | 220553_s_at | 55015 | PRPF39 | PRP39 pre-mRNA processing factor 39 homolog (yeast) |
| 3.4 | 212847_at | 8880 | FUBP1 | Nexilin (F actin binding protein) |
| 3.4 | 213025_at | 55623 | THUMPD1 | THUMP domain containing 1 |
| 3.4 | 218096_at | 55326 | AGPAT5 | 1-acylglycerol-3-phosphate O-acyltransferase 5 (lysophosphatidic acid acyltransferase, epsilon) |
| 3.4 | 216944_s_at | 3708 | ITPR1 | inositol 1,4,5-triphosphate receptor, type 1 |
| 3.4 | 219231_at | 96764 | NCOA6IP | nuclear receptor coactivator 6 interacting protein |
| 3.4 | 202832_at | 9648 | GCC2 | GRIP and coiled-coil domain containing 2 |
| 3.4 | 202550_s_at | 9217 | VAPB | VAMP (vesicle-associated membrane protein)-associated protein B and C |
| 3.4 | 201437_s_at | 1977 | EIF4E | eukaryotic translation initiation factor 4E |
| 3.4 | 200608_s_at | 5885 | RAD21 | RAD21 homolog (S. pombe) |
| 3.4 | 218352_at | 55213 | RCBTB1 | regulator of chromosome condensation (RCC1) and BTB (POZ) domain containing protein 1 |
| 3.4 | 212927_at | 23137 | SMC5L1 | SMC5 structural maintenance of chromosomes 5-like 1 (yeast) |
| 3.4 | 221007_s_at | 81608 | FIP1L1 | FIP1 like 1 (S. cerevisiae) /// FIP1 like 1 (S. cerevisiae) |
| 3.4 | 212438_at | 11017 | RY1 | putative nucleic acid binding protein RY-1 |
| 3.4 | 213128_s_at | 7337 | UBE3A | ubiquitin protein ligase E3A (human papilloma virus E6-associated protein, Angelman syndrome) |
| 3.4 | 202653_s_at | 64844 | 7-Mar | membrane-associated ring finger (C3HC4) 7 |
| 3.4 | 209330_s_at | 3184 | HNRPD | heterogeneous nuclear ribonucleoprotein D (AU-rich element RNA binding protein 1, 37kDa) |
| 3.4 | 215696_s_at | 9919 | KIAA0310 | KIAA0310 |
| 3.4 | 214678_x_at | 7543 | ZFX | zinc finger protein, X-linked |
| 3.4 | 213251_at | 8467 | SMARCA5 | Hypothetical LOC 441046 |
| 3.4 | 202660_at | 3709 | ITPR2 | Inositol 1,4,5-triphosphate receptor, type 2 |
| 3.4 | 212160_at | 11260 | XPOT | exportin, tRNA (nuclear export receptor for tRNAs) |
| 3.4 | 211698_at | 23741 | CRI1 | CREBBP/EP300 inhibitor 1 /// CREBBP/EP300 inhibitor 1 |
| 3.4 | 219031_s_at | --- | --- | comparative gene identification transcript 37 |
| 3.4 | 218236_s_at | 23683 | PRKD3 | protein kinase D3 |
| 3.4 | 214363_s_at | 9782 | MATR3 | matrin 3 |
| 3.4 | 212764_at | 6935 | TCF8 | --- |
| 3.4 | 212593_s_at | 27250 | PDCD4 | programmed cell death 4 (neoplastic transformation inhibitor) |
| 3.4 | 221918_at | 5128 | PCTK2 | PCTAIRE protein kinase 2 |
| 3.4 | 206015_s_at | 22887 | FOXJ3 | forkhead box J3 |
| 3.4 | 202880_s_at | 9267 | PSCD1 | pleckstrin homology, Sec7 and coiled-coil domains 1(cytohesin 1) |
| 3.4 | 209898_x_at | 50618 | ITSN2 | intersectin 2 |
| 3.4 | 201408_at | 5500 | PPP1CB | protein phosphatase 1, catalytic subunit, beta isoform |
| 3.4 | 209022_at | 10735 | STAG2 | stromal antigen 2 |
| 3.4 | 217819_at | 51125 | GOLGA7 | golgi autoantigen, golgin subfamily a, 7 |
| 3.4 | 204897_at | 5734 | PTGER4 | prostaglandin E receptor 4 (subtype EP4) |
| 3.4 | 212752_at | 23332 | CLASP1 | cytoplasmic linker associated protein 1 |
| 3.4 | 204651_at | 4899 | NRF1 | nuclear respiratory factor 1 |
| 3.4 | 218149_s_at | 55893 | ZNF395 | zinc finger protein 395 |
| 3.4 | 212582_at | 114882 | OSBPL8 | oxysterol binding protein-like 8 |
| 3.4 | 218381_s_at | 11338 | U2AF2 | U2 (RNU2) small nuclear RIVA auxiliary factor 2 |
| 3.4 | 212936_at | 83989 | DKFZP564D172 | hypothetical protein DKFZp564D172 |
| 3.4 | 244512_at | --- | --- | --- |
| 3.4 | 211763_s_at | 7320 | UBE2B | ubiquitin-conjugating enzyme E2B (RAD6 homolog) /// ubiquitin-conjugating enzyme E2B (RAD6 homolog) |
| 3.4 | 221502_at | 3839 | KPNA3 | karyopherin alpha 3 (importin alpha 4) |
| 3.4 | 218079_s_at | 79893 | ZNF403 | zinc finger protein 403 |
| 3.4 | 212774_at | 10472 | ZNF238 | zinc finger protein 238 |
| 3.4 | 212842_x_at | 84220 | RANBP2L1 | RAN binding protein 2-like 1 /// similar to Ran-binding protein 2 /// similar to RAN-binding protein 2-like 1 |
| 3.4 | 218256_s_at | 53371 | NUP54 | nucleoporin 54kDa |
| 3.4 | 204520_x_at | 23774 | BRD1 | bromodomain containing 1 |
| 3.4 | 212455_at | 91746 | YT521 | splicing factor YT521-B |
| 3.4 | 204742_s_at | 23047 | APRIN | androgen-induced proliferation inhibitor |
| 3.4 | 203429_s_at | 51430 | Clorf9 | chromosome 1 open reading frame 9 |
| 3.4 | 217987_at | 54529 | NS3TP1 | HCV NS3-transactivated protein 1 |
| 3.4 | 201567_s_at | 2803 | GOLGA4 | golgi autoantigen, golgin subfamily a, 4 |
| 3.4 | 202158_s_at | 10659 | CUGBP2 | CUG triplet repeat, RNA binding protein 2 |
| 3.4 | 202362_at | 5906 | RAP1A | RAP1A, member of RAS oncogene family |
| 3.4 | 218674_at | 80006 | FLJ13611 | hypothetical protein FLJ13611 |
| 3.4 | 209034_at | 10957 | PNRC1 | proline-rich nuclear receptor coactivator 1 |
| 3.4 | 202918_s_at | 25843 | PREI3 | preimplantation protein 3 |
| 3.4 | 203525_s_at | 324 | APC | adenomatosis polyposis coli |
| 3.4 | 212920_at | --- | --- | --- |
| 3.4 | 212267_at | 23063 | KIAA0261 | KIAA0261 |
| 3.4 | 203493_s_at | 9702 | PIG8 | translokin |
| 3.4 | 217142_at | --- | --- | --- |
| 3.4 | 214658_at | 51014 | TMED7 | transmembrane emp24 protein transport domain containing 7 |
| 3.4 | 222148_s_at | 55288 | RHOT1 | ras homolog gene family, member T1 |
| 3.4 | 217731_s_at | 9445 | ITM2B | integral membrane protein 2B |
| 3.4 | 205269_at | 3937 | LCP2 | lymphocyte cytosolic protein 2 (SH2 domain containing leukocyte protein of 76kDa) |
| 3.4 | 209451_at | 10010 | TANK | TRAF family member-associated NFKB |
| 3.4 | 218649_x_at | 9147 | SDCCAG1 | serologically defined colon cancer antigen 1 |
| 3.4 | 210283_x_at | 10605 /// 388345 | PAIP1 /// LOC388345 | poly(A) binding protein interacting protein 1 /// similar to poly(A) binding protein interacting protein 1 isoform 1; polyadenylate binding protein-interacting protein 1; PABC1-interacting protein 1 |
| 3.4 | 212200_at | 23141 | KIAA0692 | KIAA0692 protein |
| 3.4 | 218499_at | 51765 | MST4 | Mst3 and SOK1-related kinase |
| 3.4 | 211935_at | 23204 | ARL6IP | ADP-ribosylation factor-like 6 interacting protein |
| 3.4 | 213086_s_at | 1452 | CSNK1A1 | casein kinase 1, alpha 1 |
| 3.4 | 201699_at | 5706 | PSMC6 | proteasome (prosome, macropain) 26S subunit, ATPase, 6 |
| 3.4 | 218640_s_at | 79666 | PLEKHF2 | pleckstrin homology domain containing, family F (with FYVE domain) member 2 |
| 3.4 | 213372_at | 152559 | PAQR3 | progestin and adipoQ receptor family member III |
| 3.4 | 213360_s_at | 340318 /// 9883 | POM121 /// LOC340318 | POM121 membrane glycoprotein (rat) /// nuclear envelope pore membrane LOC340318 |
| 3.4 | 202673_at | 8813 | DPM1 | dolichyl-phosphate mannosyltransferase polypeptide 1, catalytic subunit |
| 3.4 | 208673_s_at | 6428 | SFRS3 | splicing factor, arginine/serine-rich 3 |
| 3.4 | 217822_at | 51729 | WBP11 | WW domain binding protein 11 |
| 3.4 | 218348_s_at | 29066 | ZC3HDC7 | zinc finger CCCH type domain containing 7 |
| 3.4 | 202623_at | 55837 | C14orf11 | chromosome 14 open reading frame 11 |
| 3.4 | 209064_x_at | 10605 | PAIP1 | poly(A) binding protein interacting protein 1 |
| 3.4 | 218669_at | 57826 | RAP2C | RAP2C, member of RAS oncogene family |
| 3.4 | 218696_at | 9451 | EIF2AK3 | eukaryotic translation initiation factor 2-alpha kinase 3 |
| 3.4 | 218668_s_at | 57826 | RAP2C | RAP2C, member of RAS oncogene family |
| 3.4 | 217812_at | 51441 | YTHDF2 | YTH domain family, member 2 |
| 3.4 | 215716_s_at | 490 | ATP2B1 | ATPase, Ca++ transporting, plasma |
| 3.4 | 201535_at | 5412 | UBL3 | ubiquitin-like 3 |
| 3.4 | 221596_s_at | 84060 | DKFZP56400523 | hypothetical protein DKFZp56400523 |
| 3.4 | 201409_s_at | 5500 | PPP1CB | protein phosphatase 1, catalytic subunit, beta isoform |
| 3.4 | 218247_s_at | 51320 | RKHD2 | ring finger and KH domain containing 2 |
| 3.4 | 218311_at | 8491 | MAP4K3 | mitogen-activated protein kinase kinase kinase kinase 3 |
| 3.4 | 214429_at | 9107 | MTMR6 | myotubularin related protein 6 |
| 3.4 | 203544_s_at | 8027 | STAM | signal transducing adaptor molecule (SH3 domain and ITAM motif) 1 |
| 3.4 | 212418_at | 1997 | ELF1 | E74-like factor 1 (ets domain transcription factor) |
| 3.4 | 204739_at | 1060 | CENPC1 | centromere protein C 1 |
| 3.4 | 208671_at | 57515 | TDE2 | tumor differentially expressed 2 |
| 3.4 | 206875_s_at | 9748 | SLK | STE20-like kinase (yeast) |
| 3.4 | 214467_at | 8477 | GPR65 | G protein-coupled receptor 65 |
| 3.4 | 203356_at | 23473 | CAPN7 | calpain 7 |
| 3.4 | 212990_at | 8867 | *S*YNJ1 | synaptojanin 1 |
| 3.4 | 213070_at | 5286 | PIK3C2A | Phosphoinositide-3-kinase, class 2, alpha polypeptide |
| 3.4 | 212366_at | 23036 | ZNF292 | zinc finger protein 292 |
| 3.4 | 221559_s_at | 79003 | MIS12 | MIS12 homolog (yeast) |
| 3.4 | 201603_at | 4659 | PPP1R12A | protein phosphatase 1, regulatory (inhibitor) subunit 12A |
| 3.4 | 208933_s_at | 3964 | LGALS8 | lectin, galactoside-binding, soluble, 8 (galectin 8) |
| 3.4 | 212435_at | 51592 | TRIM33 | Tripartite motif-containing 33 |
| 3.4 | 212633_at | 23376 | KIAA0776 | KIAA0776 |
| 3.4 | 218577_at | 55631 | FLJ20331 | hypothetical protein FLJ20331 |
| 3.4 | 218181_s_at | 9448 | MAP4K4 | mitogen-activated protein kinase kinase kinase kinase 4 |
| 3.4 | 214790_at | 26054 | SENP6 | SUMO1/sentrin specific protease 6 |
| 3.4 | 209689_at | 54520 | FLJ10996 | hypothetical protein FLJ10996 |
| 3.4 | 207956_x_at | 23047 | APRIN | androgen-induced proliferation inhibitor |
| 3.4 | 212635_at | 3842 | TNPO1 | Transportin 1 |
| 3.4 | 217863_at | 8554 | PIAS1 | protein inhibitor of activated STAT, 1 |
| 3.4 | 209741_x_at | 49855 | ZNF291 | zinc finger protein 291 |
| 3.4 | 203552_at | 11183 | MAP4K5 | mitogen-activated protein kinase kinase kinase kinase 5 |
| 3.4 | 203852_s_at | 6606 /// 6607 | SMIV1 /// SMIV2 | survival of motor neuron 1, telomeric /// survival of motor neuron 2, centromeric |
| 3.4 | 212293_at | 204851 | HIPK1 | homeodomain interacting protein kinase 1 |
| 3.4 | 213158_at | --- | --- | Homo sapiens, clone IMAGE:4214654, mRNA |
| 3.4 | 208841_s_at | 9908 | G3BP2 | Ras-GTPase activating protein SH3 domain-binding protein 2 |
| 3.4 | 202703_at | 8446 | DUSP11 | dual specificity phosphatase 11 (RNA/RNP complex 1-interacting) |
| 3.4 | 218566_s_at | 26973 | CHORDC1 | cysteine and histidine-rich domain (CHORD)-containing, zinc binding protein 1 |
| 3.4 | 213860_x_at | 1452 | CSNK1A1 | casein kinase 1, alpha 1 |
| 3.4 | 202536_at | 25978 | DKFZP5640123 | chromatin modifying protein 2B |
| 3.4 | 55692_at | 63916 | ELMO2 | engulfment and cell motility 2 (ced-12 homolog, C. elegans) |
| 3.4 | 203531_at | 8065 | CUL5 | Cullin 5 |
| 3.4 | 201542_at | 56681 | SARA1 | SARla gene homolog 1 (S. cerevisiae) |
| 3.4 | 221257_x_at | 81545 | FBXO38 | F-box protein 38 /// F-box protein 38 |
| 3.4 | 218432_at | 26273 | FBXO3 | F-box protein 3 |
| 3.4 | 221568_s_at | 55327 | LIN7C | lin-7 homolog C (C. elegans) |
| 3.4 | 217941_s_at | 55914 | ERBB2IP | erbb2 interacting protein |
| 3.4 | 212588_at | 5788 | PTPRC | protein tyrosine phosphatase, receptor type, C |
| 3.4 | 209115_at | 9039 | UBE1C | ubiquitin-activating enzyme E1C (UBA3 homolog, yeast) |
| 3.4 | 201865_x_at | 2908 | NR3Cl | nuclear receptor subfamily 3, group C, member 1 (glucocorticoid receptor) |
| 3.4 | 201934_at | 80335 | PR02730 | Hypothetical protein PR02730 |
| 3.4 | 217843_s_at | 29079 | MED4 | mediator of RNA polymerase II transcription, subunit 4 homolog (yeast) |
| 3.4 | 209455_at | 23291 | FBXW11 | F-box and WD-40 domain protein 11 |
| 3.4 | 212536_at | 23200 | ATP11B | ATPase, Class VI, type 11B |
| 3.4 | 212984_at | 1386 | ATF2 | activating transcription factor 2 |
| 3.4 | 216321_s_at | 2908 | NR3C1 | nuclear receptor subfamily 3, group C, member 1 (glucocorticoid receptor) |
| 3.4 | 212245_at | 90411 | MCFD2 | multiple coagulation factor deficiency 2 |
| 3.4 | 215245_x_at | 2332 | FMR1 | fragile X mental retardation 1 |
| 3.4 | 203375_s_at | 7174 | TPP2 | tripeptidyl peptidase II |
| 3.4 | 208661_s_at | 7267 | TTC3 | tetratricopeptide repeat domain 3 |
| 3.4 | 221905_at | 1540 | CYLD | cylindromatosis (turban tumor syndrome) |
| 3.4 | 212644_s_at | 93487 | C14orf32 | chromosome 14 open reading frame 32 |
| 3.4 | 209553_at | 23355 | KIAA0804 | KIAA0804 |
| 3.4 | 208761_s_at | 7341 | SUMO1 | SMT3 suppressor of mif two 3 homolog 1 (yeast) |
| 3.4 | 218911_at | 8089 | YEATS4 | YEATS domain containing 4 |
| 3.4 | 201177_s_at | 10054 | UBA2 | SUMO-1 activating enzyme subunit 2 |
| 3.4 | 218802_at | 55013 | FLJ20647 | hypothetical protein FLJ20647 |
| 3.4 | 209358_at | 6882 | TAF11 | TAF11 RNA polymerase II, TATA box binding protein (TBP)-associated factor, 28kDa |
| 3.4 | 209724_s_at | 7541 | ZFP161 | zinc finger protein 161 homolog (mouse) |
| 3.4 | 219565_at | 57404 | CYP20A1 | cytochrome P450, family 20, subfamily A, polypeptide 1 |
| 3.4 | 212493_s_at | 29072 | HYPB | huntingtin interacting protein B |
| 3.4 | 201648_at | 3716 | JAK1 | Janus kinase 1 (a protein tyrosine kinase) |
| 3.4 | 202573_at | 1455 | CSNK1G2 | casein kinase 1, gamma 2 |
| 3.4 | 37590 g at | 374655 | DKFZp547K1113 | Hypothetical protein DKFZp547Kll13 |
| 3.4 | 209609_s_at | 65005 | MRPL9 | mitochondrial ribosomal protein L9 |
| 3.4 | 211185_s_at | 23451 | SF3B1 | splicing factor 3b, subunit 1, 155kDa |
| 3.4 | 202505_at | 6629 | SNRPB2 | small nuclear ribonucleoprotein polypeptide B" |
| 3.4 | 201513_at | 7247 | TSN | translin |
| 3.4 | 205061_s_at | 5393 | EXOSC9 | exosome component 9 |
| 3.4 | 204593_s_at | 54471 | FLJ20232 | hypothetical protein FLJ20232 |
| 3.4 | 219696_at | 54530 | FLJ20054 | hypothetical protein FLJ20054 |
| 3.4 | 209025_s_at | 10492 | SYNCRIP | synaptotagmin binding, cytoplasmic RNA interacting protein |
| 3.4 | 221970_s_at | 25926 | DKFZP586L0724 | DKFZP586L0724 protein |
| 3.4 | 201385_at | 1665 | DHX15 | DEAH (Asp-Glu-Ala-His) box polypeptide 15 |
| 3.4 | 213227_at | 10424 | PGRMC2 | progesterone receptor membrane component 2 |
| 3.4 | 202386_s_at | 9665 | LKAP | limkain b1 |
| 3.4 | 213287_s_at | 3858 | KRT10 | keratin 10 (epidermolytic hyperkeratosis; keratosis palmaris et plantaris) |
| 3.4 | 214683_s_at | 1195 | CLK1 | CDC-like kinase 1 |
| 3.4 | 204299_at | 10772 | FUSIP1 | FUS interacting protein (serine-arginine rich) 1 |
| 3.4 | 204094_s_at | 9819 | KIAA0669 | TSC22 domain family 2 |
| 3.4 | 211375_s_at | 3609 | ILF3 | Interleukin enhancer binding factor 3, 90kDa |
| 3.4 | 201031_s_at | 3187 | HNRPH1 | heterogeneous nuclear ribonucleoprotein H1 (H) |
| 3.4 | 214323_s_at | 65110 | UPF3A | UPF3 regulator of nonsense transcripts homolog A (yeast) |
| 3.4 | 212706_at | 10156 | RASA4 | RAS p21 protein activator 4 |
| 3.4 | 213111_at | 200576 | PIP5K3 | phosphatidylinositol-3-phosphate/phosphatidylinositol 5-kinase, type III |
| 3.4 | 212855_at | 23142 | KIAA0276 | KIAA0276 protein |
| 3.4 | 213049_at | 253959 | GARNL1 | GTPase activating Rap/RanGAP domain-like 1 |
| 3.4 | 39582_at | 1540 | CYLD | Cylindromatosis (turban tumor syndrome) |
| 3.4 | 207606_s_at | 94134 | ARHGAP12 | Rho GTPase activating protein 12 |
| 3.4 | 210346_s_at | 57396 | CLK4 | CDC-like kinase 1 /// CDC-like kinase 4 |
| 3.4 | 200899_s_at | 10724 | MGEA5 | meningioma expressed antigen 5 (hyaluronidase) |
| 3.4 | 218067_s_at | 55082 | FLJ10154 | hypothetical protein FLJ10154 |
| 3.4 | 222182_s_at | 4848 | CNOT2 | CCR4-NOT transcription complex, subunit 2 |
| 3.4 | 211137_s_at | 27032 | ATP2C1 | ATPase, Ca++ transporting, type 2C, member 1 |
| 3.4 | 207181_s_at | 840 | CASP7 | caspase 7, apoptosis-related cysteine protease |
| 3.4 | 204369_at | 5290 | PIK3CA | phosphoinositide-3-kinase, catalytic, alpha polypeptide |
| 3.4 | 205062_x_at | 5926 | ARID4A | AT rich interactive domain 4A (RBP1-like) |
| 3.4 | 212180_at | 1399 | CRKL | v-crk sarcoma virus CT10 oncogene homolog (avian)-like |
| 3.4 | 221826_at | 90806 | LOC90806 | similar to RIKEN CDNA 2610307121 |
| 3.4 | 217952_x_at | 23469 | PHF3 | PHD finger protein 3 |
| 3.4 | 219378_at | 79612 | NARG1L | NMDA receptor regulated 1-like |
| 3.4 | 221229_s_at | 55006 | FLJ20628 | hypothetical protein FLJ20628 /// hypothetical protein FLJ20628 |
| 3.4 | 208896_at | 8886 | DDX18 | DEAD (Asp-Glu-Ala-Asp) box polypeptide 18 |
| 3.4 | 221510_s_at | 2744 | GLS | glutaminase |
| 3.4 | 229265_at | 79906 | FLJ13941 | Atrophin 1 |
| 3.4 | 208884_s_at | 51366 | EDD | E3 identified by differential display |
| 3.4 | 201829_at | 10276 | NET1 | neuroepithelial cell transforming gene 1 |
| 3.4 | 201672_s_at | 9097 | USP14 | ubiquitin specific protease 14 (tRNA-guanine transglycosylase) |
| 3.4 | 201821_s_at | 10440 | TIMM17A | translocase of inner mitochondrial membrane 17 homolog A (yeast) |
| 3.4 | 208666_s_at | 6767 | ST13 | suppression of tumorigenicity 13 (colon carcinoma) (Hsp70 interacting protein) |
| 3.4 | 218263_s_at | 58486 | LOC58486 | transposon-derived Busterl transposase-like protein gene |
| 3.4 | 201314_at | 10494 | STK25 | serine/threonine kinase 25 (STE20 homolog, yeast) |
| 3.4 | 207996_s_at | 753 | C18orf1 | chromosome 18 open reading frame 1 |
| 3.4 | 202754_at | 23518 | R3HDM | R3H domain (binds single-stranded nucleic acids) containing |
| 3.4 | 214835_s_at | 8801 | SUCLG2 | succinate-CoA ligase, GDP-forming, beta subunit |
| 3.4 | 218031_s_at | 1112 | CHES1 | checkpoint suppressor 1 |
| 3.4 | 203620_s_at | 9873 | FCHSD2 | FCH and double SH3 domains 2 |
| 3.5 | 211745_x_at | 3039 | HBA1 | hemoglobin, alpha 1 hemoglobin, alpha 1 /// hemoglobin, alpha 2 /// hemoglobin, alpha 2 |
| 3.5 | 217414_x_at | 3040 | HBA2 | hemoglobin, alpha 2 |
| 3.5 | 204018_x_at | 3039 /// 3040 | HBA1 /// HBA2 | hemoglobin, alpha 1 /// hemoglobin, alpha 1 /// hemoglobin, alpha 2 /// hemoglobin, alpha 2 |
| 3.5 | 209116_x_at | 3043 | HBB | hemoglobin, beta /// hemoglobin, beta |
| 3.5 | 209458_x_at | 3039 /// 3040 | HBA1 /// HBA2 | hemoglobin, alpha 1 /// hemoglobin, alpha 1 /// hemoglobin, alpha 2 hemoglobin, alpha 2 |
| 3.5 | 217232_x_at | 3043 | HBB | hemoglobin, beta |
| 3.5 | 214414_x_at | 3040 | HBA2 | hemoglobin, alpha 2 /// hemoglobin, alpha 2 |
| 3.5 | 243989_at | --- | --- | KU-MEL-3 protein |
| 3.5 | 211696_x_at | 3043 | HBB | hemoglobin, beta /// hemoglobin, beta |
| 3.5 | 205245_at | 50855 | PARD6A | par-6 partitioning defective 6 homolog alpha (C.elegans) |
| 3.5 | 214327_x_at | 7178 | TPT1 | tumor protein, translationally-controlled 1 |
| 3.5 | 200630_x_at | 6418 | SET | SET translocation (myeloid leukemia-associated) |
| 3.5 | 208755_x_at | 3020 | H3F3A | H3 histone, family 3A |
| 3.5 | 213828_x_at | 3020 | H3F3A | H3 histone, family 3A |
| 3.5 | 202264_s_at | 10452 | TOMM40 | translocase of outer mitochondrial membrane 40 homolog (yeast) |
| 3.5 | 208749_x_at | 10211 | FLOT1 | flotillin 1 |
| 3.5 | 220050_at | 11092 | C9orf9 | chromosome 9 open reading frame 9 |
| 3.5 | 209107_x_at | 8648 | NCOA1 | nuclear receptor coactivator 1 |
| 3.5 | 209320_at | 109 | ADCY3 | adenylate cyclase 3 |
| 3.5 | 227431_at | 222194 | RSBN1L | Round spermatid basic protein 1-like |
| 3.5 | 217052_x_at | --- | --- | --- |
| 3.5 | 243576_at | --- | --- | --- |
| 3.6 | 222683_at | 56254 | RNF20 | ring finger protein 20 |
| 3.6 | 223087_at | 55862 | ECHDC1 | enoyl Coenzyme A hydratase domain containing 1 |
| 3.6 | 224989_at | 201895 | LOC201895 | Hypothetical protein LOC201895 |
| 3.6 | 224827_at | 92181 | DC-UbP | Dendritic cell-derived ubiquitin-like protein |
| 3.6 | 226119_at | 115294 | LOC115294 | similar to hypothetical protein FLJ10883 |
| 3.6 | 230925_at | 54518 | APBB1IP | amyloid beta (A4) precursor protein-binding, family B, member 1 interacting protein |
| 3.6 | 208898_at | 51382 | ATP6V1D | ATPase, H+ transporting, lysosomal 34kDa, V1 subunit D |
| 3.6 | 223004_s_at | 51300 | C3orf1 | chromosome 3 open reading frame 1 |
| 3.6 | 200703_at | 8655 | DNCL1 | dynein, cytoplasmic, light polypeptide 1 |
| 3.6 | 229068_at | --- | --- | chaperonin containing TCP1, subunit 5 (epsilon) |
| 3.6 | 223157_at | 84273 | C4orf14 | chromosome 4 open reading frame 14 |
| 3.6 | 225921_at | 51199 | NIN | ninein (GSK3B interacting protein) |
| 3.6 | 224129_s_at | 84661 | LOC84661 | dpy-30-like protein |
| 3.6 | 226879_at | 84329 | MGC15619 | hypothetical protein MGC15619 |
| 3.6 | 204565_at | 55856 | THEM2 | thioesterase superfamily member 2 |
| 3.6 | 201742_x_at | 6426 | SFRS1 | splicing factor, arginine/serine-rich 1 (splicing factor 2, alternate splicing factor) |
| 3.6 | 231576_at | --- | --- | CDNA clone IMAGE:5261903, partial cds |
| 3.6 | 223386_at | 79607 | FLJ21103 | hypothetical protein FLJ21103 |
| 3.6 | 205202_at | 5110 | PCMT1 | protein-L-isoaspartate (D-aspartate) O-methyltransferase |
| 3.6 | 222980_at | 10890 | RAB10 | RAB10, member RAS oncogene family |
| 3.6 | 201716_at | 6642 | SNX1 | sorting nexin 1 |
| 3.6 | 223978_s_at | 54675 | C20orf155 | chromosome 20 open reading frame 155 |
| 3.6 | 225200_at | --- | --- | zinc finger, CSL domain containing 2 |
| 3.6 | 203800_s_at | 63931 | MRPS14 | mitochondrial ribosomal protein S14 |
| 3.6 | 234983_at | --- | --- | Transcribed locus, moderately similar to XP_512541.1 similar to hypothetical protein [Pan troglodytes] |
| 3.6 | 203327_at | 3416 | IDE | insulin-degrading enzyme |
| 3.6 | 224919_at | 64968 | MRPS6 | mitochondrial ribosomal protein S6 |
| 3.6 | 218026_at | 28958 | HSPCO09 | HSPCO09 protein |
| 3.6 | 202297_s_at | 11079 | RER1 | RER1 retention in endoplasmic reticulum 1 homolog (S. cerevisiae) |
| 3.6 | 223086_x_at | 51258 | MRPL51 | mitochondrial ribosomal protein L51 |
| 3.6 | 223114_at | 84274 | MGC4767 | hypothetical protein MGC4767 |
| 3.6 | 223212_at | 84287 | ZDHHC16 | zinc finger, DHHC domain containing 16 |
| 3.6 | 228799_at | --- | --- | Transcribed locus |
| 3.6 | 226923_at | 152579 | SCFD2 | sec1 family domain containing 2 |
| 3.6 | 225036_at | 401505 | C9orf105 | chromosome 9 open reading frame 105 |
| 3.6 | 222388_s_at | 55737 | VPS35 | vacuolar protein sorting 35 (yeast) |
| 3.6 | 224732_at | 54921 | DERPC | decreased expression in renal and prostate |
| 3.6 | 228418_at | 10640 | SEC10L1 | SEC10-like 1 (S. cerevisiae) |
| 3.6 | 237289_at | 1385 | CREB1 | CAMP responsive element binding protein 1 |
| 3.6 | 223335_at | 51249 | LOC51249 | hypothetical protein LOC51249 |
| 3.6 | 227167_s_at | --- | --- | Mesenchymal stem cell protein DSC96 |
| 3.6 | 225153_at | 85476 | GFM1 | G elongation factor, mitochondrial 1 |
| 3.6 | 223268_at | 28970 | PTD012 | PTD012 protein |
| 3.6 | 225371_at | 2733 | GLE1L | GLE1 RNA export mediator-like (yeast) |
| 3.6 | 229595_at | 131474 | CHCHD4 | coiled-coil-helix-coiled-coil-helix domain containing 4 |
| 3.6 | 222714_s_at | 51110 | LACTB2 | lactamase, beta 2 |
| 3.6 | 225734_at | 26263 | FBXO22 | F-box protein 22 |
| 3.6 | 225769_at | 57511 | COG6 | component of oligomeric golgi complex 6 |
| 3.6 | 226116_at | --- | --- | CDNA FLJ12540 fis, clone NT2RM4000425 |
| 3.6 | 223066_at | 23557 | SNAPAP | SNAP-associated protein |
| 3.6 | 224675_at | 23184 | MESDC2 | mesoderm development candidate 2 |
| 3.6 | 239069_s_at | --- | --- | Transcribed locus |
| 3.6 | 225528_at | 10526 | IPO8 | importin 8 |
| 3.6 | 223301_s_at | 79780 | FLJ23518 | hypothetical protein FLJ23518 |
| 3.6 | 223106_at | 51522 | TMEM14C | transmembrane protein 14C |
| 3.6 | 225232_at | 54545 | PIP3AP | myotubularin related protein 12 |
| 3.6 | 222637_at | 51397 | COMMD10 | COMM domain containing 10 |
| 3.6 | 225502_at | 81704 | DOCK8 | dedicator of cytokinesis 8 |
| 3.6 | 225411_at | 84910 | FLJ14681 | Hypothetical protein FLJ14681 |
| 3.6 | 224974_at | 64426 | SDS3 | likely ortholog of mouse Sds3 |
| 3.6 | 223361_at | 58527 | C6orf115 | chromosome 6 open reading frame 115 |
| 3.6 | 228343_at | 5452 | POU2F2 | POU domain, class 2, transcription factor 2 |
| 3.6 | 200684_s_at | 7332 | UBE2L3 | ubiquitin-conjugating enzyme E2L 3 |
| 3.6 | 226019_at | 115209 | OMA1 | OMA1 homolog, zinc metallopeptidase (S. cerevisiae) |
| 3.6 | 224723_x_at | 402587 | LOC402587 | hypothetical LOC401397 |
| 3.6 | 226335_at | 6197 | RPS6KA3 | ribosomal protein S6 kinase, 90kDa, polypeptide 3 |
| 3.6 | 224834_at | 92181 | DC-UbP | Dendritic cell-derived ubiquitin-like protein |
| 3.6 | 223223_at | 64801 | ARV1 | ARV1 homolog (yeast) |
| 3.6 | 225661_at | 3454 | IFNAR1 | interferon (alpha, beta and omega) receptor 1 |
| 3.6 | 223356_s_at | 219402 | MTIF3 | mitochondrial translational initiation factor 3 |
| 3.6 | 227293_at | 84708 | LNX | Ligand of numb-protein X |
| 3.6 | 226219_at | 257106 | LOC257106 | hypothetical protein LOC257106 |
| 3.6 | 225274_at | 51449 | PCYOX1 | prenylcysteine oxidase 1 |
| 3.6 | 226432 at | --- | --- | CDNA clone IMAGE:5261903, partial cds |
| 3.6 | 228841_at | 90624 | LOC90624 | hypothetical protein LOC90624 |
| 3.6 | 227075_at | 55140 | ELP3 | elongation protein 3 homolog (S. cerevisiae) |
| 3.6 | 240064_at | --- | --- | Transcribed locus, strongly similar to XP_529518.1 LOC452210 [Pan troglodytes] |
| 3.6 | 228075_x_at | 51106 | TFB1M | transcription factor B1, mitochondrial |
| 3.6 | 224437_s_at | 51534 | C6orf55 | chromosome 6 open reading frame 55 /// chromosome 6 open reading frame 55 |
| 3.6 | 201472_at | 7411 | VBP1 | von Hippel-Lindau binding protein 1 |
| 3.6 | 229670_at | --- | --- | 5.5 kb mRNA upregulated in retinoic acid treated HL-60 neutrophilic cells. |
| 3.6 | 218130_at | 79415 | MGC4368 | hypothetical protein MGC4368 |
| 3.6 | 200676_s_at | 7332 | UBE2L3 | ubiquitin-conjugating enzyme E2L 3 |
| 3.6 | 201518_at | 10951 | CBX1 | chromobox homolog 1 (HP1 beta homolog Drosophila) |
| 3.6 | 219547_at | 1355 | COX15 | COX15 homolog, cytochrome c oxidase assembly protein (yeast) |
| 3.6 | 223751_x_at | 81793 | TLR10 | toll-like receptor 10 |
| 3.6 | 228061_at | 90693 | LOC90693 | alpha-1,3(6)-mannosylglycoprotein beta-1,6-N-acetyl-glucosaminyltransferase-like |
| 3.6 | 223305_at | 51259 | MGC13379 | HSPC244 |
| 3.6 | 200996_at | 10096 | ACTR3 | ARP3 actin-related protein 3 homolog (yeast) |
| 3.6 | 209276_s_at | 2745 | GLRX | glutaredoxin (thioltransferase) |
| 3.6 | 208899_x_at | 51382 | ATP6V1D | ATPase, H+ transporting, lysosomal 34kDa, V1 subunit D |
| 3.6 | 223021_x_at | 51534 | C6orf55 | chromosome 6 open reading frame 55 |
| 3.6 | 231016_s_at | --- | --- | Aryl hydrocarbon receptor nuclear translocator |
| 3.6 | 233124_s_at | 55862 | ECHDC1 | enoyl Coenzyme A hydratase domain containing 1 |
| 3.6 | 223225_s_at | 81929 | SEH1L | SEH1-like (S. cerevisiae) |
| 3.6 | 231918_s_ at | 84340 | GFM2 | G elongation factor, mitochondrial 2 |
| 3.6 | 223400_s_at | 55193 | PB1 | polybromo 1 |
| 3.6 | 212216_at | 9581 | KIAA0436 | prolyl endopeptidase-like |
| 3.6 | 222585_x_at | 51315 | LOC51315 | hypothetical protein LOC51315 |
| 3.6 | 201634_s_at | 80777 | CYB5-M | outer mitochondrial membrane cytochrome b5 |
| 3.6 | 222587_s_at | 51809 | GALNT7 | UDP-N-acetyl-alpha-D-galactosamine:polypeptide N-acetylgalactosaminyltransferase 7 (GalNAc-T7) |
| 3.6 | 242317_at | --- | --- | Transcribed locus |
| 3.6 | 209600_s_at | 51 | ACOX1 | acyl-Coenzyme A oxidase 1, palmitoyl |
| 3.6 | 202874_s_at | 528 | ATP6V1C1 | ATPase, H+ transporting, lysosomal 42kDa, V1 subunit C, isoform 1 |
| 3.6 | 217905_at | 79892 | C10orf119 | chromosome 10 open reading frame 119 |
| 3.6 | 201956_s_at | 8443 | GNPAT | glyceronephosphate O-acyltransferase |
| 3.6 | 222498_at | 64343 | AZI2 | 5-azacytidine induced 2 |
| 3.6 | 217873_at | 51719 | CAB39 | calcium binding protein 39 |
| 3.6 | 230176_at | 55293 | UEV3 | Ubiquitin-conjugating enzyme E2-like |
| 3.6 | 223065_s_at | 83930 | STARD3NL | STARD3 N-terminal like |
| 3.6 | 218809_at | 80025 | PANK2 | pantothenate kinase 2 (Hallervorden-Spatz syndrome) |
| 3.6 | 227012-at | 55972 | MCFP | mitochondrial carrier family protein |
| 3.6 | 222477_s_at | 51768 | TM7SF3 | transmembrane 7 superfamily member 3 |
| 3.6 | 227107_at | 24145 | PANX1 | Pannexin 1 |
| 3.6 | 225367_at | 55276 | PGM2 | phosphoglucomutase 2 |
| 3.6 | 225633_at | 147991 | LOC147991 | hypothetical protein LOC147991 |
| 3.6 | 223077_at | 29766 | TMOD3 | tropomodulin 3 (ubiquitous) |
| 3.6 | 224961_at | 55681 | SCYL2 | SCY1-like 2 (S. cerevisiae) |
| 3.6 | 225101_s_at | 57231 | SNX14 | sorting nexin 14 |
| 3.6 | 225366_at | 55276 | PGM2 | phosphoglucomutase 2 |
| 3.6 | 202872_at | 528 | ATP6V1C1 | ATPase, H+ transporting, lysosomal 42kDa, V1 subunit C, isoform 1 |
| 3.6 | 232353_s_at | 51657 | DUSP24 | dual specificity phosphatase 24 (putative) |
| 3.6 | 224708_at | 90231 | MGC33867 | hypothetical protein MGC33867 |
| 3.6 | 209397_at | 4200 | ME2 | malic enzyme 2, IVAD(+)-dependent, mitochondrial |
| 3.6 | 201500_s_at | 6992 | PPP1R11 | protein phosphatase 1, regulatory (inhibitor) subunit 11 |
| 3.6 | 222394_at | 10015 | PDCD6IP | programmed cell death 6 interacting protein |
| 3.6 | 227220_at | 152518 | HOZFP | nuclear transcription factor, X-box binding-like 1 |
| 3.6 | 228162_at | 2098 | ESD | Esterase D/formylglutathione hydrolase |
| 3.6 | 242648_at | 57563 | KLHL8 | kelch-like 8 (Drosophila) |
| 3.6 | 229295_at | 150166 | LOC150166 | hypothetical protein LOC150166 |
| 3.6 | 227270_at | 285550 | LOC285550 | hypothetical protein LOC285550 |
| 3.6 | 228006_at | 5728 | PTEN | Phosphatase and tensin homolog (mutated in multiple advanced cancers 1) |
| 3.6 | 225558_at | 9815 | GIT2 | G protein-coupled receptor kinase interactor 2 |
| 3.6 | 227873_at | 79770 | C5orf14 | chromosome 5 open reading frame 14 |
| 3.6 | 235625_at | 27072 | VPS41 | vacuolar protein sorting 41 (yeast) |
| 3.6 | 227608_at | 55249 | YAP | YY1 associated protein 1 |
| 3.6 | 243797_at | 9262 | STK17B | serine/threonine kinase 17b (apoptosis-inducing) |
| 3.6 | 235459_at | --- | --- | Transcribed locus |
| 3.6 | 239824_s_at | 84314 | MGC10744 | hypothetical protein MGC10744 |
| 3.6 | 232432_s_at | 64924 | SLC30A5 | solute carrier family 30 (zinc transporter), member 5 |
| 3.6 | 225779_at | 10999 | SLC27A4 | solute carrier family 27 (fatty acid transporter), member 4 |
| 3.6 | 222702_x_at | 9419 | CRIPT | postsynaptic protein CRIPT |
| 3.6 | 224187_x_at | 3312 | HSPA8 | heat shock 70kDa protein 8 |
| 3.6 | 225161_at | 85476 | GFM1 | G elongation factor, mitochondrial 1 |
| 3.6 | 218160_at | 4702 | NDUFA8 | NADH dehydrogenase (ubiquinone) 1 alpha subcomplex, 8, 19kDa |
| 3.6 | 204613_at | 5336 | PLCG2 | phospholipase C, gamma 2 (phosphatidylinositol-specific) |
| 3.6 | 235346_at | 139341 | FUNDC1 | FUN14 domain containing 1 |
| 3.6 | 223286_at | 23587 | DERP6 | S-phase 2 protein |
| 3.6 | 203201_at | 5373 | PMM2 | phosphomannomutase 2 |
| 3.6 | 224634_at | 54865 | GPATC4 | G patch domain containing 4 |
| 3.6 | 226468_at | --- | --- | Transcribed locus, weakly similar to XP_517454.1 similar to hypothetical protein MGC45438 [Pan troglodytes] |
| 3.6 | 223082_at | 30011 | SH3KBP1 | SH3_domain kinase binding protein 1 |
| 3.6 | 219283_at | 29071 | C1GALT1C1 | C1GALT1-specific chaperone 1 |
| 3.6 | 226073_at | 219854 | LOC219854 | hypothetical protein LOC219854 |
| 3.6 | 225331_at | 152137 | C3orf6 | chromosome 3 open reading frame 6 |
| 3.6 | 225419_at | --- | --- | chromosome 7 open reading frame 11 |
| 3.6 | 201240_s_at | 9789 | SPCS2 | signal peptidase complex subunit 2 homolog (S. cerevisiae) |
| 3.6 | 208716_s_at | 54499 | LOC54499 | putative membrane protein |
| 3.6 | 205078_at | 5281 | PIGF | phosphatidylinositol glycan, class F |
| 3.6 | 201583_s_at | 10483 | SEC23B | Sec23 homolog B (S. cerevisiae) |
| 3.6 | 223088_x_at | 55862 | ECHDC1 | enoyl Coenzyme A hydratase domain containing 1 |
| 3.6 | 200617_at | 9761 | KIAA0152 | KIAA0152 |
| 3.6 | 225666_at | 84899 | FLJ14624 | hypothetical protein FLJ14624 |
| 3.6 | 225743_at | 285367 | RPUSD3 | RNA pseudouridylate synthase domain containing 3 |
| 3.6 | 225737_s_at | 26263 | FBXO22 | F-box protein 22 |
| 3.6 | 227942_s_at | 9419 | CRIPT | postsynaptic protein CRIPT |
| 3.6 | 213698_at | 9204 | ZNF258 | zinc finger protein 258 |
| 3.6 | 226060_at | 91869 | RFT1 | RFT1 homolog (S. cerevisiae) |
| 3.6 | 213149_at | 1737 | DLAT | dihydrolipoamide 5-acetyltransferase (E2 component of pyruvate dehydrogenase |
| 3.6 | 203008_x_at | 10190 | TXNDC9 | thioredoxin domain containing 9 |
| 3.6 | 225110_at | 55239 | FLJ10826 | hypothetical protein FLJ10826 |
| 3.6 | 228009_x_at | 30834 | ZNRD1 | zinc ribbon domain containing, 1 |
| 3.6 | 226416_at | 90459 | 3'HEXO | histone mRNA 3' end-specific exonuclease |
| 3.6 | 213102_at | 10096 | ACTR3 | ARP3 actin-related protein 3 homolog (yeast) |
| 3.6 | 225472_at | 7918 | BAT4 | HLA-B associated transcript 4 |
| 3.6 | 228495_at | 253635 | FLJ38348 | Hypothetical protein FLJ38348 |
| 3.6 | 228087_at | 90693 | LOC90693 | alpha-1,3(6)-mannosylglycoprotein beta-1,6-N-acetyl-glucosaminyltransferase-like |
| 3.6 | 214179_s_at | 4779 | NFE2L1 | nuclear factor (erythroid-derived 2)-like 1 |
| 3.6 | 226861_at | 140461 | ASB8 | ankyrin repeat and SOCS box-containing 8 |
| 3.6 | 219375_at | 10390 | CEPT1 | choline/ethanolamine phosphotransferase 1 |
| 3.6 | 222473_s_at | 55914 | ERBB2IP | erbb2 interacting protein |
| 3.6 | 231808_at | 85287 | KRTAP4-7 | keratin associated protein 4-7 |
| 3.6 | 234987_at | 25939 | SAMHD1 | SAM domain and HD domain 1 |
| 3.6 | 225144_at | 659 | BMPR2 | bone morphogenetic protein receptor, type II (serine/threonine kinase) |
| 3.6 | 228324_at | 138199 | C9orf41 | chromosome 9 open reading frame 41 |
| 3.6 | 226558_at | 441057 | LOC441057 | Ankyrin repeat domain 20B |
| 3.6 | 201491_at | 10598 | AHSA1 | AHAL, activator of heat shock 90kDa protein ATPase homolog 1 (yeast) |
| 3.6 | 228026_at | 80143 | FLJ21168 | Hypothetical protein FLJ21168 |
| 3.6 | 226831_at | --- | --- | Hypothetical protein BC017169 |
| 3.6 | 224600_at | 8545 | CGGBP1 | CGG triplet repeat binding protein 1 |
| 3.6 | 224944_at | --- | --- | Thymopoietin |
| 3.6 | 228987_at | 51571 | FAM49B | Family with sequence similarity 49, member B |
| 3.6 | 225084_at | 10640 | SEC10L1 | SEC10-like 1 (S. cerevisiae) |
| 3.6 | 224769_at | --- | --- | CDNA clone IMAGE:5263531, partial cds |
| 3.6 | 226875_at | 139818 | DOCK11 | dedicator of cytokinesis 11 |
| 3.6 | 222476_at | 57472 | CNOT6 | CCR4-NOT transcription complex, subunits 6 |
| 3.6 | 225456_at | 5469 | PPARBP | PPAR binding protein |
| 3.6 | 236539_at | 26191 | PTPN22 | protein tyrosine phosphatase, non-receptor type 22 (lymphoid) |
| 3.6 | 225512_at | 253461 | FLJ35036 | zinc finger and BTB domain containing 38 |
| 3.6 | 223104_at | 84522 | JAGN1 | jagunal homolog 1 (Drosophila) |
| 3.6 | 222762_x_at | 8994 | LIMD1 | LIM domains containing 1 |
| 3.6 | 226511_at | --- | --- | CDNA FLJ13558 fis, clone PLACE1007743 |
| 3.6 | 225225_at | 85287 | KRTAP4-7 | Homo sapiens, clone IMAGE:5274897, mRNA |
| 3.6 | 223090_x_at | 55591 | VEZATIN | transmembrane protein vezatin |
| 3.6 | 235125_x_at | 374986 | FLJ35093 | FLJ35093 protein |
| 3.6 | 228283_at | 152100 | MGC61571 | hypothetical protein MGC61571 |
| 3.6 | 224786_at | 60592 | SCOC | short coiled-coil protein |
| 3.6 | 224333_s_at | 64969 | MRPS5 | mitochondrial ribosomal protein S5 /// mitochondrial ribosomal protein S5 |
| 3.6 | 226259_at | 54536 | SEC15L1 | SEC15-like 1 (S. cerevisiae) |
| 3.6 | 231093_at | 115352 | FCRH3 | Fc receptor-like protein 3 |
| 3.6 | 226562_at | 146050 | FLJ35867 | hypothetical protein FLJ35867 |
| 3.6 | 225501_at | 84295 | PHF6 | PHD finger protein 6 |
| 3.6 | 231921_at | 80067 | FLJ13096 | hypothetical protein FLJ13096 |
| 3.6 | 227520_at | 55787 | CXorf15 | Chromosome X open reading frame 15 |
| 3.6 | 226801_s_at | 64853 | FLJ12806 | --- |
| 3.6 | 222715_s_at | 11276 | AP1GBP1 | AP1 gamma subunit binding protein 1 |
| 3.6 | 224619_at | 113201 | H63 | H63 breast cancer expressed gene |
| 3.6 | 222410_s_at | 58533 | SNX6 | sorting nexin 6 |
| 3.6 | 234947_s_at | 63877 | C10orf84 | chromosome 10 open reading frame 84 |
| 3.6 | 242140_at | 113386 | LOC113386 | similar to envelope protein |
| 3.6 | 222547_at | 9448 | MAP4K4 | mitogen-activated protein kinase kinase kinase kinase 4 |
| 3.6 | 226151_x_at | 9946 | CRYZL1 | crystallin, zeta (quinone reductase)-like 1 |
| 3.6 | 225953_at | 55197 | P15RS | hypothetical protein FLJ10656 |
| 3.6 | 232024_at | 26157 | GIMAP2 | GTPase, IMAP family member 2 |
| 3.6 | 226521_s_at | 84142 | FLJ13614 | hypothetical protein FLJ13614 |
| 3.6 | 223328_at | 83787 | SVH | SVH protein |
| 3.6 | 235010_at | --- | --- | Homo sapiens, clone IMAGE:3930408, mRNA |
| 3.6 | 224893_at | 25923 | DKFZP564J0863 | DKFZP564JO863 protein |
| 3.6 | 228289_at | --- | --- | Bromodomain containing 7 |
| 3.6 | 230003_at | --- | --- | Transcribed locus |
| 3.6 | 226785_at | 286410 | ATP11C | ATPase, Class VI, type 11C |
| 3.6 | 226421_at | 286505 | LOC286505 | hypothetical protein LOC286505 |
| 3.6 | 230009_at | 79607 | FLJ21103 | Hypothetical protein FLJ21103 |
| 3.6 | 223446_s_at | 84062 | DTNBP1 | dystrobrevin binding protein 1 |
| 3.6 | 225805_at | 3192 | HNRPU | Heterogeneous nuclear ribonucleoprotein U (scaffold attachment factor A) |
| 3.6 | 227422_at | --- | --- | Transcribed locus |
| 3.6 | 227158_at | --- | --- | Chromosome 14 open reading frame 126 |
| 3.6 | 225439_at | 84955 | NUDCD1 | NudC domain containing 1 |
| 3.6 | 230298_at | 153364 | LOC153364 | similar to metallo-beta-lactamase superfamily protein |
| 3.6 | 226779_at | --- | --- | CDNA FLJ37302 fis, clone BRAMY2016009 |
| 3.6 | 244661_at | 6646 | SOAT1 | Sterol O-acyltransferase (acyl-Coenzyme A: cholesterol acyltransferase) 1 |
| 3.6 | 229804_x_at | 150472 /// 220869 /// 55871 | CBWD1 /// CBWD2 /// | COBW domain containing 1 /// COBW domain containing 2 /// dopamine responsive protein |
| 3.6 | 224881_at | 154807 | VKORC1L1 | vitamin K epoxide reductase complex, subunit 1-like 1 |
| 3.6 | 223576_at | 51250 | C6orf203 | chromosome 6 open reading frame 203 |
| 3.6 | 225340_s_at | --- | --- | membrane component, chromosome 11, surface marker 1 |
| 3.6 | 227369_at | 26135 | PAI-RBP1 | PAI-1 mRNA-binding protein |
| 3.6 | 223046_at | 54583 | EGLN1 | egl nine homolog 1 (C. elegans) |
| 3.6 | 201661_s_at | 2181 | ACSL3 | acyl-CoA synthetase long-chain family member 3 |
| 3.6 | 224720_at | 57534 | MIB1 | mindbomb homolog 1 (Drosophila) |
| 3.6 | 227052_at | 201895 | LOC201895 | Hypothetical protein LOC201895 |
| 3.6 | 230561_s_at | 151050 | FLJ23861 | hypothetical protein FLJ23861 |
| 3.6 | 225554_s_at | 51434 | ANAPC7 | anaphase promoting complex subunit 7 |
| 3.6 | 226343_at | --- | --- | Dipeptidylpeptidase 8 |
| 3.6 | 222589_at | 51701 | NLK | nemo like kinase |
| 3.6 | 223022_s_at | 51534 | C6orf55 | chromosome 6 open reading frame 55 |
| 3.6 | 226093_at | 196513 | DCP1B | DCP1 decapping enzyme homolog B (S. cerevisiae) |
| 3.6 | 225196_s_at | 64949 | MRPS26 | mitochondrial ribosomal protein S26 |
| 3.6 | 227247_at | --- | --- | Pleckstrin homology domain containing, family A (phosphoinositide binding specific) member 8 |
| 3.6 | 226826_at | 134353 | LSM11 | LSM11, U7 small nuclear RNA associated |
| 3.6 | 226628_at | 57187 | THOC2 | THO complex 2 |
| 3.6 | 235424_at | 116224 | C9orf42 | Chromosome 9 open reading frame 42 |
| 3.6 | 225430_at | 8683 | SFRS9 | Hypothetical protein 15E1.2 |
| 3.6 | 227815_at | 65082 | VPS33A | Vacuolar protein sorting 33A (yeast) |
| 3.6 | 234311_s_at | 85865 | LOC85865 | hypothetical protein BC004923 |
| 3.6 | 229573_at | --- | --- | Transcribed locus, moderately similar to XP_521389.1 ubiquitously transcribed repeat gene, Y-linked [Pan troglodytes] |
| 3.6 | 232004_at | 10236 | HNRPR | heterogeneous nuclear ribonucleoprotein R |
| 3.6 | 213853_at | 120526 | ZCSL3 | zinc finger, CSL domain containing 3 |
| 3.6 | 211337_s_at | 27229 | 76P | gamma tubulin ring complex protein (76p gene) |
| 3.6 | 201746_at | 7157 | TP53 | tumor protein p53 (Li-Fraumeni syndrome) |
| 3.6 | 218646_at | 54969 | FLJ20534 | hypothetical protein FLJ20534 |
| 3.6 | 201756_at | 6118 | RPA2 | replication protein A2, 32kDa |
| 3.6 | 230391_at | --- | --- | Transcribed locus |
| 3.6 | 242521_at | --- | --- | CDNA clone IMAGE:30349460, partial cds |
| 3.6 | 226478_at | 51768 | TM7SF3 | Transmembrane 7 superfamily member 3 |
| 3.6 | 225509_at | 56757 | LOC56757 | hypothetical protein LOC56757 |
| 3.6 | 217990_at | 51292 | GMPR2 | guanosine monophosphate reductase 2 |
| 3.6 | 211760_s_at | 8674 | VAMP4 | vesicle-associated membrane protein 4 /// vesicle-associated membrane protein 4 |
| 3.6 | 209384_at | 11212 | PROSC | proline synthetase co-transcribed homolog (bacterial) |
| 3.6 | 203266_s_at | 6416 | MAP2K4 | mitogen-activated protein kinase kinase 4 |
| 3.6 | 221471_at | 10955 | TDE1 | tumor differentially expressed 1 |
| 3.6 | 213246_at | 26175 | C14orf109 | chromosome 14 open reading frame 109 |
| 3.6 | 200683_s_at | 7332 | UBE2L3 | ubiquitin-conjugating enzyme E2L 3 |
| 3.6 | 212412_at | 10611 | PDLIM5 | PDZ and LIM domain 5 |
| 3.6 | 202811_at | 10617 | STAMBP | STAM binding protein |
| 3.6 | 202451_at | 2965 | GTF2H1 | general transcription factor IIH, polypeptide 1, 62kDa |
| 3.6 | 204683_at | 3384 | ICAM2 | intercellular adhesion molecule 2 |
| 3.6 | 202808_at | 54838 | C10orf26 | chromosome 10 open reading frame 26 |
| 3.6 | 209879_at | 6404 | SELPLG | selectin P ligand |
| 3.7 | 225523_at | 116540 | MRPL53 | mitochondrial ribosomal protein L53 |
| 3.7 | 226257_x_at | 56945 | MRPS22 | mitochondrial ribosomal protein S22 |
| 3.7 | 224885_s_at | 200185 | KRTCAP2 | keratinocyte associated protein 2 |
| 3.7 | 222609_s_at | 51013 | EXOSC1 | exosome component 1 |
| 3.7 | 222997_s_at | 54460 | MRPS21 | mitochondrial ribosomal protein S21 |
| 3.7 | 226935_s_at | 81037 | CRR9 | cisplatin resistance related protein CRR9p |
| 3.7 | 224867_at | 440574 | LOC440574 | similar to protein of fungal metazoan origin like (11.1 kD) (2C514) |
| 3.7 | 225489_at | 129787 | TMEM18 | transmembrane protein 18 |
| 3.7 | 225422_at | 246184 | CDC26 | cell division cycle 26 |
| 3.7 | 224332_s_at | 84545 | MRPL43 | mitochondrial ribosomal protein L43 /// mitochondrial ribosomal protein L43 |
| 3.7 | 224879_at | 90871 | C9orf123 | chromosome 9 open reading frame 123 |
| 3.7 | 225082_at | 51692 | CPSF3 | cleavage and polyadenylation specific factor 3, 73kDa |
| 3.7 | 223100_s_at | 11164 | NUDT5 | nudix (nucleoside diphosphate linked moiety X)-type motif 5 |
| 3.7 | 223711_s_at | 29087 | THY28 | thymocyte protein thy28 |
| 3.7 | 225399_at | 116461 | Clorf19 | chromosome 1 open reading frame 19 |
| 3.7 | 218512_at | 55759 | WDR12 | WD repeat domain 12 |
| 3.7 | 224584_at | 29058 | C20orf30 | chromosome 20 open reading frame 30 |
| 3.7 | 229099_at | --- | --- | Clone DNA64903 DSLR655 (UNQ655) mRNA, complete cds /// Hypothetical protein MGC2477 |
| 3.7 | 231045_x_at | 280636 | C11orf31 | chromosome 11 open reading frame 31 |
| 3.7 | 224607_s_at | 6730 | SRP68 | signal recognition particle 68kDa |
| 3.7 | 224479_s_at | 84311 | MRPL45 | mitochondrial ribosomal protein L45 /// mitochondrial ribosomal protein L45 |
| 3.7 | 224312_x_at | 54973 | FLJ20542 | related to CPSF subunits 68 kDa |
| 3.7 | 225729_at | 221477 | C6orf89 | Chromosome 6 open reading frame 89 |
| 3.7 | 224366_s_at | 85021 | REPS1 | RALBP1 associated Eps domain containing /// RALBP1 associated Eps domain containing 1 |
| 3.7 | 243606 at | --- | --- | Transcribed locus, weakly similar to XP_510104.1 similar to hypothetical protein FLJ25224 [Pan troglodytes] |
| 3.7 | 228744 at | --- | --- | --- |
| 3.7 | 223230_at | 84950 | FLJ14936 | hypothetical protein FLJ14936 |
| 3.7 | 224447_s_at | 84299 | C17 or f37 | chromosome 17 open reading frame 37 /// chromosome 17 open reading frame 37 |
| 3.7 | 222832_s_at | 56947 | C2orf33 | chromosome 2 open reading frame 33 |
| 3.7 | 226236_at | 388789 | LOC388789 | hypothetical gene supported by AF147354 |
| 3.7 | 227291_s_at | 388962 | LOC388962 | similar to RIKEN 1810056020 |
| 3.7 | 225201_s_at | 64928 | MRPL14 | mitochondrial ribosomal protein L14 |
| 3.7 | 226024_at | 150684 | COMMD1 | copper metabolism (Murr1) domain containing |
| 3.7 | 228332_s_at | 280636 | Cllorf31 | 1 chromosome 11 open reading frame 31 |
| 3.7 | 226453_at | 84153 | AYP1 | AYP1 protein |
| 3.7 | 224331_s_at | 64979 | MRPL36 | mitochondrial ribosomal protein L36 /// mitochondrial ribosomal protein L36 |
| 3.7 | 224302_s_at | 92259 | MRPS36 | mitochondrial ribosomal protein S36 |
| 3.7 | 222606_at | --- | --- | --- |
| 3.7 | 225684_at | --- | --- | family with sequence similarity 33, member A |
| 3.7 | 200043_at | 2079 | ERH | enhancer of rudimentary homolog (Drosophila) /// enhancer of rudimentary homolog (Drosophila) |
| 3.7 | 223334_at | 84233 | DKFZp586C1924 | hypothetical protein DKFZp586C1924 |
| 3.7 | 225676_s_at | 25879 | DKFZP564O0463 | gene model 83 |
| 3.7 | 223156_at | 51649 | MRPS23 | mitochondrial ribosomal protein S23 |
| 3.7 | 233746_x_at | 25764 | HYPK | small EDRK-rich factor 2 /// Huntingtin interacting protein K |
| 3.7 | 223436_s_at | 83707 | MGC11134 | tRNA splicing 2' phosphotransferase 1 |
| 3.7 | 224679_at | 23184 | MESDC2 | mesoderm development candidate 2 |
| 3.7 | 231995_at | 79886 | C9orf82 | chromosome 9 open reading frame 82 |
| 3.7 | 228763_at | 145553 | MGC5987 | chromatin modifying protein 4A /// hypothetical protein MGC5987 |
| 3.7 | 225297_at | 115106 | CCDC5 | coiled-coil domain containing 5 (spindle associated) |
| 3.7 | 225865_x_at | 51497 | TH1L | TH1-like (Drosophila) |
| 3.7 | 226445_s_at | 90933 | TRIM41 | tripartite motif-containing 41 |
| 3.7 | 226780_s_at | 154791 | HSPC268 | hypothetical protein HSPC268 |
| 3.7 | 223191_at | 51241 | C14orf112 | chromosome 14 open reading frame 112 |
| 3.7 | 226896_at | 118487 | CHCHD1 | coiled-coil-helix-coiled-coil-helix domain containing 1 |
| 3.7 | 226007_at | 122961 | HBLD1 | HESB like domain containing 1 |
| 3.7 | 227042_at | 150223 | LOC150223 | hypothetical protein LOC150223 |
| 3.7 | 223040_at | 51126 | NAT5 | N-acetyltransferase 5 (ARD1 homolog, S. cerevisiae) |
| 3.7 | 223105_s_at | 51522 /// 81853 | TMEM14C /// TMEM14B | transmembrane protein 14C /// transmembrane protein 14B |
| 3.7 | 222530_s_at | 8195 | MKKS | McKusick-Kaufman syndrome |
| 3.7 | 221708_s_at | 55898 | SMAP-1 | smooth muscle cell associated protein-1 /// smooth muscle cell associated protein-1 |
| 3.7 | 230326_s_at | 51501 | HSPC138 | hypothetical protein HSPC138 |
| 3.7 | 223244_s_at | 55967 | DAP13 | 13kDa differentiation-associated protein |
| 3.7 | 232510_s_at | 10072 | DPP3 | dipeptidylpeptidase 3 |
| 3.7 | 229884_s_at | 51069 | MRPL2 | mitochondrial ribosomal protein L2 |
| 3.7 | 228019_s_at | 51023 | MRPS18C | mitochondrial ribosomal protein S18C |
| 3.7 | 234672_s_at | 55706 | FLJ10407 | hypothetical protein FLJ10407 |
| 3.7 | 225427_s_at | 128240 | APOA1BP | apolipoprotein A-I binding protein |
| 3.7 | 227442_at | 285521 | FLJ38991 | hypothetical protein FLJ38991 |
| 3.7 | 223151_at | 84259 | MGC2714 | hypothetical protein MGC2714 |
| 3.7 | 226091_s_at | 93621 | PGR1 | Mof4 family associated protein 1 |
| 3.7 | 225359_at | 131118 | TIM14 | homolog of yeast TIM14 |
| 3.7 | 226200_at | 57176 | VARS2L | valyl-tRNA synthetase 2-like |
| 3.7 | 224781_s_at | 84991 | RBM17 | RNA binding motif protein 17 |
| 3.7 | 226296_s_at | 64960 | MRPS15 | mitochondrial ribosomal protein S15 |
| 3.7 | 224972_at | 140823 | C20orf52 | chromosome 20 open reading frame 52 |
| 3.7 | 226620_x_at | 26528 | DAZAP1 | DAZ associated protein 1 |
| 3.7 | 227063_at | 254863 | MGC40107 | hypothetical protein MGC40107 |
| 3.7 | 226845_s_at | 150678 | LOC150678 | helicase/primase complex protein |
| 3.7 | 232032_x_at | 51150 | Cab45 | calcium binding protein Cab45 precursor |
| 3.7 | 225772_s_at | 84987 | MGC14288 | hypothetical protein MGC14288 |
| 3.7 | 226165_at | 401466 | LOC401466 | hypothetical gene supported by BC055092 |
| 3.7 | 228690_s_at | 126328 | NDUFA11 | NADH dehydrogenase (ubiquinone) 1 alpha subcomplex, 11, 14.7kDa |
| 3.7 | 200053_at | 9552 | SPAG7 | sperm associated antigen 7 /// sperm associated antigen 7 |
| 3.7 | 224680_at | 222068 | TMED4 | transmembrane emp24 protein transport domain containing 4 |
| 3.7 | 223445_at | 84062 | DTNBP1 | dystrobrevin binding protein 1 |
| 3.7 | 225398_at | 84881 | RPUSD4 | RNA pseudouridylate synthase domain containing 4 |
| 3.7 | 233625_x_at | 54973 | FLJ20542 | related to CPSF subunits 68 kDa |
| 3.7 | 225053_at | 29883 | CNOT7 | CCR4-NOT transcription complex, subunit 7 |
| 3.7 | 227322_s_at | 56647 | BCCIP | BRCA2 and CDKN1A interacting protein |
| 3.7 | 224664_at | 119504 | C10orf104 | chromosome 10 open reading frame 104 |
| 3.7 | 223294_at | 51260 | CXorf26 | chromosome X open reading frame 26 |
| 3.7 | 203262_s_at | 9130 | FAM50A | family with sequence similarity 50, member A |
| 3.7 | 224513_s_at | 56893 | UBQLN4 | ubiquilin 4 /// ubiquilin 4 |
| 3.7 | 226017_at | 112616 | CKLFSF7 | chemokine-like factor super family 7 |
| 3.7 | 218961_s_at | 11284 | PNKP | polynucleotide kinase 3'-phosphatase |
| 3.7 | 222403_at | 23788 | MTCH2 | mitochondrial carrier homolog 2 (C. elegans) |
| 3.7 | 206790_s_at | 4707 | NDUFB1 | NADH dehydrogenase (ubiquinone) 1 beta subcomplex, 1, 7kDa |
| 3.7 | 223892_s_at | 51643 | CGI-119 | CGI-119 protein |
| 3.7 | 228647_at | --- | --- | Homo sapiens, clone IMAGE:3621928, mRNA, partial cds |
| 3.7 | 235207_at | --- | --- | --- |
| 3.7 | 226074_at | 132160 | FLJ32332 | likely ortholog of mouse protein phosphatase 2C eta |
| 3.7 | 228622_s_at | 3338 | DNAJC4 | DnaJ (Hsp40) homolog, subfamily C, member |
| 3.7 | 224849_at | 55761 | TTC17 | tetratricopeptide repeat domain 17 |
| 3.7 | 213527_s_at | 146542 | LOC146542 | similar to hypothetical protein MGC13138 |
| 3.7 | 233350_s_at | 51368 | TEX264 | testis expressed sequence 264 |
| 3.7 | 233563_s_at | 54973 | FLJ20542 | related to CPSF subunits 68 kDa |
| 3.7 | 223322_at | 83593 | RASSF5 | Ras association (RalGDS/AF-6) domain family 5 |
| 3.7 | 225317_at | 84320 | ACBD6 | acyl-Coenzyme A binding domain containing 6 |
| 3.7 | 223516_s_at | 29964 | C6orf49 | chromosome 6 open reading frame 49 |
| 3.7 | 225541_at | 200916 | LOC200916 | similar to ribosomal protein L22 |
| 3.7 | 225797_at | 116541 | MRPL54 | mitochondrial ribosomal protein L54 |
| 3.7 | 225244_at | 116841 | IMAGE3451454 | SVAP1 protein |
| 3.7 | 225749_at | 283951 | LOC283951 | hypothetical protein LOC283951 |
| 3.7 | 222440_s_at | 9967 | THRAP3 | Thyroid hormone receptor associated protein 3 |
| 3.7 | 224671_at | 124995 | MRPL10 | mitochondrial ribosomal protein L10 |
| 3.7 | 223479_s_at | 84269 | CHCHD5 | coiled-coil-helix-coiled-coil-helix domain containing 5 |
| 3.7 | 223324_s_at | 54822 | TRPM7 | transient receptor potential cation channel, subfamily M, member 7 |
| 3.7 | 227837_at | 54891 | FLJ20309 | Hypothetical protein FLJ20309 |
| 3.7 | 234302_s_at | 54890 | FLJ20308 | hypothetical protein FLJ20308 |
| 3.7 | 228301_x_at | 4716 | NDUFB10 | NADH dehydrogenase (ubiquinone) 1 beta subcomplex, 10, 22kDa |
| 3.7 | 225148_at | 91582 | MGC52010 | hypothetical protein MGC52010 |
| 3.7 | 222398_s_at | 9343 | U5-116KD | U5 snRNP-specific protein, 116 kD |
| 3.7 | 225413_at | 84833 | USMG5 | upregulated during skeletal muscle growth 5 |
| 3.7 | 223207_x_at | 29085 | PHPT1 | phosphohistidine phosphatase 1 |
| 3.7 | 224993_at | 56930 | LOC56930 | hypothetical protein from EUROIMAGE 1669387 |
| 3.7 | 224575_at | 55845 | C3orf10 | chromosome 3 open reading frame 10 |
| 3.7 | 228053_s_at | 401505 | C9orf105 | chromosome 9 open reading frame 105 |
| 3.7 | 226006_at | --- | --- | --- |
| 3.7 | 225209_s_at | 118424 | UBE2J2 | ubiquitin-conjugating enzyme E2, J2 (UBC6 homolog, yeast) |
| 3.7 | 226976_at | 23633 | KPNA6 | Karyopherin alpha 6 (importin alpha 7) |
| 3.7 | 226011_at | 151903 | CCDC12 | coiled-coil domain containing 12 |
| 3.7 | 225515_s_at | 220906 | LOC220906 | hypothetical protein LOC220906 |
| 3.7 | 222684_s_at | 79954 | FLJ14075 | hypothetical LOC79954 |
| 3.7 | 223480_s_at | 57129 | MRPL47 | mitochondrial ribosomal protein L47 |
| 3.7 | 224413_s_at | 83877 | BLP1 | BBP-like protein 1 /// BBP-like protein 1 |
| 3.7 | 223234_at | 10459 | MAD2L2 | MAD2 mitotic arrest deficient-like 2 (yeast) |
| 3.7 | 222443_s_at | 9939 | RBM8A | RNA binding motif protein 8A |
| 3.7 | 226012_at | 29123 | ANKRD11 | ankyrin repeat domain 11 |
| 3.7 | 225143_at | 119559 | SFXN4 | sideroflexin 4 |
| 3.7 | 225591_at | 26260 | FBXO25 | F-box protein 25 |
| 3.7 | 225866_at | 84154 | BXDC1 | brix domain containing 1 |
| 3.7 | 225534_at | 114926 | LOC114926 | hypothetical protein BC013035 |
| 3.7 | 228355_s_at | 91942 | LOC91942 | Myc-induced mitochondria protein |
| 3.7 | 230449_x_at | --- | --- | Transcribed locus, weakly similar to XP_519878.1 similar to ubiquitin-conjugating enzyme E2 variant 1 isoform c; DNA-binding protein [Pan troglodytes] |
| 3.7 | 203576_at | 587 | BCAT2 | branched chain aminotransferase 2, mitochondrial |
| 3.7 | 228641_at | 22900 | CARD8 | Caspase recruitment domain family, member 8 |
| 3.7 | 224345_x_at | 26355 | E2IG5 | growth and transformation-dependent protein /// growth and transformation-dependent protein |
| 3.7 | 208739_x_at | 6613 | SUMO2 | SMT3 suppressor of mif two 3 homolog 2 (yeast) |
| 3.7 | 208985_s_at | 8669 | EIF3S1 | eukaryotic translation initiation factor 3, subunit 1 alpha, 35kDa |
| 3.7 | 210149_s_at | 10476 | ATP5H | ATP synthase, H+ transporting, mitochondrial F0 complex, subunit d |
| 3.7 | 221434_s_at | 81892 | C14orf156 | chromosome 14 open reading frame 156 /// chromosome 14 open reading frame 156 |
| 3.7 | 218830_at | 51121 | RPL26L1 | ribosomal protein L26-like 1 |
| 3.7 | 226642_s_at | 134492 | NUDCD2 | Nudc domain containing 2 |
| 3.7 | 200978_at | 4190 | MDH1 | malate dehydrogenase 1, NAD (soluble) |
| 3.7 | 209796_s_at | 10330 | TMEM4 | transmembrane protein 4 |
| 3.7 | 203401_at | 5634 | PRPS2 | phosphoribosyl pyrophosphate synthetase 2 |
| 3.7 | 218970_s_at | 51076 | CUTC | cutC copper transporter homolog (E.coli) |
| 3.7 | 218281_at | 51642 | MRPL48 | mitochondrial ribosomal protein L48 |
| 3.7 | 223743_s_at | 51073 | MRPL4 | mitochondrial ribosomal protein L4 |
| 3.7 | 218058_at | 30827 | CXXC1 | CXXC finger 1 (PHD domain) |
| 3.7 | 224637_at | 400948 | LOC400948 | similar to RIKEN CDNA 2310016E02 |
| 3.7 | 227186_s_at | 64975 | MRPL41 | mitochondrial ribosomal protein L41 |
| 3.7 | 225903_at | 128869 | CDC91L1 | CDC91 cell division cycle 91-like 1 (S. cerevisiae) |
| 3.7 | 204868_at | 3396 | ICT1 | immature colon carcinoma transcript 1 |
| 3.7 | 201241_at | 1653 | DDX1 | DEAD (Asp-Glu-Ala-Asp) box polypeptide 1 |
| 3.7 | 223180_s_at | --- | --- | --- |
| 3.7 | 203517_at | 10651 | MTX2 | metaxin 2 |
| 3.7 | 225394_s_at | 85437 | MADP-1 | MADP-1 protein |
| 3.7 | 223037_at | 51248 | PDZK11 | PDZ domain containing 11 |
| 3.7 | 201274_at | 5686 | PSMA5 | proteasome (prosome, macropain) subunit, alpha type, 5 |
| 3.7 | 203252_at | 10263 | DOC-1R | CDK2-associated protein 2 |
| 3.7 | 201316_at | 5683 | PSMA2 | proteasome (prosome, macropain) subunit, alpha type, 2 |
| 3.7 | 202520_s_at | 4292 | MLH1 | mutL homolog 1, colon cancer, nonpolyposis type 2 (E. coli) |
| 3.7 | 218597_s_at | 55847 | C10orf70 | chromosome 10 open reading frame 70 |
| 3.7 | 201966_at | 4720 | NDUFS2 | NADH dehydrogenase (ubiquinone) Fe-S protein 2, 49kDa (NADH-coenzyme Q |
| 3.7 | 222416_at | 5832 | ALDH18A1 | aldehyde dehydrogenase 18 family, member |
| 3.7 | 225686_at | 348235 | FAM33A | family with sequence similarity 33, member A |
| 3.7 | 202691_at | 6632 | SNRPD1 | small nuclear ribonucleoprotein D1 polypeptide 16kDa |
| 3.7 | 204205_at | 60489 | APOBEC3G | apolipoprotein B mRNA editing enzyme, catalytic polypeptide-like 3G |
| 3.7 | 222763 s at | 55339 | WDR33 | WD repeat domain 33 |
| 3.7 | 205644 s at | 6637 | SNRPG | small nuclear ribonucleoprotein polypeptide G |
| 3.7 | 209507_at | 6119 | RPA3 | replication protein A3, 14kDa |
| 3.7 | 215691 x at | 51668 | Clorf41 | chromosome 1 open reading frame 41 |
| 3.7 | 203613_s_at | 4712 | NDUFB6 | NADH dehydrogenase (ubiquinone) 1 beta subcomplex, 6, 17kDa |
| 3.7 | 218027_at | 29088 | MRPL15 | mitochondrial ribosomal protein L15 |
| 3.7 | 224655_at | 50808 | AK3L1 | adenylate kinase 3 |
| 3.7 | 211070_x_at | 1622 | DBI | diazepam binding inhibitor (GABA receptor modulator, acyl-Coenzyme A binding protein) /// diazepam binding inhibitor (GABA receptor modulator, acyl-Coenzyme A binding protein) |
| 3.7 | 224415_s_at | 84681 | HINT2 | histidine triad nucleotide binding protein 2 /// histidine triad nucleotide binding protein 2 |
| 3.7 | 222551_s_at | 65265 | FLJ20989 | hypothetical protein FLJ20989 |
| 3.7 | 223113_at | 51524 | HSPC196 | hypothetical protein HSPC196 |
| 3.7 | 228164_at | 23431 | AP4E1 | Transcribed locus |
| 3.7 | 223071_at | 51124 | IER3IP1 | immediate early response 3 interacting protein 1 |
| 3.7 | 208799_at | 5693 | PSMB5 | proteasome (prosome, macropain) subunit, beta type, 5 |
| 3.7 | 223329_x_at | 10910 | SUGT1 | SGT1, suppressor of G2 allele of SKP1 (S. cerevisiae) |
| 3.7 | 224809_x_at at | --- | --- | --- |
| 3.7 | 226238_at | 84693 | MCEE | methylmalonyl CoA epimerase |
| 3.7 | 225925_s_at | 84196 | USP48 | ubiquitin specific protease 48 |
| 3.7 | 224869_s_at | 64432 | MRPS25 | mitochondrial ribosomal protein S25 |
| 3.7 | 200793_s_at | 50 | ACO2 | aconitase 2, mitochondrial |
| 3.7 | 219819_s_at | 28957 | MRPS28 | mitochondrial ribosomal protein S28 |
| 3.7 | 228614_at | 205251 | LOC205251 | hypothetical protein LOC205251 |
| 3.7 | 225253_s_at | 339175 /// 55798 | METTL2 /// FLJ12760 | methyltransferase like 2 /// hypothetical protein FLJ12760 |
| 3.7 | 225695_at | 54978 | C2orf18 | chromosome 2 open reading frame 18 |
| 3.7 | 226466_s_at | 92002 | MGC29729 | hypothetical protein MGC29729 |
| 3.7 | 222701_s_at | 79145 | CHCHD7 | coiled-coil-helix-coiled-coil-helix domain containing 7 |
| 3.7 | 221622_s_at | 55863 | HT007 | uncharacterized hypothalamus protein HT007 |
| 3.7 | 235689_at | 123263 | MTFMT | methionyl-tRNA formyltransferase, mitochondrial |
| 3.7 | 224448_s_at | 84300 | C6orf125 | chromosome 6 open reading frame 125 /// chromosome 6 open reading frame 125 |
| 3.7 | 233588_x_at | 10471 | HKE2 | HLA class II region expressed gene KE2 |
| 3.7 | 222592_s_at | 51703 | ACSL5 | acyl-CoA synthetase long-chain family member 5 |
| 3.7 | 229025_s_at | 196294 | FLJ25059 | hypothetical protein FLJ25059 |
| 3.7 | 224472_x_at | 51150 | Cab45 | calcium binding protein Cab45 precursor /// calcium binding protein Cab45 precursor |
| 3.7 | 229018_at | 84190 | FLJ22789 | hypothetical protein FLJ22789 |
| 3.7 | 224452_s_at | 84792 | MGC12966 | hypothetical protein MGC12966 /// hypothetical protein MGC12966 |
| 3.7 | 222460_s_at | 80011 | NIP30 | NEFA-interacting nuclear protein NIP30 |
| 3.7 | 223273_at | 84520 | C14orf142 | chromosome 14 open reading frame 142 |
| 3.7 | 212954_at | 8798 | DYRK4 | dual-specificity tyrosine-(Y)-phosphorylation regulated kinase 4 |
| 3.7 | 226536_at | 286053 | FLJ32440 | hypothetical protein FLJ32440 |
| 3.7 | 226146_at | 255458 | LOC255458 | Hypothetical protein LOC255458 |
| 3.7 | 222487_s_at | --- | --- | ribosomal protein S27-like |
| 3.7 | 218007_s_at | 51065 | RPS27L | ribosomal protein S27-like |
| 3.7 | 225395_s_at | 158293 | C9orf10OS | chromosome 9 open reading frame 10 opposite strand |
| 3.7 | 228970_at | 339487 | ARCH | zinc finger and BTB domain containing 8 opposite strand |
| 3.7 | 224435_at | 84293 | C10orf58 | chromosome 10 open reading frame 58 /// chromosome 10 open reading frame 58 |
| 3.7 | 226161_at | --- | --- | solute carrier family 30 (zinc transporter), member 6 |
| 3.7 | 222405_at | --- | --- | --- |
| 3.7 | 229640_x_at | 440128 | --- | Similar to hypothetical protein, MGC:7199 |
| 3.7 | 225514_at | --- | --- | --- |
| 3.7 | 224740_at | 441075 | --- | LOC441075 |
| 3.7 | 226385_s_at | 115416 | C7orf30 | chromosome 7 open reading frame 30 |
| 3.7 | 225621_at | 85365 | ALG2 | asparagine-linked glycosylation 2 homolog (yeast, alpha-1,3-mannosyltransferase) |
| 3.7 | 223819_x_at | 28991 | COMMD5 | COMM domain containing 5 |
| 3.7 | 57532_at | 1856 | DVL2 | dishevelled, dsh homolog 2 (Drosophila) |
| 3.7 | 200968_s_at | 5479 | PPIB | peptidylprolyl isomerase B (cyclophilin B) |
| 3.7 | 209899_s_at | 22827 | SIAHBP1 | fuse-binding protein-interacting repressor |
| 3.7 | 214096_s_at | 6472 | SHMT2 | serine hydroxymethyltransferase 2 (mitochondrial) |
| 3.7 | 201611_s_at | 23463 | ICMT | isoprenylcysteine carboxyl methyltransferase |
| 3.7 | 218288_s_at | 60492 | MDS025 | hypothetical protein MDS025 |
| 3.7 | 225969_at | 84964 | MGC15677 | hypothetical protein MGC15677 |
| 3.7 | 228183_s_at | 84268 | RIP | RPA interacting protein |
| 3.7 | 223205_s_at | 83746 | L3MBTL2 | 1(3)mbt-like 2 (Drosophila) |
| 3.7 | 200057_s_at | 4841 | NONO | non-POU domain containing, octamer-binding /// non-POU domain containing, octamer-binding |
| 3.7 | 200060_s_at | 10921 | RNPS1 | RNA binding protein S1, serine-rich domain /// RNA binding protein S1, serine-rich domain |
| 3.7 | 224910_at | 23589 | CARHSP1 | calcium regulated heat stable protein 1, 24kDa |
| 3.7 | 224948_at | 64951 | MRPS24 | mitochondrial ribosomal protein S24 |
| 3.7 | 200066_at | 3550 | IK | IK cytokine, down-regulator of HLA II /// IK cytokine, down-regulator of HLA II |
| 3.7 | 222978_at | 6836 | SURF4 | surfeit 4 |
| 3.7 | 200045_at | 23 | ABCF1 | ATP-binding cassette, sub-family F (GCN20), member 1 /// ATP-binding cassette, sub-family F (GCN20), member 1 |
| 3.7 | 209040_s_at | 5696 | PSMB8 | proteasome (prosome, macropain) subunit, beta type, 8 (large multifunctional protease 7) |
| 3.7 | 225967_s_at | 284184 | LOC284184 | hypothetical LOC284184 |
| 3.7 | 223133_at | 81853 | TMEM14B | transmembrane protein 14B |
| 3.7 | 225006_x_at | 51497 | TH1L | TH1-like (Drosophila) |
| 3.7 | 223964_x_at | --- | --- | --- |
| 3.7 | 226241_s_at | 122704 | MRPL52 | mitochondrial ribosomal protein L52 |
| 3.7 | 232075_at | 80349 | REC14 | Recombination protein REC14 |
| 3.7 | 225304_s_at | 126328 | NDUFA11 | NADH dehydrogenase (ubiquinone) 1 alpha subcomplex, 11, 14.7kDa |
| 3.7 | 200076_s_at | 79036 | MGC2749 | hypothetical protein MGc2749 /// hypothetical protein MGC2749 |
| 3.7 | 210418_s_at | 3420 | IDH3B | isocitrate dehydrogenase 3 (NAD+) beta |
| 3.7 | 202758_s_at | 8625 | RFXANK | regulatory factor X-associated ankyrin-containing protein |
| 3.7 | 225607_at | 124808 | FLJ31795 | hypothetical protein FLJ31795 |
| 3.7 | 224695_at | 55571 | C2orf29 | chromosome 2 open reading frame 29 |
| 3.7 | 219220_x_at | 56945 | MRPS22 | mitochondrial ribosomal protein S22 |
| 3.7 | 226386_at | 115416 | C7orf30 | chromosome 7 open reading frame 30 |
| 3.7 | 223526_at | --- | --- | chromosome 18 open reading frame 21 |
| 3.7 | 209165_at | 26574 | AATF | apoptosis antagonizing transcription factor |
| 3.7 | 227249_at | 4629 | MYH11 | Myosin, heavy polypeptide 11, smooth muscle |
| 3.7 | 225298_at | 25953 | MR-1 | myofibrillogenesis regulator 1 |
| 3.7 | 223010_s_at | 54940 | OCIAD1 | OCIA domain containing 1 |
| 3.7 | 225808_at | 124512 | LOC124512 | Hypothetical protein LOC124512 |
| 3.7 | 225052_at | 94107 | MGC14327 | hypothetical protein MGC14327 |
| 3.7 | 228149_at | --- | --- | hypothetical protein FLJ31818 |
| 3.7 | 223003_at | 79002 | MGC2803 | hypothetical protein MGC2803 |
| 3.7 | 226330_s_at | 55578 | FAM48A | family with sequence similarity 48, member A |
| 3.7 | 223011_s_at | 54940 | OCIAD1 | OCIA domain containing 1 |
| 3.7 | 229980_s_at | 27131 | SNX5 | sorting nexin 5 |
| 3.7 | 235158_at | 84928 | FLJ14803 | hypothetical protein FLJ14803 |
| 3.7 | 223411_at | 57409 | AD023 | AD023 protein |
| 3.7 | 225092_at | 9135 | RABEP1 | rabaptin, RAB GTPase binding effector protein 1 |
| 3.7 | 227625_s_at | 10273 | STUB1 | STIP1 homology and U-box containing protein 1 |
| 3.7 | 224665_at | 119504 | C10orf104 | chromosome 10 open reading frame 104 |
| 3.7 | 224666_at | 197370 | NSMCE1 | non-SMC element 1 homolog (S. cerevisiae) |
| 3.7 | 233049_x_at | 10273 | STUB1 | STIP1 homology and U-box containing protein 1 |
| 3.7 | 224729_s_at | 64756 | ATPAF1 | ATP synthase mitochondrial F1 complex assembly factor 1 |
| 3.7 | 225179_at | 3093 | HIP2 | Huntingtin interacting protein 2 |
| 3.7 | 225588_s_at | 92305 | LOC92305 | hypothetical protein BC009331 |
| 3.7 | 225586_at | 92715 | C9orf112 | chromosome 9 open reading frame 112 |
| 3.7 | 220788_s_at | 55072 | RNF31 | ring finger protein 31 |
| 3.7 | 200000_s_at | 10594 | PRPF8 | PRP8 pre-mRNA processing factor 8 homolog (yeast) /// PRP8 pre-mRNA processing factor 8 homolog (yeast) |
| 3.7 | 223164_at | 83605 | CCM2 | cerebral cavernous malformation 2 |
| 3.7 | 232515_at | --- | --- | --- |
| 3.7 | 224932_at | 400916 | C22orf16 | chromosome 22 open reading frame 16 |
| 3.7 | 238518_x_at | 132158 | GLYCTK | CG9886-like |
| 3.7 | 224477_s_at | 84309 | NUDT16L1 | nudix (nucleoside diphosphate linked moiety X)-type motif 16-like 1 /// nudix (nucleoside diphosphate linked moiety X)-type motif 16-like 1 |
| 3.7 | 233929_x_at | 374666 | FLJ25222 | CXYorf1-related protein |
| 3.7 | 226659_at | 50619 | DEF6 | differentially expressed In FDCP 6 homolog (mouse) |
| 3.7 | 233132_at | 96610 | LOC96610 | Hypothetical protein similar to KIAA0187 gene product |
| 3.7 | 233540_s_at | 55755 | CDK5RAP2 | CDK5 regulatory subunit associated protein 2 |
| 3.7 | 218305_at | --- | --- | --- |
| 3.7 | 228521_s_at | 53916 | RAB4B | RAB4B, member RAS oncogene family |
| 3.7 | 223206_s_at | 57407 | HSCARG | HSCARG protein |
| 3.7 | 224298_s_at | 337867 | PHGDHL1 | phosphoglycerate dehydrogenase like 1 |
| 3.8 | 236165_at | 10943 | MSL3L1 | --- |
| 3.8 | 209007_s_at | 57035 | NPD014 | NPD014 protein |
| 3.8 | 243729_at | --- | --- | CDNA FLJ37931 fis, clone CTONG2004397 |
| 3.8 | 228023_x_at | 280 | AMY2B | --- |
| 3.8 | 205584_at | 79868 | CXorf45 | chromosome X open reading frame 45 |
| 3.8 | 225098_at | 10152 | ABI2 | Abl interactor 2 |
| 3.8 | 220349_s_at | 64772 | FLJ21865 | endo-beta-N-acetylglucosaminidase |
| 3.8 | 203117_s_at | 9924 | USP52 | ubiquitin specific protease 52 |
| 3.8 | 226316_at | --- | --- | CDNA clone IMAGE:5295896, partial cds |
| 3.8 | 220044_x_at | 51747 | CROP | cisplatin resistance-associated overexpressed protein |
| 3.8 | 214779_s_at | 27352 | RUTBC3 | RUN and TBC1 domain containing 3 |
| 3.8 | 216305_s_at | 6936 | C2orf3 | chromosome 2 open reading frame 3 |
| 3.8 | 226934_at | 11052 | CPSF6 | Cleavage and polyadenylation specific factor 6, 68kDa |
| 3.8 | 229425_at | --- | --- | Transcribed locus |
| 3.8 | 226665_at | 130872 | AHSA2 | AHA1, activator of heat shock 90kDa protein ATPase homolog 2 (yeast) |
| 3.8 | 209240_at | 8473 | OGT | O-linked N-acetylglucosamine (GlcNAc) transferase (UDP-N-acetylglucosamine:polypeptide-N-acetylglucosaminyl transferase) |
| 3.8 | 226015_at | 7559 | ZNF12 | zinc finger protein 12 (KOX 3) |
| 3.8 | 213653_at | 56339 | METTL3 | methyltransferase like 3 |
| 3.8 | 202220_at | 22889 | KIAA0907 | KIAA0907 protein |
| 3.8 | 212179_at | 25957 | C6orf111 | chromosome 6 open reading frame 111 |
| 3.8 | 206828_at | 7294 | TXK | TXK tyrosine kinase |
| 3.8 | 230408_at | --- | --- | Polycomb group ring finger 3 |
| 3.8 | 213213_at | 11083 | DATF1 | death associated transcription factor 1 |
| 3.8 | 221965_at | 10198 | MPHOSPH9 | M-phase phosphoprotein 9 |
| 3.8 | 218366_x_at | 64745 | FLJ20859 | FLJ20859 gene |
| 3.8 | 213460_x_at | 260294 | WBSCR20C | Williams Beuren syndrome chromosome region 20C |
| 3.8 | 213670_x_at | 155400 /// 260294 /// 55695 | NSUN5 /// WBSCR20B /// WBSCR20C | Williams-Beuren Syndrome critical region protein 20 copy B |
| 3.8 | 214100_x_at | 155400 /// 260294 /// 55695 | NSUN5 /// WBSCR20B /// WBSCR20C | Williams-Beuren Syndrome critical region protein 20 copy B |
| 3.8 | 213326_at | 6843 | VAMP1 | vesicle-associated membrane protein 1 (synaptobrevin 1) |
| 3.8 | 215667_x_at | 442589 /// 442593 /// 5379 /// 5380 5383 | PM52L1 /// /// PM52L2 /// PMS2LS /// LOC442589 /// LOC442593 | --- |
| 3.8 | 217895_at | 55037 | FLJ20758 | FLJ20758 protein |
| 3.8 | 205047_s_at | 440 | ASNS | asparagine synthetase |
| 3.8 | 209271_at | 2186 | FALZ | fetal Alzheimer antigen |
| 3.8 | 213186_at | 9666 | DZIP3 | zinc finger DAZ interacting protein 3 |
| 3.8 | 217902_s_at | 8924 | HERC2 | hect domain and RLD 2 |
| 3.8 | 208442_s_at | 472 | ATM | ataxia telangiectasia mutated (includes complementation groups A, C and D) |
| 3.8 | 214686_at | 10781 | ZNF266 | zinc finger protein 266 |
| 3.8 | 212980_at | 130872 | AHSA2 | Ubiquitin specific protease 34 |
| 3.8 | 221626_at | 91115 | ZNF506 | zinc finger protein 506 |
| 3.8 | 201837_s_at | 9913 | STAF65(gamma) | SPTF-associated factor 65 gamma |
| 3.8 | 243016_at | 55556 | HSRTSBETA | Thymidylate synthetase |
| 3.8 | 226344_at | 84460 | ZMAT1 | zinc finger, matrin type 1 |
| 3.8 | 221645_s_at | 55769 | ZNF83 | zinc finger protein 83 (HPF1) |
| 3.8 | 226848_at | 7182 | NR2C2 | Nuclear receptor subfamily 2, group C, member 2 |
| 3.8 | 238252_at | 11101 | ATE1 | Arginyltransferase 1 |
| 3.8 | 212454_x_at | 9987 | HNRPDL | Heterogeneous nuclear ribonucleoprotein D-like |
| 3.8 | 208206_s_at | 10235 | RASGRP2 | RAS guanyl releasing protein 2 (calcium and DAG-regulated) |
| 3.8 | 212557_at | 26036 | ZNF451 | zinc finger protein 451 |
| 3.8 | 208498_s_at | 279 | AMY2A | amylase, alpha 1A; salivary /// amylase, alpha 1B; salivary /// amylase, alpha 1C ; salivary /// amylase, alpha 2A; pancreatic /// amylase, alpha 2B; pancreatic |
| 3.8 | 213672_at | 4141 | MARS | Methionine-tRNA synthetase |
| 3.8 | 235432_at | 27031 | NPHP3 | nephronophthisis 3 (adolescent) |
| 3.8 | 203944_x_at | 11120 | BTN2A1 | butyrophilin, subfamily 2, member A1 |
| 3.8 | 32259_at | 2145 | EZH1 | enhancer of zeste homolog 1 (Drosophila) |
| 3.8 | 212176_at | 25957 | C6orf111 | chromosome 6 open reading frame 111 |
| 3.8 | 209390_at | 7248 | TSC1 | tuberous sclerosis 1 |
| 3.8 | 203164_at | 9197 | SLC33A1 | solute carrier family 33 (acetyl-CoA transporter), member 1 |
| 3.8 | 228331_at | 280636 | C11orf31 | chromosome 11 open reading frame 31 |
| 3.8 | 212944_at | 64968 | MRPS6 | Mitochondrial ribosomal protein S6 |
| 3.8 | 213322_at | 221443 | C6orf130 | chromosome 6 open reading frame 130 |
| 3.8 | 225786_at | 116228 | FAM36A | Family with sequence similarity 36, member A |
| 3.8 | 218476_at | 10585 | POMT1 | protein-O-mannosyltransferase 1 |
| 3.8 | 242389_at | 51747 | CROP | Cisplatin resistance-associated overexpressed protein |
| 3.8 | 202979_s_at | 58487 | ZF | HCF-binding transcription factor Zhangfei |
| 3.8 | 221834_at | 83752 | LONP | Peroxisomal lon protease |
| 3.8 | 227585_at | 84896 | ATAD1 | ATPase family, AAA domain containing 1 |
| 3.8 | 212232_at | 23360 | FNBP4 | formin binding protein 4 |
| 3.8 | 214093_s_at | 8880 | FUBP1 | Far upstream element (FUSE) binding protein 1 |
| 3.8 | 208798_x_at | 23015 | GOLGIN-67 | golgin-67 |
| 3.8 | 227504_s_at | 27146 | KIAA1276 | KIAA1276 protein |
| 3.8 | 231890_at | --- | --- | CDNA FLJ12742 fis, clone NT2RP2000644 |
| 3.8 | 213065_at | 196441 | MGC23401 | hypothetical protein MGC23401 |
| 3.8 | 212914_at | 23492 | CBX7 | chromobox homolog 7 |
| 3.8 | 202104_s_at | 6687 | SPG7 | spastic paraplegia 7, paraplegin (pure and complicated autosomal recessive) |
| 3.8 | 210113_s_at | 22861 | NALP1 | NACHT, leucine rich repeat and PYD (pyrin domain) containing 1 |
| 3.8 | 218456_at | 65981 | C1QDC1 | Clq domain containing 1 |
| 3.8 | 203249_at | 2145 | EZH1 | enhancer of zeste homolog 1 (Drosophila) |
| 3.8 | 213140_s_at | 26039 | SS18L1 | synovial sarcoma translocation gene on chromosome 18-like 1 |
| 3.8 | 205760_s_at | 4968 | OGG1 | 8-oxoguanine DNA glycosylase |
| 3.8 | 221971_x_at | 399761 | FLJ00312 | similar to centaurin, gamma-like family, member 1; ARF GTPase-activating protein; Em:AC012044.1 |
| 3.8 | 213474_at | 154881 | KCTD7 | Potassium channel tetramerisation domain containing 7 |
| 3.8 | 221833_at | 83752 | LONP | Peroxisomal lon protease |
| 3.8 | 229700_at | 148203 | LOC148203 | hypothetical protein LOC148203 |
| 3.8 | 203135_at | 6908 | TBP | TATA box binding protein |
| 3.8 | 228157_at | 7756 | ZNF207 | Zinc finger protein 207 |
| 3.8 | 213773_x_at | 55695 | NSUN5 | NOL1/NOP2/Sun domain family, member 5 |
| 3.8 | 223716_s_at | 9406 | ZNF265 | zinc finger protein 265 |
| 3.8 | 227485_at | 203522 | DDX26B | DEAD/H (Asp-Glu-Ala-Asp/His) box polypeptide 26B |
| 3.8 | 201394_s_at | 10181 | RBM5 | RNA binding motif protein 5 |
| 3.8 | 221740_x_at | 388397 | --- | C114 SLIT-like testicular protein /// Hypothetical LOC388397 |
| 3.8 | 218873_at | 54856 | FLJ20203 | hypothetical protein FLJ20203 |
| 3.8 | 219698_s_at | 64863 | METTL4 | methyltransferase like 4 |
| 3.8 | 234970_at | 123036 | MTAC2D1 | membrane targeting (tandem) C2 domain containing 1 |
| 3.8 | 220642_x_at | 51463 | GPR89 | G protein-coupled receptor 89 |
| 3.8 | 217122_s_at | 9906 | SLC35E2 | solute carrier family 35, member E2 |
| 3.8 | 224558_s_at | 378938 | MALAT1 | metastasis associated lung adenocarcinoma transcript 1 (non-coding RNA) |
| 3.8 | 220132_s_at | 29121 | CLEC2D | C-type lectin superfamily 2, member D |
| 3.8 | 219447_s_at | 51006 | SLC35C2 | solute carrier family 35, member C2 |
| 3.8 | 235484_at | 375743 | PTAR1 | protein prenyltransferase alpha subunit repeat containing 1 |
| 3.8 | 203578_s_at | 9057 | SLC7A6 | solute carrier family 7 (cationic amino acid transporter, y+ system), member 6 |
| 3.8 | 218557_at | 56954 | NIT2 | nitrilase family, member 2 |
| 3.8 | 206150_at | 939 | TNFRSF7 | tumor necrosis factor receptor superfamily, member 7 /// tumor necrosis factor receptor superfamily, member 7 |
| 3.8 | 219648_at | 55686 | DSU | likely ortholog of mouse dilute suppressor |
| 3.8 | 203551_s_at | 1353 | cox11 | COX11 homolog, cytochrome c oxidase assembly protein (yeast) |
| 3.8 | 43977_at | 54929 | FLJ20422 | hypothetical protein FLJ20422 |
| 3.8 | 229903_x_at | 55599 | RNP | RNA-binding region (RNP1, RRM) containing 3 |
| 3.8 | 226995_at | 257103 | C21orf86 | chromosome 21 open reading frame 86 |
| 3.8 | 239086_at | 414328 | C9orf103 | chromosome 9 open reading frame 103 |
| 3.8 | 225017_at | 64770 | CCDC14 | coiled-coil domain containing 14 |
| 3.8 | 230444_at | --- | --- | Transcribed locus, weakly similar to XP_510104.1 similar to hypothetical protein FLJ25224 [Pan troglodytes] |
| 3.8 | 232171_x_at | 54758 | KLHDC4 | kelch domain containing 4 |
| 3.8 | 228370_at | 6638 | SNRPN | SNRPN upstream reading frame |
| 3.8 | 222147_s_at | --- | --- | ARP5 actin-related protein 5 homolog (yeast) |
| 3.8 | 226508_at | 80012 | PHC3 | Polyhomeotic like 3 (Drosophila) |
| 3.8 | 235890_at | --- | --- | Transcribed locus |
| 3.8 | 210858_x_at | 472 | ATM | ataxia telangiectasia mutated (includes complementation groups A, C and D) |
| 3.8 | 218734_at | 79829 | FLJ13848 | hypothetical protein FLJ13848 |
| 3.8 | 218552_at | 55268 | ECHDC2 | enoyl Coenzyme A hydratase domain containing 2 |
| 3.8 | 218515_at | 94104 | C21orf66 | Chromosome 21 open reading frame 66 |
| 3.8 | 213505_s_at | 10147 | SFRS14 | splicing factor, arginine/serine-rich 14 |
| 3.8 | 240573_at | 374443 | LOC374443 | --- |
| 3.8 | 202515_at | 1739 | DLG1 | discs, large homolog 1 (Drosophila) |
| 3.8 | 215307_at | --- | --- | --- |
| 3.8 | 212201_at | 23141 | KIAA0692 | KIAA0692 protein |
| 3.8 | 40569_at | 7593 | ZNF42 | zinc finger protein 42 (myeloid-specific retinoic acid-responsive) |
| 3.8 | 204791_at | 7181 | NR2C1 | nuclear receptor subfamily 2, group C, member 1 |
| 3.8 | 236079_at | 202025 | DKFZp667E0512 | hypothetical protein DKFZp667E0512 |
| 3.8 | 203288_at | 9710 | KIAA0355 | KIAA0355 |
| 3.8 | 218016_s_at | 55718 | POLR3E | polymerase (RNA) III (DNA directed) polypeptide E (80kD) |
| 3.8 | 219870_at | 80063 | ATF7IP2 | activating transcription factor 7 interacting protein 2 |
| 3.8 | 207100_s_at | 6843 | VAMP1 | vesicle-associated membrane protein 1 (synaptobrevin 1) |
| 3.8 | 220155_s_at | 65980 | BRD9 | bromodomain containing 9 |
| 3.8 | 217940_s_at | 55739 | FLJ10769 | hypothetical protein FLJ10769 |
| 3.8 | 202548_s_at | 8874 | ARHGEF7 | Rho guanine nucleotide exchange factor (GEF) 7 |
| 3.8 | 204060_s_at | 5613 /// 5616 | PRKX /// PRKY | protein kinase, X-linked /// protein kinase, Y-linked |
| 3.8 | 215954_s_at | 58509 | C19orf29 | chromosome 19 open reading frame 29 |
| 3.8 | 220742_s_at | 55768 | NGLY1 | N-glycanase 1 |
| 3.8 | 226635_at | --- | --- | Hypothetical gene supported by AK091718 |
| 3.8 | 213398_s_at | 56948 | C14orf124 | chromosome 14 open reading frame 124 |
| 3.8 | 218373_at | 64400 | FTS | fused toes homolog (mouse) |
| 3.8 | 236436_at | 283130 | LOC283130 | hypothetical protein LOC283130 |
| 3.8 | 204828_at | 5883 | RAD9A | RAD9 homolog A (S. pombe) |
| 3.8 | 221293_s_at | 50619 | DEF6 | differentially expressed In FDCP 6 homolog (mouse) |
| 3.8 | 227988_s_at | 23230 | VPS13A | vacuolar protein sorting 13A (yeast) |
| 3.8 | 218370_s_at | 64766 | FLJ12903 | 5100P binding protein Riken |
| 3.8 | 207358_x_at | 23499 | MACF1 | microtubule-actin crosslinking factor 1 |
| 3.8 | 203082-at | 9790 | BMS1L | BMS1-like, ribosome assembly protein (yeast) |
| 3.8 | 228736_at | 113510 | HEL308 | DNA helicase HEL308 |
| 3.8 | 242273_at | --- | --- | Transcribed locus |
| 3.8 | 225107_at | 3181 | HNRPA2B1 | --- |
| 3.8 | 235729_at | 84874 | ZNF514 | zinc finger protein 514 |
| 3.8 | 225172_at | 57585 | CRAMP1L | Crm, cramped-like (Drosophila) |
| 3.8 | 221517_s_at | 9440 | CRSP6 | cofactor required for Sp1 transcriptional activation, subunit 6, 77kDa |
| 3.8 | 212932_at | 22930 | RAB3GAP | RAB3 GTPase-activating protein |
| 3.8 | 204143_s_at | 55556 | ENOSF1 | enolase superfamily member 1 |
| 3.8 | 212851_at | 23142 | KIAA0276 | KIAA0276 protein |
| 3.8 | 213882_at | 83941 | BBP | Beta-amyloid binding protein precursor |
| 3.8 | 228216_at | --- | --- | --- |
| 3.8 | 212307_s_at | 8473 | OGT | O-linked N-acetylglucosamine (GlcNAc) transferase (UDP-N-acetylglucosamine:polypeptide-N-acetylglucosaminyl transferase) |
| 3.8 | 229384 at | --- | --- | CDNA FLJ42101 fis, clone TESOP2006704 |
| 3.8 | 203804_s_at | 51747 | CROP | cisplatin resistance-associated overexpressed protein |
| 3.8 | 212037_at | 5411 | PNN | pinin, desmosome associated protein |
| 3.8 | 224817_at | 9644 | SH3MD1 | SH3 multiple domains 1 |
| 3.8 | 214048_at | 8930 | MBD4 | methyl-CpG binding domain protein 4 |
| 3.8 | 212819_at | 51665 | ASB1 | ankyrin repeat and SOCS box-containing 1 |
| 3.8 | 202328_s_at | 5310 | PKD1 | polycystic kidney disease 1 (autosomal dominant) |
| 3.8 | 235983_at | 66008 | ALS2CR3 | Amyotrophic lateral sclerosis 2 (Juvenile) chromosome region, candidate 3 |
| 3.8 | 232077_s_at | 83719 | YPEL3 | yippee_like 3 (Drosophila) |
| 3.8 | 227489_at | 64750 | SMURF2 | --- |
| 3.8 | 212753_at | 10336 | PCGF3 | polycomb group ring finger 3 |
| 3.8 | 219038_at | 79710 | ZCWCC2 | zinc finger, CW type with coiled-coil domain 2 |
| 3.8 | 236832_at | 221442 | LOC221442 | hypothetical protein LOC221442 |
| 3.8 | 227754_at | 159195 | USP54 | ubiquitin specific protease 54 |
| 3.8 | 222999_s_at | 81669 | CCNL2 | cyclin L2 |
| 3.8 | 228334_x_at | 80817 | KIAA1712 | KIAA1712 |
| 3.8 | 229078_s_at | 55425 | KIAA1704 | KIAA1704 |
| 3.8 | 203384_s_at | 2800 | GOLGA1 | golgi autoantigen, golgin subfamily a, 1 |
| 3.8 | 213743_at | 905 | CCNT2 | cyclin T2 |
| 3.8 | 213364_s_at | 6642 | SNX1 | Sorting nexin 1 |
| 3.8 | 202561_at | 8658 | TNKS | tankyrase, TRF1-interacting ankyrin-related ADP-ribose polymerase |
| 3.8 | 204142_at | 55556 | ENOSF1 | enolase superfamily member 1 |
| 3.8 | 228282 at | 256471 | MGC33302 | Hypothetical protein MGC33302 |
| 3.8 | 231850_x_at | 80817 | KIAA1712 | KIAA1712 |
| 3.8 | 215287_at | --- | --- | ELISC-1 |
| 3.8 | 229949_at | 8468 | FKBP6 | DKFZp434A0131 protein |
| 3.8 | 236007_at | 11216 | AKAP10 | A kinase (PRKA) anchor protein 10 |
| 3.8 | 227227_at | 388397 | --- | Hypothetical LOC388397 |
| 3.8 | 221973_at | 150759 | LOC150759 | CDNA clone IMAGE:5217021, with apparent retained intron |
| 3.8 | 228392_at | 55900 | ZNF302 | Zinc finger protein 302 |
| 3.8 | 225839_at | 155435 | LOC155435 | hypothetical protein LOC155435 |
| 3.8 | 231008_at | 222643 | UNC5CL | Unc-5 homolog C (C. elegans)-like |
| 3.8 | 204633_s_at | 9252 | RPS6KA5 | ribosomal protein S6 kinase, 90kDa, polypeptide 5 |
| 3.8 | 222266_at | 8725 | C19orf2 | Chromosome 19 open reading frame 2 |
| 3.8 | 204773_at | 3590 | IL11RA | Interleukin 11 receptor, alpha |
| 3.8 | 220143_x_at | 55692 | LUC7L | LUC7-like (S. cerevisiae) |
| 3.8 | 228465_at | --- | --- | Homo sapiens, clone IMAGE:5175565, mRNA |
| 3.8 | 215743_at | 9397 | NMT2 | N-myristoyltransferase 2 |
| 3.8 | 238620_at | --- | --- | Transcribed locus |
| 3.8 | 228999_at | 1106 | CHD2 | chromodomain helicase DNA binding protein 2 |
| 3.8 | 221264_s_at | 23435 | TARDBP | TAR DNAbinding protein///TAR DNAbinding protein |
| 3.8 | 239735_at | --- | --- | --- |
| 3.8 | 230120_s_at | 5342 | PLGL | plasminogen-like B1 |
| 3.8 | 203707_at | 10127 | ZNF263 | zinc finger protein 263 |
| 3.8 | 214473_x_at | --- | --- | Postmeiotic segregation increased 2-like 3 |
| 3.8 | 213703_at | 150759 | LOC150759 | hypothetical protein LOC150759 |
| 3.8 | 226528_at | 345778 | MTX3 | metaxin 3 |
| 3.8 | 225760_at | 114803 | KIAA1915 | KIAA1915 protein |
| 3.8 | 226474_at | 84166 | NOD27 | nucleotide-binding oligomerization domains 27 |
| 3.8 | 228001_at | 757 | C21orf4 | --- |
| 3.8 | 227603_at | 284459 | HKR1 | GLI-Kruppel family member HKR1 |
| 3.8 | 228318_s_at | 285464 | FLJ34443 | hypothetical protein FLJ34443 |
| 3.8 | 242607 at | --- | --- | Transcribed locus |
| 3.8 | 209770_at | 11119 | BTN3A1 | butyrophilin, subfamily 3, member A1 |
| 3.8 | 209049_s_at | 23613 | PRKCBP1 | protein kinase C binding protein 1 |
| 3.8 | 208718_at | 10521 | DDX17 | DEAD (Asp-Glu-Ala-Asp) box polypeptide 17 |
| 3.8 | 224876_at | 134553 | FLJ37562 | hypothetical protein FLJ37562 |
| 3.8 | 239734_at | --- | --- | Transcribed locus, weakly similar to XP_517454.1 similar to hypothetical protein MGC45438 [Pan troglodytes] |
| 3.8 | 225504-at | 79618 | FLJ21616 | Hypothetical protein FLJ21616 |
| 3.8 | 214163-at | 51668 | C1orf41 | chromosome 1 open reading frame 41 |
| 3.8 | 229366_at | 51185 | CRBN | Cereblon |
| 3.8 | 213483_at | 23398 | KIAA0073 | KIAA0073 protein |
| 3.8 | 220035_at | 23225 | NUP210 | nucleoporin 210kDa |
| 3.8 | 213278_at | 66036 | MTMR9 | myotubularin related protein 9 |
| 3.8 | 231940_at | 57711 | ZNF529 | zinc finger protein 529 |
| 3.8 | 204294_at | 275 | AMT | aminomethyltransferase (glycine cleavage system protein T) |
| 3.8 | 240155_x_at | 115648 /// 148203 /// 401905 | LOC115648 /// LOC148203 /// LOC401905 | --- |
| 3.8 | 239731_at | --- | --- | Transcribed locus |
| 3.8 | 244271 at | --- | --- | Transcribed locus, weakly similar to NP_872301.1 hypothetical protein FLJ25224 [Homo sapiens] |
| 3.8 | 218343_s_at | 9330 | GTF3C3 | general transcription factor IIIC, polypeptide 3, 102kDa |
| 3.8 | 218740_s_at | 80279 | CDK5RAP3 | CDK5 regulatory subunit associated protein 3 |
| 3.8 | 212277_at | 9110 | MTMR4 | myotubularin related protein 4 |
| 3.8 | 219151_s_at | 11158 /// 11159 | RABL2B /// | RAB, member of RAS oncogene family-like 2B /// RAB, member of RAS oncogene family-like 2A |
| 3.8 | 225724_at | 379025 | FLJ31306 | hypothetical protein FLJ31306 |
| 3.8 | 219169_s_at | 51106 | TFB1M | transcription factor B1, mitochondrial |
| 3.8 | 217810_x_at | 51520 | LARS | leucyl-tRNA synthetase |
| 3.8 | 221080_s_at | 79958 | FAM31C | family with sequence similarity 31, member C |
| 3.8 | 218315_s_at | 51654 | CDK5RAP1 | CDK5 regulatory subunit associated protein 1 |
| 3.8 | 204706_at | 56623 | INPP5E | inositol polyphosphate-5-phosphatase, 72 kDa |
| 3.8 | 215493_x_at | 11120 | BTN2Al | butyrophilin, subfamily 2, member A1 |
| 3.8 | 226040_at | --- | --- | MRNA; cDNA DKFZp762N156 (from clone DKFZp762N156) |
| 3.8 | 231838_at | 80336 | C20orf119 | chromosome 20 open reading frame 119 |
| 3.8 | 241991_at | 159091 | LOC159091 | Hypothetical protein BC017868 |
| 3.8 | 228397_at | 22880 | ZCWCC1 | Hypothetical protein FLJ20618 |
| 3.8 | 204352_at | 7188 | TRAF5 | TNF receptor-associated factor 5 |
| 3.8 | 220661_s_at | 55657 | FLJ20531 | hypothetical protein FLJ20531 |
| 3.8 | 225066 at | --- | --- | --- |
| 3.8 | 220081_x_at | 51478 | HSD17B7 | hydroxysteroid (17-beta) dehydrogenase 7 |
| 3.8 | 219627_at | 79970 | FLJ12700 | hypothetical protein FLJ12700 |
| 3.8 | 225945_at | 79027 | ZNF655 | zinc finger protein 655 |
| 3.8 | 241863_x_at | --- | --- | --- |
| 3.8 | 214717-at | 150967 | DKFZp434H1419 | hypothetical protein DKFZp434H1419 |
| 3.8 | 215358_x_at | 256112 | ZNF37B | zinc finger protein 37b (KOX 21) |
| 3.8 | 231944-at | 56605 | ER01LB | ER01-like beta (S. cerevisiae) |
| 3.8 | 228512-at | 55037 | FLJ20758 | FLJ20758 protein |
| 3.8 | 236006_s_at | 11216 | AKAP10 | A kinase (PRKA) anchor protein 10 |
| 3.8 | 213587_s_at | 155066 | C7orf32 | chromosome 7 open reading frame 32 |
| 3.8 | 212539_at | 9557 | CHD1L | chromodomain helicase DNA binding protein 1-like |
| 3.8 | 211383_s_at | 22884 | WDR37 | WD repeat domain 37 |
| 3.8 | 208072_s_at | 8527 | DGKD | diacylglycerol kinase, delta 130kDa |
| 3.8 | 219095_at | 8681 | PLA2G4B | phospholipase A2, group IVB (cytosolic) |
| 3.8 | 212126_at | 23468 | CBX5 | Chromobox homolog 5 (HP1 alpha homolog, Drosophila) |
| 3.8 | 203579_s_at | 9057 | SLC7A6 | solute carrier family 7 (cationic amino acid transporter, y+ system), member 6 |
| 3.8 | 215785_s_at | 26999 | CYFIP2 | cytoplasmic FMR1 interacting protein 2 |
| 3.8 | 220418_at | 53347 | UBASH3A | ubiquitin associated and SH3 domain containing, A |
| 3.8 | 52940_at | 59307 | SIGIRR | single Ig IL-1R-related molecule |
| 3.8 | 218921_at | 59307 | SIGIRR | single Ig IL-1R-related molecule |
| 3.8 | 220112_at | 79722 | FLJ11795 | hypothetical protein FLJ11795 |
| 3.8 | 243362_s_at | 51176 | LEF1 | lymphoid enhancer-binding factor 1 |
| 3.8 | 209782_s_at | 1628 | DBP | D site of albumin promoter (albumin D-box) binding protein |
| 3.8 | 202789_at | 5335 | PLCG1 | phospholipase C, gamma 1 |
| 3.8 | 208634_s_at | 23499 | MACF1 | microtubule-actin crosslinking factor 1 |
| 3.8 | 214870_x_at | 339047 /// 440341 /// 9284 | NPIP /// LOC339047 /// LOC440341 | nuclear pore complex interacting protein /// hypothetical protein LOC339047 /// similar to hypothetical protein LOC339047 |
| 3.8 | 204552_at | 3631 | INPP4A | Inositol polyphosphate-4-phosphatase, type I, 107kDa |
| 3.8 | 204538_x_at | 9284 | NPIP | nuclear pore complex interacting protein /// hypothetical protein LOC339047 /// similar to hypothetical protein LOC339047 |
| 3.8 | 203014_x_at | 27352 | RUTBC3 | RUN and TBC1 domain containing 3 |
| 3.8 | 226334_s_at | 130872 | AHSA2 | AHAL, activator of heat shock 90kDa protein ATPase homolog 2 (yeast) |
| 3.8 | 208089_s_at | 81550 | TDRD3 | tudor domain containing 3 /// tudor domain containing 3 |
| 3.8 | 219147_s_at | 54981 | C9orf95 | chromosome 9 open reading frame 95 |
| 3.8 | 202098_s_at | 3275 | HRMT1L1 | HMT1 hnRNP methyltransferase-like 1 (S. cerevislae) |
| 3.8 | 218505_at | 79726 | FLJ12270 | WD repeat domain 59 |
| 3.8 | 220607_x_at | 51497 | TH1L | TH1-like (Drosophila) |
| 3.8 | 225112_at | 10152 | ABI2 | Abl interactor 2 |
| 3.8 | 221564_at | 3275 | HRMT1L1 | HMT1 hnRNP methyltransferase-like 1 (S. cerevislae) |
| 3.8 | 227751_at | 9141 | PDCD5 | Programmed cell death 5 |
| 3.8 | 203802_x_at | 55695 | NSUN5 | NOL1/NOP2/Sun domain family, member 5 |
| 3.9 | 204236_at | 2313 | FLI1 | Friend leukemia virus integration 1 |
| 3.9 | 222103_at | 466 | ATF1 | Activating transcription factor 1 |
| 3.9 | 212530_at | 140609 | IVEK7 | NIMA (never in mitosis gene a)-related kinase 7 |
| 3.9 | 201424_s_at | 8451 | CUL4A | cullin 4A |
| 3.9 | 203049_s_at | --- | --- | --- |
| 3.9 | 202038_at | 9354 | UBE4A | ubiquitination factor E4A (UFD2 homolog, yeast) |
| 3.9 | 212513_s_at | 23032 | USP33 | ubiquitin specific protease 33 |
| 3.9 | 203689_s_at | 2332 | FMR1 | fragile X mental retardation 1 |
| 3.9 | 213027_at | 6738 | SSA2 | Sjogren syndrome antigen A2 (60kDa, ribonucleoprotein autoantigen SS-A/Ro) |
| 3.9 | 204831_at | 1024 | CDK8 | Cyclin-dependent kinase 8 |
| 3.9 | 203513_at | 80208 | FLJ21439 | hypothetical protein FLJ21439 |
| 3.9 | 205052_at | 549 | AUH | AU RNA binding protein/enoyl-Coenzyme A hydratase |
| 3.9 | 209630_s_at | 26190 | FBXW2 | F-box and WD-40 domain protein 2 |
| 3.9 | 220079_s_at | 84196 | USP48 | ubiquitin specific protease 48 |
| 3.9 | 212352_s_at | 10972 | TMP21 | transmembrane trafficking protein |
| 3.9 | 34031_1_at | 889 | CCM1 | KRIT1, ankyrin repeat containing |
| 3.9 | 212718_at | 10914 | PAPOLA | poly(A) polymerase alpha |
| 3.9 | 212507_at | 23505 | RW1 | RW1 protein |
| 3.9 | 221931_s_at | 81929 | SEH1L | SEHl-like (S. cerevisiae) |
| 3.9 | 218262_at | 64777 | FLJ22318 | hypothetical protein FLJ22318 |
| 3.9 | 215548_s_at | 23256 | SCFD1 | sec1 family domain containing 1 |
| 3.9 | 218700_s_at | 8934 | RAB7L1 | RAB7, member RAS oncogene family-like 1 |
| 3.9 | 218043_s_at | 64343 | AZI2 | 5-azacytidine induced 2 |
| 3.9 | 212585_at | 114882 | OSBPL8 | oxysterol binding protein-like 8 |
| 3.9 | 218047_at | 114883 | OSBPL9 | oxysterol binding protein-like 9 |
| 3.9 | 212648_at | 54505 | DHX29 | DEAH (Asp-Glu-Ala-His) box polypeptide 29 |
| 3.9 | 200994_at | 10527 | IPO7 | Importin 7 |
| 3.9 | 226441_at | 10746 | MAP3K2 | Mitogen-activated protein kinase kinase kinase 2 |
| 3.9 | 203253_s_at | 23262 | KIAA0433 | KIAA0433 protein |
| 3.9 | 206989_s_at | 9169 | SFRS2IP | splicing factor, arginine/serine-rich 2, interacting protein |
| 3.9 | 203738_at | 55322 | FLJ11193 | hypothetical protein FLJ11193 |
| 3.9 | 218536_at | 57380 | MRS2L | MRS2-like, magnesium homeostasis factor (S. cerevisiae) |
| 3.9 | 210891_s_at | 2969 /// 2970 | GTF2I /// GTF2IP1 | general transcription factor II, 1 III general transcription factor II, 1, pseudogene 1 |
| 3.9 | 202318_s_at | 26054 | SENP6 | SUMO1/sentrin specific protease 6 |
| 3.9 | 203629_s_at | 10466 | COGS | component of oligomeric golgi complex 5 |
| 3.9 | 213064_at | 79882 | FLJ11806 | nuclear protein UKp68 |
| 3.9 | 201833_at | 3066 | HDAC2 | histone deacetylase 2 |
| 3.9 | 201855_s_at | 23300 | KIAA0431 | KIAA0431 protein |
| 3.9 | 217832_at | 10492 | SYNCRIP | synaptotagmin binding, cytoplasmic RNA interacting protein |
| 3.9 | 212904_at | 57470 | KIAA1185 | KIAA1185 protein |
| 3.9 | 218460_at | 54919 | FLJ20397 | hypothetical protein FLJ20397 |
| 3.9 | 201326_at | 908 | CCT6A | chaperonin containing TCP1, subunit 6A (zeta 1) |
| 3.9 | 201384_s_at | 4077 | M17S2 | neighbor of BRCAL gene 1 |
| 3.9 | 213518_at | 5584 | PRKCI | protein kinase C, iota |
| 3.9 | 201528_at | 6117 | RPAL | replication protein A1, 70kDa |
| 3.9 | 65472_at | 388969 | --- | Hypothetical LOC388969 |
| 3.9 | 218842_at | 79657 | FLJ21908 | hypothetical protein FLJ21908 |
| 3.9 | 219972_s_at | 64430 | C14orf135 | chromosome 14 open reading frame 135 |
| 3.9 | 221532_s_at | 80349 | REC14 | recombination protein REC14 |
| 3.9 | 36920_at | 4534 | MTM1 | myotubularin 1 |
| 3.9 | 214113_s_at | 9939 | RBM8A | RNA binding motif protein 8A |
| 3.9 | 221094_s_at | 55140 | ELP3 | elongation protein 3 homolog (S. cerevisiae) |
| 3.9 | 214198_s_at | 9993 | DGCR2 | DiGeorge syndrome critical region gene 2 |
| 3.9 | 218698_at | 51074 | MMRP19 | likely ortholog of mouse monocyte macrophage 19 |
| 3.9 | 209285_s_at | 23272 | RAP140 | retinoblastoma-associated protein 140 |
| 3.9 | 218179_s_at | 60684 | FLJ12716 | FLJ12716 protein |
| 3.9 | 203690_at | 10426 | TUBGCP3 | tubulin, gamma complex associated protein 3 |
| 3.9 | 203048_s_at | 9652 | KIAA0372 | KIAA0372 |
| 3.9 | 200877_at | 10575 | CCT4 | chaperonin containing TCP1, subunit 4 (delta) |
| 3.9 | 208802_at | 6731 | SRP72 | signal recognition particle 72kDa |
| 3.9 | 205668_at | 4065 | LY75 | lymphocyte antigen 75 |
| 3.9 | 218147_s_at | 55830 | GLT8D1 | glycosyltransferase 8 domain containing 1 |
| 3.9 | 213208_at | 23506 | KIAA0240 | KIAA0240 |
| 3.9 | 203566_s_at | 178 | AGL | amylo-1, 6-glucosidase, 4-alpha-(glycogen glucanotransferase (glycogen debranching enzyme, glycogen storage disease type III) |
| 3.9 | 212299_at | 91754 | IVEK9 | IVIMA (never In mitosis gene a)- related kinase 9 |
| 3.9 | 212754_s_at | 23041 | KIAA1040 | KIAA1040 protein |
| 3.9 | 204354_at | 25913 | POT1 | POT1 protection of telomeres 1 homolog (S. pombe) |
| 3.9 | 200979_at | 389840 | FLJ16518 | Mitogen-activated protein kinase kinase kinase 15 |
| 3.9 | 203284_s_at | 9653 | HS2ST1 | heparan sulfate 2-O-sulfotransferase 1 |
| 3.9 | 200988_s_at | 10197 | PSME3 | proteasome (prosome, macropain) activator subunit 3 (PA28 gamma; K1) |
| 3.9 | 206542_s_at | 6595 | SMARCA2 | SWI/SNF related, matrix associated, actin dependent regulator of chromatin, subfamily a, member 2 |
| 3.9 | 201624_at | 1615 | DARS | aspartyl-tRNA synthetase |
| 3.9 | 219289_at | 55027 | FLJ20718 | hypothetical protein FLJ20718 |
| 3.9 | 202664_at | 7456 | WASPIP | Wiskott-Aldrich syndrome protein interacting protein |
| 3.9 | 203741_s_at | 113 | ADCY7 | adenylate cyclase 7 |
| 3.9 | 207238_s_at | 5788 | PTPRC | protein tyrosine phosphatase, receptor type, C |
| 3.9 | 204314_s_at | 1385 | CREB1 | CAMP responsive element binding protein 1 |
| 3.9 | 200760_s_at | 10550 | ARL6IP5 | ADP-ribosylation-like factor 6 interacting protein 5 |
| 3.9 | 201612_at | 223 | ALDH9A1 | aldehyde dehydrogenase 9 family, member A1 |
| 3.9 | 204662_at | 9738 | CP110 | CP110 protein |
| 3.9 | 206308_at | 1787 | DNMT2 | DNA (cytosine-5-)-methyltransferase 2 |
| 3.9 | 218981_at | 57001 | ACN9 | ACN9 homolog (S. cerevisiae) |
| 3.9 | 218989_x_at | 64924 | SLC30A5 | solute carrier family 30 (zinc transporter), member 5 |
| 3.9 | 218701_at | 51110 | LACTB2 | lactamase, beta 2 |
| 3.9 | 218090_s_at | 55717 | WDR11 | WD repeat domain 11 |
| 3.9 | 212652_s_at | 8723 | SNX4 | sorting nexin 4 |
| 3.9 | 218158_s_at | 26060 | APPL | adaptor protein containing pH domain, PTB domain and leucine zipper motif 1 |
| 3.9 | 201339_s_at | 6342 | SCP2 | sterol carrier protein 2 |
| 3.9 | 204634_at | 6787 | NEK4 | NIMA (never in mitosis gene a)-related kinase 4 |
| 3.9 | 201296_s_at | 26118 | WSB1 | WD repeat and SOCS box-containing 1 |
| 3.9 | 213883_s_at | 83941 | BBP | beta-amyloid binding protein precursor |
| 3.9 | 213154_s_at | 23299 | BICD2 | bicaudal D homolog 2 (Drosophila) |
| 3.9 | 201375_s_at | 5516 | PPP2CB | protein phosphatase 2 (formerly 2A), catalytic subunit, beta isoform |
| 3.9 | 201218_at | 1488 | CTBP2 | --- |
| 3.9 | 202215_s_at | 4802 | NFYC | nuclear transcription factor Y, gamma |
| 3.9 | 219649_at | 29929 | ALG6 | asparagine-linked glycosylation 6 homolog (yeast, alpha-1,3-glucosyltransferase) |
| 3.9 | 201985_at | 9897 | KIAA0196 | KIAA0196 gene product |
| 3.9 | 218323_at | 55288 | RHOT1 | ras homolog gene family, member T1 |
| 3.9 | 209206_at | 9554 | SEC22L1 | SEC22 vesicle trafficking protein-like 1 (S. cerevislae) |
| 3.9 | 218264_at | 56647 | BCCIP | BRCA2 and CDKN1A interacting protein |
| 3.9 | 203211_s_at | 8898 | MTMR2 | myotubularin related protein 2 |
| 3.9 | 217921_at | --- | --- | --- |
| 3.9 | 212248_at | 92140 | LYRIC | LYRIC/3D3 |
| 3.9 | 203743_s_at | 6996 | TDG | thymine-DNA glycosylase |
| 3.9 | 209551_at | 84272 | MGC11061 | hypothetical protein MGC11061 |
| 3.9 | 204297_at | 5289 | PIK3C3 | phosphoinositide-3-kinase, class 3 |
| 3.9 | 200857_s_at | 9611 | NCOR1 | nuclear receptor co-repressor 1 |
| 3.9 | 209523_at | 6873 | TAF2 | TAF2 RNA polymerase II, TATA box binding protein (TBP)-associated factor, 150kDa |
| 3.9 | 208882_s_at | 51366 | EDD | E3 identified by differential display |
| 3.9 | 222111_at | 54629 | KIAA1164 | Hypothetical protein KIAA1164 |
| 3.9 | 218593_at | 55131 | RBM28 | RNA binding motif protein 28 |
| 3.9 | 204075_s_at | --- | --- | glycine-, glutamate-, thienylcyclohexylpiperidine-binding protein |
| 3.9 | 219363_s_at | 51001 | CGI-12 | CGI-12 protein |
| 3.9 | 210962_s_at | 10142 | AKAP9 | A kinase (PRKA) anchor protein (yotiao) 9 |
| 3.9 | 211987_at | 7155 | TOP2B | topoisomerase (DNA) II beta 180kDa |
| 3.9 | 202491_s_at | 8518 | IKBKAP | inhibitor of kappa light polypeptide gene enhancer in B-cells, kinase complex-associated protein |
| 3.9 | 201493_s_at | 23369 | PUM2 | pumilio homolog 2 (Drosophila) |
| 3.9 | 212476_at | 23527 | CENTB2 | centaurin, beta 2 |
| 3.9 | 212919_at | 167227 | DCP2 | DCP2 decapping enzyme homolog (S. ceremsnae) |
| 3.9 | 202956_at | 10565 | ARFGEF1 | ADP-ribosylation factor guanine nucleotide-exchange factor 1 (brefeldin A-inhibited) |
| 3.9 | 213313_at | 23637 | RABGAP1 | RAB GTPase activating protein 1 |
| 3.9 | 201166_s_at | 9698 | PUM1 | pumilio homolog 1 (Drosophila) |
| 3.9 | 203983_at | 7257 | TSNAX | translin-associated factor X |
| 3.9 | 38290_at | 10636 | RGS14 | regulator of G-protein signalling 14 |
| 3.9 | 221873_at | 7702 | ZNF143 | zinc finger protein 143 (clone pHZ-1) |
| 3.9 | 200992_at | 10527 | IPO7 | importin 7 |
| 3.9 | 212637_s_at | 11059 | WWP1 | WW domain containing E3 ubiquitin protein ligase 1 |
| 3.9 | 201849_at | 664 | BNIP3 | BCL2/adenovirus E1B 19kDa interacting protein 3 |
| 3.9 | 204020_at | 5813 | PURA | purine-rich element binding protein A |
| 3.9 | 240824_at | 79991 | OBFC1 | Oligonucleotide/oligosaccharide-binding fold containing 1 |
| 3.9 | 212150_at | 23167 | KIAA0143 | KIAA0143 protein |
| 3.9 | 211727_s_at | 1353 | COX11 | COX11 homolog, cytochrome c oxidase assembly protein (yeast) /// COX11 homolog, cytochrome c oxidase assembly protein (yeast) |
| 3.9 | 208694_at | 5591 | PRKDC | protein kinase, DNA-activated, catalytic polypeptide |
| 3.9 | 217887_s_at | 2060 | EPS15 | epidermal growth factor receptor pathway substrate 15 |
| 3.9 | 200902_at | 9403 | 15-Sep | 15 kDa selenoprotein |
| 3.9 | 219137_s_at | 56947 | C2orf33 | chromosome 2 open reading frame 33 |
| 3.9 | 32042_at | 10495 | COVA1 | cytosolic ovarian carcinoma antigen 1 |
| 3.9 | 203845_at | 8850 | PCAF | p300/CBP-associated factor |
| 3.9 | 211954_s_at | 3843 | RANBP5 | RAN binding protein 5 |
| 3.9 | 203537_at | 5636 | PRPSAP2 | phosphoribosyl pyrophosphate synthetase-associated protein 2 |
| 3.9 | 202602_s_at | 27336 | HTATSF1 | HIV TAT specific factor 1 |
| 3.9 | 218545_at | 55297 | FLJ11088 | GGA binding partner |
| 3.9 | 210970_s_at | 25998 | IBTK | inhibitor of Bruton agammaglobulmemna tyrosine kinase |
| 3.9 | 212137_at | 23367 | LARP | likely ortholog of mouse la related protein |
| 3.9 | 208070_s_at | 5980 | REV3L | REV3-like, catalytic subunit of DNA polymerase zeta (yeast) |
| 3.9 | 212138_at | 23244 | SCC-112 | SCC-112 protein |
| 3.9 | 217886_at | 2060 | EPS15 | epidermal growth factor receptor pathway substrate 15 |
| 3.9 | 208877_at | 5062 | PAK2 | p21 (CDKN1A)-actmated kinase 2 |
| 3.9 | 203077_s_at | 4087 | SMAD2 | SMAD, mothers against DPP homolog 2 (Drosophila) |
| 3.9 | 209092_s_at | 51031 | C17orf25 | chromosome 17 open reading frame 25 |
| 3.9 | 217815_at | 11198 | SUPT16H | suppressor of Ty 16 homolog (S. cerevisiae) |
| 3.9 | 218212_s_at | 4338 | MOCS2 | molybdenum cofactor synthesis 2 |
| 3.9 | 221744_at | 10238 | HAN11 | WD-repeat protein |
| 3.9 | 201503_at | 10146 | G3BP | Ras-GTPase-activating protein SH3-domain-binding protein |
| 3.9 | 219296_at | 54503 | ZDHHC13 | zinc finger, DHHC domain containing 13 |
| 3.9 | 213390_at | 23211 | C19orf7 | chromosome 19 open reading frame 7 |
| 3.9 | 218491_s_at | 29087 | THY28 | thymocyte protein thy28 |
| 3.9 | 211971_s_at | 10128 | LRPPRC | leucine-rich PPR-motif containing |
| 3.9 | 201034_at | 120 | ADD3 | adducin 3 (gamma) |
| 3.9 | 212692_s_at | 987 | LRBA | LPS-responsive vesicle trafficking, beach and anchor containing |
| 3.9 | 212140_at | 23244 | SCC-112 | SCC-112 protein |
| 3.9 | 212780_at | 6654 | SOS1 | son of sevenless homolog 1 (Drosophila) |
| 3.9 | 218699_at | 8934 | RAB7L1 | RAB7, member RAS oncogene family-like 1 |
| 3.9 | 204980_at | 9575 | CLOCK | clock homolog (mouse) |
| 3.9 | 211615_s_at | 10128 | LRPPRC | leucine-rich PPR-motif containing /// leucine-rich PPR-motif containing |
| 3.9 | 201817_at | 9690 | UBE3C | ubiquitin protein ligase E3C |
| 3.9 | 201036_s_at | 3033 | HADHSC | L-3-hydroxyacyl-Coenzyme A dehydrogenase, short chain |
| 3.9 | 217724_at | 26135 | PAI-RBP1 | PAI-1 mRNA-binding protein |
| 3.9 | 200860_s_at | 23019 | CNOT1 | CCR4-NOT transcription complex, subunit 1 |
| 3.9 | 202214_s_at | 8450 | CUL4B | cullin 4B |
| 3.9 | 202346_at | 3093 | HIP2 | huntingtin interacting protein 2 |
| 3.9 | 202432_at | 5532 | PPP3CB | protein phosphatase 3 (formerly 2B), catalytic subunit, beta isoform (calcineurin A beta) |
| 3.9 | 203465_at | 9801 | MRPL19 | mitochondrial ribosomal protein L19 |
| 3.9 | 202567_at | 6634 | SNRPD3 | small nuclear ribonucleoprotein D3 polypeptide 18kDa |
| 3.9 | 202606_s_at | 9874 | TLK1 | tousled-like kinase 1 |
| 3.9 | 204172_at | 1371 | CPOX | coproporphyrinogen oxidase |
| 3.9 | 202663_at | 7456 | WASPIP | Wiskott-Aldrich syndrome protein interacting protein |
| 3.9 | 202261_at | 6944 | TCFL1 | transcription factor-like 1 |
| 3.9 | 218538_s_at | 57380 | MRS2L | MRS2-like, magnesium homeostasis factor (S. cerevisiae) |
| 3.9 | 202926_at | 51594 | NAG | neuroblastoma-amplified protein |
| 3.9 | 220329_s_at | 55005 | C6orf96 | chromosome 6 open reading frame 96 |
| 3.9 | 208809_s_at | 81688 | C6orf62 | chromosome 6 open reading frame 62 |
| 3.9 | 214789_x_at | 10929 | SRP46 | Splicing factor, arginine/serine-rich, 46kD |
| 3.9 | 218684_at | 55144 | LRRC5 | leucine rich repeat containing 5 |
| 3.9 | 213238_at | 57205 | ATP10D | ATPase, Class V, type 10D |
| 3.9 | 201734_at | 1182 | CLCN3 | Chloride channel 3 |
| 3.9 | 226685_at | 6645 | SNTB2 | Syntrophin, beta 2 (dystrophin-associated protein Al, 59kDa, basic component 2) |
| 3.9 | 213375_s_at | 90634 | CG018 | hypothetical gene CG018 |
| 3.9 | 201990_s_at | 1389 | CREBL2 | CAMP responsive element binding protein-like 2 |
| 3.9 | 214695_at | 9898 | UBAP2L | ubiquitin associated protein 2-like |
| 3.9 | 202850_at | 5825 | ABCD3 | ATP-binding cassette, sub-family D (ALD), member 3 |
| 3.9 | 202603_at | 102 | ADAM10 | A disintegrin and metalloproteinase domain 10 |
| 3.9 | 203073_at | 22796 | COG2 | component of oligomeric golgi complex 2 |
| 3.9 | 205771_s_at | 9465 | AKAP7 | A kinase (PRKA) anchor protein 7 |
| 3.9 | 217800_s_at | 80762 | NDFIP1 | Nedd4 family interacting protein 1 |
| 3.9 | 203787_at | 23635 | SSBP2 | single-stranded DNA binding protein 2 |
| 3.9 | 209786_at | 10473 | HMGN4 | high mobility group nucleosomal binding domain 4 |
| 3.9 | 209943_at | 26235 | FBXL4 | F-box and leucine-rich repeat protein 4 |
| 3.9 | 219013_at | 63917 | GALNT11 | UDP-N-acetyl-alpha-D-galactosamme:polypeptnde N-acetylgalactosaminyltransferase 11 (GalVAc-T11) |
| 3.9 | 218322_s_at | 51703 | ACSL5 | acyl-CoA synthetase long-chain family member 5 |
| 3.9 | 212943_at | 9847 | KIAA0528 | KIAA0528 gene product |
| 3.9 | 218962_s_at | 64418 | FLJ13576 | hypothetical protein FLJ13576 |
| 3.9 | 204521_at | 29902 | HSU79274 | protein predicted by clone 23733 |
| 3.9 | 212640_at | 201562 | PTPLB | protein tyrosine phosphatase-like (proline instead of catalytic arginine), member b |
| 3.9 | 218095_s_at | 55858 | TPARL | TPA regulated locus |
| 3.9 helix) | 204849_at | 10732 | TCFL5 | transcription factor-like 5 (basic helix-loop- |
| 3.9 | 209459_s_at | 18 | ABAT | 4-aminobutyrate aminotransferase |
| 3.9 | 207305_s_at | 22878 | KIAA1012 | KIAA1012 |
| 3.9 | 217501_at | 9391 | WDR39 | WD repeat domain 39 |
| 3.9 | 209199_s_at | 4208 | MEF2C | MADS box transcription enhancer factor 2, polypeptide C (myocyte enhancer factor 2C) |
| 3.9 | 217970_s_at | 57472 | CNOT6 | CCR4-NOT transcription complex, subunit 6 |
| 3.9 | 204702_s_at | 9603 | NFE2L3 | nuclear factor (erythroid-derived 2)-like 3 |
| 3.9 | 210111_s_at | --- | --- | KIAA0265 protein |
| 3.9 | 203415_at | 10016 | PDCD6 | programmed cell death 6 |
| 3.9 | 212877_at | 3831 | KNS2 | kinesin 2 60/70kDa |
| 3.9 | 221196_x_at | 79184 | CXorf53 | chromosome X open reading frame 53 |
| 3.9 | 222369_at | --- | --- | --- |
| 3.9 | 211784_s_at | 6426 | SFRS1 | factor, arginine/serine-rich 1 (splicing factor 2, alternate splicing factor) /// splicing factor, arginine/serine-rich 1 (splicing factor 2, alternate splicing factor) |
| 3.9 | 212486_s_at | 2534 | FYN | FYN oncogene related to SRC, FGR, YES |
| 3.9 | 209382_at | 10623 | POLR3C | polymerase (RNA) III (DNA directed) polypeptide C (62kD) |
| 3.9 | 203067_at | 8050 | PDHX | pyruvate dehydrogenase complex, component X |
| 3.9 | 204274_at | 9166 | EBAG9 | estrogen receptor binding site associated, antigen, 9 |
| 3.9 | 203688_at | 5311 | PKD2 | polycystic kidney disease 2 (autosomal dominant) |
| 3.9 | 38892_at | 23506 | KIAA0240 | KIAA0240 |
| 3.9 | 209200_at | --- | --- | MADS box transcription enhancer factor 2, polypeptide C (myocyte enhancer factor 2C) |
| 3.9 | 213090_s_at | 6874 | TAF4 | TAF4 RNA polymerase II, TATA box binding protein (TBP)-associated factor, 135kDa |
| 3.9 | 221213_s_at | 54816 | SUHW4 | suppressor of hairy wing homolog 4 (Drosophila) |
| 3.9 | 212499_s_at | 51077 /// 93487 | C14orf111 /// C14orf32 | chromosome 14 open reading frame 111 /// chromosome 14 open reading frame 32 |
| 3.9 | 203791_at | 1657 | DMXL1 | Dmx-like 1 |
| 3.9 | 222201_s_at | 9994 | CASP8AP2 | CASP8 associated protein 2 |
| 3.9 | 214670_at | 7586 | ZKSCAN1 | zinc finger with KRAB and SCAN domains 1 |
| 3.9 | 208953_at | 23185 | KIAA0217 | KIAA0217 |
| 3.9 | 218351_at | 54951 | COMMD8 | COMM domain containing 8 |
| 3.9 | 210849_s_at | 27072 | VPS41 | vacuolar protein sorting 41 (yeast) |
| 3.9 | 209247_s_at | 10061 | ABCF2 | ATP-binding cassette, sub-family F (GCIV20), member 2 |
| 3.9 | 218396_at | 54832 | VP513C | vacuolar protein sorting 13C (yeast) |
| 3.9 | 204333_s_at | 175 | AGA | aspartylglucosaminidase |
| 3.9 | 209539_at | 9459 | ARHGEF6 | Rac/Cdc42 guanine nucleotide exchange factor (GEF) 6 |
| 3.9 | 218128_at | 4801 | NFYB | nuclear transcription factor Y, beta |
| 3.9 | 201501_s_at | 2926 | GRSF1 | G-rich RNA sequence binding factor 1 |
| 3.9 | 209143_s_at | 1207 | CLNS1A | chloride channel, nucleotide-sensitive, 1A /// chromosome 3 open reading frame 4 |
| 3.9 | 218838_s_at | 64427 | FLJ12788 | hypothetical protein FLJ12788 |
| 3.9 | 209268_at | 11311 | VPS45A | vacuolar protein sorting 45A (yeast) |
| 3.9 | 201448_at | 7072 | TIA1 | TIA1 cytotoxic granule-associated RNA binding protein |
| 3.9 | 208861_s_at | 546 | ATRX | alpha thalassemia/mental retardation syndrome X-linked (RAD54 homolog, S. cerevisiae) |
| 3.9 | 219481_at | 79573 | TTC13 | tetratricopeptide repeat domain 13 |
| 3.9 | 201589_at | 8243 | SMC1L1 | SMC1 structural maintenance of chromosomes 1-like 1 (yeast) |
| 3.9 | 214531_s_at | 6642 | SNX1 | sorting nexin 1 |
| 3.9 | 209702_at | 79068 | FTO | fatso |
| 3.9 | 227987_at | 23230 | VPS13A | vacuolar protein sorting 13A (yeast) |
| 3.9 | 221079_s_at | 339175 /// 55798 | METTL2 /// FLJ12760 | methyltransferase like 2 /// hypothetical protein FLJ12760 |
| 3.9 | 218452_at | 50485 | SMARCAL1 | SWI/SNF related, matrix associated, actin dependent regulator of chromatin, subfamily a-like 1 |
| 3.9 | 201517_at | 22916 | NCBP2 | nuclear cap binding protein subunit 2, 20kDa |
| 3.9 | 209846_s_at | 11118 | BTN3A2 | butyrophilin, subfamily 3, member A2 |
| 3.9 | 219007_at | 348995 | NUP43 | nucleoporin 43kDa |
| 3.9 | 201967_at | 10180 | RBM6 | RNA binding motif protein 6 |
| 3.9 | 229342_at | --- | --- | CDNA FLJ32162 fis, clone PLACE6000325 |
| 3.9 | 228548_at | 5906 | RAP1A | RAP1A, member of RAS oncogene family |
| 3.9 | 201778_s_at | 9813 | KIAA0494 | KIAA0494 gene product |
| 3.9 | 212189_s_at | 25839 | COG4 | component of oligomeric golgi complex 4 |

### REFERENCES

1. Golub, T.R. et al. Molecular classification of cancer: class discovery and class prediction by gene expression monitoring. Science 286, 531-7 (1999).
2. Alizadeh, A.A. et al. Distinct types of diffuse large B-cell lymphoma identified by gene expression profiling. Nature 403, 503-11 (2000).
3. Garber, K. Genomic medicine. Gene expression tests foretell breast cancer's future. Science 303, 1754-5 (2004).
4. van de Vijver, M.J. et al. A gene-expression signature as a predictor of survival in breast cancer. N Engl J Med 347, 1999-2009 (2002).
5. Pascual, V., Allantaz, F., Arce, E., Punaro, M. & Banchereau, J. Role of interleukin-1 (IL-1) in the pathogenesis of systemic onset juvenile idiopathic arthritis and clinical response to IL-1 blockade. J Exp Med 201, 1479-86 (2005).
6. Michiels, S., Koscielny, S. & Hill, C. Prediction of cancer outcome with microarrays: a multiple random validation strategy. *Lancet* **365**, 488-92 (2005).
7. loannidis, J.P. Microarrays and molecular research: noise discovery? *Lancet* **365**, 454-5 (2005).
8. Jarvinen, A.K. et al. Are data from different gene expression microarray platforms comparable? *Genomics* **83**, 1164-8 (2004).
9. Tan, P.K. et al. Evaluation of gene expression measurements from commercial microarray platforms. *Nucleic Acids Res* **31**, 5676-84 (2003).
10. Bammler, T. et al. Standardizing global gene expression analysis between laboratories and across platforms. *Nat Methods* **2**, 351-6 (2005).
11. Irizarry, R.A. et al. Multiple-laboratory comparison of microarray platforms. *Nat Methods* **2*,*** 345-50 (2005).
12. Larkin, J.E., Frank, B.C., Gavras, H., Sultana, R. & Quackenbush, J. Independence and reproducibility across microarray platforms. Nat Methods 2, 337-44 (2005).
13. Chaussabel, D. Biomedical literature mining: challenges and solutions in the 'omics' era. Am J Pharmacogenomics 4, 383-93 (2004).
14. Rhodes, D.R. et al. Mining for regulatory programs in the cancer transcriptome. Nat Genet 37, 579-83 (2005).
15. Segal, E., Friedman, N., Koller, D. & Regev, A. A module map showing conditional activity of expression modules in cancer. Nat Genet 36, 1090-8 (2004).
16. Mootha, V.K. et al. PGC-lalpha-responsive genes involved in oxidative phosphorylation are coordinately downregulated in human diabetes. Nat Genet 34, 267-73 (2003).
17. Segal, E., Friedman, N., Kaminski, N., Regev, A. & Koller, D. From signatures to models: understanding cancer using microarrays. Nat Genet 37 Suppl, S38-45 (2005).
18. Chaussabel, D. & Sher, A. Mining microarray expression data by literature profiling. Genome Biol 3, RESEARCH0055 (2002).
19. Bennett, L. et al. Interferon and granulopoiesis signatures in systemic lupus erythematosus blood. J Exp Med 197, 711-23 (2003).
20. Connolly, P.H. et al. Effects of exercise on gene expression in human peripheral blood mononuclear cells. J Appl Physiol 97, 1461-9 (2004).
21. Barrett, T. et al. NCBI GEO: mining millions of expression profiles--database and tools. Nucleic Acids Res 33, D562-6 (2005).
22. Ogawa, K., Oka, J., Yamakawa, J. & Higuchi, M. A single bout of exercise influences natural killer cells in elderly women, especially those who are habitually active. J Strength Cond Res 19, 45-50 (2005).
23. Woods, J.A., Evans, J.K., Wolters, B.W., Ceddia, M.A. & McAuley, E. Effects of maximal exercise on natural killer (NK) cell cytotoxicity and responsiveness to interferon-alpha in the young and old. J Gerontol A Biol Sci Med Sci 53, B430-7 (1998).
24. Tuma, R.S. Efforts aimed at reducing noise, data overload in microarrays. J Natl Cancer Inst 97, 1173-5 (2005).

## Claims

1. A method for identifying transcriptional modules comprising the steps of:
providing individual gene expression levels from cells of one or more patients with a disease or condition;
recording the expression value for each gene in a table that is divided into clusters;
iteratively selecting gene expression values for one or more transcriptional modules by:
selecting for the module the genes from each cluster that match in every disease or condition;
removing the selected genes from the analysis; and
repeating the process of gene expression value selection for genes that cluster in a sub-fraction of the diseases or conditions; and
iteratively repeating the generation of modules for each clusters until all gene clusters are exhausted, **characterized in that** the one or more transcriptional modules are selected from:
| Transcriptional modules |
|---|
| **Plasma cells:** genes encoding for Immunoglobulin chains (IGHM, IGJ, IGLL1, IGKC, IGHD) and the plasma cell marker CD38.; |
| **Platelets:** genes encoding for platelet glycoproteins (ITGA2B, ITGB3, GP6, GP1A/B), and platelet-derived immune mediators such as PPPB (pro-platelet basic protein) and PF4 (platelet factor 4); |
| **B-cells:** genes encoding for B-cell surface markers (CD72, CD79A/B, CD19, CD22) and other B-cell associated molecules: Early B-cell factor (EBF), B-cell linker (BLNK) and B lymphoid tyrosine kinase (BLK); |
| genes encoding regulators and targets of cAMP signaling pathway (JUND, ATF4, CREM, PDE4, NR4A2, VIL2), as well as repressors of TNF-alpha mediated NF-KB activation (CYLD, ASK, TNFAIP3); |
| **Myeloid lineage:** genes encoding molecules expressed by cells of the myeloid lineage (CD86, CD 163, FCGR2A), some of which being involved in pathogen recognition (CD14, TLR2, MYD88). This set also includes TNF family members (TNFR2, BAFF); |
| genes encoding for signaling molecules, the zinc finger containing inhibitor of activated STAT (PIAS1 and PIAS2), or the nuclear factor of activated T-cells NFATC3; |
| **MHC/Ribosomal proteins:** genes encoding MHC class I molecules (HLA-A,B,C,G,E)+ Beta 2-microglobulin (B2M) or Ribosomal proteins (RPLs, RPSs); |
| genes encoding metabolic enzymes (GLS, NSF1, NAT1) and factors involved in DNA replication (PURA, TERF2, EIF2S1); |
| **Cytotoxic cells:** genes encoding Cytotoxic T-cells amd NK-cells surface markers (CD8A, CD2, CD160, NKG7, KLRs), cytolytic molecules (granzyme, perforin, granulysin), chemokines (CCL5, XCL1) and CTL/NK-cell associated molecules (CTSW); |
| **Neutrophils:** genes encoding innate molecules that are found in neutrophil granules (Lactotransfernn: LTF, defensin: DEAF1, Bacterial Permeability Increasing protein: BPI, Cathelicidin antimicrobial protein: CAMP...); |
| **Erythrocytes:** genes encoding hemoglobin genes (HGBs) and other erythrocyte-associated genes (erythrocytic alkirin:ANK1, Glycophorin C: GYPC, hydroxymethylbilane synthase: HMBS, erythroid associated factor: ERAF); |
| **Ribosomal proteins:** genes encoding ribosomal proteins (RPLs, RPSs), Eukaryotic Translation Elongation factor family members (EEFs) and Nucleolar proteins (NPM1, NOAL2, NAP1L1); |
| genes encoding immune-related (CD40, CD80, CXCL12, IFNA5, IL4R) as well as cytoskeleton-related molecules (Myosin, Dedicator of Cytokenesis, Syndecan 2, Plexin C1, Distrobrevin); |
| **Myeloid lineage:** Related to M 1.5. Includes genes expressed in myeloid lineage cells (IGTB2/CD18, Lymphotoxin beta receptor, Myeloid related proteins 8/14 Formyl peptide receptor 1), such as Monocytes and Neutrophils; |
| genes encoding chemokine-like factor superfamily (CKLFSF8); |
| **T-cells:** genes encoding T-cell surface markers (CD5, CD6, CD7, CD26, CD28, CD96) and molecules expressed by lymphoid lineage cells (lymphotoxin beta, IL2-inducible T-cell kinase, TCF7, T-cell differentiation protein mal, GATA3, STAT5B); |
| genes encoding molecules that associate to the cytoskeleton (Actin related protein 2/3, MAPK1, MAP3K1, RAB5A). Also present are T-cell expressed genes (FAS, ITGA4/CD49D, ZNF1A1); |
| genes encoding for Immune-related cell surface molecules (CD36, CD86, LILRB), cytokines (IL15) and molecules involved in signaling pathways (FYB, TICAM2-Toll-like receptor pathway); |
| genes encoding kinases (UHMK1, CSNK1G1, CDK6, WNK1, TAOK1, CALM2, PRKCI, ITPKB, SRPK2, STK17B, DYRK2, PIK3R1, STK4, CLK4, PKN2) and RAS family members (G3BP, RAB14, RASA2, RAP2A, KRAS); |
| **Interferon-inducible:** genes encoding interferon-inducible genes: antiviral molecules (OAS1/2/3/L, GBP1, G1P2, EIF2AK2/PKR, MX1, PML), chemokines (CXCL10/IP-10), signaling molecules (STAT1, STAt2, IRF7, ISGF3G); |
| **Inflammation I:** genes encoding molecules involved in inflammatory processes (IL8, ICAM1, C5R1, CD44, PLAUR, IL1A, CXCL16), and regulators of apoptosis (MCL1, FOXO3A, RARA, BCL3/6/2A1, GADD45B); |
| **Inflammation II:** genes encoding molecules inducing or inducible by Granulocyte-Macrophage CSF (SPI1, IL18, ALOX5, ANPEP), as well as lysosomal enzymes (PPT1, CTSB/S, CES1, NEU1, ASAH1, LAMP2, CAST); |
| genes encoding protein phosphates (PPP1R12A, PTPRC, PPP1CB, PPM1B) and phosphoinositide 3-kinase (PI3K) family members (PIK3CA, PIK32A, PIP5K3); |
| genes encoding hemoglobin genes (HBA1, HBA2, HBB); |
| genes encoding T-cell surface markers (CD101, CD102, CD103) as well as molecules ubiquitously expressed among blood leukocytes (CXRCR1: fraktalkine receptor, CD47, P-selectin ligand); |
| genes encoding proteasome subunits (PSMA2/5, PSMB5/8); ubiquitin protein ligases HIP2, STUB1, as well as components ofubiqutin ligase complexes (SUGT1); |
| genes encoding for several enzymes: aminomethyltransferase, arginyltransferase, asparagines synthetase, diacylglycerol kinase, inositol phosphatases, methyltransferases, helicases; and |
| genes encoding for protein kinases (PRKPIR, PRKDC, PRKCI) and phosphatases (PTPLB, PPP1R8/2CB). Also includes RAS oncogene family members and the NK cell receptor 2B4 (CD244); |
and combinations thereof, wherein the level of expression of genes in a sample is charted to the modules to determine a disease or condition.

2. The method of claim 1, **characterized in that** the clusters are selected from expression value clusters, keyword clusters, metabolic clusters, disease clusters, infection clusters, transplantation clusters, signaling clusters, transcriptional clusters, replication clusters, cell-cycle clusters, siRNA clusters, miRNA clusters, mitochondrial clusters, T cell clusters, B cell clusters, cytokine clusters, lymphokine clusters, heat shock clusters and combinations thereof.

3. The method of claim 1 or 2, **characterized in that** the one or more diseases or conditions are selected from one or more of the following conditions: systemic juvenile idiopathic arthritis, systemic lupus erythematosus, type I diabetes, liver transplant recipients, melanoma patients, and patients bacterial infections such as Escherichia coli, Staphylococcus aureus, viral infections such as influenza A, infections with a bioterror agent and combinations thereof.

4. The method of claims 1 to 3, **characterized in that** the cells comprise:
• peripheral blood mononuclear cells (PBMCs), blood cells, fetal cells, peritoneal cells, solid organ biopsies, resected tumors, primary cells, cells lines, cell clones and combinations thereof, or
• single cells, a collection of cells, tissue, cell culture, urine and blood, or
• a tissue biopsy, one or more sorted cell populations, cell culture, cell clones, transformed cells, biopies or a single cell, or
• brain, liver, heart, kidney, lung, spleen, retina, bone, neural, lymph node, endocrine gland, reproductive organ, blood, nerve scular tissue, and olfactory epithelium cells.

5. The method of claims 1 to 4, **characterized in that** the step of obtaining individual gene expression levels is performed using an array of oligonucleotides, or using hybridization of nucleic acids on a solid support, or using cDNA from mRNA collected from the cells as a template.

6. A method for diagnosing a disease or condition comprising the steps of:
analyzing the transcriptome from a patient based on one or more transcriptional modules that are indicative of a disease or condition; and
determining the patient's disease or condition based on the presence, absence or level of expression of genes within one or more transcriptional modules of the transcriptome, preferably identified by claims 1 to 5.

7. A disease analysis tool comprising:
one or more gene modules selected from the group consisting of:
| Transcriptional modules |
|---|
| **Plasma cells:** genes encoding for Immunoglobulin chains (IGHM, IGJ, IGLL1, IGKC, IGHD) and the plasma cell marker CD38.; |
| **Platelets:** genes encoding for platelet glycoproteins (ITGA2B, ITGB3, GP6, GP1A/B), and platelet-derived immune mediators such as PPPB (pro-platelet basic protein) and PF4 (platelet factor 4); |
| **B-cells:** genes encoding for B-cell surface markers (CD72, CD79A/B, CD19, CD22) and other B-cell associated molecules: Early B-cell factor (EBF), B-cell linker (BLNK) and B lymphoid tyrosine kinase (BLK); |
| Genes encoding regulators and targets of cAMP signaling pathway (JUND, ATF4, CREM, PDE4, NR4A2, VIL2), repressors of TNF-alpha mediated NF-KB activation (CYLD, ASK, TNFAIP3); |
| **Myeloid lineage:** Genes encoding molecules expressed by cells of the myeloid lineage (CD86, CD 163, FCGR2A), some of which being involved in pathogen recognition (CD14, TLR2, MYD88). This set also includes TNF family members (TNFR2, BAFF); |
| genes encoding for signaling molecules, the zinc finger containing inhibitor of activated STAT (PIAS1 and PIAS2), or the nuclear factor of activated T-cells NFATC3; |
| **MHC/Ribosomal proteins:** genes encoding MHC class I molecules (HLA-A,B,C,G,E)+ Beta 2-microglobulin (B2M) or Ribosomal proteins (RPLs, RPSs); |
| genes encoding metabolic enzymes (GLS, NSF1, NAT1) and factors involved in DNA replication (PURA, TERF2, EIF2S1); |
| **Cytotoxic cells:** Gene encoding for Cytotoxic T-cells and NK-cells surface markers (CD8A, CD2, CD160, NKG7, KLRs), cytolytic molecules (granzyme, perforin, granulysin), chemokines (CCL5, XCL1) and CTL/NK-cell associated molecules (CTSW); |
| **Neutrophils:** Gene encoding innate molecules that are found in neutrophil granules (Lactotransferrin: LTF, defensin: DEAF1, Bacterial Permeability Increasing protein: BPI, Cathelicidin antimicrobial protein: CAMP); |
| **Erythrocytes:** Gene encoding hemoglobin genes (HGBs) and other erythrocyte-associated genes (erythrocytic alkirin:ANK1, Glycophorin C: GYPC, hydroxymethylbilane synthase: HMBS, erythroid associated factor: ERAF); |
| **Ribosomal proteins:** genes encoding ribosomal proteins (RPLs, RPSs), Eukaryotic Translation Elongation factor family members (EEFs) and Nucleolar proteins (NPM1, NOAL2, NAP1L1); |
| genes encoding immune-related (CD40, CD80, CXCL12, IFNA5, IL4R) as well as cytoskeleton-related molecules (Myosin, Dedicator of Cytokenesis, Syndecan 2, Plexin C1, Distrobrevin); |
| **Myeloid lineage:** Related to M 1.5. Includes genes expressed in myeloid lineage cells (IGTB2/CD18, Lymphotoxin beta receptor, Myeloid related proteins 8/14 Formyl peptide receptor 1), such as Monocytes and Neutrophils; |
| genes encoding members of the chemokine-like factor superfamily (CKLFSF8); |
| **T-cells:** genes encoding T-cell surface markers (CD5, CD6, CD7, CD26, CD28, CD96) and molecules expressed by lymphoid lineage cells (lymphotoxin beta, IL2-inducible T-cell kinase, TCF7, T-cell differentiation protein mal, GATA3, STAT5B); |
| genes encoding molecules that associate to the cytoskeleton (Actin related protein 2/3, MAPK1, MAP3K1, RAB5A). Also present are T-cell expressed genes (FAS, ITGA4/CD49D, ZNF1A1); |
| genes encoding for immune-related cell surface molecules (CD36, CD86, LILRB), cytokines (IL15) and molecules involved in signaling pathways (FYB, TICAM2-Toll-like receptor pathway); |
| genes encoding kinases (UHMK1, CSNK1G1, CDK6, WNK1, TAOK1, CALM2, PRKCI, ITPKB, SRPK2, STK17B, DYRK2, PIK3R1, STK4, CLK4, PKN2) and RAS family members (G3BP, RAB14, RASA2, RAP2A, KRAS); |
| **Interferon-inducible:** genes encoding interferon-inducible genes: antiviral molecules (OAS1/2/3/L, GBP1, G1P2, EIF2AK2/PKR, MX1, PML), chemokines (CXCL10/IP-10), signaling molecules (STAT1, STAt2, IRF7, ISGF3G); |
| **Inflammation I:** genes encoding molecules involved in inflammatory processes (IL8, ICAM1, C5R1, CD44, PLAUR, IL1A, CXCL16), and regulators of apoptosis (MCL1, FOXO3A, RARA, BCL3/6/2A1, GADD45B); |
| **Inflammation II:** genes encoding molecules inducing or inducible by Granulocyte-Macrophage CSF (SPI1, IL18, ALOX5, ANPEP), as well as lysosomal enzymes (PPT1, CTSB/S, CES1, NEU1, ASAH1, LAMP2, CAST); |
| genes encoding protein phosphates (PPP1R12A, PTPRC, PPP1CB, PPM1B) and phosphoinositide 3-kinase (PI3K) family members (PIK3CA, PIK32A, PIP5K3); |
| genes encoding hemoglobin genes (HBA1, HBA2, HBB); |
| genes encoding T-cell surface markers (CD101, CD102, CD103) as well as molecules ubiquitously expressed among blood leukocytes (CXRCR1, fraktalkine receptor, CD47, P-selectin ligand); |
| genes encoding proteasome subunits (PSMA2/5, PSMB5/8); ubiquitin protein ligases HIP2, STUB1, as well as components of ubiqutin ligase complexes (SUGT1); |
| genes encoding for several enzymes: aminomethyltransferase, arginyltransferase, asparagines synthetase, diacylglycerol kinase, inositol phosphatases, methyltransferases, helicases; and |
| genes encoding for protein kinases (PRKPIR, PRKDC, PRKCI) and phosphatases (PTPLB, PPP1R8/2CB), RAS oncogene family members and the NK cell receptor 2B4 (CD244); |
and are sufficient to distinguish between an autoimmune disease, a viral infection a bacterial infection, cancer and transplant rejection.

8. Use of a tool of claim 7, **characterized in that** the modules are used to distinguish between Systemic Lupus erythematosus, Influenza infection, melanoma and transplant rejection, preferably wherein the modules selected are selected from:
**Plasma cells:** genes encoding for Immunoglobulin chains (IGHM, IGJ, IGLL1, IGKC, IGHD) and the plasma cell marker CD38; and
**Platelets:** genes encoding for platelet glycoproteins (ITGA2B, ITGB3, GP6, GP1A/B), and platelet-derived immune mediators such as PPPB (pro-platelet basic protein) and PF4 (platelet factor 4); and
• the modules are used to identify Systemic Lupus erythematosus by having a positive vector at these two modules, or
• the modules are used to identify Influenza infection by having neither a positive nor a negative vector at these two modules, or
• the modules are used to identify melanoma by having a negative vector for the plasma cell markers and a positive vector for the platelet markers, or
• the modules are used to identify transplant rejection by having a negative vectors at these two modules, or
• the modules are used to identify Influenza infection by having a negative vector at these two modules.

9. A prognostic gene array comprising:
a customized gene array that comprises a combination of genes that are representative of one or more transcriptional modules, wherein the transcriptome of a patient that is contacted with the customized gene array is prognostic of one or more disease or conditions that match the transcriptional modules, wherein preferably the patient's immune response to the disease or condition is determined based on the presence, absence or level of expression of genes of the transcriptome based on a correlation of the transcriptional modules with a specific disease or condition.

10. The array of claim 9, **characterized in that** the array can distinguish between an autoimmune disease, a viral infection a bacterial infection, cancer and transplant rejection.

11. The array of claim 9 or 10, **characterized in that** the array is organized into two or more transcriptional modules, preferably wherein the array is organized into three transcriptional modules comprising one or more submodules selected from:
| **Submodule** | **Number of probe sets** | **Keyword selection** | **Assessment** |
|---|---|---|---|
| M 1.1 | 69 | Ig, Immunoglobulin, Bone, Marrow, PreB, IgM, Mu. | **Plasma cells:** genes encoding for Immunoglobulin chains (IGHM, IGJ, IGLL1, IGKC, IGHD) and the plasma cell marker CD38; |
| M 1.2 | 96 | Platelet, Adhesion, Aggregation, Endothelial, Vascular | **Platelets:** genes encoding for platelet glycoproteins (ITGA2B, ITGB3, GP6, GP1A/B), and platelet-derived immune mediators such as PPPB (pro-platelet basic protein) and PF4 (platelet factor 4); |
| M 1.3 | 47 | Immunoreceptor, BCR, B-cell, IgG | **B-cells:** genes encoding for B-cell surface markers (CD72, CD79A/B, CD19, CD22) and other B-cell associated molecules: Early B-cell factor (EBF), B-cell linker (BLNK) and B lymphoid tyrosine kinase (BLK); |
| M 1.4 | 87 | Replication, Repression, Repair, CREB, Lymphoid, TNF-alpha | genes encoding regulators and targets of cAMP signaling pathway (JUND, ATF4, CREM, PDE4, NR4A2, VIL2), as well as repressors of TNF-alpha mediated NF-KB activation (CYLD, ASK, TNFAIP3); |
| M 1.5 | 130 | Monocytes, Dendritic, MHC, Costimulatory, TLR4, MYD88 | **Myeloid lineage:** Molecules expressed by cells of the myeloid lineage (CD86, CD163, FCGR2A), some of which being involved in pathogen recognition (CD14, TLR2, MYD88). This set also includes TNF family members (TNFR2, BAFF); |
| M 1.6 | 28 | Zinc, Finger, P53, RAS | genes encoding for signaling molecules, the zinc finger containing inhibitor of activated STAT (PIAS 1 and PIAS2), or the nuclear factor of activated T-cells NFATC3; |
| M 1.7 | 127 | Ribosome, Translational, 40S, 60S, HLA | **MHC/Ribosomal proteins:** genes encoding MHC class I molecules (HLA-A,B,C,G,E)+ Beta 2-microglobulin (B2M) or Ribosomal proteins (RPLs, RPSs); |
| M 1.8 | 86 | Metabolism, Biosynthesis, Replication, Helicase | genes encoding metabolic enzymes (GLS, NSF1, NAT1) and factors involved in DNA replication (PURA, TERF2, EIF2S 1); |
| M 2.1 | 72 | NK, Killer, Cytolytic, CD8, Cell-mediated, T-cell, CTL, IFN-g | **Cytotoxic cells:** genes encoding cytotoxic T-cell and NK-cell surface markers (CD8A, CD2, CD160, NKG7, KLRs), cytolytic molecules (granzyme, perforin, granulysin), chemokines (CCL5, XCL1) and CTL/NK-cell associated molecules (CTSW); |
| M 2.2 | 44 | Granulocytes, Neutrophils, Defense, Myeloid, Marrow | **Neutrophils:** genes encoding innate molecules that are found in neutrophil granules (Lactotransferrin: LTF, defensin: DEAF 1, Bacterial Permeability Increasing protein: BPI, Cathelicidin antimicrobial protein: CAMP); |
| M 2.3 | 94 | Erythrocytes, Red, Anemia, Globin, Hemoglobin | **Erythrocytes:** hemoglobin genes (HGBs) and other erythrocyte-associated genes (erythrocytic alkirin:ANK1, Glycophorin C: GYPC, hydroxymethylbilane synthase: HMBS, erythroid associated factor: ERAF); |
| M 2.4 | 118 | Ribonucleoprotein, 60S, nucleolus, Assembly, Elongation | **Ribosomal proteins:** genes encoding ribosomal proteins (RPLs, RPSs), Eukaryotic Translation Elongation factor family members (EEFs) and Nucleolar proteins (NPM1, NOAL2, NAP1L1); |
| M 2.5 | 242 | Adenoma, Interstitial, Mesenchyme, Dendrite, Motor | genes encoding immune-related (CD40, CD80, CXCL12, IFNA5, IL4R) as well as cytoskeleton-related molecules (Myosin, Dedicator of Cytokenesis, Syndecan 2, Plexin C1, Distrobrevin); |
| M 2.6 | 110 | Granulocytes, Monocytes, Myeloid, ERK, Necrosis | genes encoding molecules expressed in myeloid lineage cells (IGTB2/CD18, Lymphotoxin beta receptor, Myeloid related proteins 8/14 Formyl peptide receptor 1), Monocytes and Neutrophils; |
| M 2.7 | 43 | No keywords extracted. | genes encoding one or more members of the chemokine-like factor superfamily (CKLFSF8); |
| M 2.8 | 104 | Lymphoma, T-cell, CD4, CD8, TCR, Thymus, Lymphoid, IL2 | **T-cells:** genes encoding T-cell surface markers (CD5, CD6, CD7, CD26, CD28, CD96) and molecules expressed by lymphoid lineage cells (lymphotoxin beta, IL2-inducible T-cell kinase, TCF7, T-cell differentiation protein mal, GATA3, STAT5B) ; |
| M 2.9 | 122 | ERK, Transactivation, Cytoskeletal, MAPK, JNK | genes encoding molecules that associate to the cytoskeleton (Actin related protein 2/3, MAPK1, MAP3K1, RAB5A). Also present are T-cell expressed genes (FAS, ITGA4/CD49D, ZNF1A1); |
| M2.10 | 44 | Myeloid, Macrophage, Dendritic, Inflammatory, Interleukin | genes encoding for Immune-related cell surface molecules (CD36, CD86, LILRB), cytokines (IL15) and molecules involved in signaling pathways (FYB, TICAM2-Toll-like receptor pathway); |
| M 2.11 | 77 | Replication, Repress, RAS, Autophosphorylati on, Oncogenic | genes encoding kinases (UHMK1, CSNK1G1, CDK6, WNK1, TAOK1, CALM2, PRKCI, ITPKB, SRPK2, STK17B, DYRK2, PIK3R1, STK4, CLK4, PKN2) and RAS family members (G3BP, RAB14, RASA2, RAP2A, KRAS); |
| M 3.1 | 80 | ISRE, Influenza, Antiviral, IFN-gamma, IFN-alpha, Interferon | **Interferon-inducible:** genes encoding interferon-inducible genes: antiviral molecules (OAS1/2/3/L, GBP1, G1P2, EIF2AK2/PKR, MX1, PML), chemokines (CXCL10/IP-10), signaling molecules (STAT1, STAt2, IRF7, ISGF3G); |
| M 3.2 | 230 | TGF-beta, TNF, Inflammatory, Apoptotic, Lipopolysaccharid e | **Inflammation I:** genes encoding molecules involved in inflammatory processes (IL8, ICAM1, C5R1, CD44, PLAUR, IL1A, CXCL16), and regulators of apoptosis (MCL1, FOXO3A, RARA, BCL3/6/2A1, GADD45B); |
| M 3.3 | 230 | Granulocyte, Inflammatory, Defense, Oxidize, Lysosomal | **Inflammation II:** genes encoding molecules inducing or inducible by Granulocyte-Macrophage CSF (SPI1, IL18, ALOX5, ANPEP), as well as lysosomal enzymes (PPT1, CTSB/S, CES1, NEU1, ASAH1, LAMP2, CAST); |
| M 3.4 | 323 | No keyword extracted | genes encoding protein phosphates (PPP1R12A, PTPRC, PPP1CB, PPM1B) and phosphoinositide 3-kinase (PI3K) family members (PIK3CA, PIK32A, PIP5K3); |
| M 3.5 | 19 | No keyword extracted | genes encoding hemoglobin genes (HBA1, HBA2, HBB); |
| M 3.6 | 233 | Complement, Host, Oxidative, Cytoskeletal, T-cell | genes encoding T-cell surface markers (CD101, CD102, CD103) as well as molecules ubiquitously expressed among blood leukocytes (CXRCR1: fraktalkine receptor, CD47, P-selectin ligand); |
| M 3.7 | 80 | Spliceosome, Methylation, Ubiquitin, Beta-catenin | genes encoding proteasome subunits (PSMA2/5, PSMB5/8); ubiquitin protein ligases HIP2, STUB1, as well as components of ubiqutin ligase complexes (SUGT1); |
| M 3.8 | 182 | CDC, TCR, CREB, Glycosylase | genes encoding for several enzymes: aminomethyltransferase, arginyltransferase, asparagines synthetase, diacylglycerol kinase, inositol phosphatases, methyltransferases, helicases; and |
| M 3.9 | 261 | Chromatin, Checkpoint, Replication, Transactivation | genes encoding for protein kinases (PRKPIR, PRKDC, PRKCI) and phosphatases (PTPLB, PPP1R8/2CB). Also includes RAS oncogene family members and the NK cell receptor 2B4 (CD244); |
and comprising probes that bind specifically one or more of the genes in the module.

12. A method for selecting patients for a clinical trial comprising the steps of:
comparing the transcriptome of a prospective patient to one or more transcriptional modules that are indicative of a disease or condition that is to be treated in the clinical trial; and
determining the likelihood that a patient is a good candidate for the clinical trial based on the presence, absence or level of one or more genes that are expressed in the patient's transcriptome within one or more transcriptional modules that are correlated with success in a clinical trial.

13. The method of claim 12, **characterized in that** each module comprises a vector that correlates with a sum of the proportion of transcripts in a sample, or a vector and wherein one or more diseases or conditions is associated with the one or more vectors, or a vector that correlates to the expression level of one or more genes within each module or a vector and wherein the modules selected are:
**Plasma cells:** genes encoding for Immunoglobulin chains (IGHM, IGJ, IGLL1, IGKC, IGHD) and the plasma cell marker CD38; and
**Platelets:** genes encoding for platelet glycoproteins (ITGA2B, ITGB3, GP6, GP1A/B), and platelet-derived immune mediators such as PPPB (pro-platelet basic protein) and PF4 (platelet factor 4);
and the modules are used to distinguish between Systemic Lupus erythematosus by having a positive vector at these two modules; Influenza infection by having neither a positive nor a negative vector at these two modules; melanoma by having a negative vector for the plasma cell markers and a positive vector for the platelet markers; identify transplant rejection by having a negative vectors at these two modules.

14. An array of nucleic acid probes immobilized on a solid support comprising sufficient probes from one or more modules to provide a sufficient proportion of differentially expressed genes to distinguish between one or more diseases, the probes being specific for modules, wherein data obtained from a sample contacted with the nucleic acid probes immobilized on the solid support, is sorted by the modules selected from:
| Module I.D. | Transcriptional Modules |
|---|---|
| M 1.1 | Plasma cells: genes encoding for Immunoglobulin chains (IGHM, IGJ, IGLL1, IGKC, IGHD) and the plasma cell marker CD38. |
| M 1.2 | Platelets: genes encoding for platelet glycoproteins (ITGA2B, ITGB3, GP6, GP1A/B), and platelet-derived immune mediators such as PPPB (pro-platelet basic protein) and PF4 (platelet factor 4). |
| M 1.3 | B-cells: genes encoding for B-cell surface markers (CD72, CD79AB, CD19, CD22) and other B-cell associated molecules: Early B-cell factor (EBF), B-cell linker (BLNK) and B lymphoid tyrosine kinase (BLK). |
| M 1.4 | genes encoding regulators and targets of cAMP signaling pathway (JUND, ATF4, CREM, PDE4, NR4A2, VIL2), as well as repressors of TNF-alpha mediated NF-KB activation (CYLD, ASK, TNFAIP3). |
| M 1.5 | Myeloid lineage: genes encoding molecules expressed by cells of the myeloid lineage (CD86, CD163, FCGR2A), some of which being involved in pathogen recognition (CD14, TLR2, MYD88). This set also includes TNF family members (TNFR2, BAFF). |
| M 1.6 | genes encoding for signaling molecules, the zinc finger containing inhibitor of activated STAT (PIAS 1 and PIAS2), or the nuclear factor of activated T-cells NFATC3. |
| M 1.7 | MHC/Ribosomal proteins: genes encoding MHC class I molecules (HLA-A,B,C,G,E)+ Beta 2-microglobulin (B2M) or Ribosomal proteins (RPLs, RPSs). |
| M 1.8 | Undetermined. genes encoding metabolic enzymes (GLS, NSF1, NAT1) and factors involved in DNA replication (PURA, TERF2, EIF2S1). |
| M 2.1 | Cytotoxic cells: genes encoding cytotoxic T-cells amd NK-cells surface markers (CD8A, CD2, CD160, NKG7, KLRs), cytolytic molecules (granzyme, perforin, granulysin), chemokines (CCL5, XCL1) and CTL/NK-cell associated molecules (CTSW). |
| M 2.2 | Neutrophils: genes encoding innate molecules that are found in neutrophil granules (Lactotransferrin: LTF, defensin: DEAF1, Bacterial Permeability Increasing protein: BPI, Cathelicidin antimicrobial protein: CAMP...). |
| M 2.3 | Erythrocytes: genes encoding hemoglobin genes (HGBs) and other erythrocyte-associated genes (erythrocytic alkirin:ANK1, Glycophorin C: GYPC, hydroxymethylbilane synthase: HMBS, erythroid associated factor: ERAF). |
| M 2.4 | Ribosomal proteins: genes encoding ribosomal proteins (RPLs, RPSs), Eukaryotic Translation Elongation factor family members (EEFs) and Nucleolar proteins (NPM1, NOAL2, NAP1L1). |
| M 2.5 | genes encoding immune-related (CD40, CD80, CXCL12, IFNA5, IL4R) as well as cytoskeleton-related molecules (Myosin, Dedicator of Cytokenesis, Syndecan 2, Plexin C1, Distrobrevin). |
| M 2.6 | Myeloid lineage: genes expressed in myeloid lineage cells (IGTB2/CD18, Lymphotoxin beta receptor, Myeloid related proteins 8/14 Formyl peptide receptor 1), such as Monocytes and Neutrophils: |
| M 2.7 | genes encoding one or more members of the chemokine-like factor superfamily (CKLFSF8). |
| M 2.8 | T-cells: genes encoding T-cell surface markers (CD5, CD6, CD7, CD26, CD28, CD96) and molecules expressed by lymphoid lineage cells (lymphotoxin beta, IL2-inducible T-cell kinase, TCF7, T-cell differentiation protein mal, GATA3, STAT5B). |
| M 2.9 | genes encoding molecules that associate to the cytoskeleton (Actin related protein 2/3, MAPK1, MAP3K1, RAB5A). Also present are T-cell expressed genes (FAS, ITGA4/CD49D, ZNF1A1). |
| M2.10 | genes encoding for Immune-related cell surface molecules (CD36, CD86, LILRB), cytokines (IL15) and molecules involved in signaling pathways (FYB, TICAM2-Toll-like receptor pathway). |
| M 2.11 | genes encoding kinases (UHMK1, CSNK1G1, CDK6, WNK1, TAOK1, CALM2, PRKCI, ITPKB, SRPK2, STK17B, DYRK2, PIK3R1, STK4, CLK4, PKN2) and RAS family members (G3BP, RAB14, RASA2, RAP2A, KRAS). |
| M 3.1 | Interferon-inducible: genes encoding interferon-inducible genes: antiviral molecules (OAS1/2/3/L, GBP1, G1P2, EIF2AK2/PKR, MX1, PML), chemokines (CXCL10/IP-10), signaling molecules (STAT1, STAt2, IRF7, ISGF3G). |
| M 3.2 | Inflammation I: genes encoding molecules involved in inflammatory processes (IL8, ICAM1, C5R1, CD44, PLAUR, IL1A, CXCL16), and regulators of apoptosis (MCL1, FOXO3A, RARA, BCL3/6/2A1, GADD45B). |
| M 3.3 | Inflammation II: genes encoding molecules inducing or inducible by Granulocyte-Macrophage CSF (SPI1, IL18, ALOX5, ANPEP), as well as lysosomal enzymes (PPT1, CTSB/S, CES1, NEU1, ASAH1, LAMP2, CAST). |
| M 3.4 | genes encoding protein phosphates (PPP1R12A, PTPRC, PPP1CB, PPM1B) and phosphoinositide 3-kinase (PI3K) family members (PIK3CA, PIK32A, PIP5K3). |
| M 3.5 | genes encoding hemoglobin genes (HBA1, HBA2, HBB). |
| M 3.6 | genes encoding T-cell surface markers (CD101, CD102, CD103) as well as molecules ubiquitously expressed among blood leukocytes (CXRCR1: fraktalkine receptor, CD47, P-selectin ligand). |
| M 3.7 | genes encoding proteasome subunits (PSMA2/5, PSMB5/8); ubiquitin protein ligases HIP2, STUB1, and ubiqutin ligase complexes (SUGT1). |
| M 3.8 | genes encoding for several enzymes: aminomethyltransferase, arginyltransferase, asparagines synthetase, diacylglycerol kinase, inositol phosphatases, methyltransferases, helicases |
| M 3.9 | genes encoding for protein kinases (PRKPIR, PRKDC, PRKCI) and phosphatases (PTPLB, PPP1R8/2CB), RAS oncogenes and the NK cell receptor 2B4 (CD244). |
wherein the probes in the first probe set have one or more interrogation positions respectively corresponding to one or more diseases.

15. The array of claim 14, **characterized in that** the array has between 100 and 100,000 probes, and/or each probe is 9-21 nucleotides.

16. The array of claim 14 or 15, **characterized in that** the probes in the second, third and fourth probe sets positioned to be interrogated.

17. An array of claim 14 to 16 comprising nucleic acid probes immobilized on a solid support, the array comprising at least one pair of first and second probe groups, each group comprising one or more probes specific for said modules.

18. The array of claim 17, **characterized in that** the groups provide a composite transcriptional marker vector that is consistent across microarray platforms, preferably further displayed in a summary for regulatory approval.
